(19) Europäisches Patentamt
European Patent Office
Office européen des brevets

(11) EP 2 177 505 A1

(12) EUROPEAN PATENT APPLICATION
published in accordance with Art. 153(4) EPC

(43) Date of publication:
**21.04.2010 Bulletin 2010/16**

(21) Application number: **08792314.0**

(22) Date of filing: **07.08.2008**

(51) Int Cl.:
***C07C 279/22*** (2006.01) ***A61K 31/341*** (2006.01)
***A61K 31/352*** (2006.01) ***A61K 31/495*** (2006.01)
***A61P 25/28*** (2006.01) ***A61P 43/00*** (2006.01)
***C07D 295/12*** (2006.01) ***C07D 307/52*** (2006.01)
***C07D 311/04*** (2006.01)

(86) International application number:
**PCT/JP2008/064257**

(87) International publication number:
**WO 2009/022633 (19.02.2009 Gazette 2009/08)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **10.08.2007 JP 2007208796**

(71) Applicant: **Astellas Pharma Inc.**
**Tokyo 103-8411 (JP)**

(72) Inventors:
- **KINOYAMA, Isao**
  **Tokyo 103-8411 (JP)**
- **MIYAMOTO, Satoshi**
  **Tokyo 103-8411 (JP)**
- **MIYAZAKI, Takehiro**
  **Tokyo 103-8411 (JP)**
- **KOGANEMARU, Yohei**
  **Tokyo 103-8411 (JP)**
- **KAWAMOTO, Yuichiro**
  **Tokyo 103-8411 (JP)**
- **SHIRAISHI, Nobuyuki**
  **Tokyo 103-8411 (JP)**
- **HOSHII, Hiroaki**
  **Tokyo 103-8411 (JP)**
- **KURODA, Akio**
  **Tokyo 103-8411 (JP)**
- **YAMAZAKI, Mayako**
  **Tokyo 103-8411 (JP)**
- **YASUDA, Minoru**
  **Tokyo 103-8411 (JP)**
- **MIZUNO, Hiroaki**
  **Osaka-shi**
  **Osaka 547-0022 (JP)**

(74) Representative: **HOFFMANN EITLE**
**Patent- und Rechtsanwälte**
**Arabellastrasse 4**
**81925 München (DE)**

# (54) BICYCLIC ACYLGUANIDINE DERIVATIVE

(57) An object of the present invention is to provide a novel and excellent agent for treating or preventing dementia, schizophrenia, and the like, based on the serotonin 5-$HT_{5A}$ receptor modulating action.
It was confirmed that a bicyclic acylguanidine derivative which has a characteristic structure that guanidine is bonded to one ring of a bicyclic structure such as chromene and dihydronaphthalene through a carbonyl group and a cyclic group is bonded on the other ring, has a potent 5-$HT_{5A}$ receptor modulating action and an excellent pharmacological action based on this mechanism. The present invention is useful as an excellent agent for treating or preventing dementia, schizophrenia, bipolar disorder, or attention deficit hyperactivity disorder.

EP 2 177 505 A1

## Description

Technical Field

The present invention relates to a medicine, and particularly to a substituted guanidine derivative that has a 5-$HT_{5A}$ receptor modulating action, and is useful as an agent for treating or preventing dementia, schizophrenia, and the like.

Background Art

In recent years, it has been suggested that the 5-$HT_{5A}$ receptor which is one of the subtypes of serotonin receptors plays an important role in dementia and schizophrenia. For example, it has been reported that new exploratory behaviors are increased in the 5-$HT_{5A}$ receptor knock-out mice, and hyperactivity by LSD is inhibited in the 5-$HT_{5A}$ receptor knock-out mice (Neuron, 22, 581-591, 1999). From the results of gene expression analysis, it has been reported that the 5-$HT_{5A}$ receptor is highly expressed in the brains of humans and rodents, and expression is high in the brains, hippocampal CA1 and CA3 pyramidal cells which are related to memory, and frontal lobe (cerebral cortex) which is deeply related to schizophrenia (Molecular Brain Research, 56, 1-8, 1998). Furthermore, it has been reported that gene polymorphism of the 5-$HT_{5A}$ receptor relates to schizophrenia (Neuroreport 11, 2017-2020, 2000; Mol. Psychiatr. 6, 217-219, 2001; and J. Psychiatr. Res. 38, 371-376, 2004). Accordingly, it has been suggested that regulation of the action of the 5-$HT_{5A}$ receptor leads to the improvement of dementia and schizophrenia. Therefore, there is a need for a compound having such a function.

There have been hitherto reported several kinds of compounds having high affinity for the 5-$HT_{5A}$ receptor. For example, it has been described that a guanidine derivative represented by the following general formula binds to the 5-$HT_{5A}$ receptor and thus is used for treating multiple central diseases such as a neurodegenerative disease and a neurophychiatric disease (Patent Document 1).

[Chem. 1]

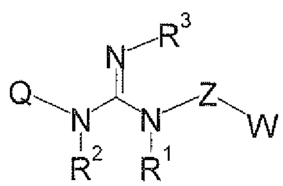 W: 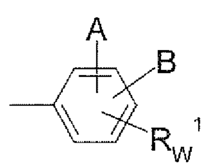 or 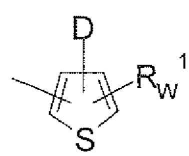

(wherein A represents $NO_2$, $NH_2$, or the like; B represents a hydrogen atom, or the like; $R_w1$ represents a hydrogen atom, or the like; D represents a group represented by A; Q represents a di-substituted 5-membered heteroaryl; $R^1$, $R^2$, and $R^3$ each represent a hydrogen atom, or the like; and Z represents .$(CR_z^1R_z^2)_a$-$(V_z)_b$-$(CR_z^3/R_z^4)_c$-, in which a and c each represent 0 to 4, b represents 0 or 1, $R_z1$, $R_z^2$, $R_z^3$ and $R_z^4$ each represents a hydrogen atom, or the like, and $V_z$ represents CO, or the like. For details on these, refer to the publication).

None of the S-$HT_{5A}$ receptor modulators which have been reported until now has a bicyclic acylguanidine structure. On the other hand, several kinds of compounds having bicyclic acylguanidine structures that are used in other uses have been known.

For example, it has been reported that a derivative represented by the following general formula has an antiviral activity, and is useful in the treatment of infections with HIV, HCV, and the like (Patent Document 2).

[Chem. 2]

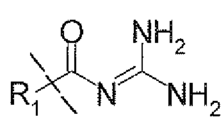 R1: 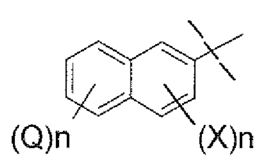 ,

and the like
(wherein $R^1$ represents phenyl, substituted phenyl, naphthyl, substituted naphthyl, or a structure shown above; n represents 1, 2, 3 or 4; Q independently represents hydrogen, cycloalkyl, thienyl, furyl, pyrazolyl, pyridyl, substituted pyridyl, phenyl, substituted phenyl, or the like; and X represents hydrogen or alkoxy. For details on these, refer to the publication.)
The publication has no description concerning the 5-$HT_{5A}$ receptor modulating action regarding the derivative, dementia, and schizophrenia.

A benzopyran derivative having a cyclic structure at the 4-position has been reported. For example, a compound represented by the following formula, and a derivative thereof are know as a K-channel opener (Non-Patent Document 1).

[Chem. 3]

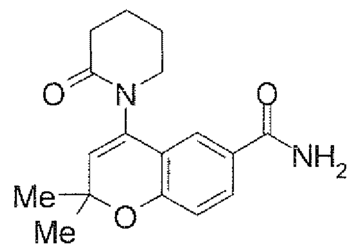

In addition, there has been reported a benzoxazine derivative that has an $Na^+/H^+$-exchanger inhibiting action, and is useful for the treatment of myocardial infarction and angina pectoris (Patent Document 3).
Neither Non-Patent Document 1 nor Patent Document 3 has a description concerning the 5-$HT_{5A}$ receptor modulating action, dementia, or schizophrenia.

[Patent Document 1] Pamphlet of International Publication 05/082871
[Patent Document 2] Pamphlet of International Publication 06/135978
[Patent Document 3] JP-A-9-77753
[Non-Patent Document 1] Rolf Bergmann, et al., Journal of Medicinal Chemistry (1990), Vol. 33, p. 492-504

DISCLOSURE OF THE INVENTION

PROBLEM THAT THE INVENTION IS TO SOLVE

An object of the present invention is to provide a novel and excellent agent for treating or preventing dementia, schizophrenia, or the like, based on the 5-$HT_{5A}$ receptor modulating action.

MEANS FOR SOLVING THE PROBLEM

The present inventors have extensively studied on a compound having a 5-$HT_{5A}$ receptor modulating action, and as a result, they have found that a bicyclic acylguanidine derivative which has a characteristic structure that guanidine is bonded to one ring of a bicyclic structure such as chromene and dihydronaphthalene through a carbonyl group and, a cyclic group is bonded on the other ring, has a potent 5-$HT_{5A}$ receptor modulating action and excellent pharmacological action based on this mechanism, and thus it can be an excellent agent for treating or preventing dementia, schizophrenia, and the like, thereby completing the present invention.
Namely, the present invention relates to a bicyclic acylguanidine derivative represented by the following general formula (1), or a salt thereof.

[Chem. 4]

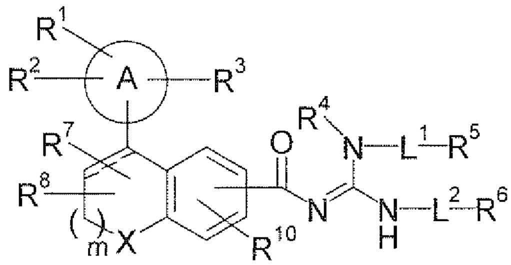

(I)

(the symbols in the formula have the following meanings:

[Chem. 5]

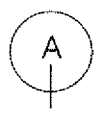

:

phenyl, cycloalkyl, monocyclic or bicyclic heteroaryl, monocyclic oxygen-containing saturated heterocyclic group or monocyclic nitrogen-containing saturated heterocyclic group,
$R^1$, $R^2$, and $R^3$: the same with or different from each other, each representing H, lower alkyl, halogen, halogeno-lower alkyl, -CN, $-NO_2$, $-NR^bR^c$, $-OR^a$, -O-halogeno-lower alkyl,
$-SR^a$, $-C(O)R^a$, $-CO_2R^a$, $-C(O)NR^bR^c$, $-SO_2$-lower alkyl,
$-NR^bC(O)R^a$, lower alkylene-$OR^a$, lower alkylene-$NR^bR^c$, lower alkylene-CN, phenyl, or -O-phenyl, or $R^1$ and $R^2$ in combination represent oxo or $-O-(CH_2)_n-O-$,

n: 1, 2, for 3,
$R^a$, $R^b$, and $R^c$: the same with or different from each other, each representing H or lower alkyl,
$R^7$ and $R^8$: the same with or different from each other, each representing H, lower alkyl, halogen, or lower alkylene-$OR^a$, or $R^7$ and $R^8$ in combination represent oxo, or $R^7$ and $R^8$ may be combined together to form a $C_{2-5}$ alkylene chain which forms a $C_{3-6}$ cycloalkyl ring with a carbon atom to which they bond,
dotted line: a bond or inexistence, and it represents, together with the solid line, that a ring bond at this moiety is a single bond or a double bond,
X: O, S or $CR^{9a}R^{9b}$,
$R^{9a}$ and $R^{9b}$: the same with or different from each other, each representing H or lower alkyl,
m: 0, 1, or 2,
$R^4$: H or lower alkyl,
$L^1$ and $L^2$: the same with or different from each other, each representing a bond or lower alkylene,
$R^5$ and $R^6$: the same with or different from each other, each representing H, $-OR^a$, $-NR^bR^c$, phenyl, or cycloalkyl, in which $R^5$ may form a monocyclic nitrogen-containing heterocyclic group together with $R^4$ and L', and a nitrogen atom to which they are bonded, in which phenyl, cycloalkyl, and a monocyclic nitrogen-containing heterocyclic group may be substituted with lower alkyl, halogen, or $-OR^a$, and
$R^{10}$: H, halogen, or $-OR^a$.)

Furthermore, the present invention relates to a pharmaceutical composition comprising a bicyclic acylguanidine derivative represented by the general formula (I) or a salt thereof, and a pharmaceutically acceptable carrier. Preferably, it relates to the pharmaceutical composition which is a 5-$HT_{5A}$ receptor modulator, more preferably, the pharmaceutical composition for dementia, schizophrenia, bipolar disorder, or attention deficit hyperactivity disorder, and even more preferably, the pharmaceutical composition which is an agent for preventing or treating dementia or schizophrenia. Furthermore, other embodiments include; use of the bicyclic acylguanidine derivative represented by the general formula (I) or a salt thereof for the manufacture of a S-$HT_{5A}$ receptor modulator, preferably, an agent for preventing or treating dementia, schizophrenia, bipolar disorder, or attention deficit hyperactivity disorder, and more preferably, an agent for

preventing or treating dementia or schizophrenia; and a method for preventing or treating dementia, schizophrenia, bipolar disorder, or attention deficit hyperactivity disorder, and preferably, a method for preventing or treating dementia or schizophrenia, comprising administering a therapeutically effective amount of the bicyclic acylguanidine represented by the general formula (I) or a salt thereof to a mammal.

EFFECT OF THE INVENTION

The compound of the present invention has an advantage that it has a potent 5-$HT_{5A}$ receptor modulating action, and an excellent pharmacological action based on it. The pharmaceutical composition of the present invention is useful for treatment or prevention of 5-$HT_{5A}$ receptor-related diseases, and particularly, for treatment or prevention of dementia, schizophrenia, bipolar disorder, or attention deficit hyperactivity disorder.

BEST MODE FOR CARRYING OUT THE INVENTION

Hereinafter, the present invention will be described in more detail.
In this specification, the "5-$HT_{5A}$ receptor modulator" is a generic term referring to a compound that inhibits activation of the 5-$HT_{5A}$ receptor by antagonizing with an endogenous ligand (5-$HT_{5A}$ antagonist), and a compound shows function by activation of the 5-$HT_{5A}$ receptor (5-$HT_{5A}$ agonist). The "5-$HT_{5A}$ receptor modulating action" is preferably a 5-$HT_{5A}$ antagonist.
The "lower alkyl" is preferably a linear or branched alkyl having 1 to 6 carbon atoms (hereinafter simply referred to as $C_{1-6}$), and specifically, is methyl, ethyl, n-propyl, isopropyl, n-butyl, isobutyl, sec-butyl, tert-butyl, n-pentyl, n-hexyl groups, and the like. More preferably, it is $C_{1-4}$ alkyl, and even more preferably, it is methyl, ethyl, n-propyl, and isopropyl.
The "lower alkylene" is preferably means a linear or branched $C_{1-6}$ alkylene, and specifically, it is methylene, ethylene, trimethylene, tetramethylene, pentamethylene, hexamethylene, propylene, methylmethylene, ethylethylene, 1,2-dimethylethylene, 1,1,2,2-tetramethylethylene groups, and the like. More preferably, it is $C_{1-4}$ alkylene, and even more preferably, it is methylene, ethylene, trimethylene, and propylene groups.
The "halogen" means F, Cl, Br, or I.
The "halogeno-lower alkyl" is $C_{1-6}$ alkyl substituted with one or more halogen. Preferably, it is $C_{1-6}$ alkyl substituted with 1 to 5 halogens, and more preferably difluoromethyl and trifluoromethyl groups.
The "cycloalkyl," is a $C_{3-10}$ saturated hydrocarbon ring group, which may have a bridge. Specifically, it is cyclopropyl, cyclobutyl, cyclopentyl, cyclohexyl, cycloheptyl, cyclooctyl, and adamantyl groups. Preferably, it is $C_{3-8}$ cycloalkyl, more preferably $C_{3-6}$ cycloalkyl, and even more preferably, it is cyclopropyl, cyclobutyl, cyclopentyl, and cyclohexyl groups.
The "heterocyclic" group is a 3- to 15-membered, preferably 5- to 10-membered, monocyclic to tricyclic heterocyclic group containing 1 to 4 hetero atoms selected from oxygen, sulfur, and nitrogen, and includes a saturated ring, an aromatic ring, and a partially hydrogenated ring group thereof. Sulfur or nitrogen which is a ring atom may be oxidized to form an oxide or a dioxide. Specifically, it is pyridyl, pyrrolyl, pyrazinyl, pyrimidinyl, pyridazinyl, imidazolyl, triazolyl, triazinyl, thienyl, furyl, thiazolyl, pyrazolyl, isothiazolyl, oxazolyl, isooxazolyl, thiadiazolyl, oxadiazolyl, tetrazolyl, azetidinyl, pyrrolidinyl, piperidyl, piperazinyl, azepanyl, diazepanyl, azocanyl, morpholinyl, thiomorpholinyl, tetrahydropyridinyl, oxiranyl, oxetanyl, dihydropyridyl, tetrahydrofuryl, tetrahydropyranyl, 1,4-dioxoranyl, dioxanyl, tetrahydrothiopyranyl, quinolyl, isoquinolyl, tetrahydroquinolyl, tetrahydroisoquinolyl, quinazolinyl, quinoxalinyl, phthalazinyl, benzoimidazolyl, imidazopyridyl, benzofuryl, benzothienyl, benzothiadiazolyl, benzothiazolyl, benzoisothiazolyl, benzooxazolyl, benzoisooxazolyl, methylenedioxyphenyl, ethylenedioxyphenyl, indolyl, isoindolyl, indolinyl, indazolyl, tetrahydrobenzoimidazolyl, dihydrobenzofuryl, chromanyl, chromonyl, and 1,4-dithiaspiro[4.5]decanyl groups. More preferably, it is a 5- to 10-membered monocyclic or bicyclic heterocyclic group, and even more preferably, a 5- to 6-membered monocyclic heterocyclic group.
The "monocyclic heteroaryl" is, among the above-described heterocyclic groups, a 5- to 6-membered monocyclic aromatic ring group, and preferably, it is pyridyl, pyrrolyl, pyrazinyl, pyrimidinyl, pyridazinyl, imidazolyl, triazolyl, thienyl, furyl, thiazolyl, pyrazolyl, isothiazolyl, oxazolyl, isooxazolyl, and tetrazolyl, more preferably, pyridyl, pyrazinyl, pyrimidinyl, thienyl, furyl, thiazolyl, and pyrazolyl, and even more preferably, pyridyl and thiazolyl.
The "bicyclic heteroaryl," is a ring group formed by fusion of the above-described "monocyclic heteroaryl" rings, or a ring group formed by fusion of the "monocyclic heteroaryl" ring and a benzene ring, and preferably, quinolyl, isoquinolyl, quinazolinyl, quinoxalinyl, phthalazinyl, benzoimidazolyl, imidazopyridyl, benzofuryl, benzothienyl, benzothiadiazolyl, benzothiazolyl, benzoisothiazolyl, benzooxazolyl, benzoisooxazolyl, indolyl, isoindolyl, indolinyl, and indazolyl, more preferably, a ring group containing nitrogen atom among these ring groups, and even more preferably, quinolyl and isoquinolyl.
The "monocyclic nitrogen-containing heterocyclic group" means a 5- to 8-membered monocyclic group that contains one nitrogen atom, and may contain one hetero atom selected from nitrogen, oxygen, and sulfur, among the above-described heterocyclic groups, and is a generic term referring to a "monocyclic nitrogen-containing saturated

heterocyclic group" that is a saturated or partially unsaturated ring group, and a "monocyclic nitrogen-containing heteroaryl" that is an aromatic ring group. The monocyclic nitrogen-containing saturated heterocyclic group is preferably azetidinyl, pyrrolidinyl, piperidyl, piperazinyl, azepanyl, diazepanyl, azocanyl, morpholinyl, thiomorpholinyl, and tetrahydropyridinyl groups, and more preferably, piperazinyl, and morpholinyl. The monocyclic nitrogen-containing heteroaryl is preferably pyridyl, thiazolyl, and pyrazolyl, and more preferably, pyrazolyl.
The "monocyclic oxygen-containing saturated heterocyclic group" means a 3- to 7-membered saturated monocyclic group that contains one oxygen atom, and may contain one hetero atom selected from nitrogen, oxygen, and sulfur, among the above-described heterocyclic groups. Preferably, it is oxiranyl, oxetanyl, tetrahydrofuryl, tetrahydropyranyl, and 1,4-dioxanyl groups, and particularly preferably a tetrahydropyranyl group.

The ring group, A, is preferably phenyl, pyridyl, pyrazolyl, pyrimidinyl, pyrazinyl, thiazolyl, thienyl, furyl, piperazinyl, tetrahydropyranyl, imidazopyridyl, and quinolyl, more preferably, phenyl, pyridyl, thiazolyl, and tetrahydropyranyl, even more preferably phenyl, and pyridyl, and particularly preferably phenyl.
The groups represented by $R^1$, $R^2$, and $R^3$ is preferably H, lower alkyl, halogen, halogeno-lower alkyl, -CN, $-NO_2$, $-OR^a$, $-C(O)R^a$, $-C(O)NR^bR^c$, lower alkylene-$OR^a$, lower alkylene-$NR^bR^c$, phenyl, -O-phenyl, oxo, and $-O-CH_2-O-$, and more preferably, H, lower alkyl, -CN, halogen, and - $OR^a$.
The group represented by $R^4$ and $R^5$ is preferably H or methyl, and more preferably H.
The group represented by $R^6$ is preferably H, methyl, or methoxy, and more preferably H.
The group represented by $R^7$ and $R^8$ is preferably, H, lower alkyl, or fluoro.
The group represented by $R^{9a}$ and $R^{9b}$ is preferably H or lower alkyl.
$L^1$ and $L^2$ are each preferably a bond or ethylene, and more preferably a bond.
The group represented by $R^{10}$ is preferably, H, F, or $-OR^a$.

Preferred embodiments in the compound of the present invention represented by the general formula (I) (which is hereinafter referred to as the compound (I)) are the compound or a salt thereof as follows.

(1) A compound of the formula (I), in which $R^4$ and $R^5$ are each H, $R^6$ is H, methyl or methoxy, and $L^1$ and $L^2$ are each a bond.
(2) The compound as described in (1), in which $R^6$ is H.
(3) The compound as described in (2), in which A is phenyl or pyridyl.
(4) The compound as described in (3), in which X is $CR^{9a}R^{9b}$.
(5) The compound as described in (3), in which X is O.
(6) The compound as described in (3), in which X is S.
(7) The compound as described in (4) to (6), in which m is 1.
(8) The compound as described in (7), in which the dotted line is a bond, and together with the solid line, a ring bond of the moiety represents a double bond.
(9) A compound represented by the following general formula (II):

[Chem. 6]

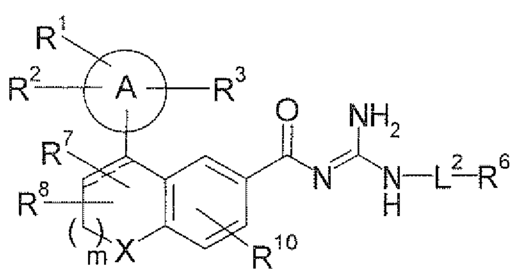

(II)

(wherein symbols in the formula have the same meanings as in the formula (I)).
Preferred ranges for the symbols in the formula (II) are the same as described above.

(10) The compound as described in (9), in which $R^6$ is H, methyl, or methoxy.

(11) The compound as described in (10), in which $R^6$ is H.
(12) The compound as described in (11), in which A is phenyl or pyridyl.
(13) The compound as described in (12), in which X is O or $CR^{9a}R^{9b}$.
(14) A compound selected from the group consisting of N-(diaminomethylene)-4-(4-fluorophenyl)-2H-chromene-6-carboxamide, N-(diaminomethylene)-4-(2-methylphenyl)-2H-chromene-6-carboxamide, 4-(2-chlorophenyl)-N-(diaminomethylene)-2H-chromene-6-carboxamide, N-(diaminomethylene)-4-(2,4,6-trifluorophenyl)-2H-chromene-6-carboxamide, N-(diaminomethylene)-4-(2,6-difluorophenyl)-2H-chromene-6-carboxamide, N-(diaminomethylene)-4-(2-fluoro-4-methylphenyl)-2H-chromene-6-carboxamide, N-(diaminomethylene)-4-(2,4-dichiorophenyl)-2H-chromene-6-carboxamide, N-(diaminomethylene)-4-(2,6-difluoro-4-methoxyphenyl)-2H-chromene-6-carboxamide, 4-(2-chloro-6-fluorophenyl)-N-(diaminomethylene)-2H-chromene-6-carboxamide, N-(diaminomethylene)-4-(2,4-dichlorophenyl)-2-methyl-2H-chromene-6-carboxamide, N-(diaminomethylene)-8-(4-fluorophenyl)-5,6-dihydronaphthalene-2-carboxamide, N-(diaminomethylene)-8-(2-methoxyphenyl)-5,6-dihydronaphthalene-2-carboxamide, N-(diaminomethylene)-8-(3-methylphenyl)-5,6-dihydronaphthalene-2-carboxamide, 8-(2-cyanophenyl)-N-(diaminomethylene)-5,6-dihydronaphthalene-2-carboxamide, N-(diaminomethylene)-8-phenyl-5,6-dihydronaphthalene-2-carboxamide, N-(diaminomethylene)-7-fluoro-8-(2-methoxyphenyl)-5,6-dihydronaphthalene-2-carboxamide, 8-(4-cyanophenyl)-N-(diaminomethylene)-7-methyl-5,6-dihydronaphthalene-2-carboxamide, N-(diaminomethylene)-8-(2,4,6-trifluorophenyl)-5,6-dihydronaphthalene-2-carboxamide, 8-(5-eyano-2-methoxyphenyl)-N-(diaminomethylene)-5,6-dihydronaphthalene-2-carboxamide, 8-(2-chloro-4-fluorophenyl)-N-(diaminomethylene)-5,6-dihydronaphthalene-2-carboxamide, 8-(4-chloro-2,6-difluorophenyl)-N-diaminomethylene)-5,6-dihydronaphthalene-2-carboxamide, N-(diaminomethylene)-8-(2,6-difluoro-4-methoxyphenyl)-5,6-dihydronaphthalene-2-carboxamide, N-(diaminomethylene)-8-(2,6-difluorophenyl)-5,6-dihydronaphthalene-2-carboxamide, N-{(1E)-amino[(2-methoxyethyl)amino]methylene}-8-(2-methoxyphenyl)-5,6-dihydronaphthalene-2-carboxamide, N-(diaminomethylene)-8-(3-fluoro-2-methoxyphenyl)-5,6-dihydranaphthalene-2-carboxamide, N-(diaminomethylene)-8-(2-fluoro-6-methoxyphenyl)-5,6-dihydronaphthalene-2-carboxamide, N-(diaminomethylene)-8-(3,5-difluoropyridin-4-yl)-5,6-dihydranaphthalene-2-carboxamide, N-(diaminomethylene)-3-(2-methoxyphenyl)-1-benzothiophene5-carboxamide, and N-(diaminomethylene)-3-(2-methoxyphenyl)-2-methyl-1-benzothiophene5-carboxamide.

Other preferred embodiments in the compound (I) are the following compounds or salts thereof.

(15) The compound, in which $R^1$, $R^2$, and $R^3$ are the same with or different from each other, and each represent H, lower alkyl, halogen, halogeno-lower alkyl, -CN, $-NO0_2$, $-NR^bR^c$, $-OR^a$, $-C(O)R^a$, $-CO_2R^a$, $-C(O)NR^bR^c$, $-SO_2$-lower alkyl,
$-NR^bC(O)R^a$, lower alkylene-$OR^a$, lower alkylene-$NR^bR^c$, lower alkylene-CN, phenyl,
or -O-phenyl; $R^7$ and $R^8$ are the same with or different from each other, and each represent H, lower alkyl or halogen; and $R^{10}$ represents H or halogen.

The preferred embodiments in (15) are the compounds that are defined in the same manner as in (1) to (13) above.
Furthermore, the compound (I) may exist in the form of other tautomers, geometrical isomers, or optical isomers, depending on the kind of the substituents. Although in the specification, one form of the isomers may be described, the present invention includes these isomers, isolated forms thereof, or a mixture therof. For example, in the acylguanidine sites of the compound (I), two isomers can be present in which the sites of a double bond differ as shown in the following scheme. Also, each of the isomers may be present in the form of an E-isomer and a Z-isomer, based on the geometrical configuration of double bonds. The present invention includes all of these isomers.

[Chem. 7]

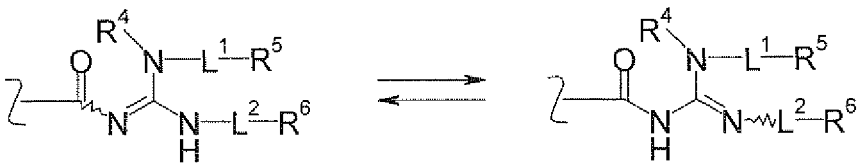

(wherein the structure in the formula partially shows the acylguanidine moiety of the compound (I). The bond represented by the wavy line represents that either of E and Z configurations can be taken).
Furthermore, a pharmaceutically acceptable prodrug of the compound (I) is also included in the present invention. The pharmaceutically acceptable prodrug refers to the compound which has a group that can be converted into an amino

group, OH, $CO_2H$, or the like by solvolysis or under a physiological condition, and produces the compound (I) *in vivo* after administration. Examples of the group forming a prodrug include the groups described in "Prog. Med., 5, 2157-2161 (1985), and "Iyakuhin no Kaihatsu (Development of Medicines)" (Hirokawa Shoten, 1990), vol. 7, Bunshi Sekkei (Molecular Design)", 163-198.

Furthermore, the compound (I) may form an acid addition salt, or may form a salt with a base depending on the kind of substituents, and any salt that is pharmaceutically acceptable is included in the present invention. Specifically, examples of the salts include acid addition salts with inorganic acids such as hydrochloric acid, hydrobromic acid, hydroiodic acid, sulfuric acid, nitric acid, and phosphoric acid, and with organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, and glutamic acid, salts with inorganic bases such as sodium, potassium, magnesium, calcium, and aluminum, and organic bases such as methylamine, ethylamine, ethanolamine, lysine, and ornithine, and ammonium salts.

In addition, the compound (I) and a salt thereof also include various hydrates, solvates, and substances of crystalline polymorphism. Also, the compound (I) and a salt thereof also include various compounds labeled with radioactive isotopes or nonradioactive isotopes.

(Production Processes)

The compound (I) of the present invention may be produced by applying various known synthetic methods, using the characteristics based on their basic skeletons or the kind of the substituents. Further, depending on the kind of a functional group, it is sometimes effective from the viewpoint of the production techniques to protect the functional group with an appropriate protecting group (a group which may be easily converted into the functional group), during the steps of from starting materials to intermediates. Examples of such a functional group include an amino group, a hydroxyl group, and a carboxyl group, and examples of such a protecting group include protecting groups described in "Green's Protective Groups in Organic Synthesis", edited by P. G. M. Wuts and T. W. Greene, 4th Edition, 2006, which may be optionally selected and used in response to the reaction conditions. By such a method, a desired compound can be obtained by introducing a protecting group to carry out the reaction, and then, removing the protecting group as needed.

In addition, a prodrug of the compound (I) can be produced by introducing a specific group during the steps from starting materials to intermediates, in a similar way to the aforementioned protecting groups, or by carrying out a reaction using the obtained compound (I). The reaction may be carried out by employing a method known to a skilled person in the art, such as ordinary esterification, amidation, and dehydration.

Hereinbelow, the representative production processes of the compounds of the present invention are described. Each of the production processes can be carried out with reference to the references cited in the description. Further, the production processes of the present invention are not limited to the examples as shown below.

(Production Process 1)

[Chem. 8]

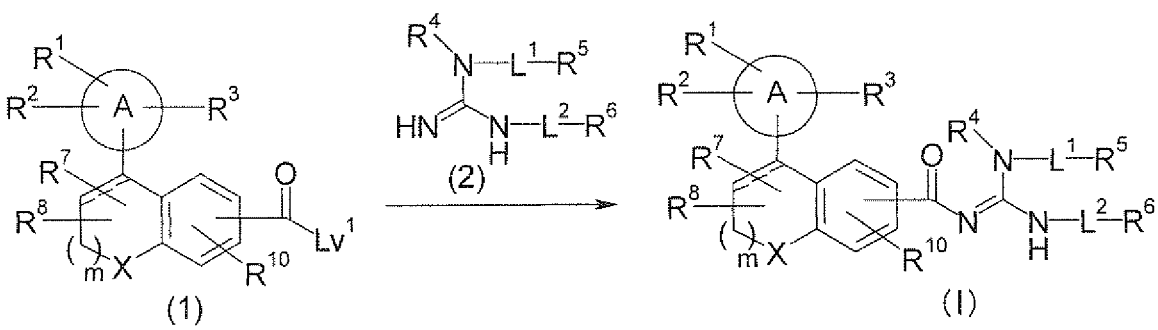

(wherein $Lv^1$ represents -OH or a leaving group).

The compound (I) of the present invention can be produced by the reaction of a carboxylic acid or a reactive derivative thereof (1) with guanidine (2) or a salt thereof.

The reaction can be carried out using equivalent amounts of the carboxylic acid or a reactive derivative thereof (1) and guanidine (2), or an excess amount of guanidine. It can be carried out under cooling to under heating, preferably at from -20˚C to 80˚C, in a solvent which is inert to the reaction, such as aromatic hydrocarbons such as benzene, toluene, or xylene, halogenated hydrocarbons such as dichloromethane, 1,2-dichloromethane, or chloroform, ethers such as diethylether, tetrahydrofuran (THF), dioxane, or dimethoxyethane (DME), N,N-dimethylformamide (DMF), dimethylsulfoxide (DMSO), N-methylpyrolidone (NMP), ethyl acetate, acetonitrile, or water, or a mixture thereof.

When a free carboxylic acid wherein $Lv^1$ is OH is used as the starting compound (1), it is desirable to carry out the reaction in the presence of a condensing agent. In that case, examples of the condensing agent include N,N'-dicyclohexylcarbodiimide (DCC), 1-[3-(dimethylamino)propyl]-3-ethylcarbodiimide (WSC), 1,1'-carbonyldiimidazole (CDI), 2-(1H-benzotriazol-1-yl)-1,1,3,3-tetramethyluronium hexafluorophosphate (HBTU), diphenylphosphoryl azide (DPPA), and phosphorous oxychloride. In some cases, it is preferred to further add an additive agent (e.g., N-hydroxysuccinimide (HONSu), 1-hydroxybenzotriazole (HOBt)), and the like. Normaly, the condensing agent is used in an equivalent amount or excess amount based on the carboxylic acid.

As the reactive derivative of the carboxylic acid wherein $Lv^1$ is a leaving group regarding the starting compound (1), an acid halide (acid chloride, acid bromide, or the like), an acid anhydride (a mixed acid anhydride with phenyl chlorocarbonate, p-toluenesulfonic acid, or isovaleric acid, or the like or a symmetric acid anhydride), an active ester (an ester which can be prepared using phenol that may be substituted with an electron withdrawing group such as a nitro group or a fluorine atom, HOBt, HONSu and the like), a lower alkyl ester and the like may be exemplified, and each of them can be produced from carboxylic acid using a reaction obvious to those skilled in the art. Depending on the kind of a reactive derivative, it is sometimes advantageous for smooth progress of the reaction to carry out the reaction in the presence of a base (organic bases such as triethylamine, diisopropylethylamine (DIPEA), N-methylmorpholine, pyridine, or 4-(N,N-dimethylamino)pyridine, or inorganic bases such as sodium hydrogen carbonate, or the like). Pyridine can also serve as a solvent. In this connection, when a lower alkyl ester is used as the reactive derivative, it is desirable to carry out the reaction under room temperature to under heating to reflux.

(Production Process 2)

[Chem. 9]

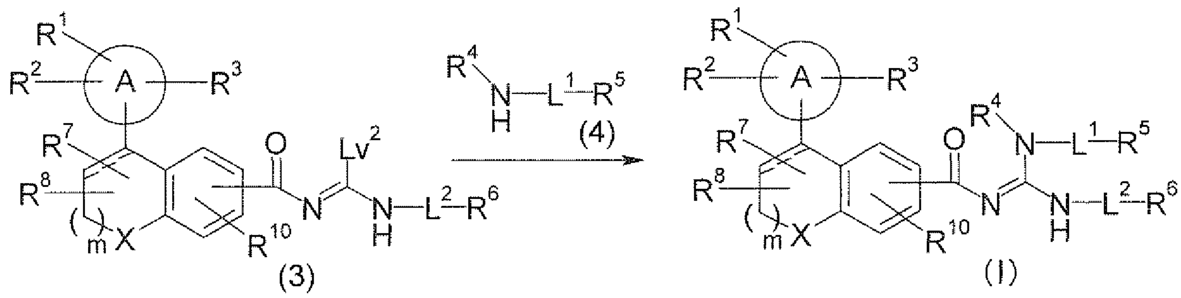

(3) (4) (I)

(wherein $Lv^2$ represents a leaving group such as pyrazol-1-yl which may be substituted with lower alkyl, -S-lower alkyl, -O-phenyl, -Br, and -Cl)

The compound (I) of the present invention can be produced by the reaction of an amidine compound (3) having a leaving group with an amine compound (4).

This reaction can be carried out using equivalent amounts of the compound (3) and the compound (4), or either thereof in an excess amount. The mixture of these compounds is stirred under from cooling to heating under reflux, preferably at from 0˚C to 80˚C, in a solvent inert to reaction or without a solvent, usually for 0.1 hour to 5 days. Examples of the solvent used herein are not limited, but include aromatic hydrocarbons, ethers, halogenated hydrocarbons, DMF, DMSO, NMP, ethyl acetate, acetonitrile, and a mixture thereof. It is sometimes advantageous for smooth progress of the reaction to carry out the reaction in the presence of organic bases such as triethylamine, N,N-diisopropylethylamine, or N-methylmorpholine, or inorganic bases such as potassium carbonate, sodium carbonate, or potassium hydroxide.

(Production Process 3: Other Production Processes)

The compounds of the present invention having various functional groups such as an amino group, a carboxyl

group, an amido group, a hydroxyl group, and an alkylamino group can be easily synthesized by methods which are obvious to those skilled in the art, or modified methods thereof, using the compounds of the present invention having a corresponding nitro group, ester group, carboxyl group, amino group, and the like, as the starting materials. For example, these can be produced by the following reactions.

3-a: Reduction

A compound having an amino group can be produced by reducing a compound having a nitro group. For example, the reaction can be carried out using a hydrogenation reaction which uses palladium-carbon, Raney nickel, or the like as the catalyst.

3-b: Hydrolysis

A compound having a carboxyl group can be produced by hydrolyzing a compound having an ester group. For example, this may be carried out in accordance with the deprotection reaction described in the aforementioned "Green's Protective Groups in Organic Synthesis".

3-c: Alkylation

A compound having an alkylamino group can be produced by alkylating a compound having an amino group. As the alkylation reaction, the reaction can be carried out by a general method using various alkylating agents (for example, an alkyl halide, an alkyl sulfonic acid ester, and the like). In addition, a compound having an alkylamino group can be produced by carrying out reductive alkylation of a compound having an amino group with a carbonyl compound. The method described in "Jikken Kagaku Koza (Experimental Chemistry Course) (vol. 20) Yuki Gosei (Organic Synthesis) 2", edited by The Chemical Society of Japan, 4th Edition, Maruzen, 1992, p. 300; or the like can be applied to the reaction.

The starting compounds (1) to (4) in the Production Processes described above can be produced, by a conventionally known method, or a modified method thereof. For example, the starting compound (1) can be produced directly by the following manner (a reaction route shown in the production process for the starting compound), or produced by removing the protecting group of $-CO_2R^{11}$ of the compound (1a) or (1b) obtained by the route.

(Production Process of Starting Compound)

[Chem. 10]

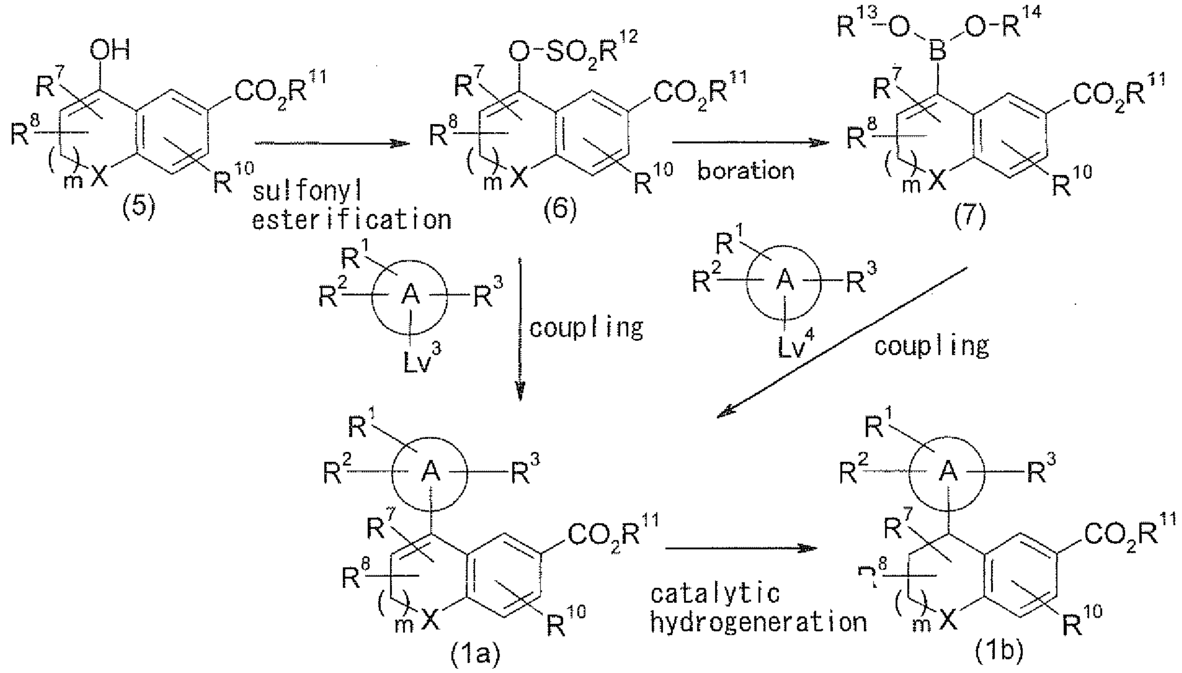


(in the formula, $R^{11}$ represents a protecting group for a carboxylic group, such as lower alkyl or benzyl, or H; $R^{12}$ represents halogeno-lower alkyl; $R^{13}$ and $R^{14}$ are the same with or different from each other, and each represents lower alkyl, or $R^{13}$ and $R^{14}$ may be combined with each other to form lower alkylene. $Lv^3$ and $Lv^4$ each represent a leaving group).

The leaving group represented by $Lv^3$ can be exemplified by $-B(OH)_2$, - $B(OR^{13})(OR^{14})$ or the like, and the leaving group represented by $Lv^4$ can be exemplified by halogen, a trifluoromethanesulfonyloxy group or the like.

Here, the sulfonyl esterification, boration, and coupling reactions can be carried out by the methods described in "Metal-Catalyzed Cross-Coupling Reactions" edited by A. d. Meijere and F. Diederich, 1st Edition, VCH Publishers Inc., 1997. Furthermore, the catalytic hydrogenation can be carried out by the methods described in "Reductions in Organic Chemistry, 2nd ed (ACS Monograph: 188)" edited by M. Hudlicky, ACS, 1996.

In the above-described starting compound (1a), a compound in which m is 0, and X is O and S, can be produced by the methods described in J. Chem. Soc., Perkin Trans. 1, 2421-2423 (1999). The starting compound (1a) in which m is 1, X is O, and, together with a benzene ring, these atoms form a chromene ring which has a lower alkyl group at the 2-position, can be produced by referring to the methods described in Tetrahedron Asymmetry 14, 1529-1534 (2003). Furthermore, a compound having a lower alkyl group at the 3-position of a chromene ring can be produced by referring to the methods described in Tetrahedron 59, 9641-9648 (2003).

Thus obtained compounds (I) is isolated and purified as their free compounds, or pharmaceutically acceptable salts, hydrates, or crystalline polymorphism thereof. The pharmaceutically acceptable salt of the compound (I) can be produced by a conventional salt formation treatment which is technical common knowledge among those skilled in the art. The isolation and purification can be carried out by employing common chemical operations such as extraction, fractional crystallization, and various fractionation chromatography.

Various isomers can be isolated by selecting appropriate starting compounds, or by making use of the differences in the physicochemical properties among the isomers. For example, the optical isomers can be separated to a stereochemically pure isomer by general optical separations (for example, a fractional crystallization from which a diastereomeric salt with an optically active base or acid is derived, and chromatography using a chiral column). In addition, they can also be produced from appropriate starting compounds that are optically active.

Examples

Hereinbelow, the processes for producing the compound of the present invention will be described with reference to Examples. Also, the processes for producing the compounds used as starting materials will be described with reference to Preparative Examples. In addition, the production process for producing the compound (I) is not limited to the production

processes in specific Examples, and can be produced by combination of these production processes, or by a known production process.

Preparative Example 1

To a solution of methyl 2,2-dimethyl-4-oxochromane-6-carboxylate (1.0 g) in dichloromethane (20 mL) were added 2,6-di-tert-butyl-4-methylpyridine (1.7 g) and trifluoromethane sulfonic anhydride (2.4 g) at 0˚C, followed by stirring at the same temperature for 10 minutes, and then stirring at room temperature for additional 5 hours. The reaction mixture was diluted with hexane, and the insoluble materials were then separated by filtration, and the filtrate was concentrated under reduced pressure. The resulting residue was purified by silica gel column chromatography (hexane/ethyl acetate=4/1) to obtain methyl 2,2-dimethyl-4-{[(trifluoromethyl)sulfonyl]oxy}-2H-chromene-6-carboxylate (1.47 g).

Preparative Example 2

A mixed solution of methyl 2,2-dimethyl-4-{[(trifluoromethyl)sulfonyl]oxy}-2H-chromene-6-carboxylate (584 mg), 2-methoxyphenyl boric acid (291 mg), tetrakis(triphenylphosphine)palladium (46 mg), and DIPEA (412 mg) in NMP (3 mL) was heated under stirring with microwave at 170˚C for 10 minutes. The reaction mixture was returned to room temperature, diluted with water, and then extracted with ethyl acetate. The organic layer was washed with water, dried, and then concentrated under reduced pressure. The resulting residue was purified by silica gel column chromatography (hexane/ethyl acetate=9/1) to obtain methyl 4-(2-methoxyphenyl)-2,2-dimethyl-2H-chromene-6-carboxylate (467 mg).

Preparative Example 3

A mixture of methyl 4-(2-methoxyphenyl)-2,2-dimethyl-2H-chromene-6-carboxylate (450 mg), a 1 M aqueous sodium hydroxide solution (3 mL), THF (3 mL), and methanol (3 mL) was heated under stirring at 60˚C for 14 hours. The reaction mixture was returned to room temperature, and neutralized with hydrochloric acid, and the solution was then concentrated under reduced pressure. The resulting residue was washed with water, and collected by filtration to obtain 4-(2-methoxyphenyl)-2,2-dimethyl-2H-chromene-6-carboxylic acid (370 mg).

Preparative Example 4

A mixed solution of methyl 2,2-dimethyl-4-{[(trifiuoromethyl)sulfonyl]oxy}-2H-chromene-6-carboxylate (3.0 g), bis(pinacolato)diboron (2.48 g), bis(triphenylphosphine)palladium chloride (311 mg), triphenylphosphine (233 mg), and potassium acetate (2.61 g) in 1,4-dioxane (60 mL) was heated under stirring at 100˚C for 18 hours. The reaction mixture was returned to room temperature, the insoluble materials were separated by filtration, and the filtrate was concentrated under reduced pressure. The resulting residue was purified by silica gel column chromatography (hexane/ethyl acetate=4/1) to obtain methyl 4-(4,4,5,5-tetramethyl-1,3,2-dioxaborolan-2-yl)-2H-chromene-6-carboxylate (970 mg).

Preparative Example 5

A mixed solution of methyl 4-(4,4,5,5-tetramethyl-1,3,2-dioxaborolan-2-yl)-2H-chroinene-6-carboxylate (400 mg), 4-bromo-3-methylbenzonitrile (372 mg), 1,1'-bis(diphenylphosphino) ferrocene dichloropalladium (46 mg), and cesium fluoride (384 mg) in DME (10 mL) was stirred at 100˚C for 3 days under an argon atmosphere. The reaction mixture was diluted with water, and then extracted with ethyl acetate. The organic layer was washed with water, dried, and then concentrated under reduced pressure. The resulting residue was purified by silica gel column chromatography (hexane/ethyl acetate=4/1) to obtain methyl 4-(4-cyano-2-methylphenyl)-2H-chromene-6-carboxylate (253 mg).

Preparative Example 6

To a mixed liquid of methyl 8-{[(trifluoromethyl)sulfonyl]oxy}-5,6-dihydronaphthalene-2-carboxylate (1.0 g) and iron (III) acetylacetonate (53 mg) in THF (60 mL) and NMP (3 mL) was added chloro(tetrahydro-2H-pyran-4-yl) magnesium (a 1 M THF solution, 4.46 mL) at -30˚C, followed by stirring at the same temperature for 15 minutes. The reaction mixture was diluted with a saturated aqueous ammonium chloride solution, and then extracted with ethyl acetate. The organic layer was washed with water, dried, and then concentrated under reduced pressure. The resulting residue was purified by silica gel column chromatography (hexane/ethyl acetate=85/15) to obtain methyl 8-(tetrahydro-2H-pyran-4-yl)-5,6-dihydronaphthalene-2-carboxylate (298 mg).

Preparative Example 7

To a solution of 2-hydroxy-5-methylbenzonitrile (2.0 g) in dichloromethane (40 mL) were added triethylamine (1.8 g) and trifluoromethanesulfonic anhydride (5.1 g) at 0˚C, followed by stirring at the same temperature for 30 minutes, and then stirring at room temperature for additional 1 hour. The reaction mixture was diluted with water, and the organic layer was then washed with water, dried, and concentrated under reduced pressure. The resulting residue was purified by silica gel column chromatography (chloroform) to obtain 2-cyano-4-methylphenyltrifluoromethanesulfonate (2.7 g).

Preparative Example 8

2-Cyano-4-methylphenyltrifluoromethanesulfonate (2.6 g), bis(pinacolato)diboron (2.74 g), bis(triphenylphosphine)palladium chloride (344 mg), triphenylphosphine (257 mg), and potassium acetate (2.89 g) were heated in 1,4-dioxane in the same manner as in Preparative Example 4 to obtain 5-methyl-2-(4,4,5,5-tetramethyl-1,3,2-dioxaborolan-2-yl)benzonitrile (2.38 g).

Preparative Example 9

To a solution of diisopropyl amine (1.5 mL) in THF (40 mL) was added n-butyl lithium (a 1.58 M n-hexane solution, 6.5 mL) at -78˚C, followed by stirring at 0˚C for 30 minutes. To the solution was added methyl 8-oxo-5,6,7,8-tetrahydronaphthatene-2-carboxylate (2.0 g) at -78˚C, followed by stirring at the same temperature for 1 hour. To the solution were further added hexamethylphosphoramide (5 mL) and methyl iodide (1 mL), followed by stirring at room temperature for 1 hour. The reaction mixture was diluted with water, and then extracted with ethyl acetate. The organic layer was washed with water, dried, and then concentrated under reduced pressure. The resulting residue was purified by silica gel column chromatography (hexane/ethyl acetate=9/1) to obtain methyl 7-methyl-8-oxo-5,6,7,8-tetrahydronaphthalene-2-carboxylate (897 mg).

Preparative Example 10

A mixed solution of methyl 8-oxo-5,6,7,8-tetrahydronaphthalene-2-carboxylate (3.0 g) and 1-fluoro-4-hydroxy-1,4-diazoniabicyclo[2,2,2]octane bis(tetrafluoroborate) (5.2 g) in methanol (140 mL) was heated under reflux for 3 hours. The reaction mixture was concentrated under reduced pressure, and diluted with dichloromethane, and the insoluble materials were separated by filtration. The filtrate was washed with water, dried, and then concentrated under reduced pressure. The resulting residue was purified by silica gel column chromatography (hexane/ethyl acetate=4/1) to obtain methyl 7-fluoro-8-oxo-5,6,7,8-tetrahydronaphthalene-2-carboxylate (2.8 g).

Preparative Example 11

To a solution of methyl p-hydroxybenzoate (17.7 g), 4-penten-2-ol (10 g) and triphenylphosphine (33.5 g) in THF (175 mL) was added dropwise diethyl azodicarboxylate (a 40% toluene solution, 55 mL) under ice-cooling, followed by stirring at room temperature for 3 days. The reaction mixture was concentrated, and the resulting residue was then added with diethyl ether/hexane, the insoluble materials were separated by filtration, and the filtrate was concentrated under reduced pressure. The resulting residue was purified by silica gel column chromatography (hexane/ethyl acetate=201/1) to obtain methyl 4-[(1-methylbut-3-en-1-yl)oxy]benzoate (19.2 g).

Preparative Example 12

To a solution of methyl 4-[(1-methylbut-3-en-1-yl)oxy]benzoate (11.5 g) in dichloromethane/acetonitrile/water (2/2/3, 100 mL) were added sodium metaperiodate (44.5 g) and ruthenium chloride (III) hydride (235 mg), followed by stirring at room temperature for 14 hours. The insoluble materials were separated by filtration, and the mother liquor was then extracted with ethyl acetate. The organic layer was washed with an aqueous sodium sulfite solution, and concentrated under reduced pressure. The resulting residue was purified by silica gel column chromatography (chloroform/methanol=9/1) to obtain 3-[4-(methoxycarbonyl)phenoxy]butanoic acid (8.4 g).

Preparative Example 13

A mixture of 3-[4-(methoxycarbonyl)phenoxy]butanoic acid (8.4 g) and trifluoromethanesulfonic acid (75 g) was stirred at room temperature for 1 hour. The solution was diluted with water, and then extracted with ethyl acetate. The organic layer was washed with water, dried, and then concentrated under reduced pressure. The resulting residue was

purified by silica gel column chromatography (chloroform) to obtain methyl 2-methyl-4-oxochromane-6-carboxylate (2.8 g).

Preparative Example 14

To a solution of 4-mercaptobenzoic acid (1.5 g) in ethanol (36 mL) were added 2-bromo-2'-methoxyacetophenone (2.3 g) and potassium carbonate (4.0 g), followed by stirring at room temperature for 3 days. The reaction mixture was diluted with water, and neutralized with 1 M hydrochloric acid, and the precipitate was collected by filtration to obtain 4-{[2-(2-methoxyphenyl)-2-oxo ethyl]sulfanyl}benzoic acid (2.94 g).

Preparative Example 15

To a solution of 4-{[2-(2-methoxyphenyl)-2-oxo ethyl]sulfanyl}benzoic acid (1.0 g) in toluene (30 mL) was added Amberlyst 15 (registered trademark) (3.0 g), followed by heating under reflux for 3 days. The reaction mixture was returned to room temperature, the insoluble materials were separated by filtration, and the mother liquid was then concentrated under reduced pressure to obtain 3-(2-methoxyphenyl)-1-benzothiophene-5-carboxylic acid (900 mg).

Preparative Example 16

To a solution of methyl 8-hydroxy-5,6,7,8-tetrahydronaphthalene-2-carboxylate (1.76 g) and pyridine (743 mg) in dichloromethane (10 mL) was added thionyl chloride (1.32 g) under ice-cooling, followed by stirring at room temperature for 2 hours. The reaction mixture was concentrated under reduced pressure. The resulting residue was diluted with water, and then extracted with ethyl acetate. The organic layer was washed with water, dried, and then concentrated under reduced pressure to obtain methyl 8-chloro-5,6,7,8-tetrahydronaphthalene-2-carboxylate (1.6 g).

Preparative Example 17

A mixed solution of methyl 8-chloro-5,6,7,8-tetrahydronaphthalene-2-carboxylate (1.57 g), 1-(tert-butoxycarbonyl)piperazine (1.56 g), sodium iodide (209 mg), and potassium carbonate (1.26 g) in DMF (30 mL) was heated under stirring at 70°C for 5 hours. The reaction mixture was returned to room temperature, diluted with water, and then extracted with ethyl acetate. The organic layer was washed with water, dried, and then concentrated under reduced pressure. The resulting residue was purified by silica gel column chromatography (hexane/ethyl acetate=4/1) to obtain tert-butyl 4-[7-(methoxycarbonyl)-1,2,3,4-tetrahydronaphthalen-1-yl]piperazine-1-carboxylate (2.15 g).

Preparative Example 18

To a solution of methyl 8-phenyl-5,6-dihydronaphthalene-2-carboxylate (500 mg) in methanol (40 mL) was added 10% palladium carbon (100 mg), followed by stirring at room temperature for 2 days under a 1 atm hydrogen gas atmosphere. The insoluble materials were separated by filtration, and the filtrate was concentrated under reduced pressure. Then, the resulting residue was purified by silica gel column chromatography(hexane/ethyl acetate=4/1) to obtain methyl 8-phenyl-5,6,7,8-tetrahydronaphthalene-2-carboxylate (349 mg).

Preparative Example 19

To a solution of DIPEA (545 mg) in THF (30 mL) was added n-butyl lithium (a 1.57 M hexane solution, 3.4 mL) at -70°C, followed by stirring at 0°C for 30 minutes. Then, the reaction mixture was cooled to -70°C, and added dropwise with a solution of methyl 8-oxo-5,6,7,8-tetrahydronaphthalene-2-carboxylate (1.0 g) in THF. Then, it was stirred at the same temperature for 30 minutes, and then added with acetaldehyde (237 mg), followed by stirring for 2 hours. The reaction mixture was diluted with acetic acid and water, and then extracted with diethyl ether, and the organic layer was concentrated under reduced pressure. The resulting residue was purified by silica gel column chromatography (hexane/ethyl acetate=1/1) to obtain methyl 7-(1-hydroxyethyl)-8-oxo-5,6,7,8-tetrahydronaphthalene-2-carboxylate (570 mg).

Preparative Example 20

To a solution of methyl 7-(1-hydroxyethyl)-8-oxo-5,6,7,8-tetrahydronaphthalene-2-carboxylate (570 mg) in dichloromethane (20 mL) were added anhydrous acetic acid (469 mg) and pyridine (400 mg), followed by stirring at room temperature for 14 hours. The reaction mixture was diluted with water, and then extracted with ethyl acetate. The organic layer was washed with 1 M hydrochloric acid and then with a saturated aqueous sodium bicarbonate solution,

dried, and then concentrated under reduced pressure. The resulting residue was dissolved in 1,2-dichloroethane (20 mL), and then added with triethylamine (465 mg), followed by stirring at 60˚C for 14 hours. The reaction mixture was returned to room temperature, diluted with water, and then extracted with ethyl acetate. The organic layer was washed with 1 M hydrochloric acid, dried, concentrated under reduced pressure, and then purified by silica gel column chromatography(hexane/ethyl acetate=3/1) to obtain methyl 7-ethylidene-8-oxo-5,6,7,8-tetrahydronaphthalene-2-carboxylate (300 mg).

Preparative Example 21

By using methyl 7-ethylidene-8-oxo-5,6,7,8-tetrahydronaphthalene-2-carboxylate (300 mg), and carrying out the catalytic hydrogenation as in Preparative Example 18, methyl 7-ethyl-8-oxo-5,6,7,8-tetrahydronaphthalene-2-carboxylate (115 mg) was obtained.

Preparative Example 22

By using ethyl 2-(acetoxymethyl)acrylate (6.0 g), methyl p-hydroxybenzoate (7.95 g), bis(dibcnzylideneacetone) palladium (501 mg), 1,2-bis(diphenylphosphino)ethane (694 mg), and potassium fluoride/alumina (20 g), and carrying out the same synthesis method as in Tetrahedron 56,8133-8140 (2000), methyl 4-{[2-(ethoxycarbonyl)prop-2-en-1-yl]oxy}benzoate (8.22 g) was obtained.

Preparative Example 23

By using methyl 4-{[2-(ethoxycarbonyl)prop-2-en-1-yl]oxy}benzoate (2.0 g) and carrying out the catalytic hydrogenation as in Preparative Example 18, methyl 4-(3-ethoxy-2-methyl-3-oxopropoxy)benzoate (1.67 g) was obtained.

Preparative Example 24

A mixture of phosphorus pentoxide (3.0 g) and methanesulfonic acid (20 mL) was stirred at 50˚C for 1 hour. To this solution was added 4-(2-carboxypropoxy) benzoic acid (3.0 g), followed by heating under stirring for additional 1 hour. The reaction mixture was poured into iced water, and extracted with ethyl acetate. The organic layer was dried, and then concentrated under reduced pressure. To the resulting residue were added methanol (60 mL) and concentrated sulfuric acid (6 mL), followed by heating under reflux for 14 hours. This solution was concentrated under reduced pressure. The resulting residue was diluted with water, and then extracted with ethyl acetate. The organic layer was dried, and then concentrated under reduced pressure. The resulting residue was purified by silica gel column chromatography (hexane/ethyl acetate=4/1) to obtain methyl 3-methyl-4-oxochromane-6-carboxylate (868 mg).

Preparative Example 25

A mixture of 4-[(2-carboethoxy)sulfanyl]benzoic acid (5.0 g) and trifluoromethanesulfonic acid (25 g) was stirred at room temperature for 1 hour. The solution was diluted with water, and the precipitate was then collected by filtration. In addition, it was heated under reflux for 14 hours in a mixed solution of concentrated sulfuric acid (30 mL) and methanol (300 mL). The reaction mixture was concentrated under reduced pressure, diluted with water, and then extracted with ethyl acetate. The organic layer was washed with a saturated aqueous sodium bicarbonate solution, dried, and then concentrated under reduced pressure. The resulting residue was purified by silica gel column chromatography (hexane/ethyl acetate=4/1) to obtain methyl 4-oxothiochromane-6-carboxylate (3.58 g).

Preparative Example 26

A mixture of phosphorus pentoxide (100 g) and phosphoric acid (50 mL) was heated under stirring at 130˚C for 1 hour. To this solution was added 5-[4-(methoxycarbonyl)phenyl]valeric acid (4.8 g), and then heated under stirring for additional 2 hours. The reaction mixture was poured into iced water, followed by extraction with ethyl acetate. The organic layer was dried, and then concentrated under reduced pressure. To the resulting residue were added methanol (100 mL) and concentrated sulfuric acid (10 mL), followed by heating under reflux for 14 hours. The reaction mixture was concentrated under reduced pressure. The resulting residue was diluted with water, followed by extraction with ethyl acetate. The organic layer was dried, and then concentrated under reduced pressure. The resulting residue was purified by silica gel column chromatography (hexane/ethyl acetate=9/1) to obtain methyl 9-oxo-6,7,8,9-tetrahydro-5H-benzo[7]annulene -2-carboxylate (703 mg).

Preparative Example 27

A mixture of 7-bromo-3,4-dihydro-1-benzooxepin-5(2H)-one (3.26 g), palladium acetate (II) (607 mg), 1,1'-bis (diphenylphosphino)ferrocene (1.5 g), triethylamine (4.1 g), NMP (30 mL), and methanol (45 mL) was stirred at room temperature for 15 minutes while penetrating a carbon monoxide gas thereinto, and heated under stirring at 80˚C for 16 hours under a 1 atm carbon monoxide gas atmosphere. The reaction mixture was returned to room temperature, diluted with water, and then extracted with ethyl acetate. The organic layer was dried, and then concentrated under reduced pressure. The resulting residue was purified by silica gel column chromatography (hexane/ethyl acetate=4/1) to obtain methyl 5-oxo-2,3,4,5-teirahydro-1-benzooxepine-7-carboxylate (1.65 g).

Preparative Example 28

To a mixture of methyl 8-(4-formylphenyl)-5,6-dihydronaphthalene-2-carboxylate (220 mg), dimethylamine hydrochloride (92 mg), acetic acid (68 mg), triethylamine (114 mg), and 1,2-dichloroethane (5 mL) was added sodium triacetoxyborohydride (239 mg), followed by stirring at room temperature for 14 hours. The reaction mixture was diluted with a 1 M aqueous sodium hydroxide solution, and then extracted with chloroform. The organic layer was dried, and then concentrated under reduced pressure. The resulting residue was purified by silica gel column chromatography (hexane/ethyl acetate-2/1) to obtain methyl 8-{4-[(dimethylamino)methyl]phenyl}-5,6-dihydronaphthalene-2-carboxylate (217 mg).

Preparative Example 29

To a solution of methyl 8-[2-(methylsulfanyl)phenyl]-5,6-dihydronaphthalene-2-carboxylate (200 mg) in dichloromethane (10 mL) was added m-chloroperbenzoic acid (355 mg) under ice-cooling, followed by stirring at room temperature for 7 hours. The reaction mixture was diluted with an aqueous sodium hydrogen sulfite solution, and then extracted with chloroform, and the organic layer was concentrated under reduced pressure. The resulting residue was purified by silica gel column chromatography (hexane/ethyl acetate=1/0 to 0/1) to obtain methyl 8-[2-(methylsulfonyl) phenyl]-5,6-dihydronaphthalene-2-carboxylate (85 mg).

Preparative Example 30 to 484

The compounds of Preparative Examples as shown in the following Tables 1 to 31 were prepared in the same manner as the methods in Preparative Examples 1 to 29, using each of the corresponding starting materials.

Preparative Example 485

To a mixture of methyl 8-oxo-5,6,7,8-tetrahydronaphthalene-2-carboxylate (1.0 g) and THF (30 mL) were added 60% sodium hydride (450 mg) and dimethyl carbonate(1.65 mL), followed by heating under stirring in an oil bath at 60˚C for 3 hours. The reaction mixture was returned to room temperature, diluted with a saturated aqueous ammonium chloride solution, and then extracted with ethyl acetate. The organic layer was washed with water, dried over magnesium sulfate, and then concentrated under reduced pressure. The resulting residue was purified by silica gel column chromatography (hexane/ethyl acetate=7/3) to obtain dimethyl 1-oxo-1,2,3,4-tetrahydronaphthalene-2,7-dicarboxylate (814mg).

Preparative Example 486

A mixture of 1-phenyl-3,4-dihydronaphthalene-2,7-dicarboxylic acid (404 mg), potassium carbonate (228 mg), benzyl bromide (0.18 mL), and DMF (15 mL) was stirred at room temperature for 2 hours. The reaction mixture was diluted with ethyl acetate, washed with water, dried over magnesium sulfate, and then concentrated under reduced pressure. The resulting residue was purified by silica gel column chromatography (hexane/ethyl acetate=1/1) to obtain 7-[(benzyloxy)carbonyl]-1-phenyl-3,4-dihydronaphthalene-2-carboxylic acid (30 mg).

Preparative Example 487

To a mixture of 7-[(benzyloxy)carbonyl]-1-phenyl-3,4-dihydronaphthalene-2-carboxylic acid (49 mg) and THF (4 mL) were added isobutyl chlorocarbonate (21 mg) and triethylamine (15 mg), followed by stirring at room temperature for 1 hour. The resulting insoluble materials were separated by filtration, and then added with sodium tetrahydroborate (10 mg), followed by stirring at room temperature for 3 hours. The reaction mixture was diluted with a saturated aqueous ammonium chloride solution, and then extracted with ethyl acetate. The organic layer was washed with water, dried

over magnesium sulfate, and then concentrated under reduced pressure. The resulting residue was purified by silica gel column chromatography (hexane/ethyl acetate=7/3) to obtain benzyl 7-(hydroxymethyl)-8-phenyl-5,6-dihydronaphthalene-2-carboxylate (28 mg).

Preparative Example 488

To a mixture of methyl 2-(hydroxymethyl)-1-benzothiophene-5-carboxylate (1.0 g) and THF (20 mL) were added 55% sodium hydride (234 mg) and methyl iodide (0.84 mL) in this order at 0˚C under an argon gas atmosphere, followed by stirring at room temperature for 3 hours. The reaction mixture was diluted with 1 M hydrochloric acid, and then extracted with ethyl acetate. The organic layer was washed with water, dried over magnesium sulfate, and then concentrated under reduced pressure. The resulting residue was purified by silica gel column chromatography (hexane/ethyl acetate=100/0 to 80/20) to obtain methyl 2-(methoxymethyl)-1-benzothiophene-5-carboxylate (426 mg).

Preparative Example 489

To a mixture of methyl 2-(methoxymethyl)-1-benzothiophene-5-carboxylate (426 mg), sodium acetate (370 mg), and chloroform(10 mL) was added bromine (0.1 mL) at 0˚C, followed by stirring at room temperature for 2 hours. The solution was diluted with water, and then extracted with chloroform. The organic layer was washed with a saturated aqueous sodium bicarbonate solution, dried over magnesium sulfate, and then concentrated under reduced pressure to obtain methyl 3-bromo-2-(methoxymethyl)-1-benzothiophene-5-carboxylate (568 mg).

Preparative Example 490

To a mixture of methyl 3-bromo-2-(hydroxymethyl)-1-benzothiophene-5-carboxylate (800 mg), THF (15 mL), and methylene chloride (15 mL) was added manganese dioxide (2.3 g), followed by heating under stirring for 2 days in an oil bath at 50˚C. The insoluble materials were separated by filtration, and the mother liquid was then concentrated under reduced pressure to obtain methyl 3-bromo-2-formyl-1-benzothiophene-5-carboxylate (581 mg).

Preparative Example 491

A mixture of methyl 3-bromo-2-formyl-1-benzothiophene-5-carboxylate (580 mg), ammonium hydroxylchloride (269 mg), sodium formate (2.64 g), and formic acid (15 mL) was heated under reflux for 8 hours. This solution was returned to room temperature, diluted with water, and then extracted with diethyl ether. The organic layer was washed with an aqueous sodium bicarbonate solution, dried over magnesium sulfate, and then concentrated under reduced pressure to obtain methyl 3-bromo-2-cyano-1-benzothiophene-5-carboxylate (489 mg).

Preparative Example 492

A mixture ofmethyl 3-bromo-1-benzothiophene-5-carboxylate (300 mg), (2,4-dimethoxyphenyl) boronic acid (503 mg), tetrakis(triphenylphosphine)palladium (128 mg), 2 M aqueous sodium carbonate solution (2.2 mL), ethylene glycol dimethylether (9 mL), and ethanol (0.9 mL) was heated under reflux for 18 hours under an argon gas atmosphere. The reaction mixture was returned to room temperature, diluted with water, and then extracted with ethyl acetate. The organic layer was washed with water, dried over magnesium sulfate, and then concentrated under reduced pressure. The resulting residue was purified by silica gel column chromatography (hexane/ethyl acetate=4/1) to obtain methyl 3-(2,4-dimethoxyphenyl)-1-benzothiophene-5-carboxylate (182 mg).

Preparative Example 493

A mixture of methyl 8-(4,4,5,5-tetramethyl-1,3,2-dioxaborolan-2-yl)-5,6-dihydronaphthalene-2-earboxylate (400 mg), 4-chloro-3-fluoropyridine (402 mg), palladium (II) acetate (14 mg), dicyclohexyl(2',6'-dimethoxybiphenyl-2-yl) phosphine (52 mg), tripotassium phosphate (540 mg), and ethylene glycoldimethylether (15 mL) was heated under reflux for 1 day under an argon gas atmosphere. The reaction mixture was returned to room temperature, diluted with water, and then extracted with ethyl acetate. The organic layer was concentrated under reduced pressure. The resulting residue was purified by silica gel column chromatography (hexane/ethyl acetate=1/1) to obtain methyl 8-(3-fluoropyridin-4-yl)-5,6-dihydronaphthalene-2-carboxylate (81 mg).

Preparative Example 494

To a mixture of methyl 8-(6-methoxypyridin-3-yl)-5,6-dihydronaphthalene-2-carboxylate (203 mg) and methylene chloride (75 mL) were added trimethylchlorosilane (0.52 mL) and sodium iodide (618 mg), followed by heating under reflux for 7 hours. The reaction mixture was returned to room temperature, diluted with water, and then extracted with chloroform. The organic layer was washed with water, dried over magnesium sulfate, and then concentrated under reduced pressure to obtain methyl 8-(6-oxo-1,6-dihydropyridin-3-yl)-5,6-dihydronaphthalene-2-carboxylate (193 mg).

Preparative Example 495

A mixture of methyl 4-[3-(benzyloxy)propoxy]-2-methoxybenzoate (18.38 g), palladium hydroxide (1.7 g), and methanol (200 mL) was stirred at room temperature for 5 hours under a 1 atm hydrogen gas atmosphere. The insoluble materials were separated by filtration, the mother liquid was then concentrated under reduced pressure. The resulting residue was purified by silica gel column chromatography (hexane/ethyl acetate=1/1) to obtain methyl 4-(3-hydroxypropoxy)-2-methoxybenzoate (12.76 g).

Preparative Example 496

To chromium oxide (VI) (10.9 g) was added water (99 mL), followed by adding dropwise concentrated sulfuric acid (9.9 mL) under ice-cooling, and stirring at room temperature for 30 minutes to prepare a Jones reagent. To a mixture of methyl 4-(3-hydroxypropoxy)-2-methoxybenzoate (12.7 g) and acetone (450 mL) was added the prepared Jones reagent, followed by stirring at room temperature for 2 hours. Isopropyl alcohol and sodium sulfite were added thereto, followed by stirring for 1 hour, and the reaction mixture was then concentrated. The resulting residue was extracted with ethyl acetate, and the organic layer was washed with water, dried over magnesium sulfate, and then concentrated under reduced pressure to obtain 3-[3-methoxy-4-(methoxycarbonyl)phenoxy]propanoic acid (10.69 g).

Preparative Example 497

To a mixture of methyl 2,2-dimethyl-4-oxochromane-6-carboxylate (1.0 g) and THF (10 mL) was added dropwise a 1 M lithium bis(trimethylsilyl)amide/THF solution (5 mL) at -78˚C, followed by stirring at the same temperature for 1 hour. Methyl iodide (2 mL) was added thereto, followed by stirring at room temperature for 19 hours. The reaction mixture was diluted with a saturated aqueous ammonium chloride solution, and then extracted with ethyl acetate. The organic layer was washed with water, dried over magnesium sulfate, and then concentrated under reduced pressure. The resulting residue was purified by silica gel column chromatography (hexane/ethyl acetate=4/1) to obtain methyl 2,2,3-trimethyl-4-oxochromane-6-carboxylate (760 mg).

Preparative Example 498

A mixture of methyl 4-{2-[(benzoyloxy)methyl]-3-methylbutoxy}benzoate (912 mg), potassium carbonate (424 mg) and methanol (20 mL) was stirred at room temperature for 2 hours. The reaction mixture was diluted with ethyl acetate, washed with water, dried over magnesium sulfate, and then concentrated under reduced pressure. The resulting residue was purified by silica gel column chromatography (hexane/ethyl acetate=1/1) to obtain methyl 4-[2-(hydroxymethyl)-3-methylbutoxy]benzoate (551 mg).

Preparative Example 499

To a solution of diisopropyl amine (4 mL) in THF (60 mL) was added dropwise a 1.55 M n-butyl lithium/n-hexane solution (20 mL) at -78˚C, followed by stirring at the same temperature for 30 minutes. To this solution was added dropwise a mixture of 2,6-dichloropyridine (2.0 g) and THF (10 mL), followed by stirring at the same temperature for additional 1 hour. A mixture of triisopropyl borate (6.8 mL) and THF (10 mL) was added dropwise thereto, followed by stirring at room temperature for 20 hours. The reaction mixture was diluted with water, and then neutralized with hydrochloric acid, and extracted with ethyl acetate. The organic layer was washed with water, dried over magnesium sulfate, and then concentrated under reduced pressure to obtain (2,6-dichloropyridin-3-yl)boronic acid (2.6 g).

Preparative Example 500

To a mixture of N,N,N',N'-tetramethylethylenediamine (1.9 mL) and diethylether(40 mL) was added n-butyl lithium (a 1.55 M n-hexane solution, 7.5 mL) at -78˚C under an argon gas atmosphere, followed by stirring at the same

temperature for 30 minutes. To this solution was slowly added a solution of 3,5-difluoropyridine (1.21 g) in diethyl ether (10 mL), followed by stirring at -78˚C for 2 hours. To this solution was added iodine (4.0 g), followed by stirring at the same temperature for 1 hour, and then warming to room temperature. The reaction mixture was diluted with water, and the insoluble materials were then separated by filtration, and the filtrate was extracted with diethyl ether. The organic layer was washed with an aqueous sodium bicarbonate solution, dried over magnesium sulfate, and then concentrated under reduced pressure to obtain 3,5-difluoro-4-iodopyridine (820 mg).

Preparative Examples 501 to 853

The compounds of Preparative Examples as shown in the following Tables 32 to 59 were prepared in the same manner as the methods in Preparative Examples 1 to 29, and 485 to 500, using each of the corresponding starting materials.
The production processes and the physicochemical data of the compounds of Preparative Examples 1 to 853 are shown in Tables 60 to 69, respectively.

Example 1-01

A mixed solution of 4-(2-methoxyphenyl)-2,2-dimethyl-2H-chromene-6-carboxylic acid (188 mg) and CDI (148 mg) in DMF (5 mL) was heated under stirring at 60˚C for 30 minutes, the solution was returned to room temperature, and guanidine carbonate (273 mg) was added thereto, followed by stirring at room temperature for additional 15 hours. The reaction mixture was diluted with a saturated aqueous sodium bicarbonate solution, and then extracted with ethyl acetate. The organic layer was washed with water, dried over magnesium sulfate, and then concentrated under reduced pressure. The resulting residue was purified by silica gel column chromatography ("Chromatorex (registered trademark, NH", chloroform/methanol=100/0 to 50/1). To a solution of the purified product in methanol was added an excessive amount of 4 M hydrochloric acid/ethyl acetate, followed by concentrating under reduced pressure. The resulting residue was solidified by methanol/diethyl ether, and collected by filtration to obtain N-(diaminomethylene)-4-(2-methoxyphenyl)-2,2-dimethyl-2H-chromene-6-carboxamide hydrochloride (193 mg).

Examples 1-02

A mixed solution of 4-(2,6-difluorophenyl)-2H-chromene-6-carboxylic acid (1.5 g), WSC hydrochloride (1.5 g), and HOBt (0.49 g) in DMF (45 mL) was stirred at room temperature for 5 minutes, and then 3,5-dimethyl-1H-pyrazole-1-carboxyimidamide nitrate (1.26 g) and DIPEA (1.36 mL) were added thereto, followed by stirring for additional 12 hours. The reaction mixture was diluted with a saturated aqueous sodium bicarbonate solution, and extracted with ethyl acetate. The organic layer was washed with water, dried over sodium sulfate, and then concentrated under reduced pressure. The resulting residue was washed with diisopropyl ether, and collected by filtration to obtain N-[1-amino(3,5-dimethyl-1H-pyrazol-1-yl)methylene]-4-(2,6-difluorophenyl)-2H-chromene-6-carboxamide (1.34 g).

Example 1-03

A mixed solution of N-[1-amino(3,5-dimethyl-1H-pyrazol-1-yl)methylene]-4-(2,6-difluorophenyl)-2H-chromene-6-carboxamide (100 mg) and 2-methoxyethanamine(92 mg) in DMF (3 mL) was stirred at room temperature for 36 hours. The reaction mixture was diluted with water, and extracted with ethyl acetate. The organic layer was washed with water, dried over sodium sulfate, and then concentrated under reduced pressure. The resulting residue was purified by silica gel column chromatography ("Chromatorex (registered trademark), NH", chloroform/methanol=9/1). To a solution of the purified product in methanol was added an excessive amount of a 4 M hydrochloric acid/ethyl acetate solution, followed by concentrating under reduced pressure. The resulting residue was washed with diisopropyl ether, and collected by filtration to obtain N-{1-amino[(2-methoxyethyl)amino]methylene}-4-(2,6-difluorophenyl)-2H-chromene-6-carboxamide hydrochloride (49 mg).

Example 2-01

To a solution of guanidine hydrochloride (1.07 g) in methanol (30 mL) was added sodium methoxide (573 mg), followed by stirring at room temperature for 30 minutes. The reaction mixture was concentrated under reduced pressure, and a mixture of the resulting residue, methyl 8-(3-furyl)-5,6-dihydronaphthalene-2-carboxylate (270 mg) and NMP (20 mL) was heated under stirring at 80˚C for 1 day. The reaction mixture was returned to room temperature, diluted with water, and then extracted with ethyl acetate. The organic layer was concentrated under reduced pressure. The resulting residue was purified by silica gel column chromatography ("Silica Gel 60 N, spherical, neutral", chloroform/methanol/29% aqueous ammonia solution=10/1/0.1). To a solution of the purified product in ethyl acetate was added an excessive amount of methanesulfonic acid, and the precipitate was collected by filtration to obtain N-(diaminomethylene)-8-(3-furyl)-5,6-dihydronaphthalene-2-carboxamide methane sulfonate (145 mg).

Example 3-01

A mixed solution of 8-[4-(tert-butoxycarbonyl)piperazin-1-yl]-5,6,7,8-tetrahydronaphthalene-2-carboxylic acid (164 mg) and CDI (111 mg) in DMF (5 mL) was heated under stirring at 60˚C for 30 minutes. The solution was returned to room temperature, and guanidine carbonate (205 mg) was added thereto, followed by stirring at room temperature for additional 15 hours. The reaction mixture was diluted with a saturated aqueous sodium bicarbonate solution, and then extracted with ethyl acetate. The organic layer was washed with water, dried over magnesium sulfate, and then concentrated under reduced pressure. The resulting residue was purified by silica gel column chromatography ("Chromatorex (registered trademark), NH", chloroform/methanol=9/1). A suspension of the purified product in methanol was treated with an excessive amount of a 4 M hydrochloric acid/ethyl acetate solution, and the precipitate was collected by filtration to obtain N-(diaminoniethylene)-8-piperazin-1-yl-5,6,7,8 -tetrahydronaphthalene-2-carboxamide trihydrochloride (137 mg).

The compounds of Examples as shown in the following Tables 70 to 100 were prepared in the same manner as the methods in above-described Examples 1-01 to 3-01, using each of the corresponding starting materials. The production processes and physicochemical data of the compounds of Examples 1-01 to 3-39 are shown in Tables 101 to 109, respectively.

The following abbreviations are used in the following Tables.

Prep: Preparative Example number, Ex: Example number, No: compound number, Str: structural formula, Dat: Physicochemical data (ESI+: ESI-MS$[M+H]^+$ or ESI-MS$[M]^+$; ESI-: ESI-MS$[M-H]^-$; FAB+: FAB-MS$[M+H]^+$ or FAB-MS$[M]^+$; FAB-: FAB-MS$[M-H]^-$; APCI+: APCI-MS$[M+H]^+$; APCI-: APCI-MS$[M-H]^-$; EI+: EI$[M]^+$; A/E+: APCI/ESI-MS$[M+H]^+$ (APCI/ESI indicates measurements mixed of APCI and ESI); NMR: δ (ppm) of characteristic peaks in $CDCl_3$ or DMSO-$d_6$ by $^1$HNMR, Sal: salt (Blank space or no description indicates that it is a salt free, and the numeral before the acid component shows a molar ratio. For example, when 2HCl is described, it means that the compound is dihydrochloride.), Me: methyl, Et: ethyl, iPr: isopropyl, Ph: phenyl, Tf: trifluoromethanesulfonyl, Boc: tert-butoxycarbonyl, PSyn and Syn: Production process (The numeral indicates that the compound was produced using a corresponding starting material by the same methods as the compound having the numeral as Preparative Example number or Example number. When two or more numbers are described, the compound is produced by the same method as in Preparative Example or Example having the numbers, in the order). In the structural formulae, a compound in which a bond is described by two cross lines, it indicates that the bond is a double bond and the geometrical configuration is not clear.

In the column "Syn" for the Production Processes in the following Tables, identical Example number is given to the each compound with various salt form which is prepared by a different salt forming process, but a same kind of the reaction.

[Table 1]

| Prep | Str | | Prep | Str |
|---|---|---|---|---|
| 1 | 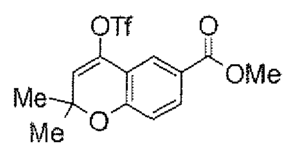 | | 10 | 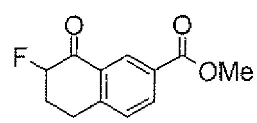 |
| 2 | 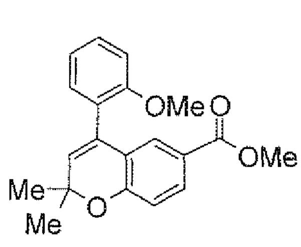 | | 11 | 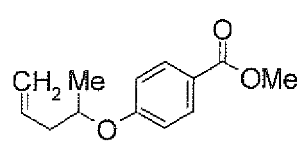 |
| | | | 12 | 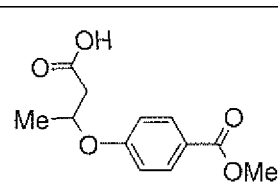 |

(continued)

| Prep | Str | Prep | Str |
|---|---|---|---|
| 3 | 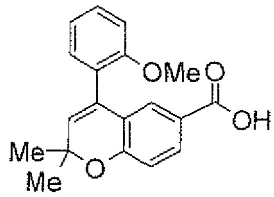 | 13 | 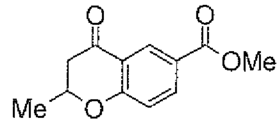 |
| 4 | 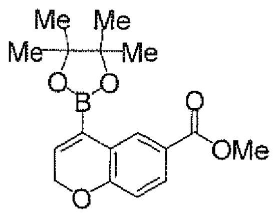 | 14 | 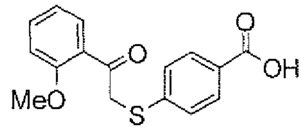 |
| | | 15 | 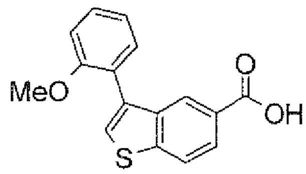 |
| 5 | 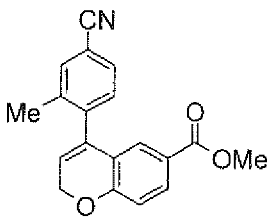 | 16 | 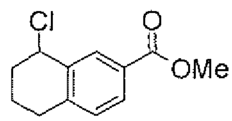 |
| 6 | 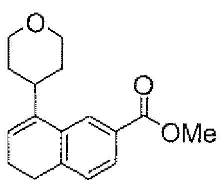 | 17 | 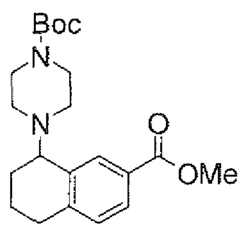 |
| 7 | 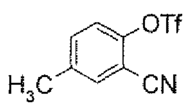 | 18 | 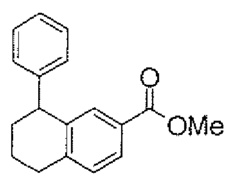 |
| 8 | 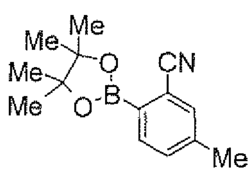 | | |

(continued)

| Prep | Str | | Prep | Str |
|---|---|---|---|---|
| 9 | 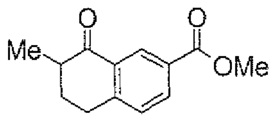 | | 19 | 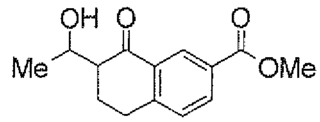 |
| | | | 20 | 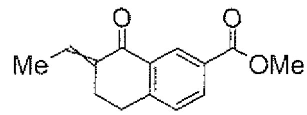 |

[Table 2]

| Prep | Str | | Prep | Str |
|---|---|---|---|---|
| 21 | 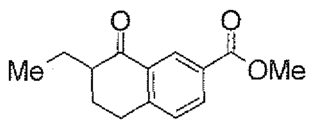 | | 31 | 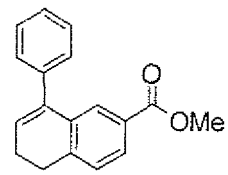 |
| 22 | 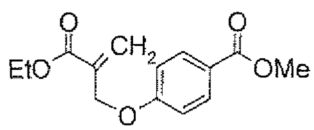 | | 32 | 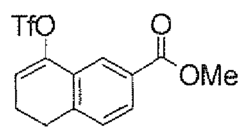 |
| 23 | 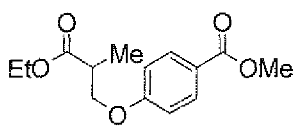 | | | |
| 24 | 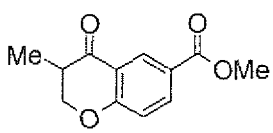 | | 33 | 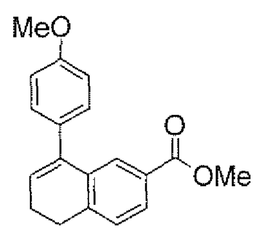 |
| 25 | 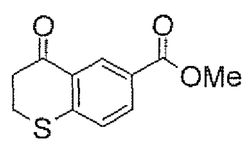 | | | |
| 26 | 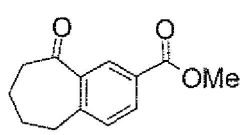 | | 34 | 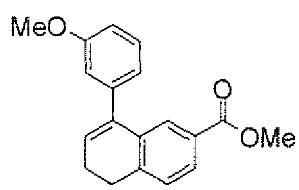 |

(continued)

| Prep | Str | | Prep | Str |
|---|---|---|---|---|
| 27 | 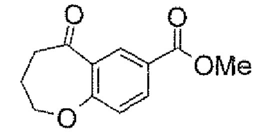 | | 35 | 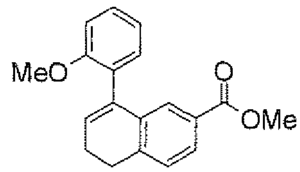 |
| 28 | 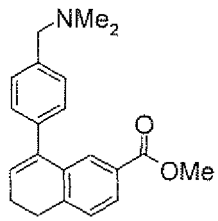 | | 36 | 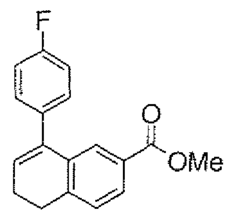 |
| 29 | 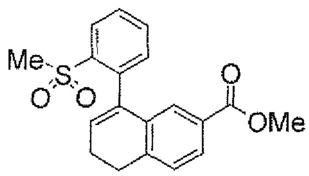 | | 37 | 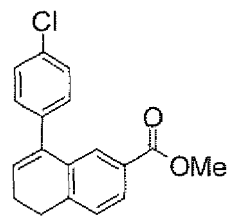 |
| 30 | 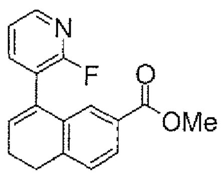 | | 38 | 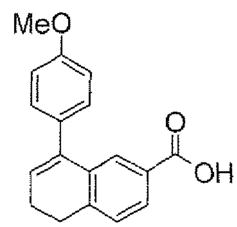 |

[Table 3]

| Prep | Str | | Prep | Str |
|---|---|---|---|---|
| 39 | 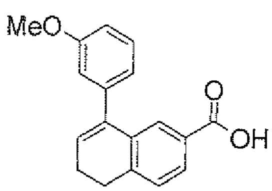 | | 47 | 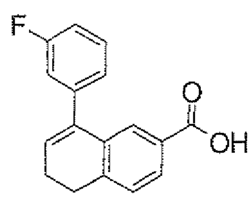 |
| 40 | 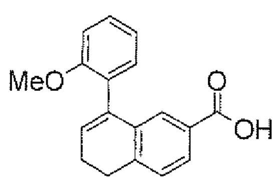 | | 48 | 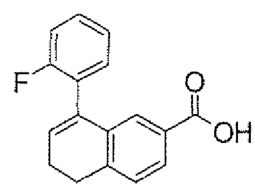 |

(continued)

| Prep | Str | | Prep | Str |
|---|---|---|---|---|
| 41 | 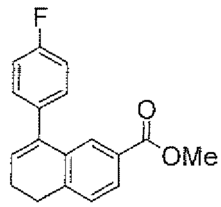 | | 49 | 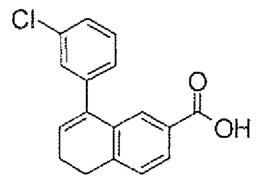 |
| 42 | 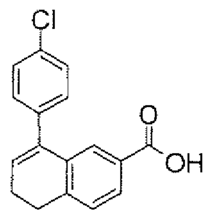 | | 50 | 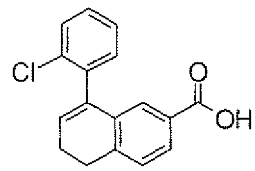 |
| 43 | 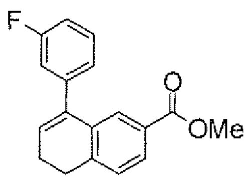 | | 51 | 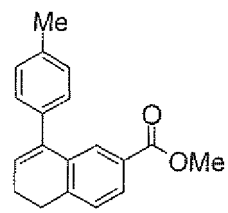 |
| 44 | 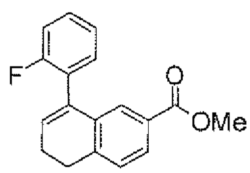 | | 52 | 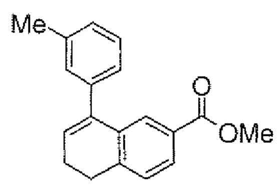 |
| 45 | 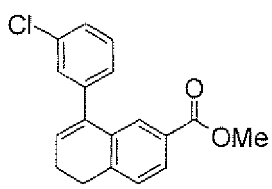 | | 53 | 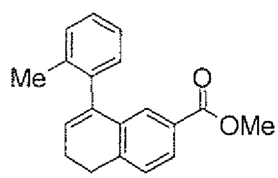 |
| 46 | 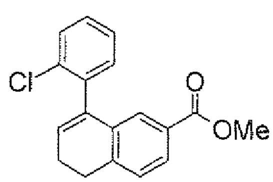 | | 54 | 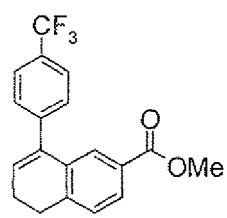 |

[Table 4]

| Prep | Str | | Prep | Str |
|---|---|---|---|---|
| 55 | 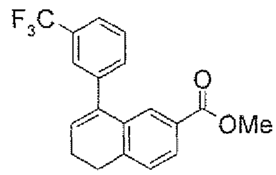 | | 63 | 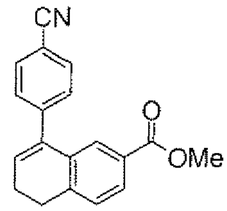 |
| 56 | 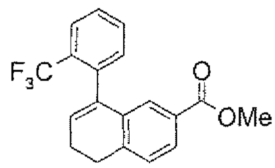 | | 64 | 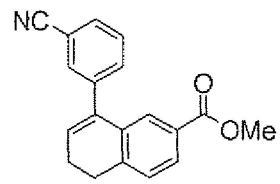 |
| 57 | 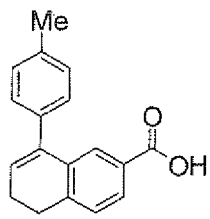 | | 65 | 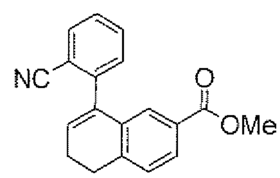 |
| 58 | 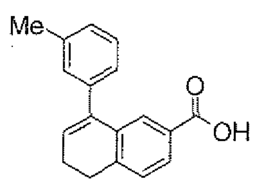 | | 66 | 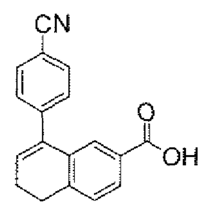 |
| 59 | 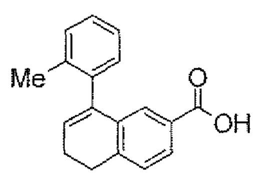 | | 67 | 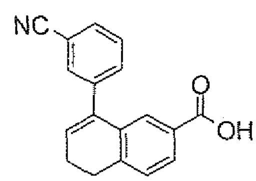 |
| 60 | 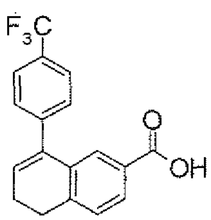 | | 68 | 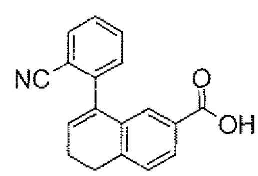 |
| 61 | 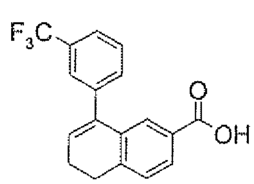 | | 69 | 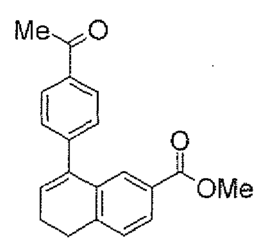 |

(continued)

| Prep | Str | | Prep | Str |
|---|---|---|---|---|
| 62 | 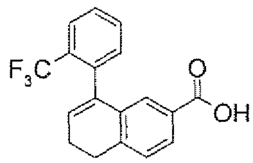 | | 70 | 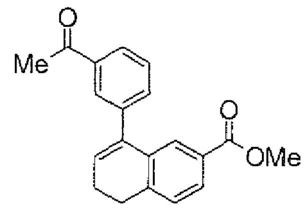 |

[Table 5]

| Prep | Str | | Prep | Str |
|---|---|---|---|---|
| 71 | 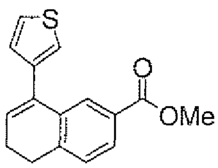 | | 79 | 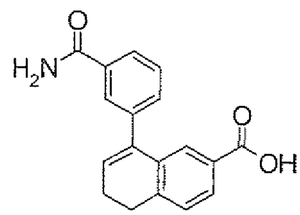 |
| 72 | 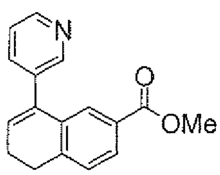 | | 80 | 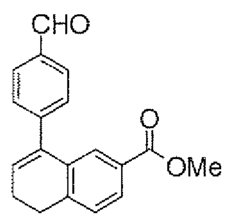 |
| 73 | 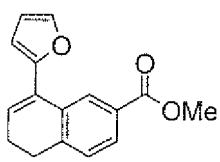 | | 81 | 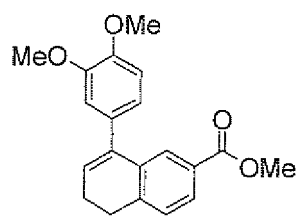 |
| 74 | 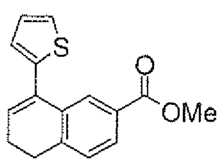 | | | |
| 75 | 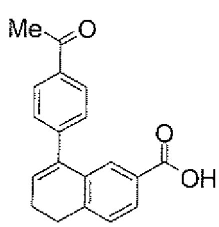 | | 82 | 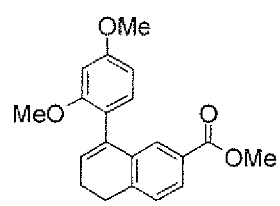 |

(continued)

| Prep | Str | | Prep | Str |
|---|---|---|---|---|
| 76 | 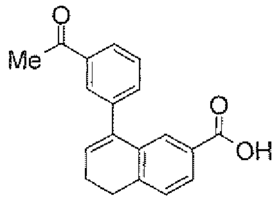 | | 83 | 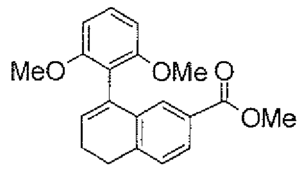 |
| 77 | 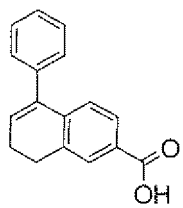 | | 84 | 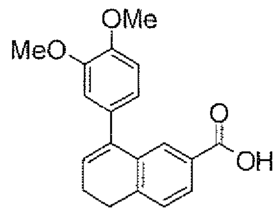 |
| 78 | 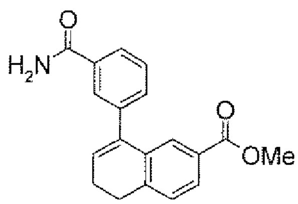 | | 85 | 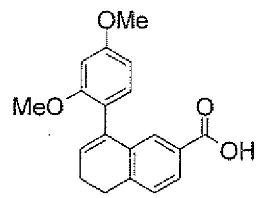 |
| | | | 86 | 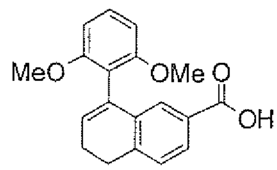 |

[Table 6]

| Prep | Str | | Prep | Str |
|---|---|---|---|---|
| 87 | 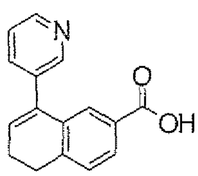 | | 96 | 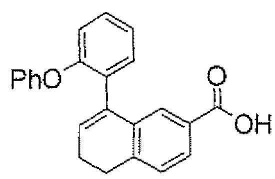 |
| 88 | 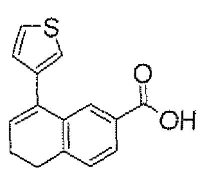 | | 97 | 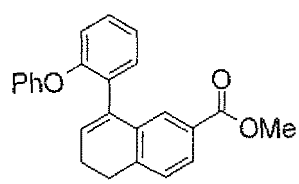 |

(continued)

| Prep | Str | | Prep | Str |
|---|---|---|---|---|
| 89 | 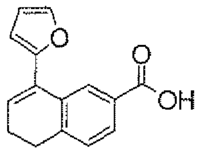 | | 98 | 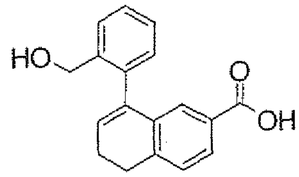 |
| 90 | 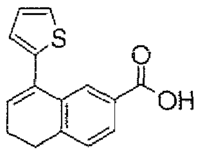 | | 99 | 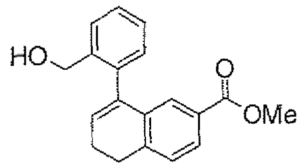 |
| 91 | 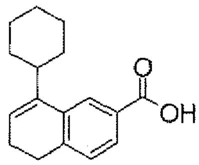 | | 100 | 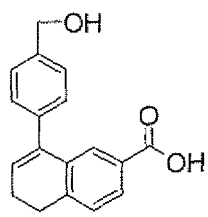 |
| 92 | 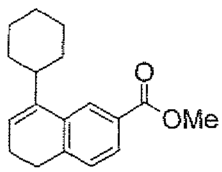 | | | |
| 93 | 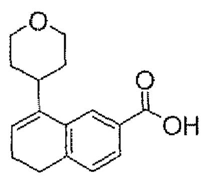 | | 101 | 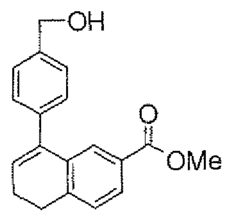 |
| 94 | 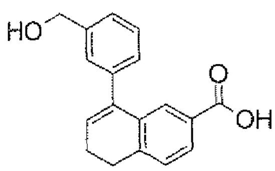 | | 102 | 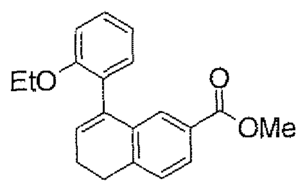 |
| 95 | 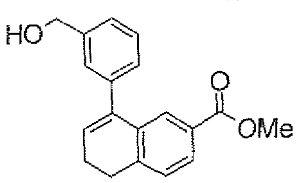 | | 103 | 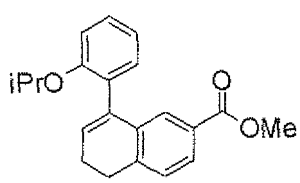 |

[Table 7]

| Prep | Str | Prep | Str |
|---|---|---|---|
| 104 | 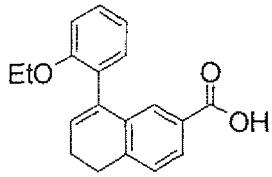 | 112 | 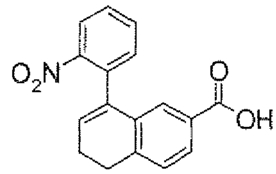 |
| 105 | 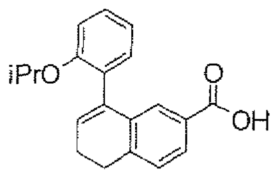 | 113 | 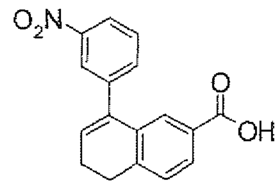 |
| 106 | 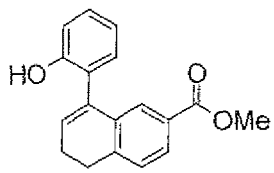 | 114 | 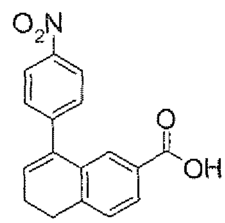 |
| 107 | 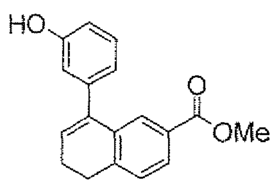 | 115 | 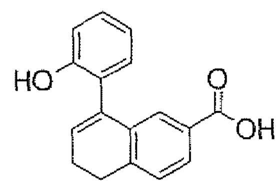 |
| 108 | 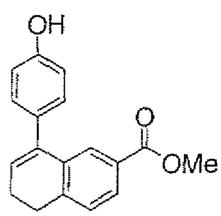 | 116 | 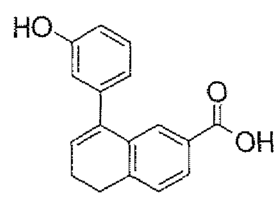 |
| 109 | 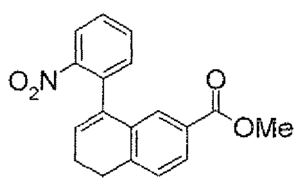 | 117 | 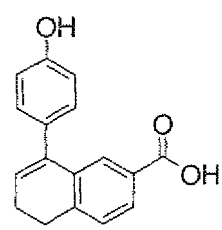 |
| 110 | 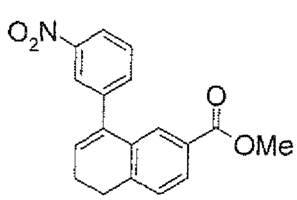 | 118 | 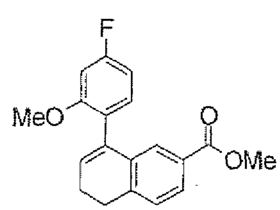 |

(continued)

| Prep | Str | | Prep | Str |
|---|---|---|---|---|
| 111 | 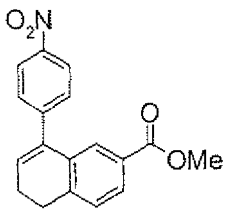 | | 119 | 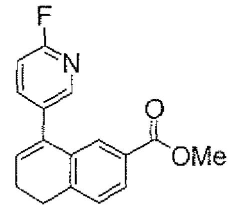 |

[Table 8]

| Prep | Str | | Prep | Str |
|---|---|---|---|---|
| 120 | 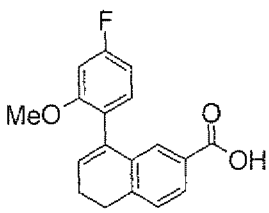 | | 128 | 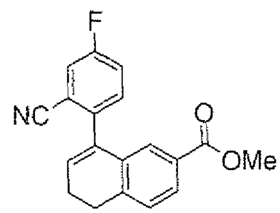 |
| 121 | 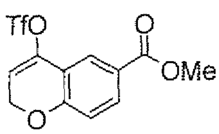 | | 129 | 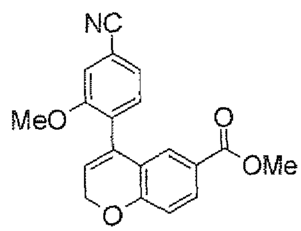 |
| 122 | 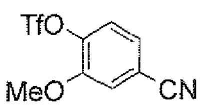 | | | |
| 123 | 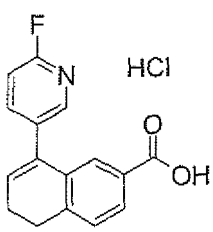 | | 130 | 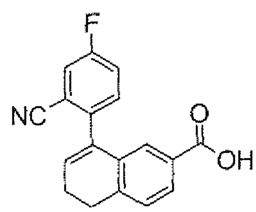 |
| 124 | 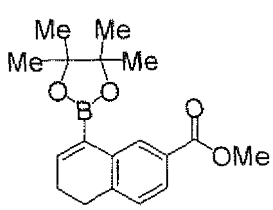 | | 131 | 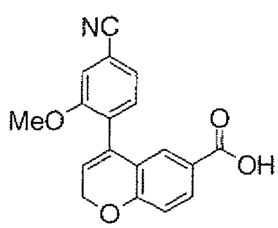 |

(continued)

| Prep | Str | | Prep | Str |
|---|---|---|---|---|
| 125 | 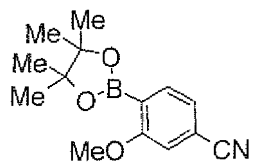 | | 132 | 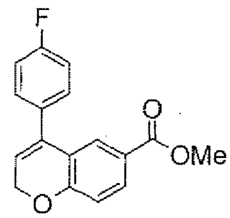 |
| 126 | 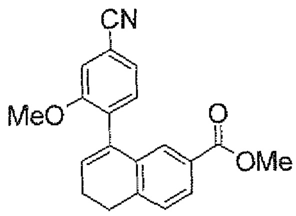 | | 133 | 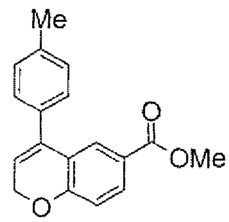 |
| 127 | 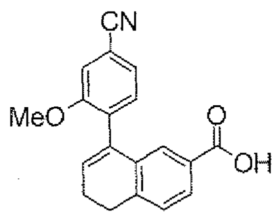 | | 134 | 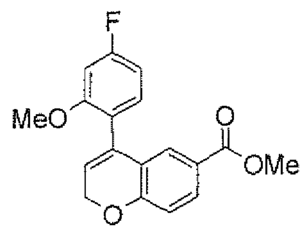 |

[Table 9]

| Prep | Str | | Prep | Str |
|---|---|---|---|---|
| 135 | 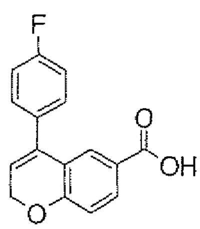 | | 142 | 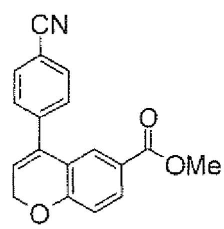 |
| 136 | 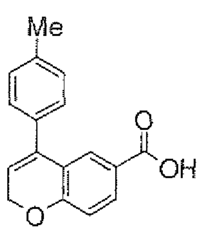 | | 143 | 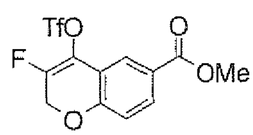 |
| | | | 144 | 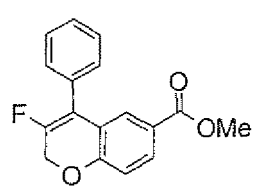 |

(continued)

| Prep | Str | | Prep | Str |
|---|---|---|---|---|
| 137 | 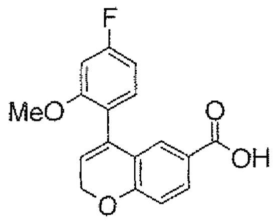 | | 145 | 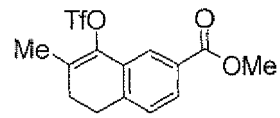 |
| 138 | 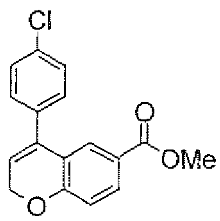 | | 146 | 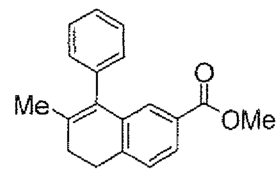 |
| 139 | 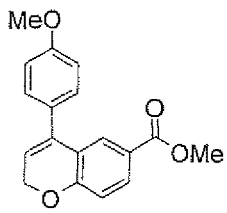 | | 147 | 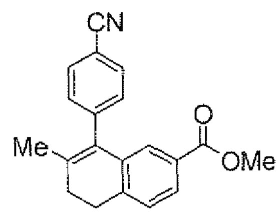 |
| 140 | 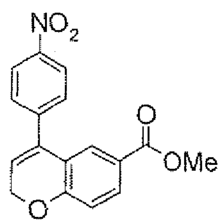 | | 148 | 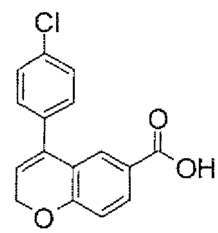 |
| 141 | 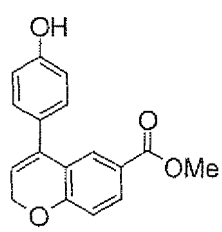 | | 149 | 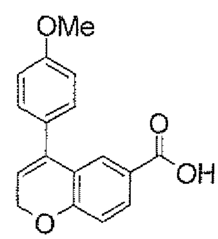 |

[Table 10]

| Prep | Str | | Prep | Str |
|---|---|---|---|---|
| 150 | 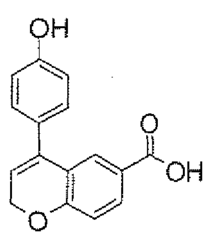 | | 157 | 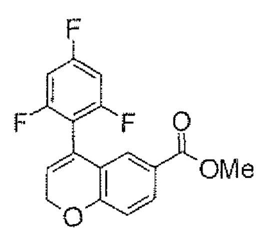 |

(continued)

| Prep | Str |
|---|---|
| 151 | 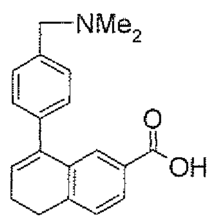 |
| 152 | 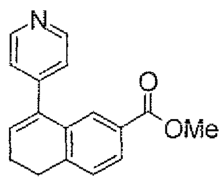 |
| 153 | 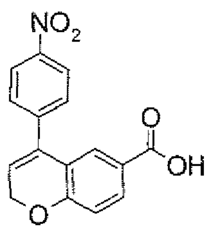 |
| 154 | 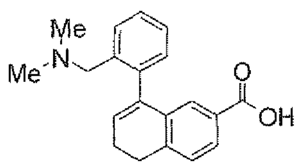 |
| 155 | 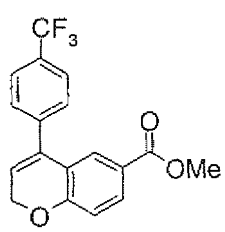 |
| 156 | 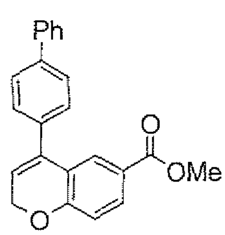 |
| 158 | 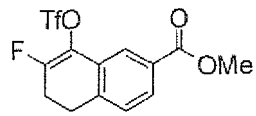 |
| 159 | 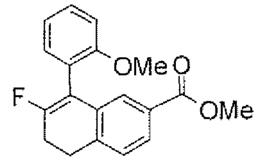 |
| 160 | 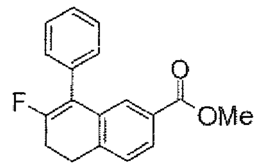 |
| 161 | 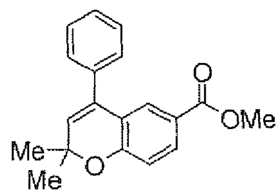 |
| 162 | 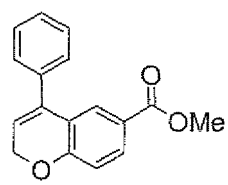 |
| 163 | 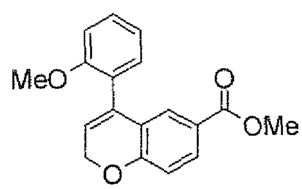 |
| 164 | 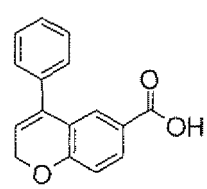 |
| 165 | 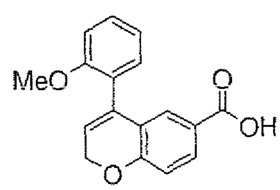 |

[Table 11]

| Prep | Str | Prep | Str |
|---|---|---|---|
| 166 | 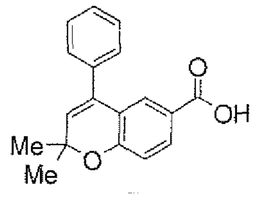 | 174 | 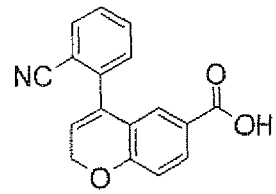 |
| 167 | 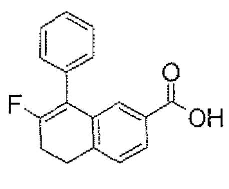 | 175 | 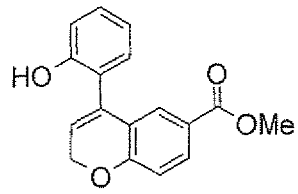 |
| 168 | 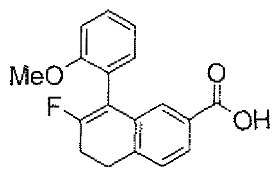 | 176 | 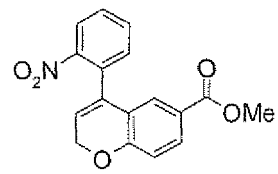 |
| 169 | 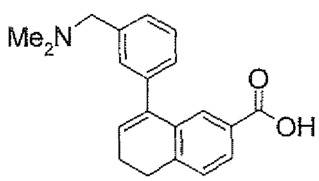 | 177 | 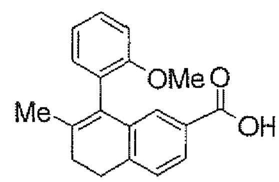 |
| 170 | 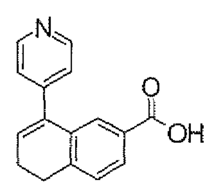 | 178 | 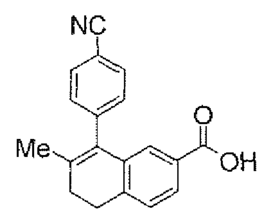 |
| 171 | 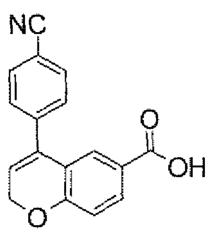 | 179 | 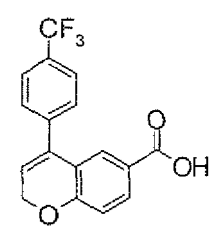 |
| 172 | 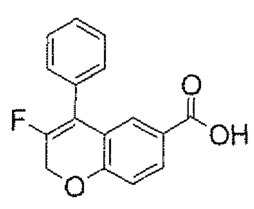 | 180 | 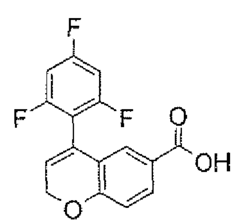 |

(continued)

| Prep | Str | | Prep | Str |
|---|---|---|---|---|
| 173 | 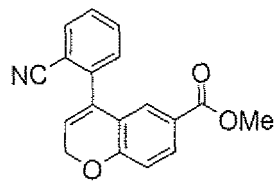 | | 181 | 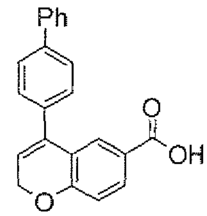 |

[Table 12]

| Prep | Str | | Prep | Str |
|---|---|---|---|---|
| 182 | 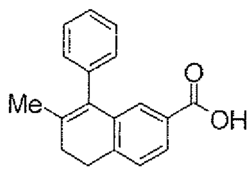 | | 191 | 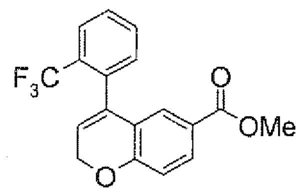 |
| 183 | 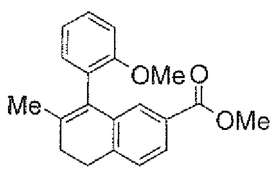 | | 192 | 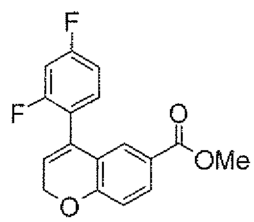 |
| 184 | 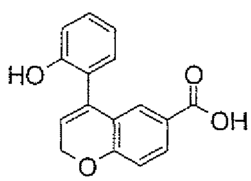 | | 193 | 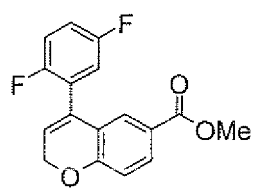 |
| 185 | 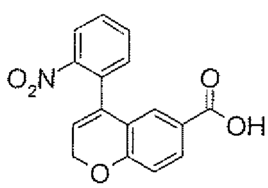 | | 194 | 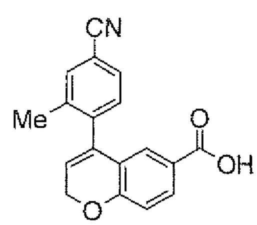 |
| 186 | 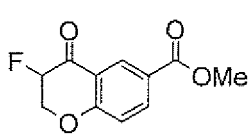 | | | |
| 187 | 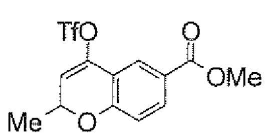 | | 195 | 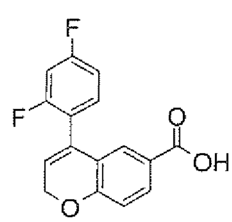 |

(continued)

| Prep | Str | | Prep | Str |
|---|---|---|---|---|
| 188 | 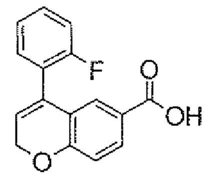 | | 196 | 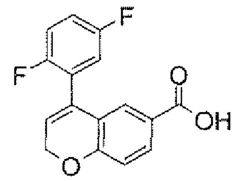 |
| 189 | 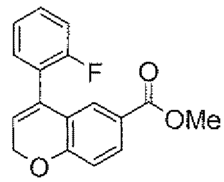 | | 197 | 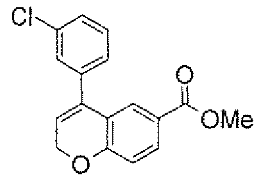 |
| 190 | 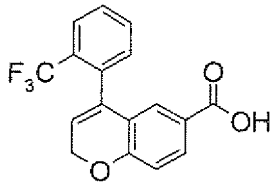 | | 198 | 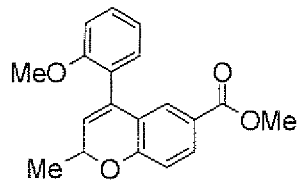 |

[Table 13]

| Prep | Str | | Prep | Str |
|---|---|---|---|---|
| 199 | 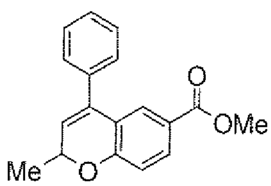 | | 207 | 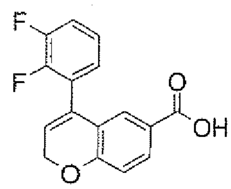 |
| 200 | 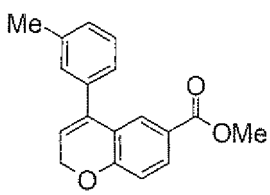 | | 208 | 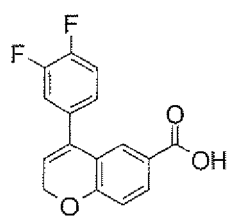 |
| 201 | 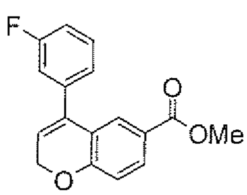 | | 209 | 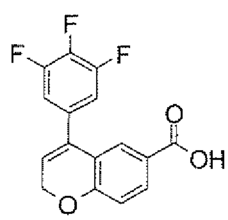 |

(continued)

| Prep | Str | | Prep | Str |
|---|---|---|---|---|
| 202 | 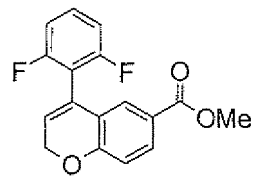 | | 210 | 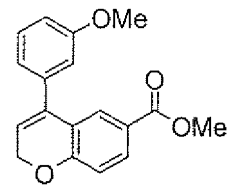 |
| 203 | 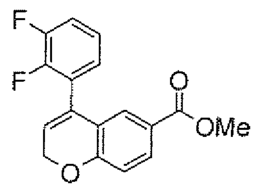 | | 211 | 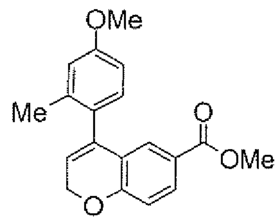 |
| 204 | 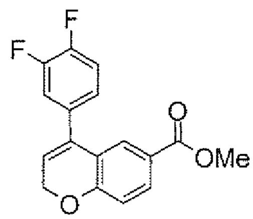 | | 212 | 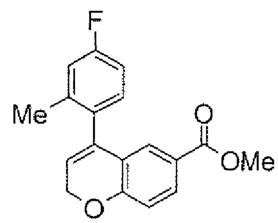 |
| 205 | 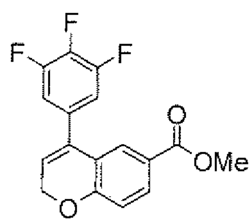 | | 213 | 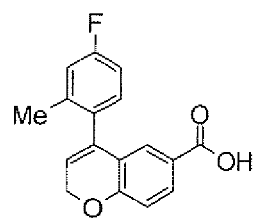 |
| 206 | 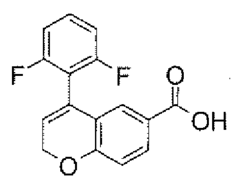 | | | |

[Table 14]

| Prep | Str | | Prep | Str |
|---|---|---|---|---|
| 214 | 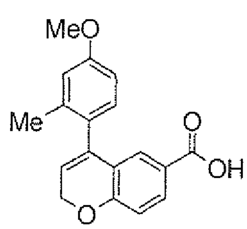 | | 222 | 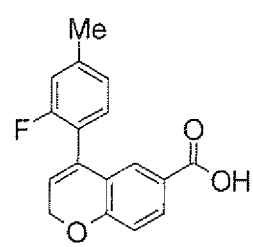 |

(continued)

| Prep | Str | | Prep | Str |
|---|---|---|---|---|
| 215 | 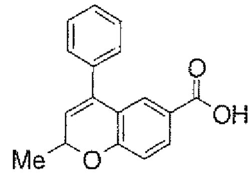 | | 223 | 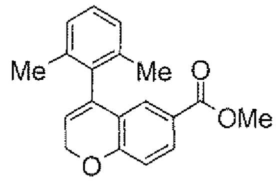 |
| 216 | 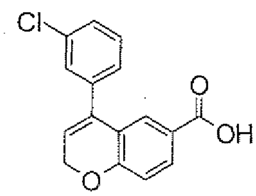 | | 224 | 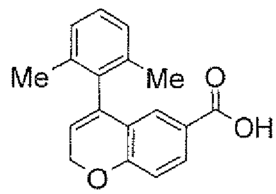 |
| 217 | 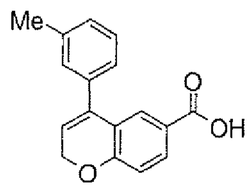 | | 225 | 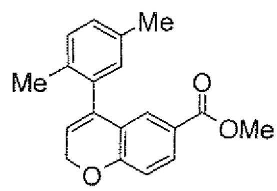 |
| 218 | 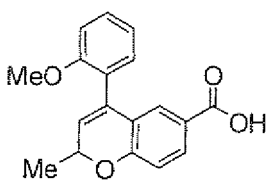 | | 226 | 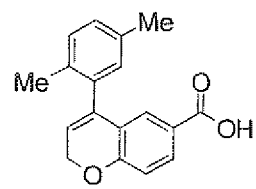 |
| 219 | 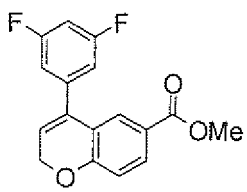 | | 227 | 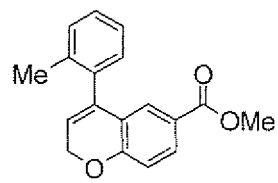 |
| 220 | 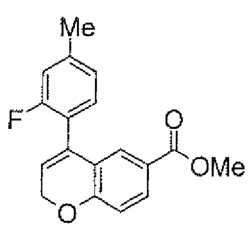 | | 228 | 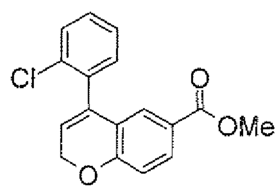 |
| 221 | 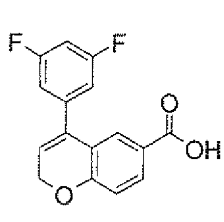 | | 229 | 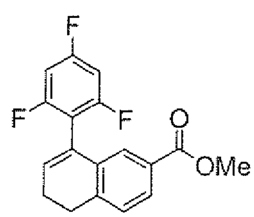 |

[Table 15]

| Prep | Str | | Prep | Str |
|---|---|---|---|---|
| 230 | 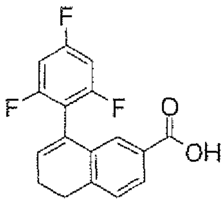 | | 239 | 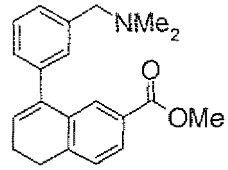 |
| 231 | 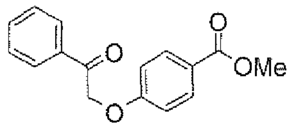 | | 240 | 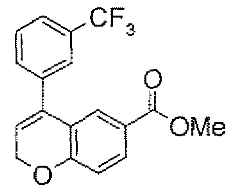 |
| 232 | 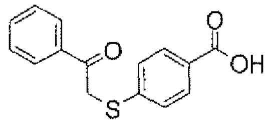 | | 241 | 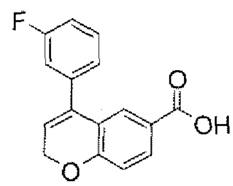 |
| 233 | 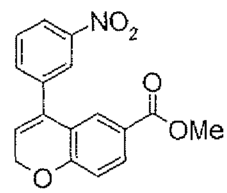 | | 242 | 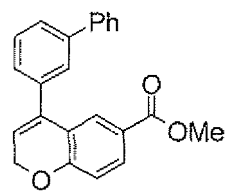 |
| 234 | 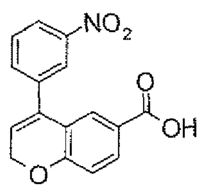 | | 243 | 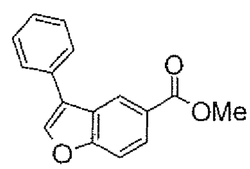 |
| 235 | 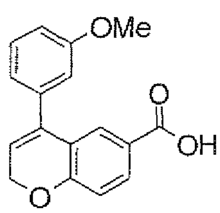 | | 244 | 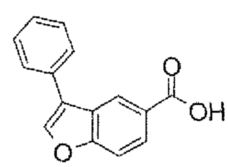 |
| 236 | 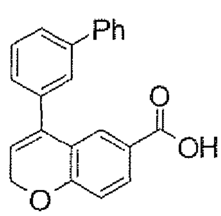 | | 245 | 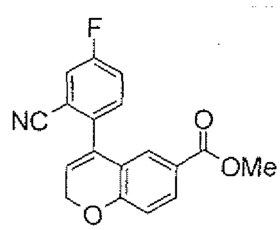 |

(continued)

| Prep | Str | | Prep | Str |
|---|---|---|---|---|
| 237 | 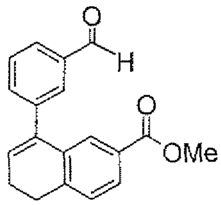 | | 246 | 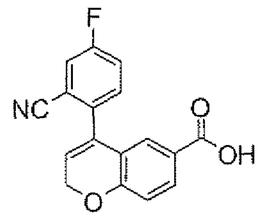 |
| 238 | 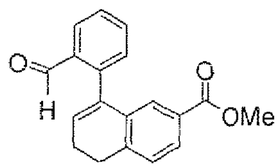 | | | |

[Table 16]

| Prep | Str | | Prep | Str |
|---|---|---|---|---|
| 247 | 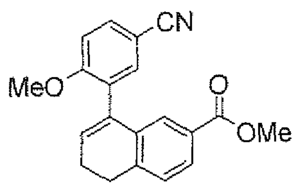 | | 255 | 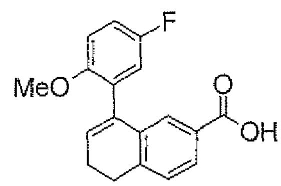 |
| 248 | 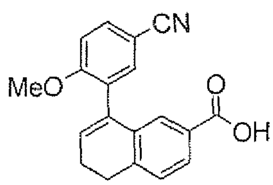 | | 256 | 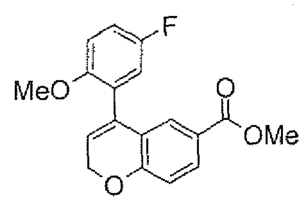 |
| 249 | 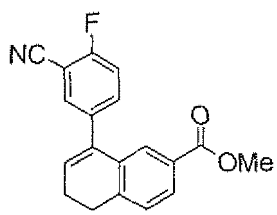 | | 257 | 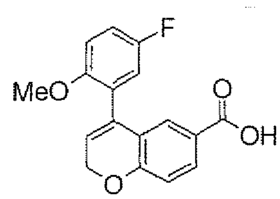 |
| 250 | 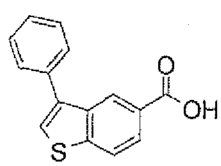 | | 258 | 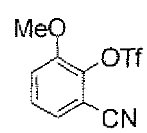 |

(continued)

| Prep | Str | | Prep | Str |
|---|---|---|---|---|
| 251 | 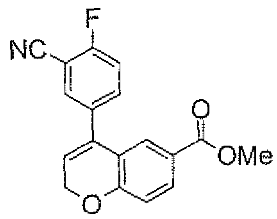 | | 259 | 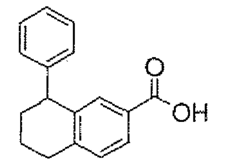 |
| | | | 260 | 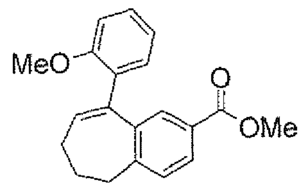 |
| 252 | 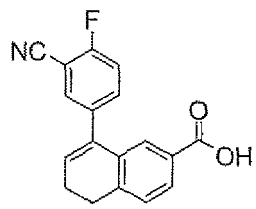 | | 261 | 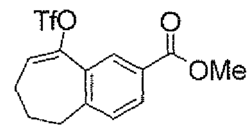 |
| 253 | 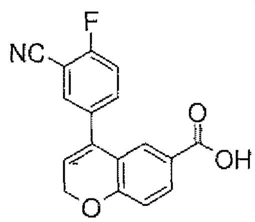 | | 262 | 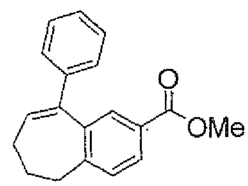 |
| 254 | 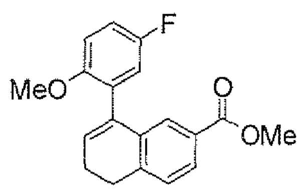 | | 263 | 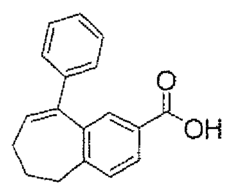 |

[Table 17]

| Prep | Str | | Prep | Str |
|---|---|---|---|---|
| 264 | 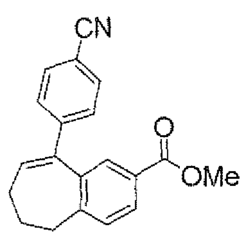 | | 271 | 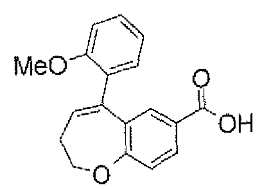 |

(continued)

| Prep | Str | | Prep | Str |
|---|---|---|---|---|
| 265 | 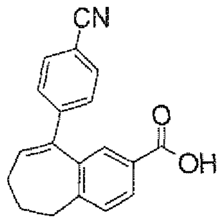 | | 272 | 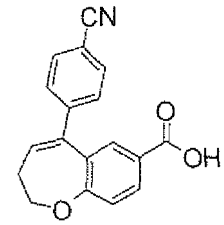 |
| 266 | 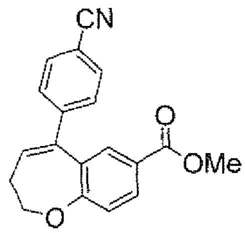 | | 273 | 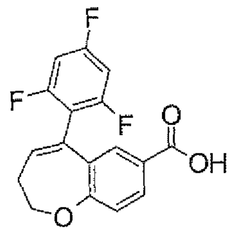 |
| 267 | 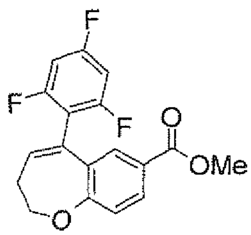 | | 274 | 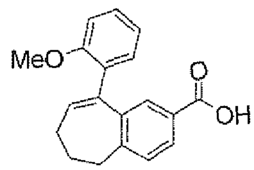 |
| 268 | 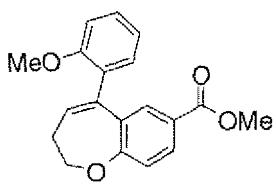 | | 275 | 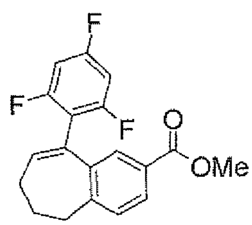 |
| 269 | 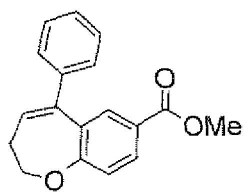 | | 276 | 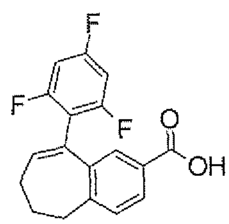 |

(continued)

| Prep | Str | | Prep | Str |
|---|---|---|---|---|
| 270 | 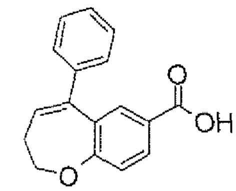 | | 277 | 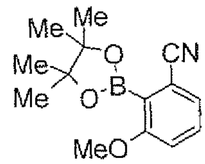 |
| | | | 278 | 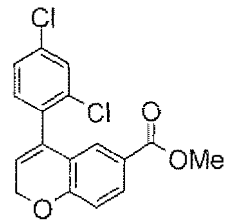 |

[Table 18]

| Prep | Str | | Prep | Str |
|---|---|---|---|---|
| 279 | 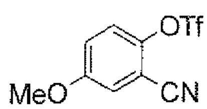 | | 287 | 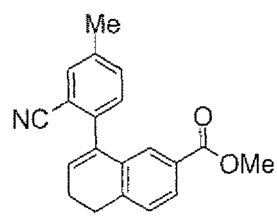 |
| 280 | 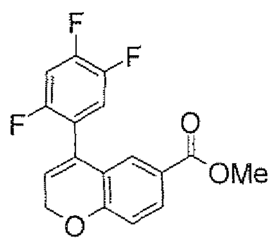 | | 288 | 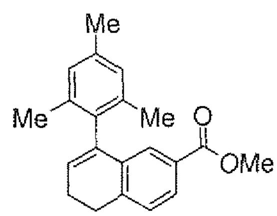 |
| 281 | 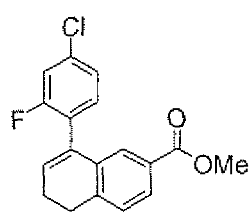 | | 289 | 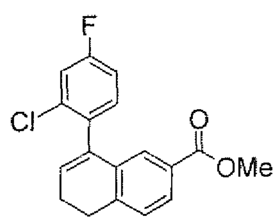 |
| 282 | 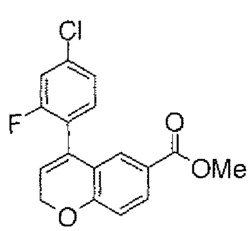 | | 290 | 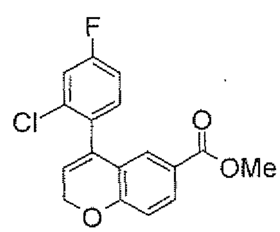 |

(continued)

| Prep | Str | | Prep | Str |
|---|---|---|---|---|
| 283 | 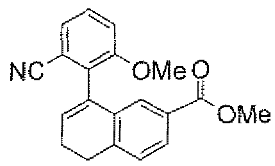 | | 291 | 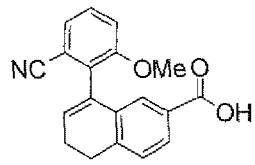 |
| 284 | 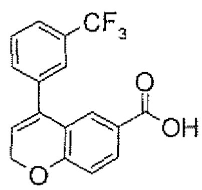 | | 292 | 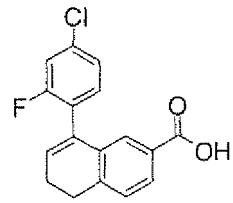 |
| 285 | 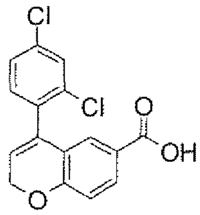 | | 293 | 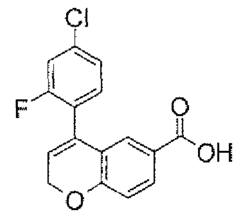 |
| 286 | 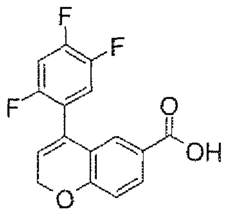 | | 294 | 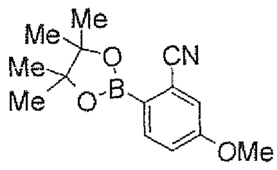 |

[Table 19]

| Prep | Str | | Prep | Str |
|---|---|---|---|---|
| 295 | 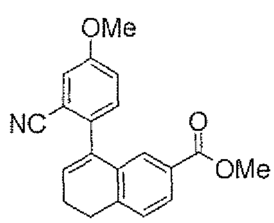 | | 302 | 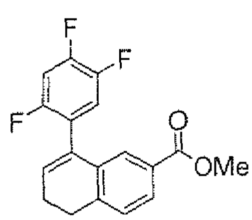 |
| 296 | 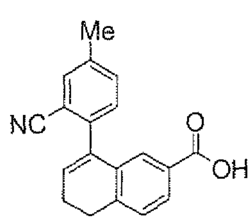 | | 303 | 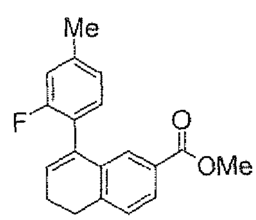 |

(continued)

| Prep | Str | | Prep | Str |
|---|---|---|---|---|
| 297 | 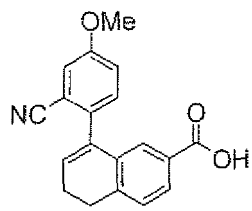 | | 304 | 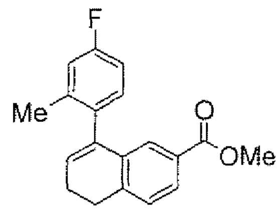 |
| 298 | 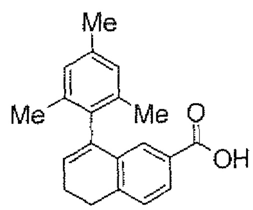 | | 305 | 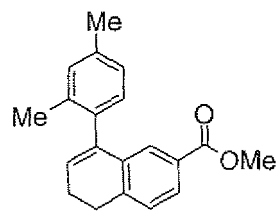 |
| 299 | 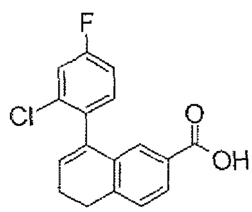 | | 306 | 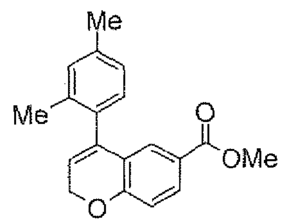 |
| 300 | 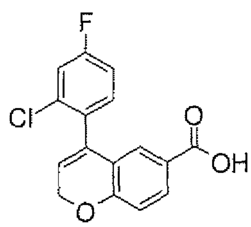 | | 307 | 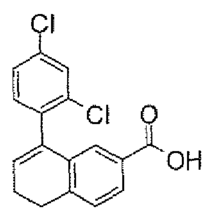 |
| 301 | 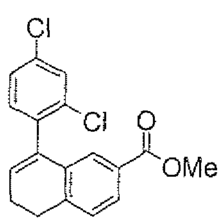 | | 308 | 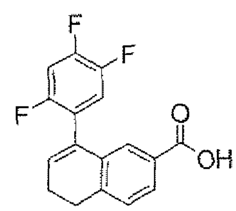 |
| | | | 309 | 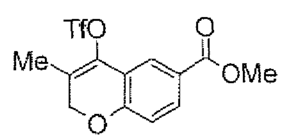 |

[Table 20]

| Prep | Str | Prep | Str |
|---|---|---|---|
| 310 | 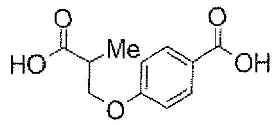 | 318 | 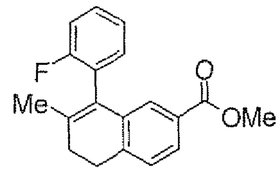 |
| 311 | 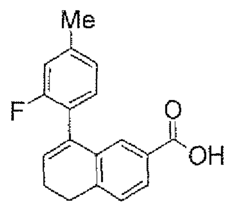 | 319 | 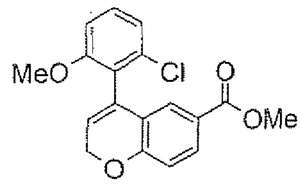 |
| 312 | 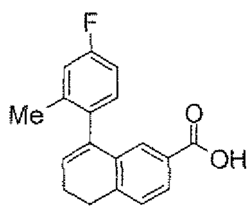 | 320 | 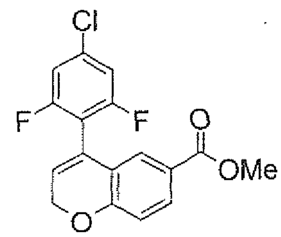 |
| 313 | 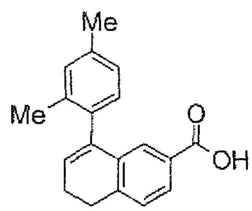 | 321 | 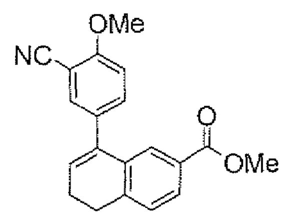 |
| 314 | 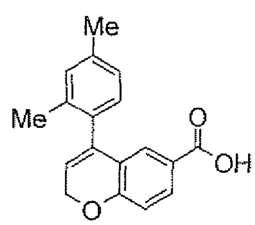 | 322 | 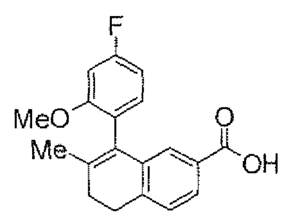 |
| 315 | 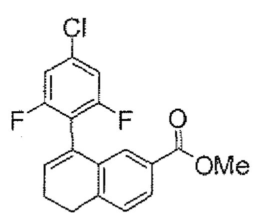 | 323 | 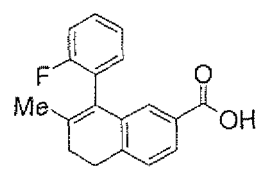 |
| 316 | 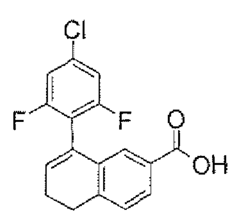 | 324 | 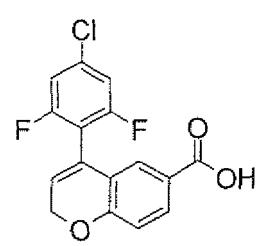 |

(continued)

| Prep | Str | | Prep | Str |
|---|---|---|---|---|
| 317 | 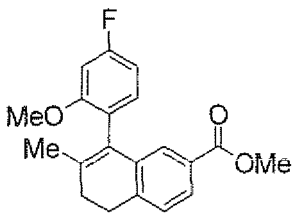 | | 325 | 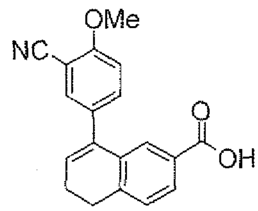 |

[Table 21]

| Prep | Str | | Prep | Str |
|---|---|---|---|---|
| 326 | 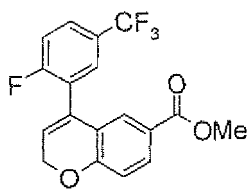 | | 334 | 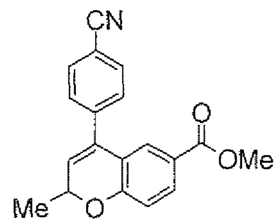 |
| 327 | 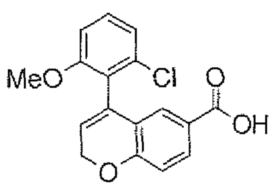 | | 335 | 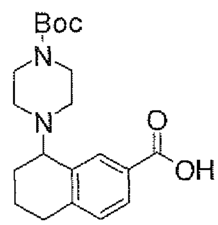 |
| 328 | 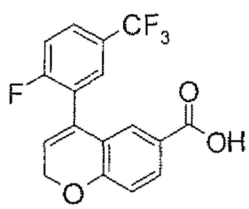 | | 336 | 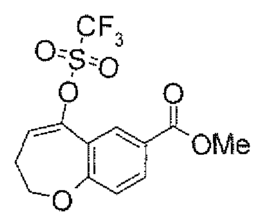 |
| 329 | 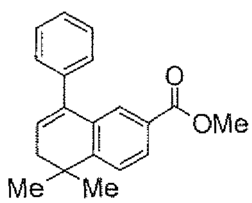 | | 337 | 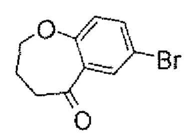 |
| 330 | 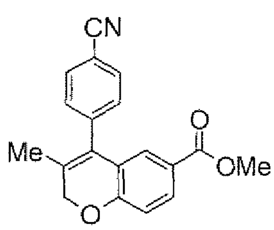 | | 338 | 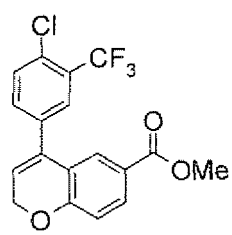 |

(continued)

| Prep | Str | | Prep | Str |
|---|---|---|---|---|
| 331 | 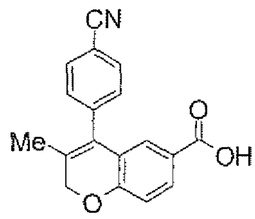 | | 339 | 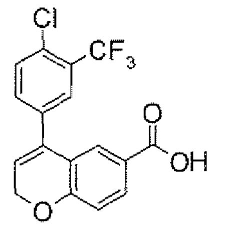 |
| 332 | 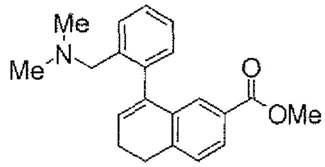 | | 340 | 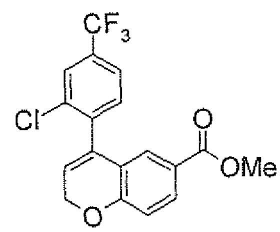 |
| 333 | 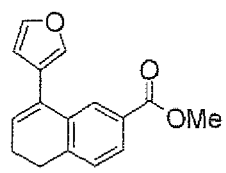 | | | |

[Table 22]

| Prep | Str | | Prep | Str |
|---|---|---|---|---|
| 341 | 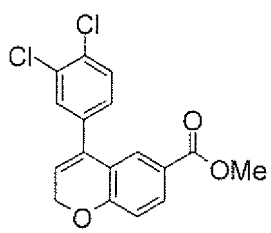 | | 348 | 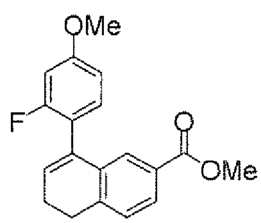 |
| 342 | 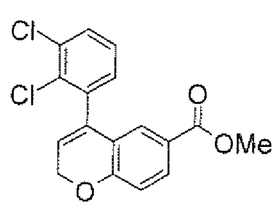 | | 349 | 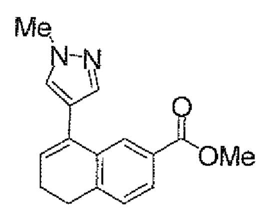 |
| 343 | 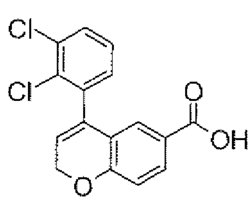 | | 350 | 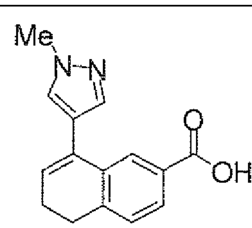 |

(continued)

| Prep | Str | | Prep | Str |
|---|---|---|---|---|
| 344 | 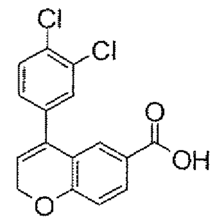 | | 351 | 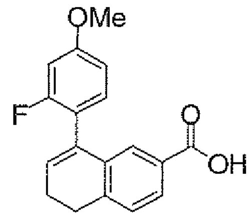 |
| 345 | 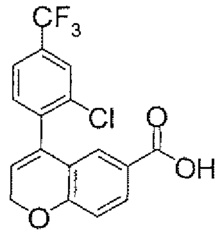 | | 352 | 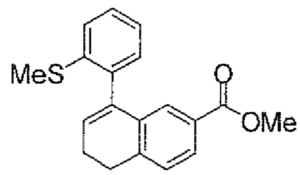 |
| 346 | 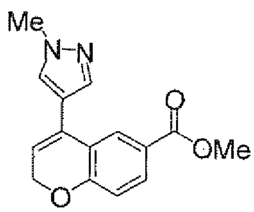 | | 353 | 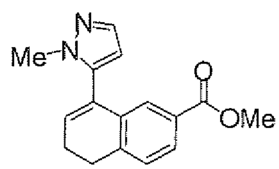 |
| 347 | 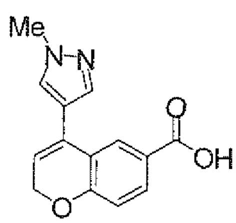 | | 354 | 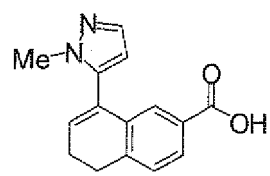 |
| | | | 355 | 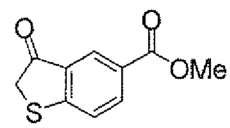 |

[Table 23]

| Prep | Str | | Prep | Str |
|---|---|---|---|---|
| 356 | 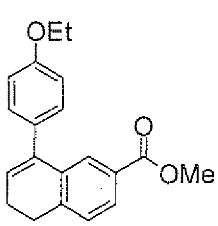 | | 364 | 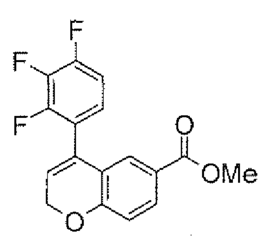 |

(continued)

| Prep | Str | | Prep | Str |
|---|---|---|---|---|
| 357 | 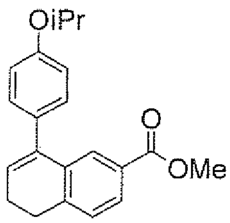 | | 365 | 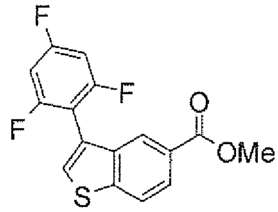 |
| 358 | 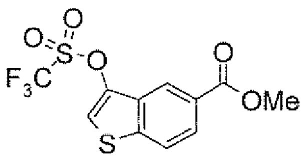 | | 366 | 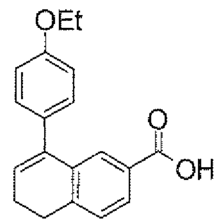 |
| 359 | 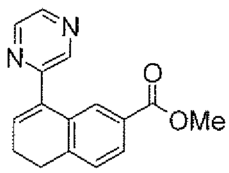 | | 367 | 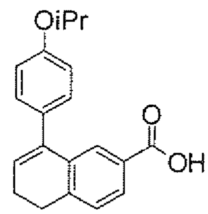 |
| 360 | 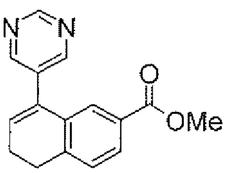 | | 368 | 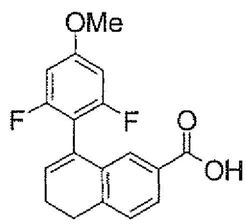 |
| 361 | 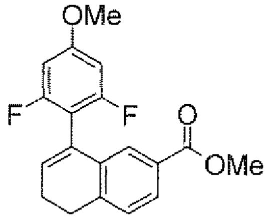 | | 369 | 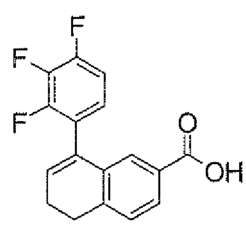 |
| 362 | 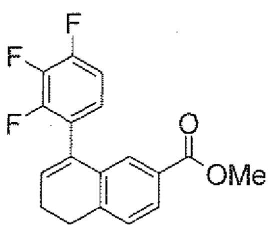 | | | |

(continued)

| Prep | Str | | Prep | Str |
|---|---|---|---|---|
| 363 | 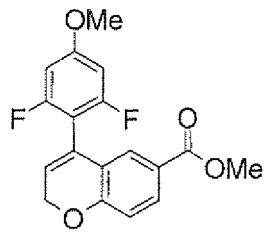 | | 370 | 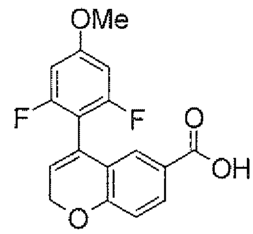 |

[Table 24]

| Prep | Str | | Prep. | Str |
|---|---|---|---|---|
| 371 | 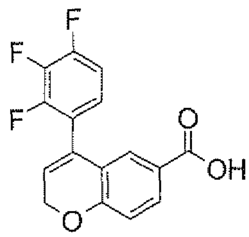 | | 379 | 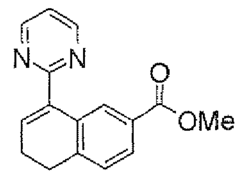 |
| 372 | 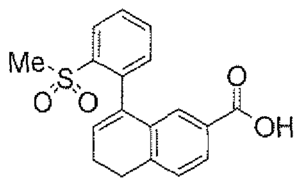 | | 380 | 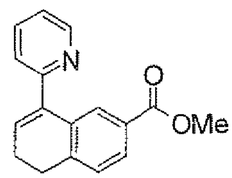 |
| 373 | 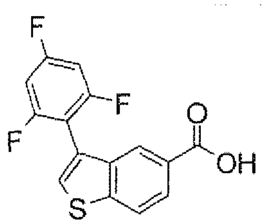 | | 381 | 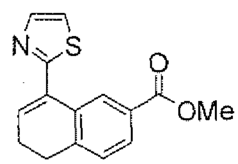 |
| 374 | 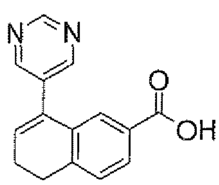 | | 382 | 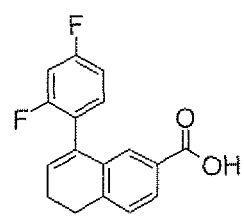 |
| 375 | 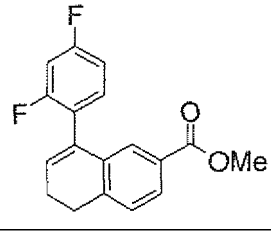 | | 383 | 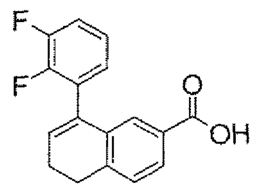 |

(continued)

| Prep | Str | | Prep. | Str |
|---|---|---|---|---|
| 376 | 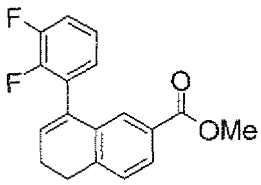 | | 384 | 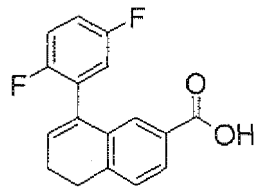 |
| 377 | 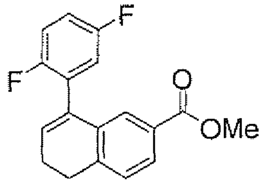 | | 385 | 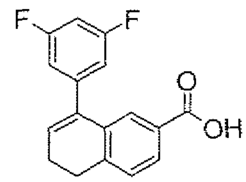 |
| 378 | 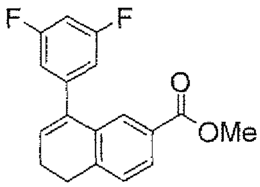 | | 386 | 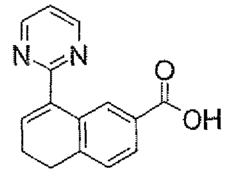 |

[Table 25]

| Prep | Str | | Prep | Str |
|---|---|---|---|---|
| 387 | 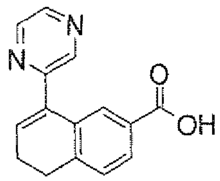 | | 395 | 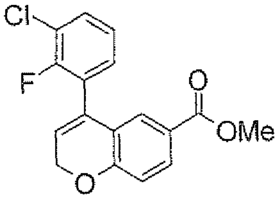 |
| 388 | 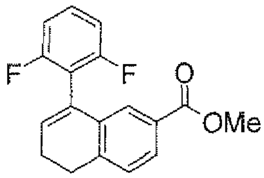 | | 396 | 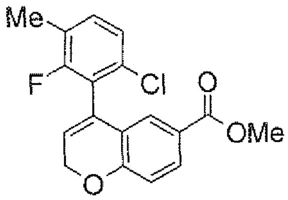 |
| 389 | 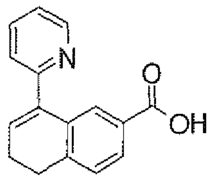 | | 397 | 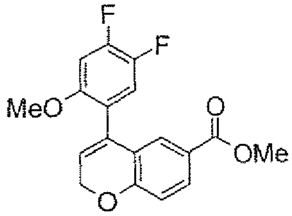 |

(continued)

| Prep | Str | | Prep | Str |
|---|---|---|---|---|
| 390 | 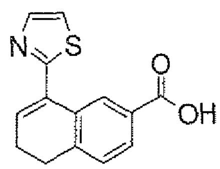 | | 398 | 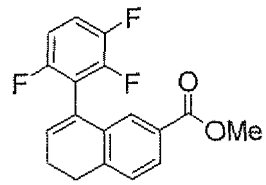 |
| 391 | 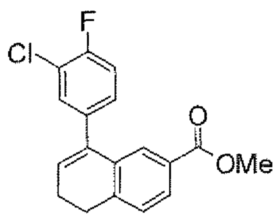 | | 399 | 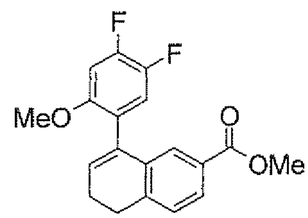 |
| 392 | 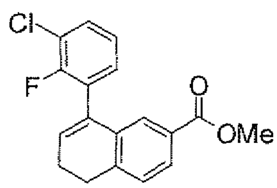 | | 400 | 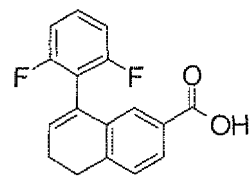 |
| 393 | 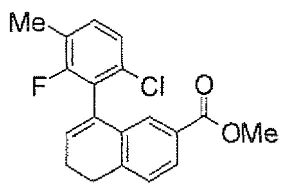 | | 401 | 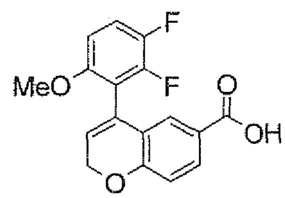 |
| 394 | 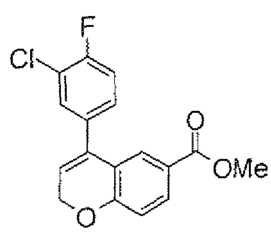 | | 402 | 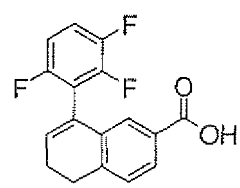 |

[Table 26]

| Prep | Str | | Prep | Str |
|---|---|---|---|---|
| 403 | 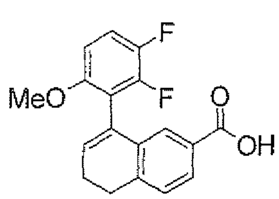 | | 411 | 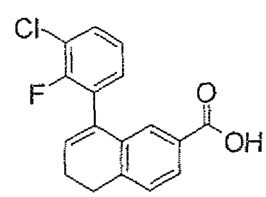 |

(continued)

| Prep | Str | Prep | Str |
|---|---|---|---|
| 404 | 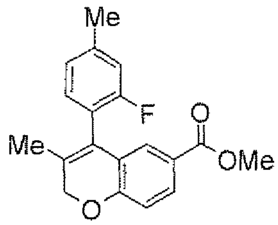 | 412 | 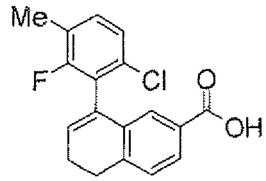 |
| 405 | 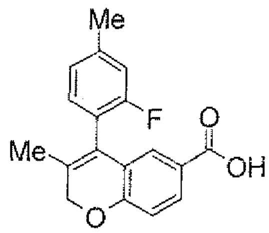 | 413 | 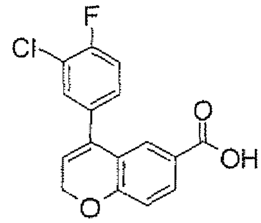 |
| 406 | 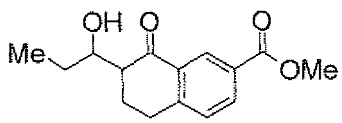 | 414 | 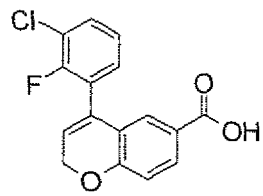 |
| 407 | 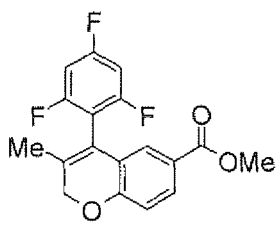 | 415 | 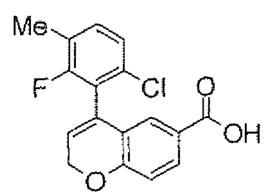 |
| 408 | 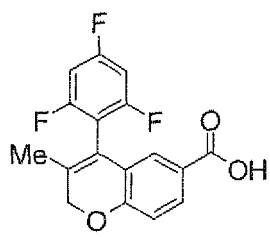 | 416 | 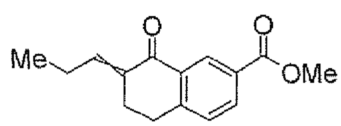 |
| 409 | 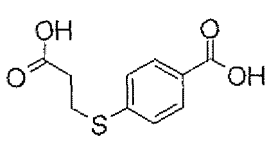 | 417 | 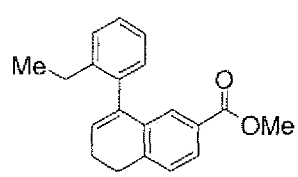 |
| 410 | 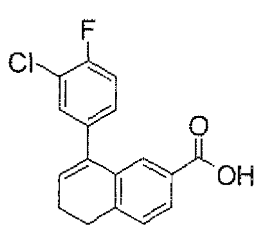 | 418 | 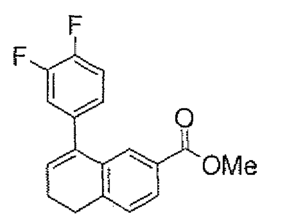 |

[Table 27]

| Prep | Str | | Prep | Str |
|---|---|---|---|---|
| 419 | 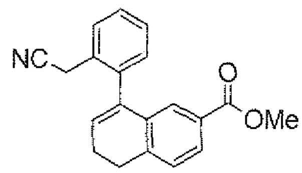 | | 427 | 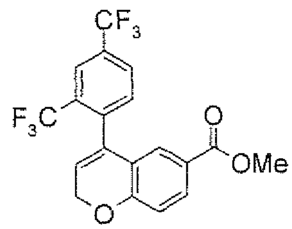 |
| 420 | 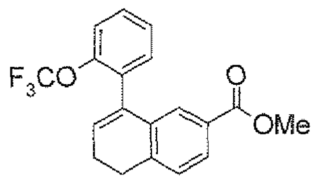 | | 428 | 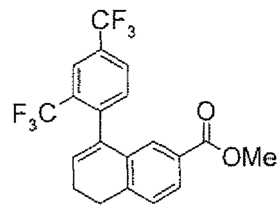 |
| 421 | 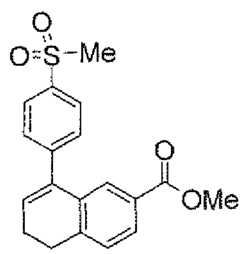 | | 429 | 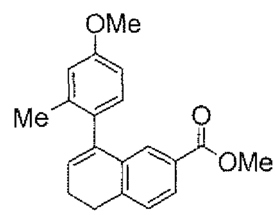 |
| 422 | 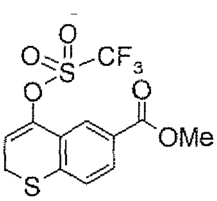 | | 430 | 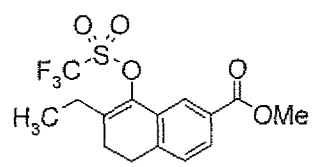 |
| 423 | 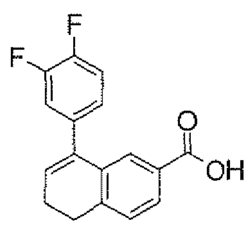 | | 431 | 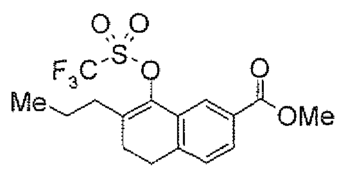 |
| 424 | 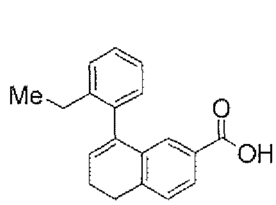 | | 432 | 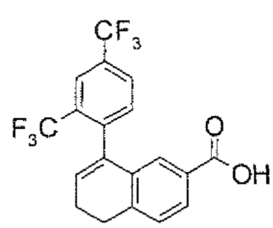 |

(continued)

| Prep | Str | | Prep | Str |
|---|---|---|---|---|
| 425 | 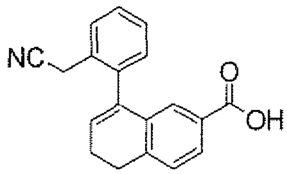 | | 433 | 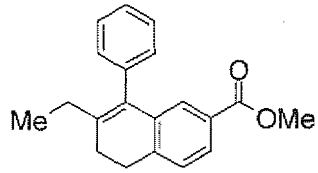 |
| 426 | 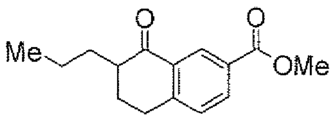 | | 434 | 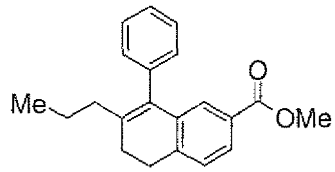 |

[Table 28]

| Prep | Str | | Prep | Str |
|---|---|---|---|---|
| 435 | 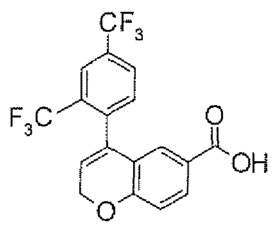 | | 443 | 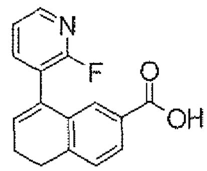 |
| 436 | 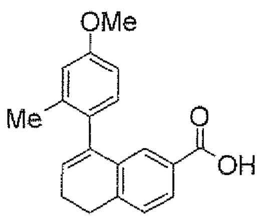 | | 444 | 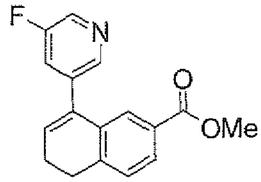 |
| 437 | 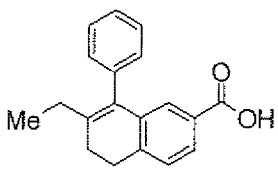 | | 445 | 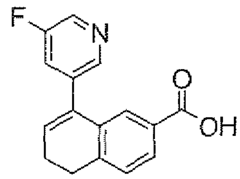 |
| 438 | 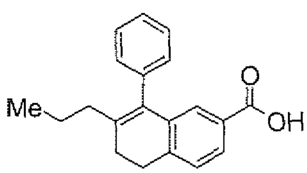 | | 446 | 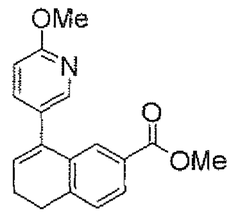 |

(continued)

| Prep | Str | | Prep | Str |
|---|---|---|---|---|
| 439 | 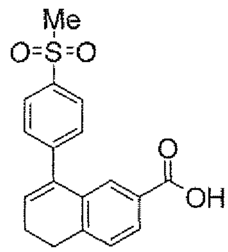 | | 447 | 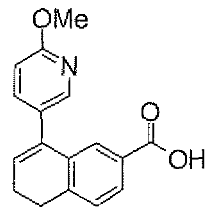 |
| 440 | 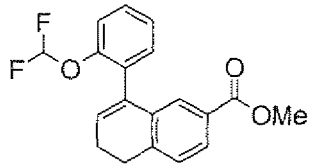 | | 448 | 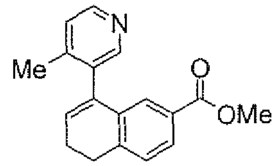 |
| 441 | 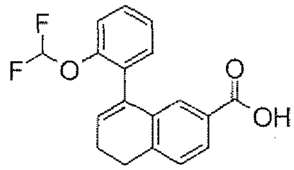 | | 449 | 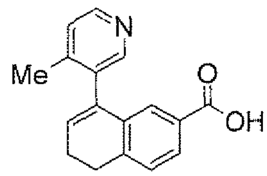 |
| 442 | 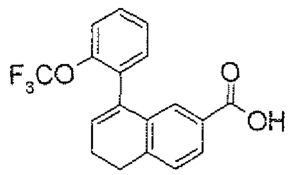 | | 450 | 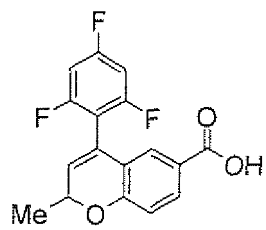 |

[Table 29]

| Prep | Str | | Prep | Str |
|---|---|---|---|---|
| 451 | 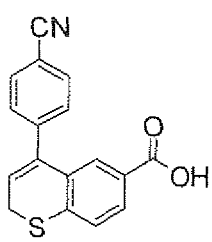 | | 458 | 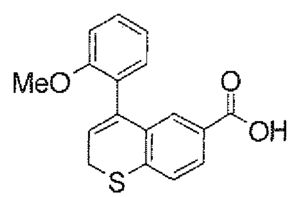 |
| 452 | 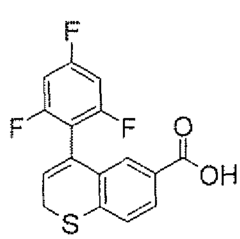 | | 459 | 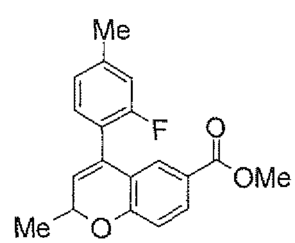 |

(continued)

| Prep | Str | | Prep | Str |
|---|---|---|---|---|
| 453 | 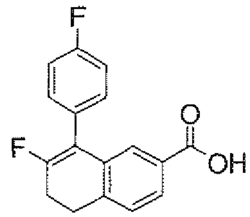 | | 460 | 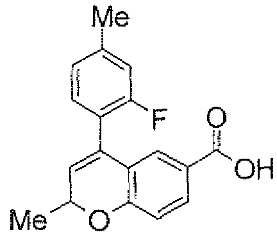 |
| 454 | 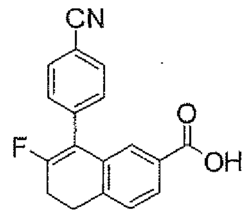 | | 461 | 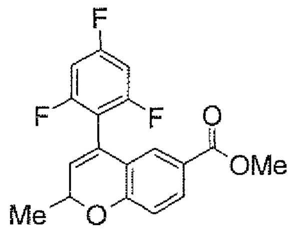 |
| 455 | 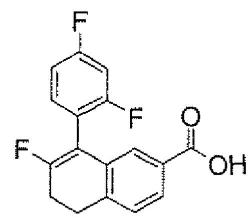 | | 462 | 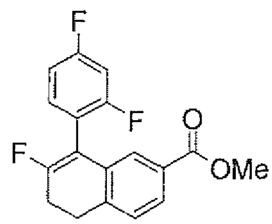 |
| 456 | 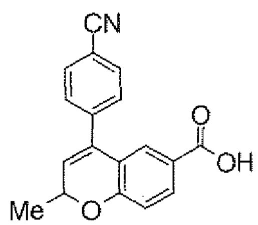 | | 463 | 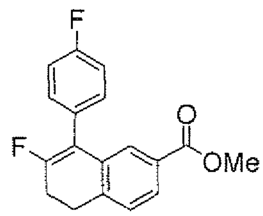 |
| 457 | 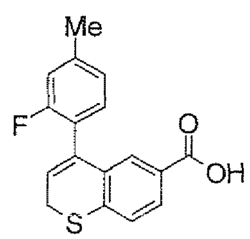 | | 464 | 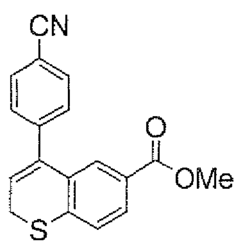 |

[Table 30]

| Prep | Str | | Prep | Str |
|---|---|---|---|---|
| 465 | 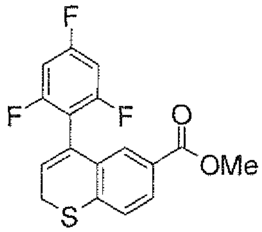 | | 472 | 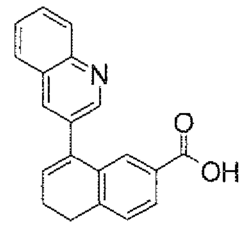 |
| 466 | 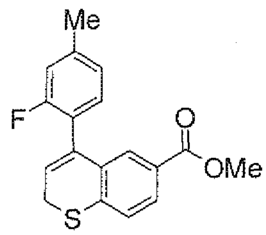 | | 473 | 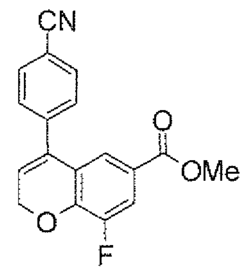 |
| 457 | 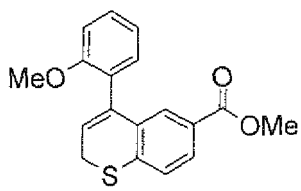 | | 474 | 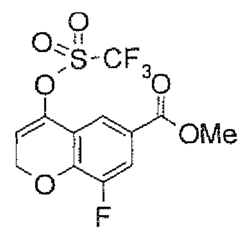 |
| 468 | 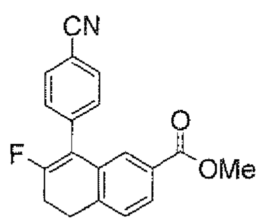 | | 475 | 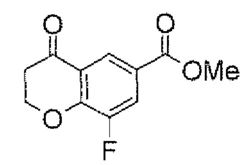 |
| 469 | 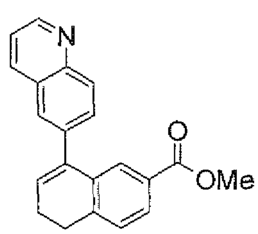 | | 476 | 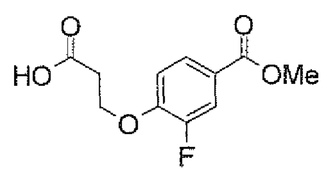 |
| 470 | 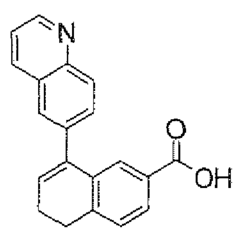 | | 477 | 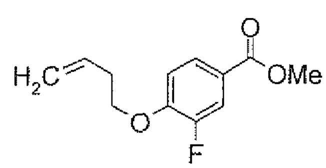 |

(continued)

| Prep | Str | | Prep | Str |
|---|---|---|---|---|
| 471 | 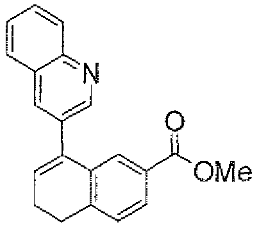 | | 478 | 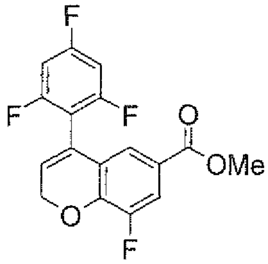 |

[Table 31]

| Prep | Str |
|---|---|
| 479 | 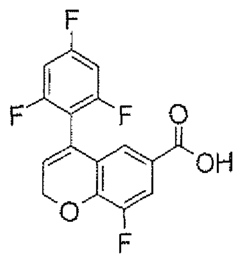 |
| 480 | 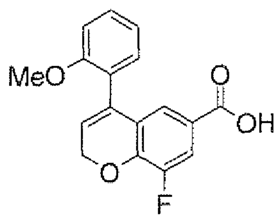 |
| 481 | 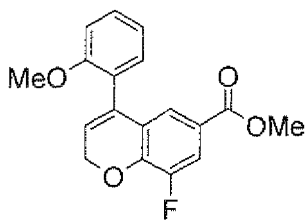 |
| 482 | 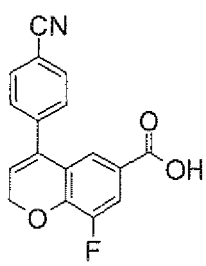 |

(continued)

| Prep | Str |
|---|---|
| 483 | 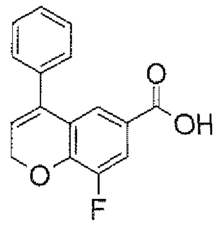 |
| 484 | 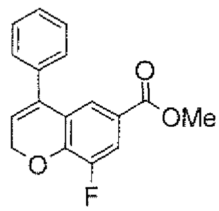 |

[Table 32]

| Prep | Str | | Prep | Str |
|---|---|---|---|---|
| 485 | 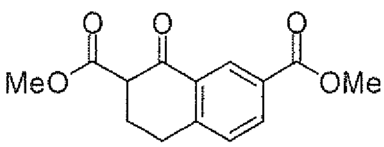 | | 493 | 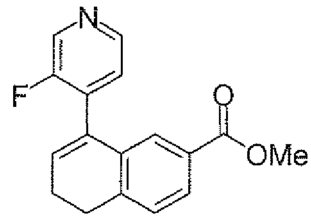 |
| 486 | 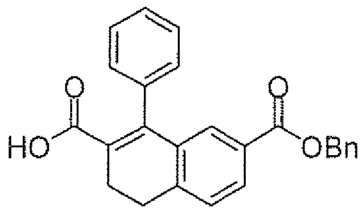 | | 494 | 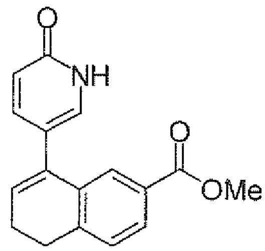 |
| 487 | 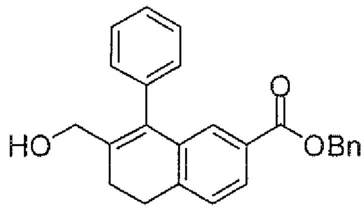 | | 495 | 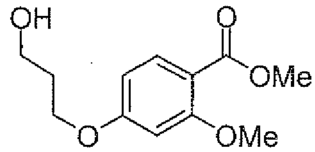 |
| 488 | 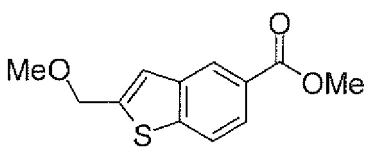 | | 496 | 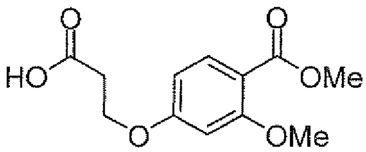 |

(continued)

| Prep | Str | | Prep | Str |
|---|---|---|---|---|
| 489 | 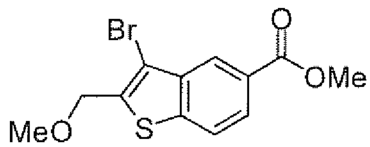 | | 497 | 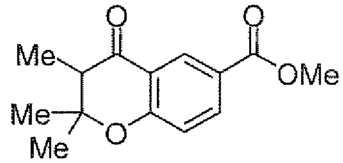 |
| 490 | 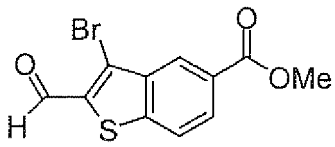 | | 498 | 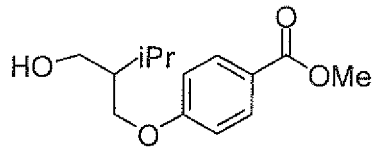 |
| 491 | 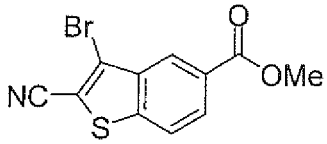 | | 499 | 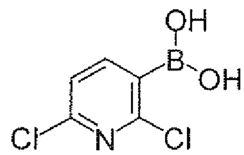 |
| 492 | 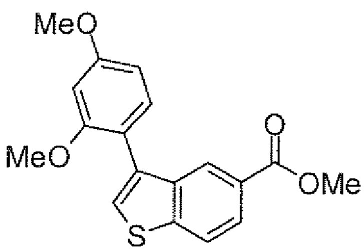 | | 500 | 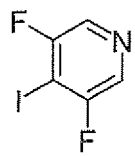 |
| | | | 501 | 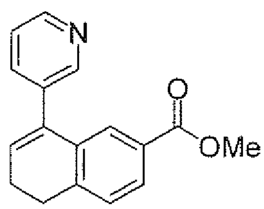 |

[Table 33]

| Prep | Str | | Prep | Str |
|---|---|---|---|---|
| 502 | 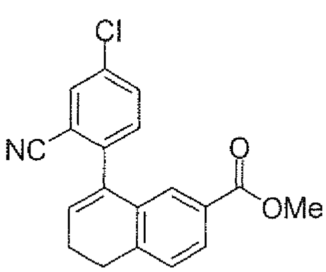 | | 510 | 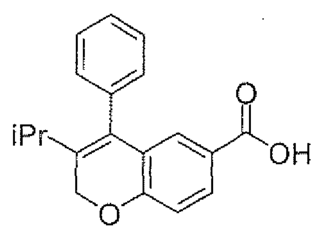 |
| 503 | 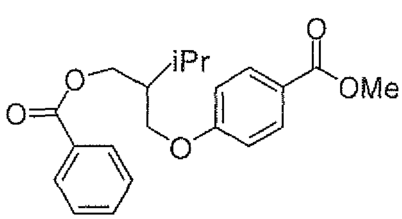 | | 511 | 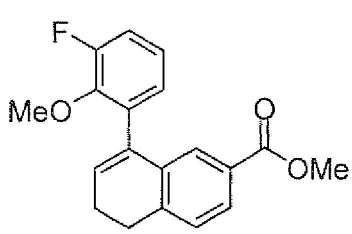 |

(continued)

| Prep | Str | Prep | Str |
|---|---|---|---|
| 504 | 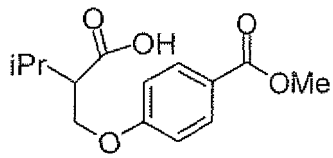 | 512 | 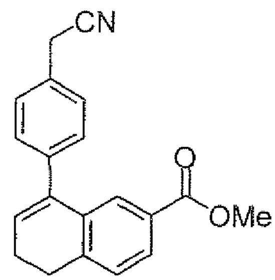 |
| 505 | 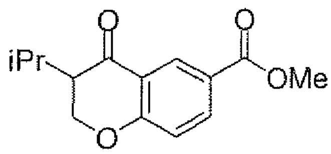 | | |
| 506 | 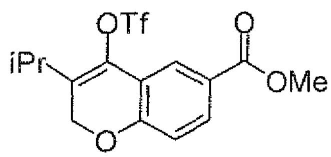 | 513 | 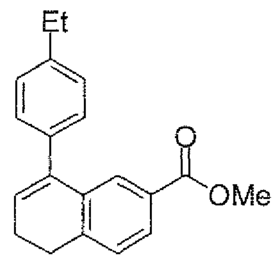 |
| 507 | 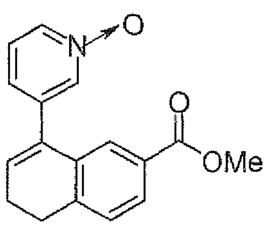 | 514 | 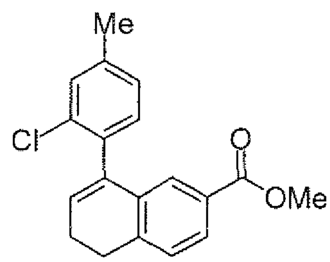 |
| 508 | 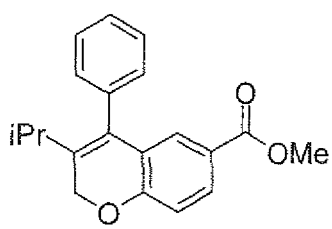 | | |
| 509 | 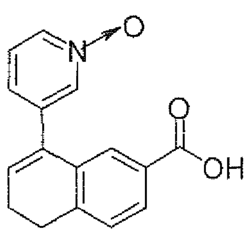 | 515 | 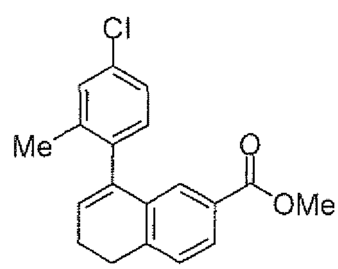 |

[Table 34]

| Prep | Str | Prep | Str |
|---|---|---|---|
| 516 | 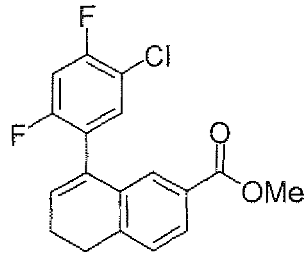 | 522 | 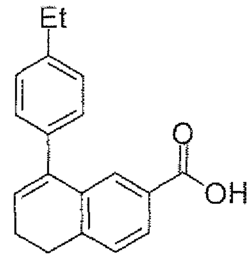 |
| 517 | 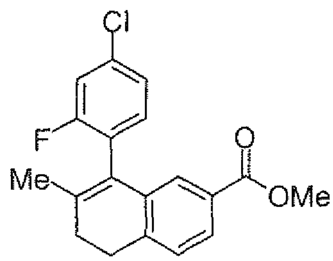 | 523 | 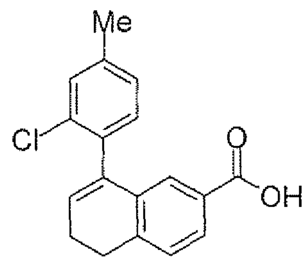 |
| 518 | 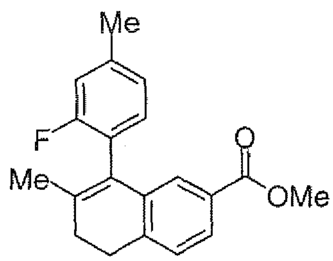 | 524 | 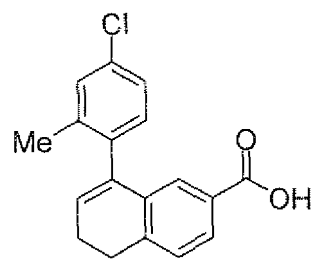 |
| 519 | 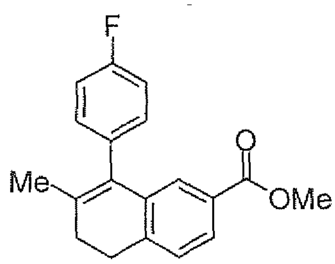 | 525 | 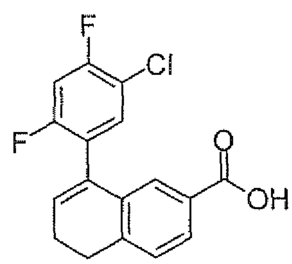 |
| 520 | 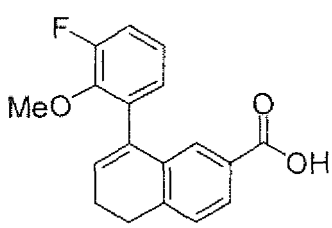 | 526 | 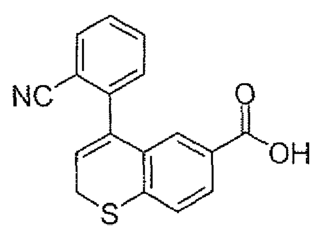 |

(continued)

| Prep | Str | Prep | Str |
|---|---|---|---|
| 521 | 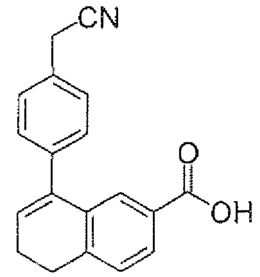 | 527 | 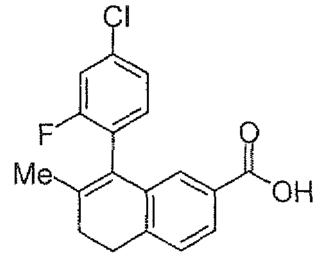 |

[Table 35]

| Prep | Str | Prep | Str |
|---|---|---|---|
| 528 | 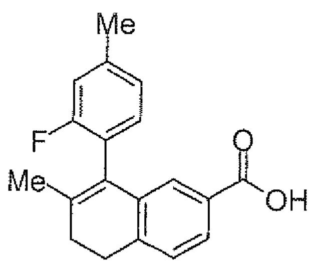 | 534 | 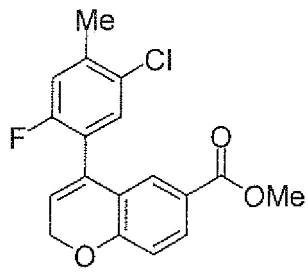 |
| 529 | 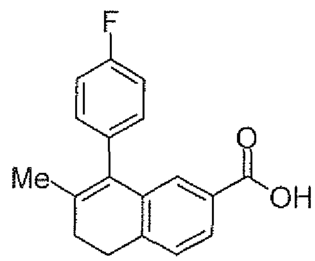 | 535 | 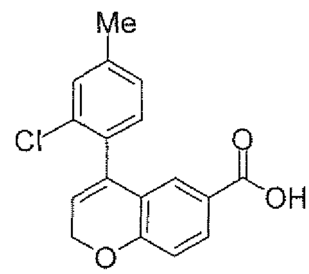 |
| 530 | 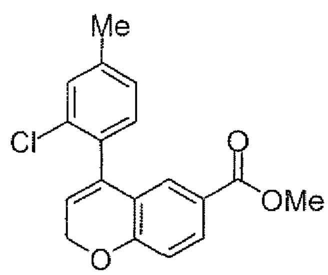 | 536 | 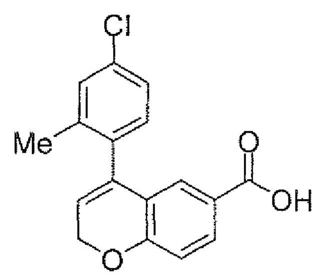 |
| 531 | 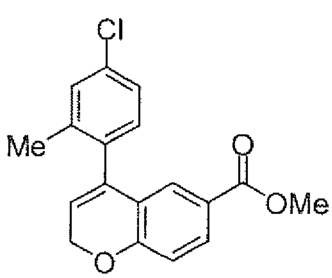 | 537 | 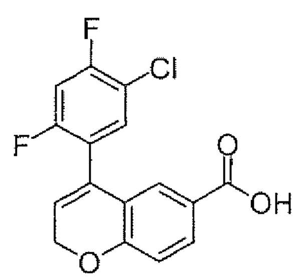 |

(continued)

| Prep | Str | | Prep | Str |
|---|---|---|---|---|
| 532 | 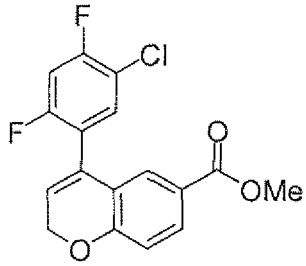 | | 538 | 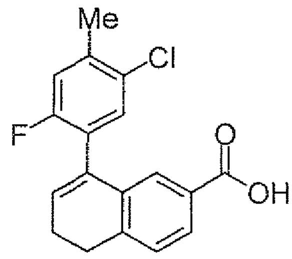 |
| 533 | 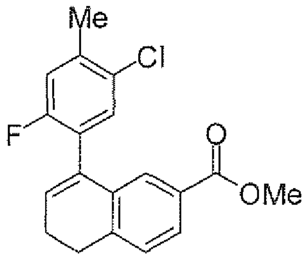 | | 539 | 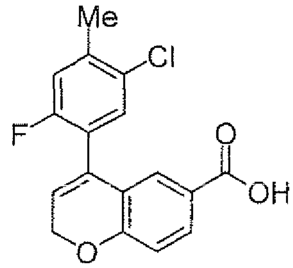 |

[Table 36]

| Prep | Str | | Prep | Str |
|---|---|---|---|---|
| 540 | 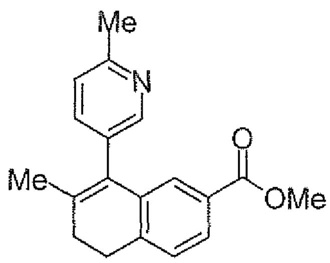 | | 546 | 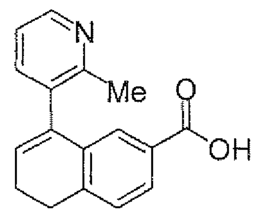 |
| 541 | 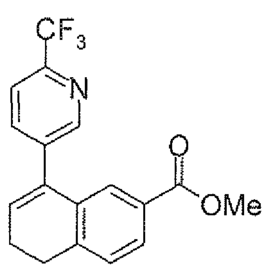 | | 547 | 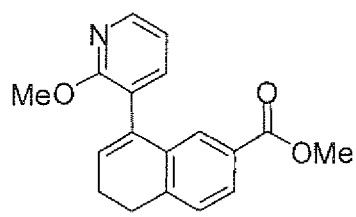 |
| | | | 548 | 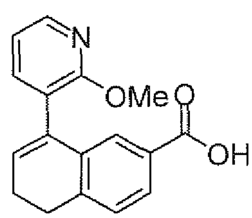 |

(continued)

| Prep | Str | | Prep | Str |
|---|---|---|---|---|
| 542 | 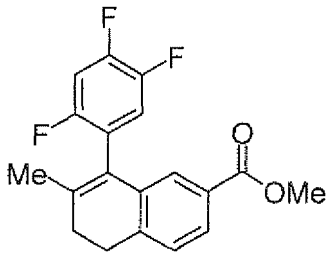 | | 549 | 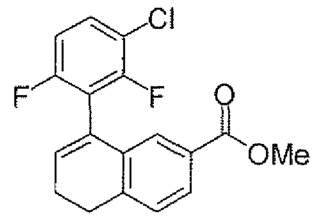 |
| 543 | 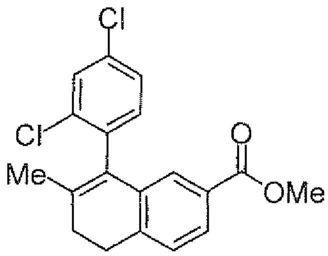 | | 550 | 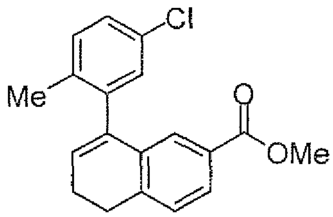 |
| 544 | 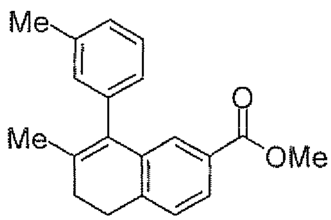 | | 551 | 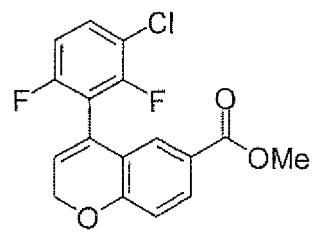 |
| 545 | 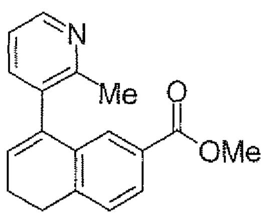 | | 552 | 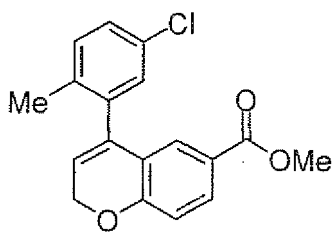 |

[Table 37]

| Prep | Str | | Prep | Str |
|---|---|---|---|---|
| 553 | 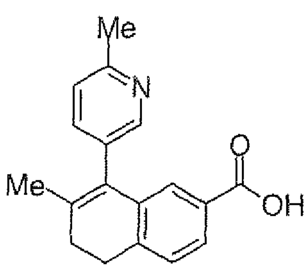 | | 560 | 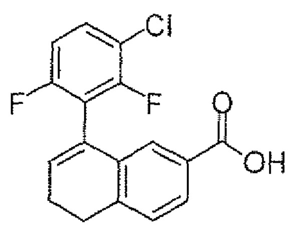 |

(continued)

| Prep | Str | Prep | Str |
|---|---|---|---|
| 554 | 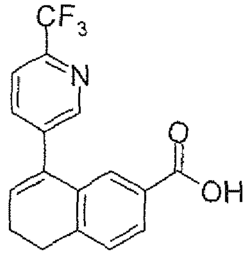 | 561 | 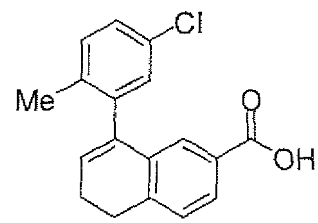 |
| 555 | 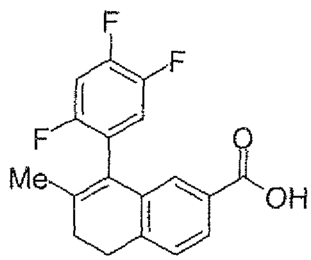 | 562 | 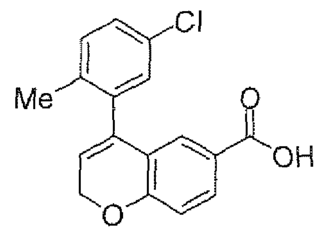 |
| 556 | 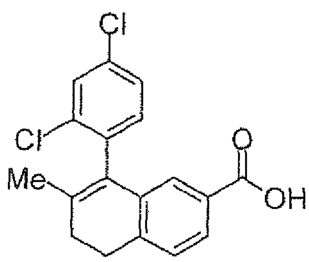 | 563 | 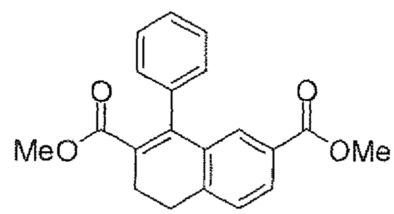 |
| 557 | 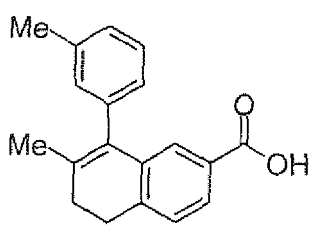 | 564 | 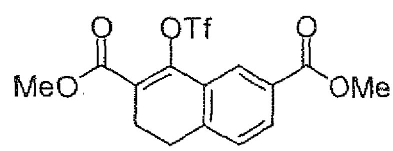 |
| 558 | 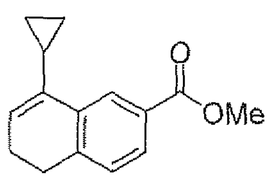 | 565 | 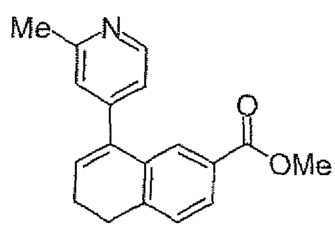 |
| 559 | 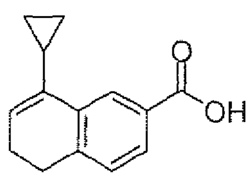 | 566 | 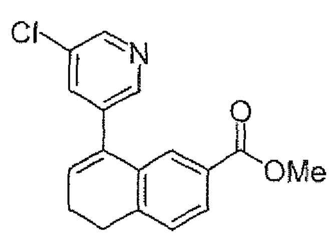 |

[Table 38]

| Prep | Str | | Prep | Str |
|---|---|---|---|---|
| 567 | 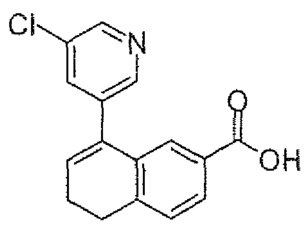 | | 574 | 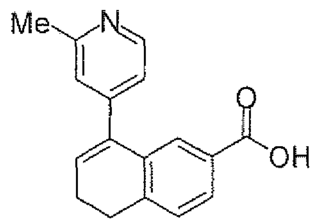 |
| S68 | 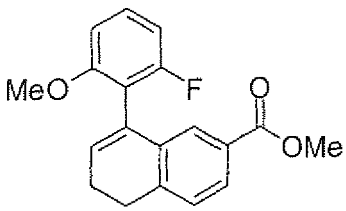 | | 575 | 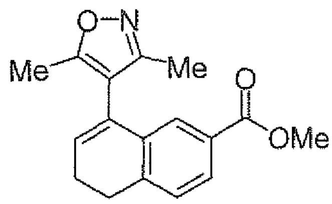 |
| 569 | 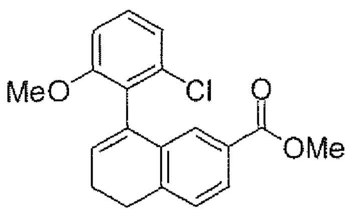 | | 576 | 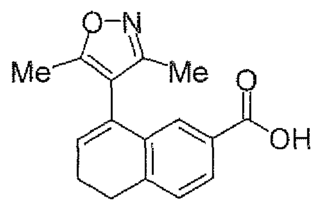 |
| 570 | 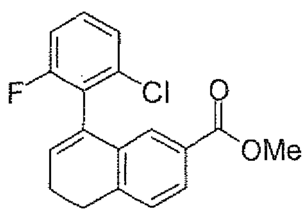 | | 577 | 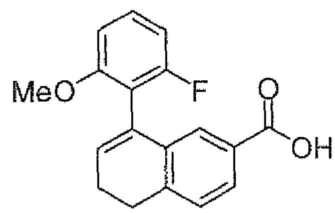 |
| 571 | 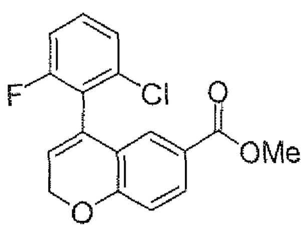 | | 578 | 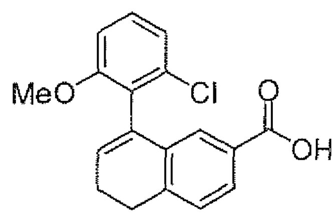 |
| 572 | 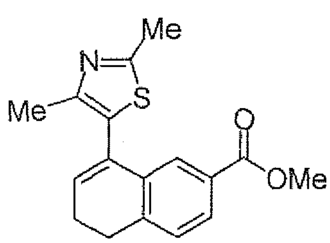 | | 579 | 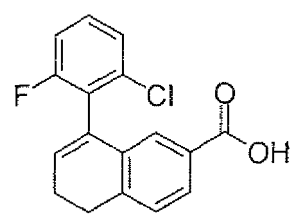 |

(continued)

| Prep | Str | | Prep | Str |
|---|---|---|---|---|
| 573 | 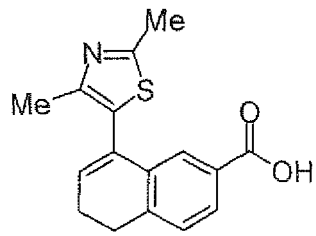 | | 580 | 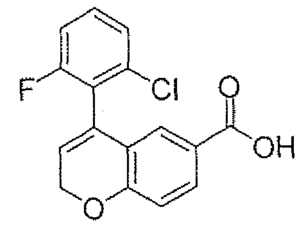 |

[Table 39]

| Prep | Str | | Prep | Str |
|---|---|---|---|---|
| 581 | 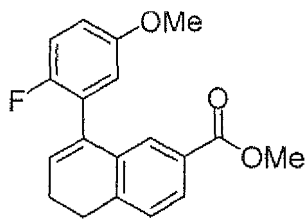 | | 588 | 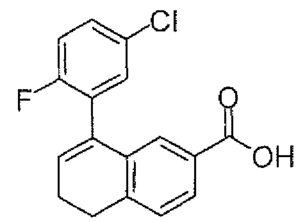 |
| 582 | 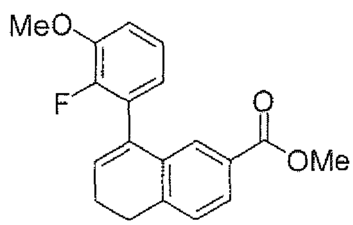 | | 589 | 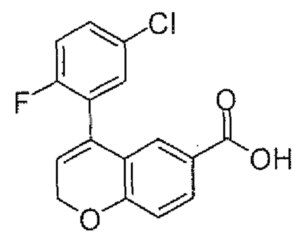 |
| 583 | 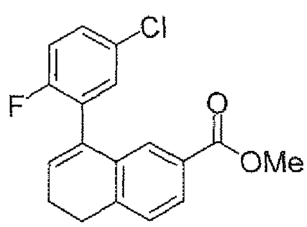 | | 590 | 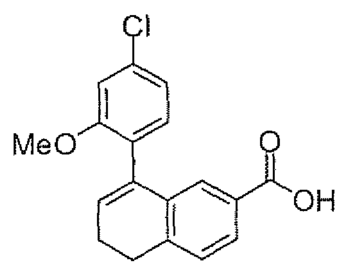 |
| 584 | 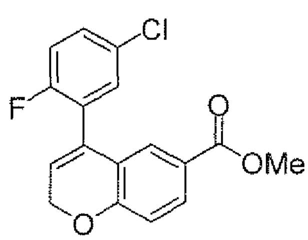 | | 591 | 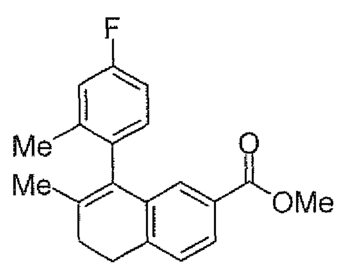 |

(continued)

| Prep | Str | | Prep | Str |
|---|---|---|---|---|
| 585 | 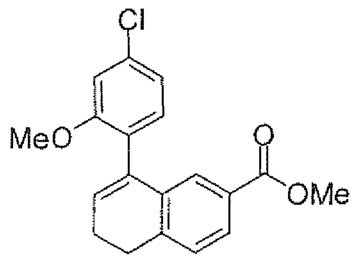 | | 592 | 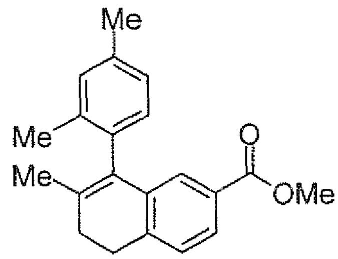 |
| 586 | 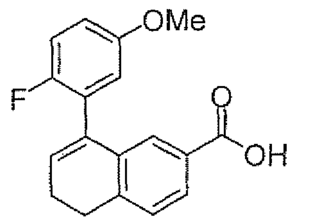 | | 593 | 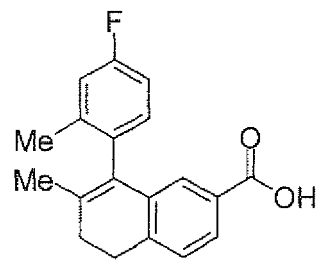 |
| 587 | 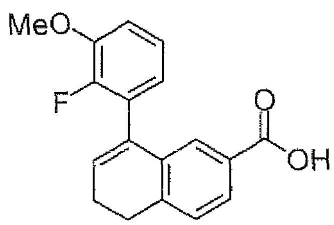 | | | |

[Table 40]

| Prep | Str | | Prep | Str |
|---|---|---|---|---|
| 594 | 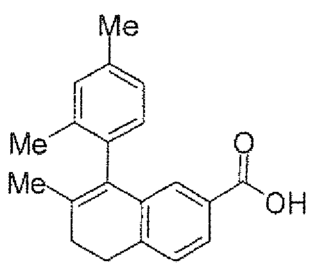 | | 600 | 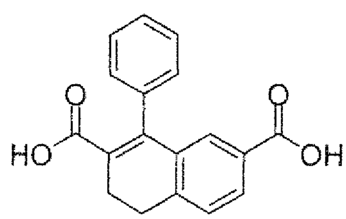 |
| 595 | 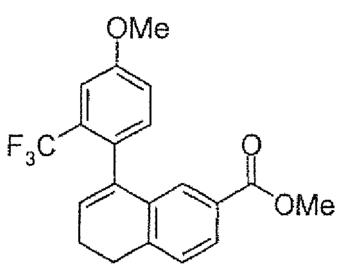 | | 601 | 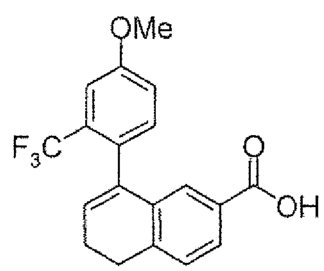 |

(continued)

| Prep | Str | | Prep | Str |
|---|---|---|---|---|
| 596 | 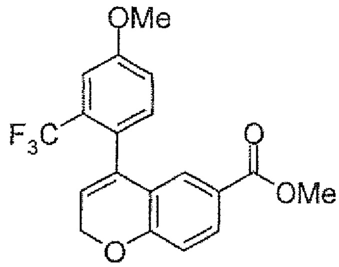 | | 602 | 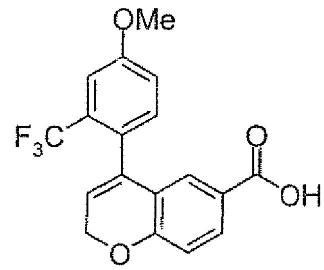 |
| 597 | 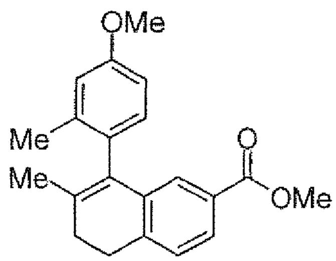 | | 603 | 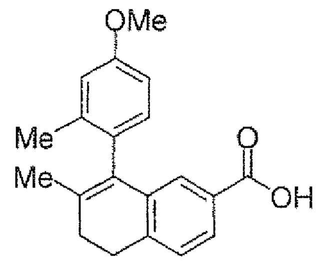 |
| 598 | 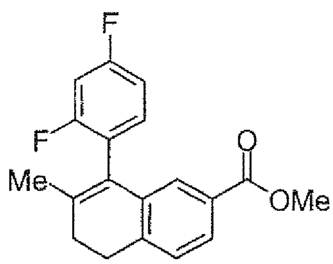 | | 604 | 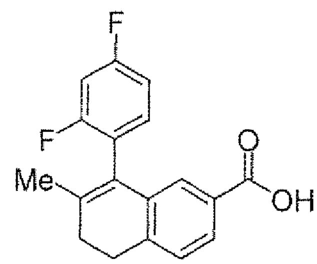 |
| 599 | 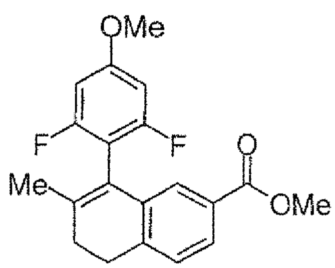 | | 605 | 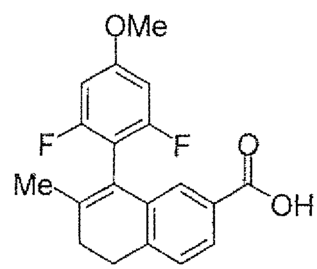 |

[Table 41]

| Prep | Str | | Prep | Str |
|---|---|---|---|---|
| 606 | 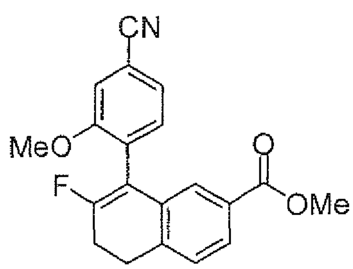 | | 612 | 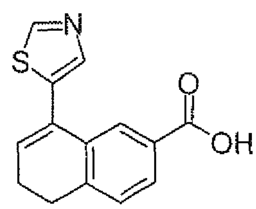 |

(continued)

| Prep | Str | Prep | Str |
|---|---|---|---|
| 607 | 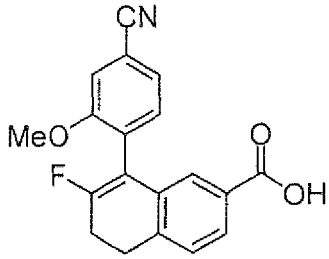 | 613 | 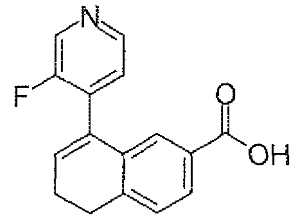 |
| 608 | 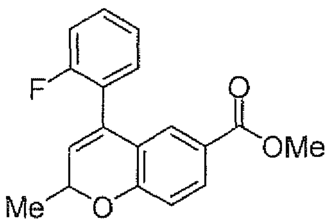 | 614 | 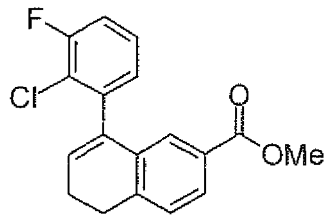 |
| | | 615 | 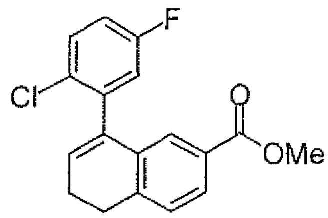 |
| 609 | 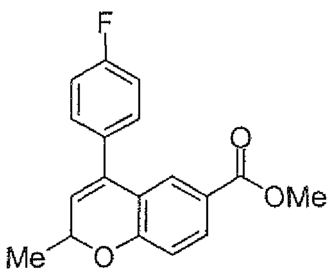 | 616 | 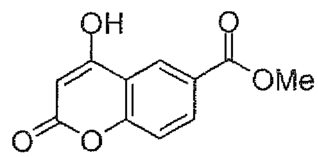 |
| 610 | 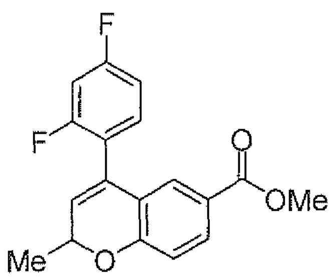 | 617 | 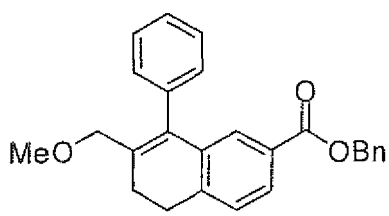 |
| 611 | 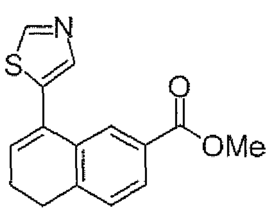 | 618 | 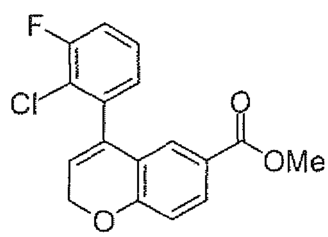 |

[Table 42]

| Prep | Str | | Prep | Str |
|---|---|---|---|---|
| 619 | 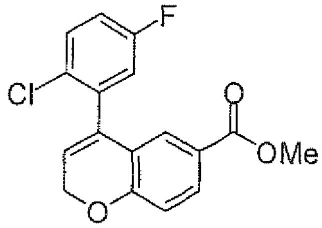 | | 626 | 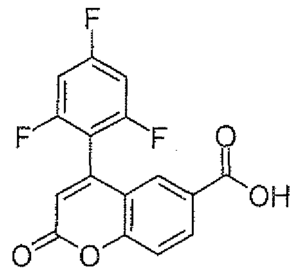 |
| 620 | 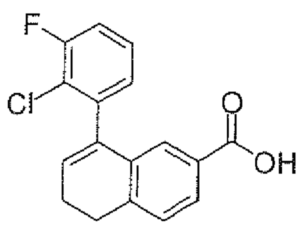 | | 627 | 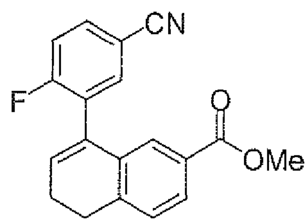 |
| 621 | 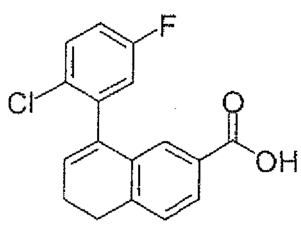 | | 628 | 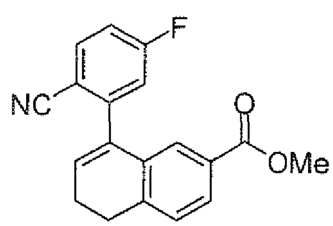 |
| 622 | 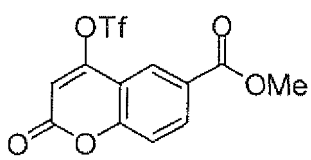 | | 629 | 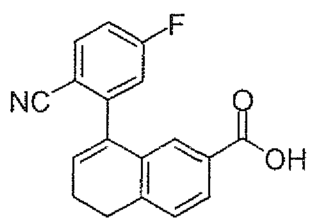 |
| 623 | 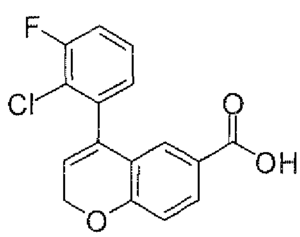 | | 630 | 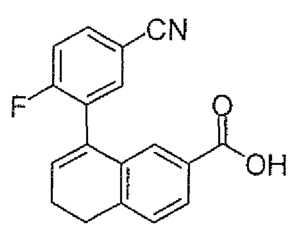 |
| 624 | 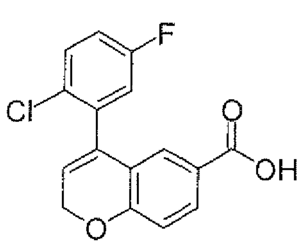 | | 631 | 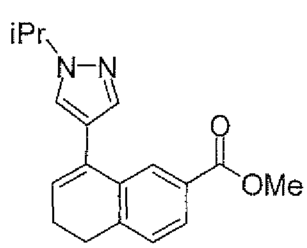 |

(continued)

| Prep | Str | | Prep | Str |
|---|---|---|---|---|
| 625 | 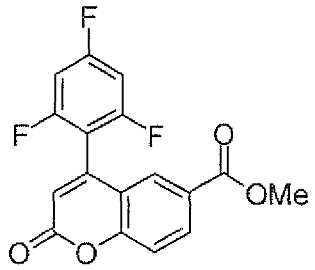 | | 632 | 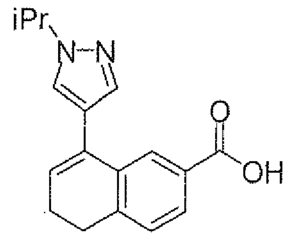 |

[Table 43]

| Prep | Str | | Prep | Str |
|---|---|---|---|---|
| 633 | 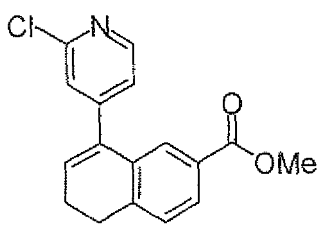 | | 639 | 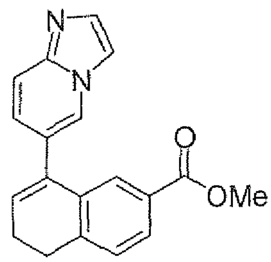 |
| 634 | 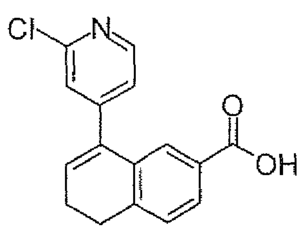 | | 640 | 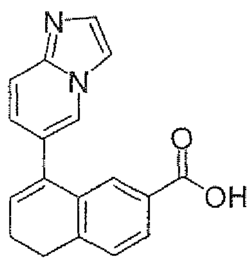 |
| 635 | 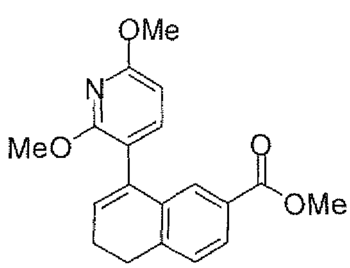 | | 641 | 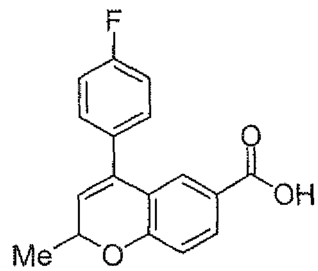 |
| 636 | 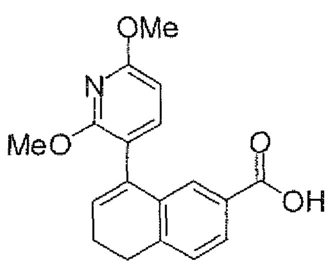 | | 642 | 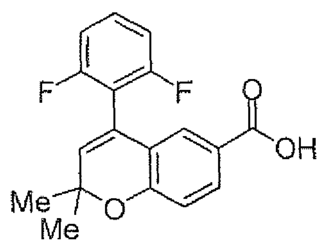 |

(continued)

| Prep | Str | | Prep | Str |
|---|---|---|---|---|
| 637 | 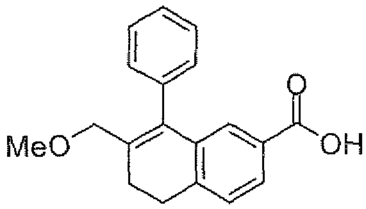 | | 643 | 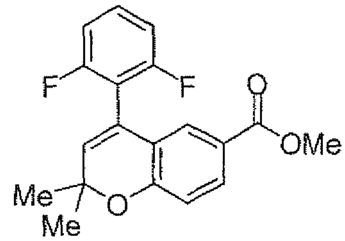 |
| 638 | 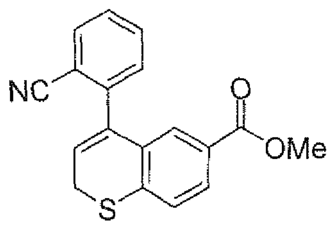 | | 644 | 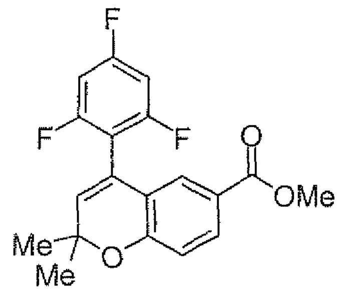 |

[Table 44]

| Prep | Str | | Prep | Str |
|---|---|---|---|---|
| 645 | 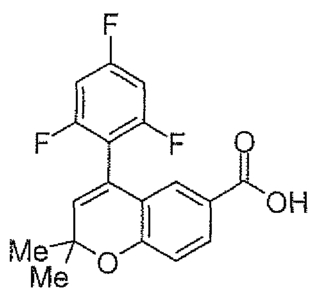 | | 651 | 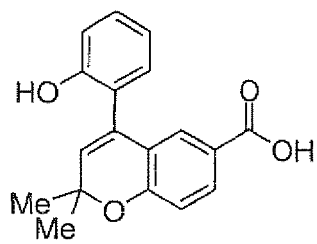 |
| 646 | 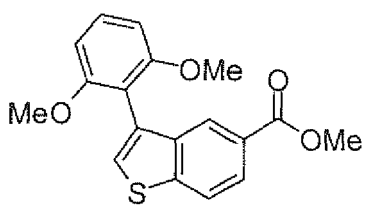 | | 652 | 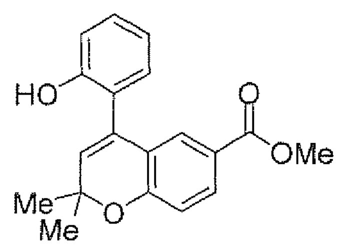 |
| 647 | 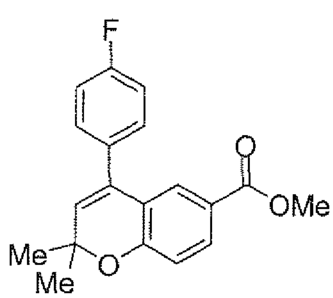 | | 653 | 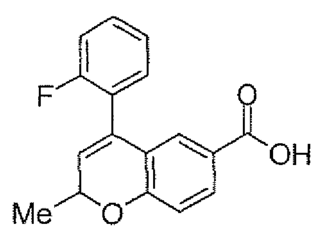 |

(continued)

| Prep | Str | | Prep | Str |
|---|---|---|---|---|
| 648 | 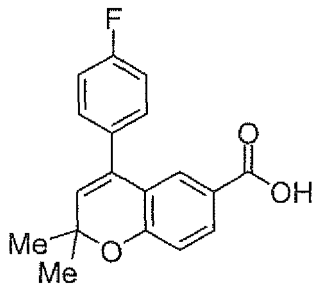 | | 654 | 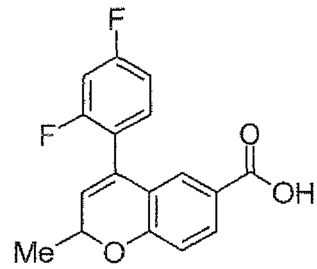 |
| 649 | 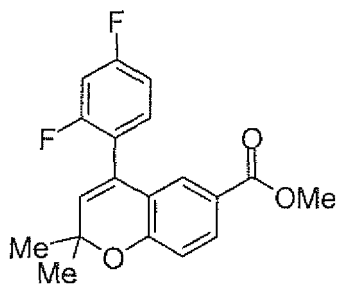 | | 655 | 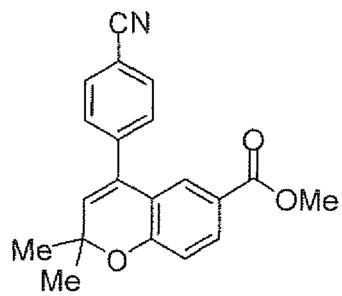 |
| 650 | 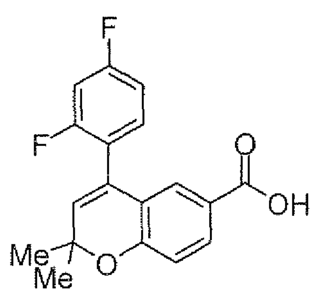 | | 656 | 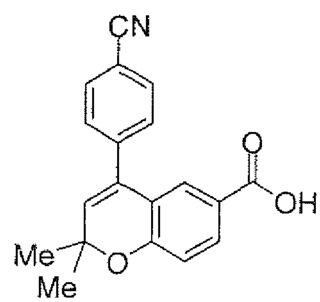 |

[Table 45]

| Prep | Str | | Prep | Str |
|---|---|---|---|---|
| 657 | 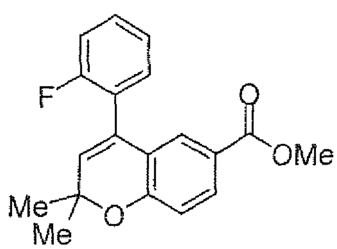 | | 664 | 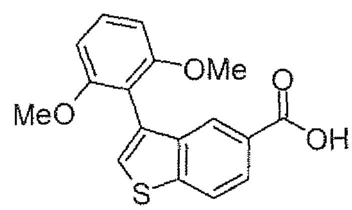 |
| 658 | 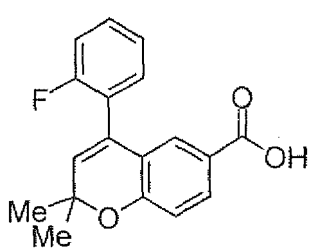 | | 665 | 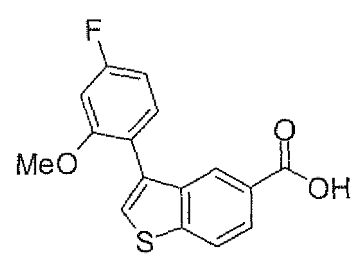 |

(continued)

| Prep | Str | | Prep | Str |
|---|---|---|---|---|
| 659 | 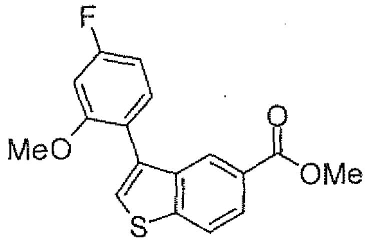 | | 666 | 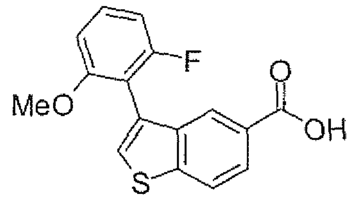 |
| 660 | 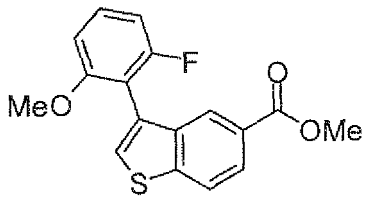 | | 667 | 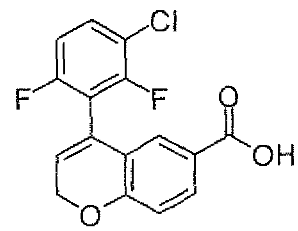 |
| 661 | 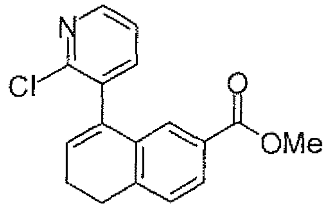 | | 668 | 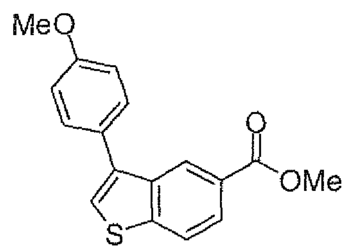 |
| 662 | 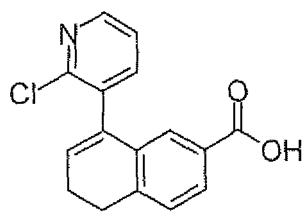 | | 669 | 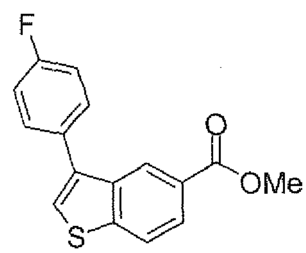 |
| 663 | 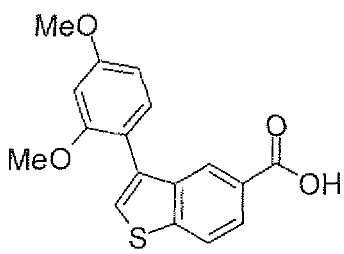 | | 670 | 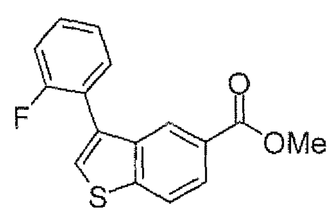 |

[Table 46]

| Prep | Str | Prep | Str |
|---|---|---|---|
| 671 | 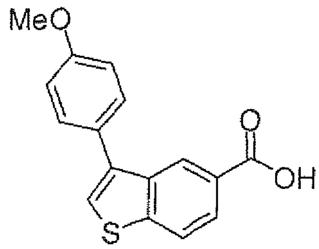 | 678 | 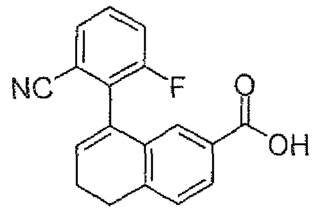 |
| 672 | 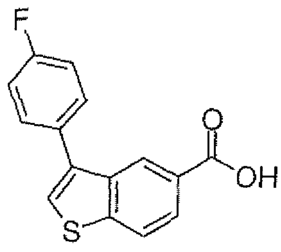 | 679 | 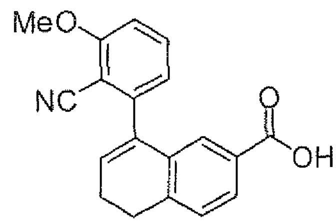 |
| 673 | 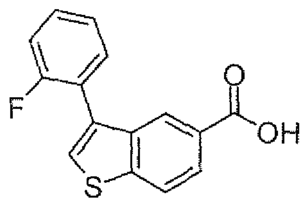 | 680 | 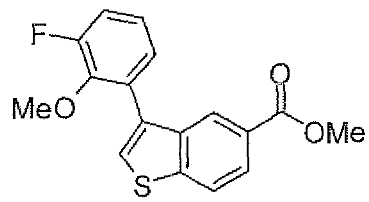 |
| 674 | 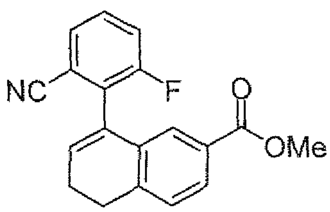 | 681 | 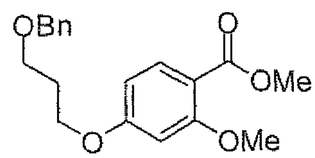 |
| 675 | 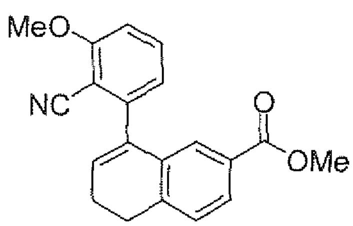 | 682 | 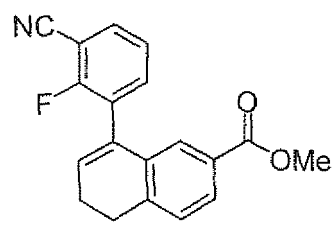 |
| 676 | 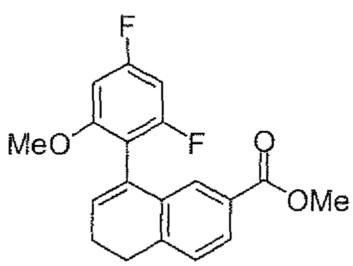 | 683 | 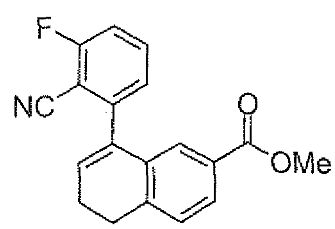 |

(continued)

| Prep | Str | | Prep | Str |
|---|---|---|---|---|
| 677 | 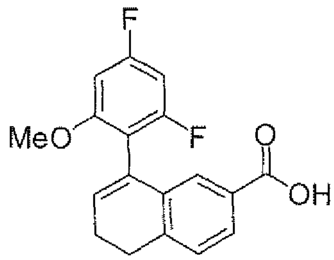 | | 684 | 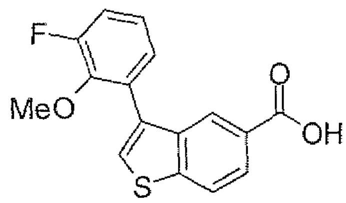 |

[Table 47]

| Prep | Str | | Prep | Str |
|---|---|---|---|---|
| 685 | 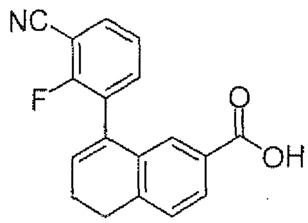 | | 693 | 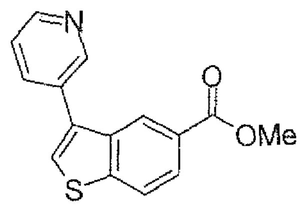 |
| 686 | 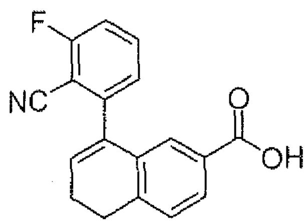 | | 694 | 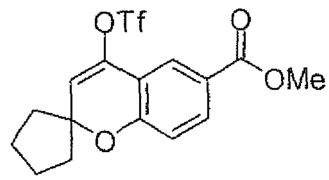 |
| 687 | 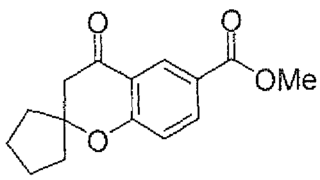 | | 695 | 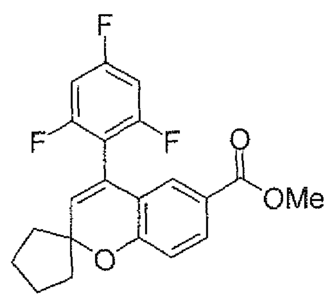 |
| 688 | 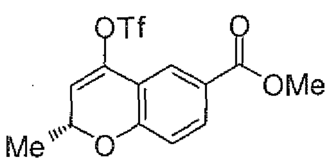 | | | |
| 689 | 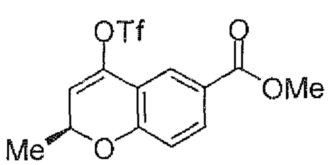 | | 696 | 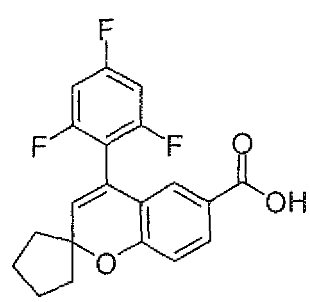 |

(continued)

| Prep | Str | | Prep | Str |
|---|---|---|---|---|
| 690 | 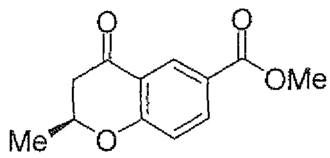 | | 697 | 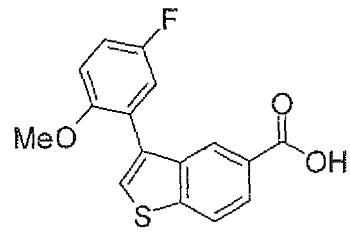 |
| 691 | 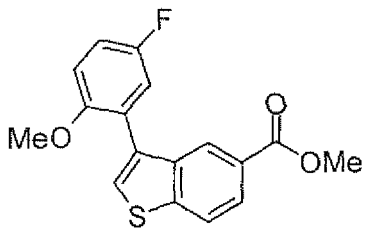 | | 698 | 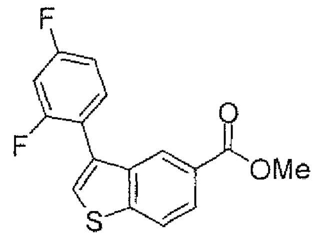 |
| 692 | 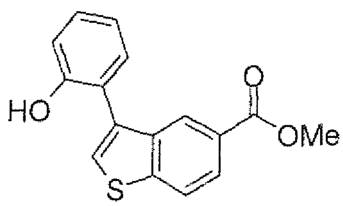 | | 699 | 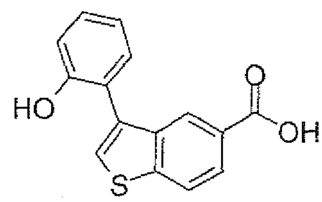 |

[Table 48]

| Prep | Str | | Prep | Str |
|---|---|---|---|---|
| 700 | 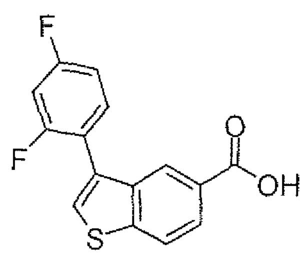 | | 708 | 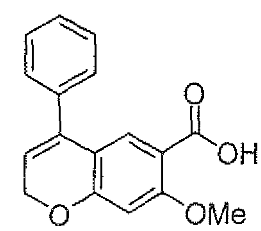 |
| 701 | 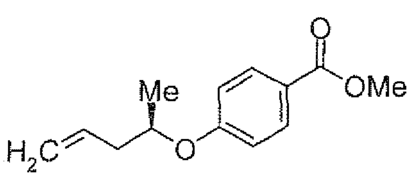 | | 709 | 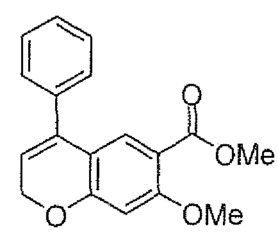 |

(continued)

| Prep | Str |
|---|---|
| 702 | 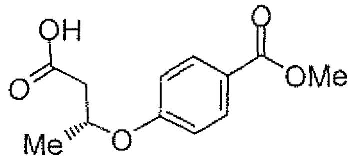 |
| 703 | 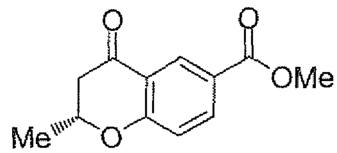 |
| 704 | 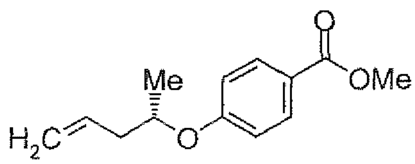 |
| 705 | 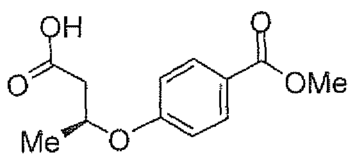 |
| 706 | 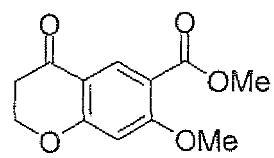 |
| 707 | 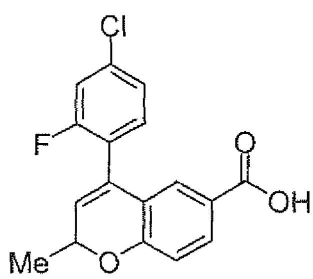 |

| Prep | Str |
|---|---|
| 710 | 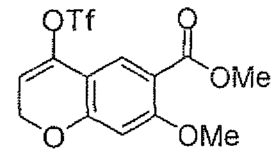 |
| 711 | 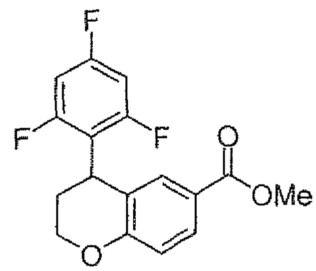 |
| 712 | 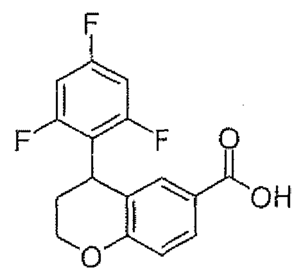 |
| 713 | 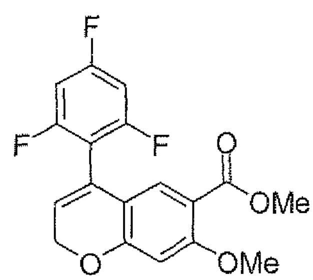 |

[Table 49]

| Prep | Str | Prep | Str |
|---|---|---|---|
| 714 | 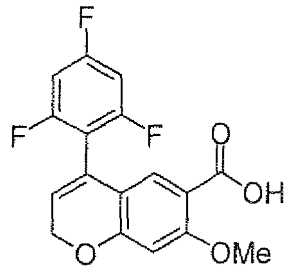 | 720 | 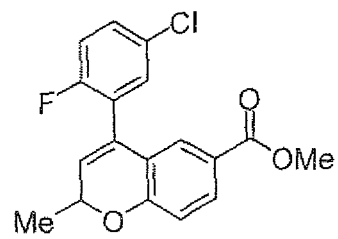 |
| 715 | 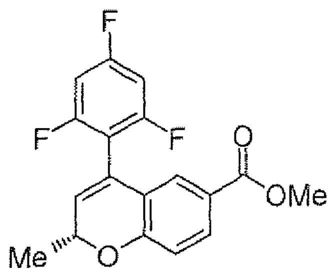 | 721 | 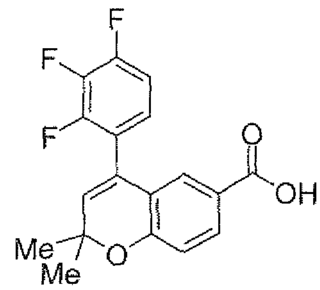 |
| 716 | 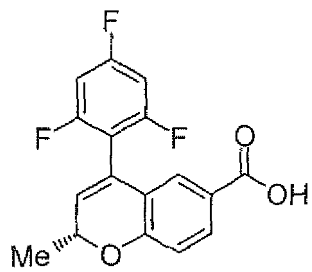 | 722 | 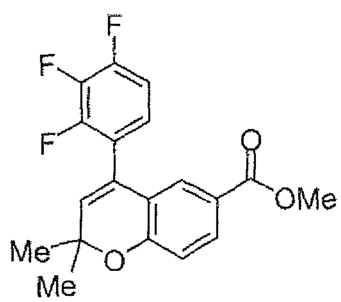 |
| 717 | 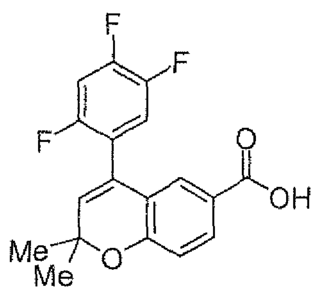 | 723 | 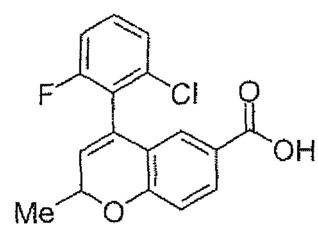 |
| 718 | 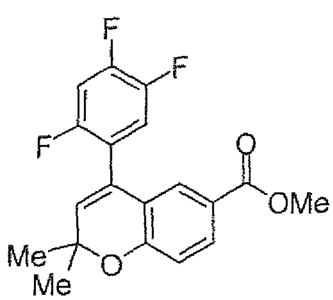 | 724 | 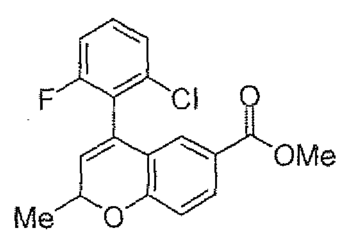 |

(continued)

| Prep | Str | | Prep | Str |
|---|---|---|---|---|
| 719 | 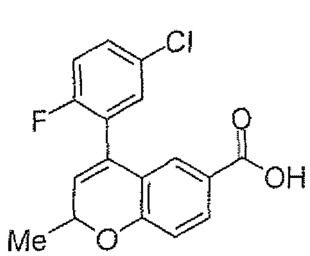 | | 725 | 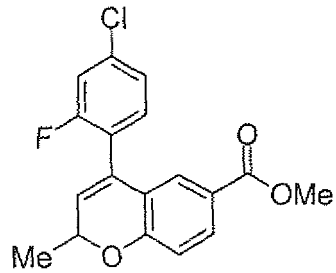 |

[Table 50]

| Prep | Str | | Prep | Str |
|---|---|---|---|---|
| 726 | 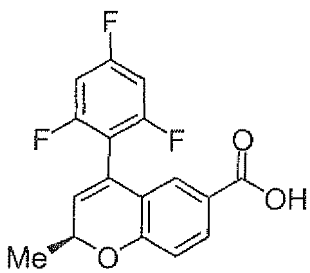 | | 733 | 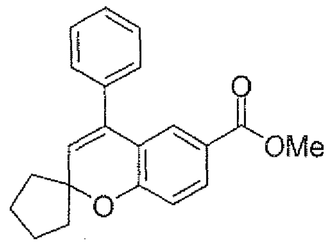 |
| 727 | 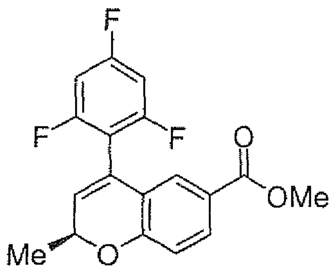 | | 734 | 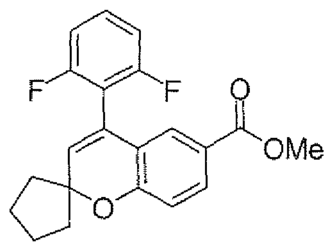 |
| 728 | 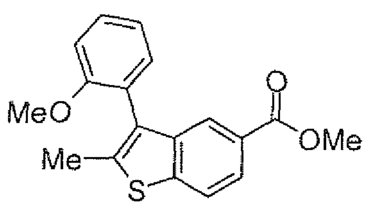 | | 735 | 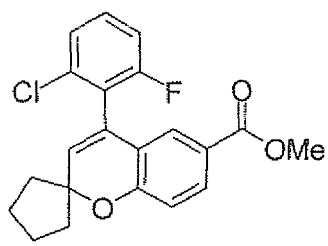 |
| 729 | 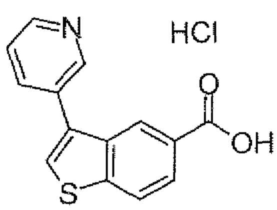 | | 736 | 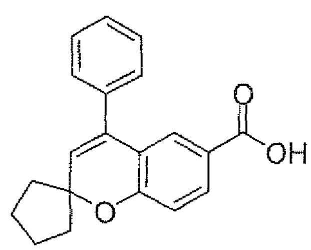 |

(continued)

| Prep | Str | | Prep | Str |
|---|---|---|---|---|
| 730 | 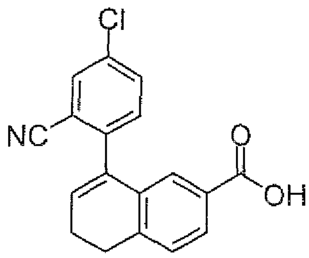 | | 737 | 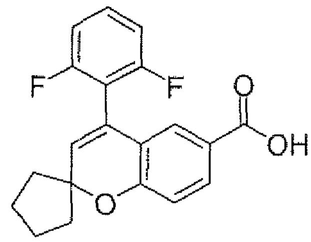 |
| 731 | 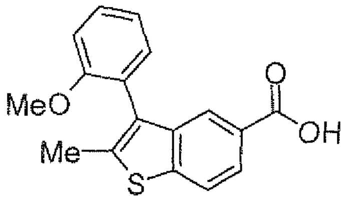 | | 738 | 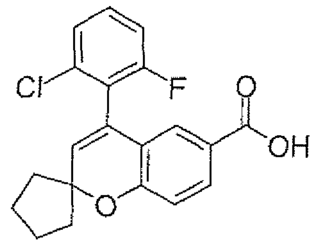 |
| 732 | 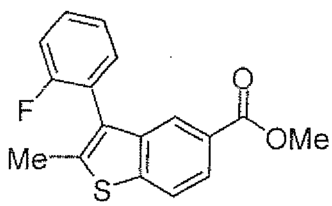 | | | |

[Table 51]

| Prep | Str | | Prep | Str |
|---|---|---|---|---|
| 739 | 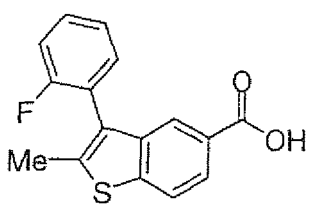 | | 746 | 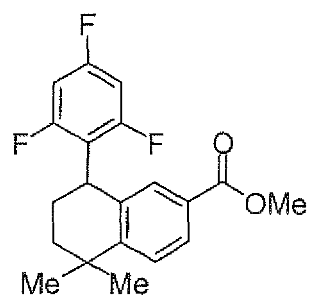 |
| 740 | 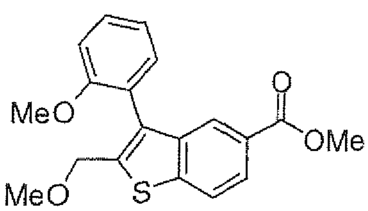 | | | |

(continued)

| Prep | Str | | Prep | Str |
|---|---|---|---|---|
| 741 | 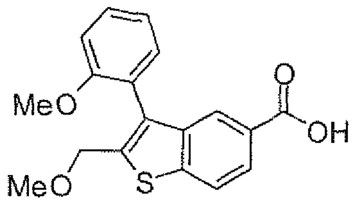 | | 747 | 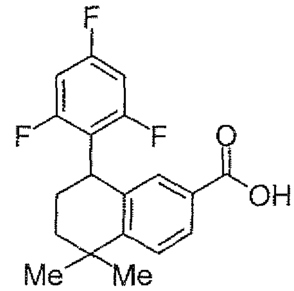 |
| 742 | 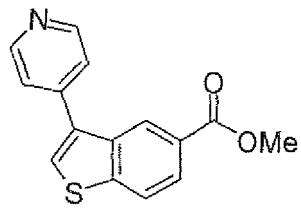 | | 748 | 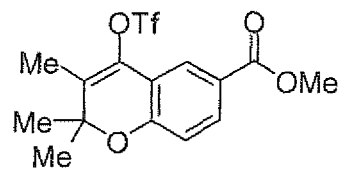 |
| 743 | 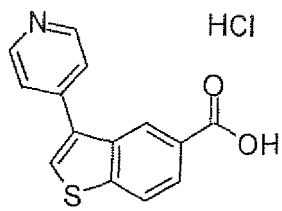 | | 749 | 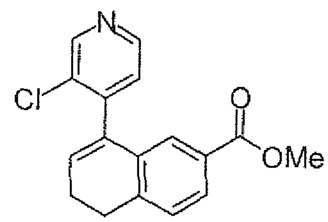 |
| 744 | 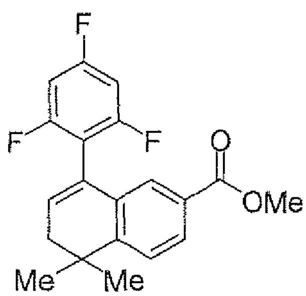 | | 750 | 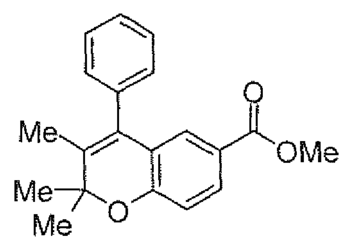 |
| 745 | 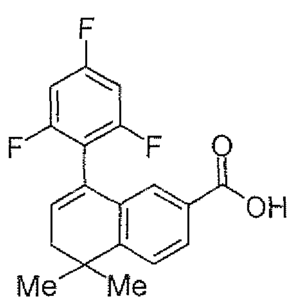 | | 751 | 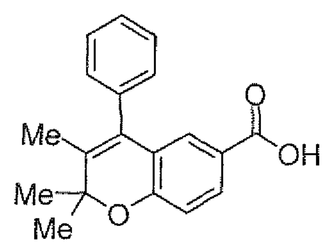 |

[Table 52]

| Prep | Str | Prep | Str |
|---|---|---|---|
| 752 | 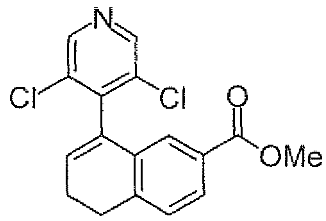 | 760 | 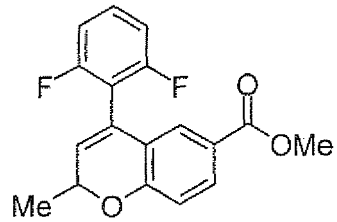 |
| 753 | 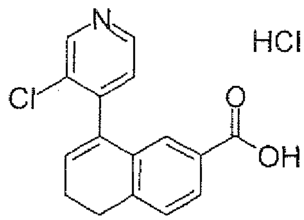 | 761 | 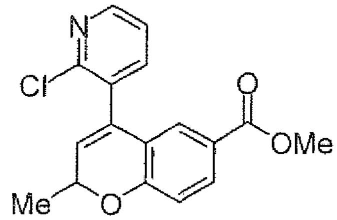 |
| 754 | 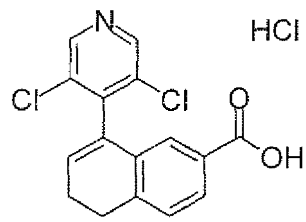 | 762 | 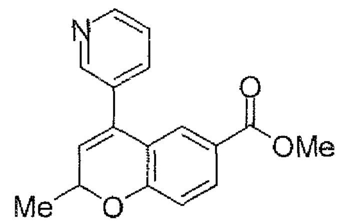 |
| 755 | 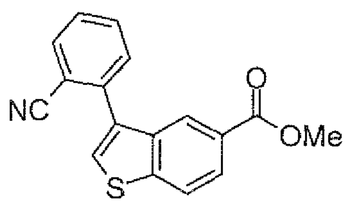 | 763 | 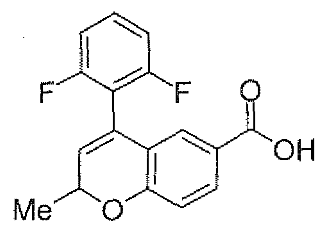 |
| 756 | 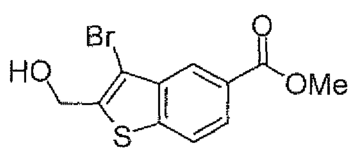 | | |
| 757 | 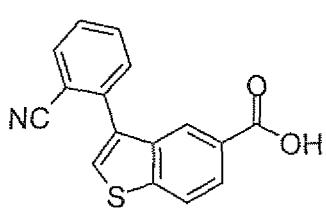 | 764 | 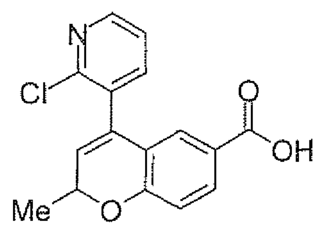 |
| 758 | 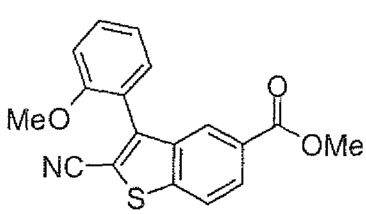 | 765 | 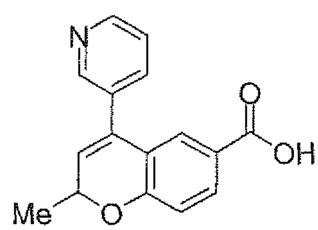 |

(continued)

| Prep | Str | | Prep | Str |
|---|---|---|---|---|
| 759 | 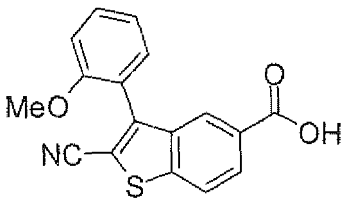 | | 766 | 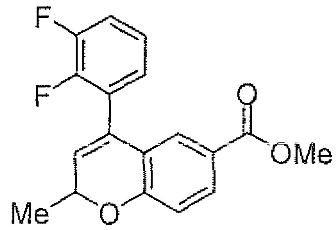 |

[Table 53]

| Prep | Str | | Prep | Str |
|---|---|---|---|---|
| 767 | 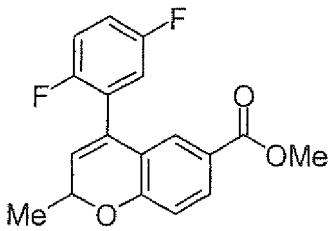 | | 774 | 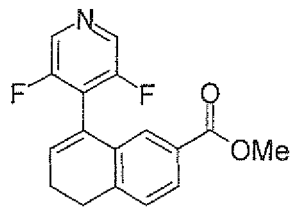 |
| 768 | 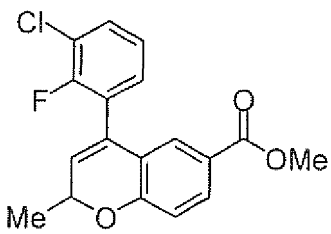 | | 775 | 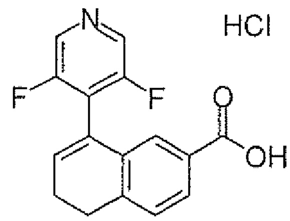 |
| 769 | 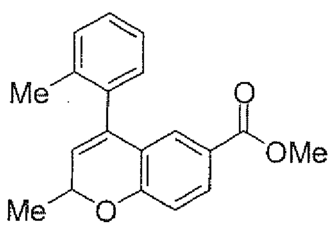 | | 776 | 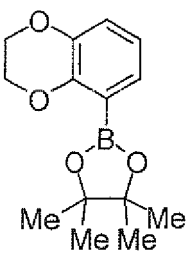 |
| 770 | 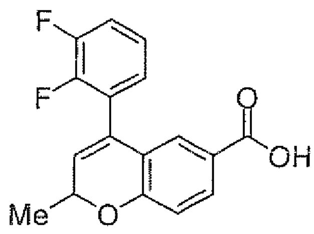 | | 777 | 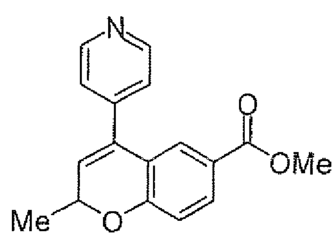 |

(continued)

| Prep | Str | | Prep | Str |
|---|---|---|---|---|
| 771 | 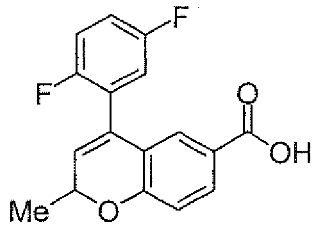 | | 778 | 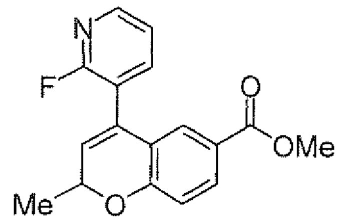 |
| 772 | 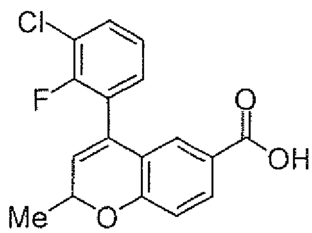 | | 779 | 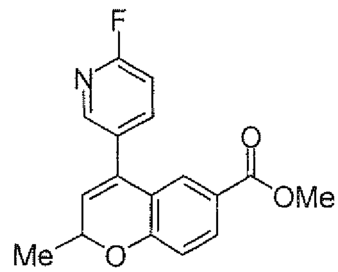 |
| 773 | 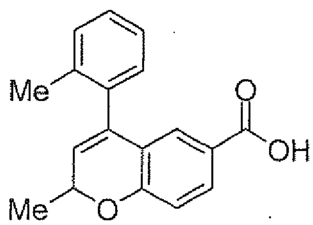 | | 780 | 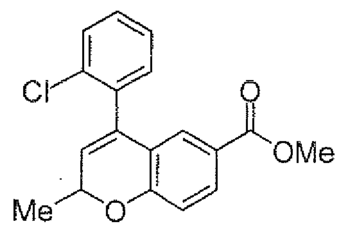 |

[Table 54]

| Prep | Str | | Prep | Str |
|---|---|---|---|---|
| 781 | 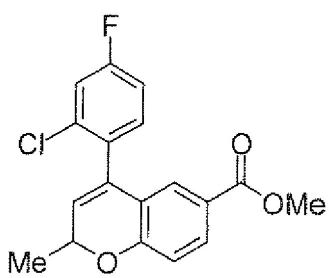 | | 787 | 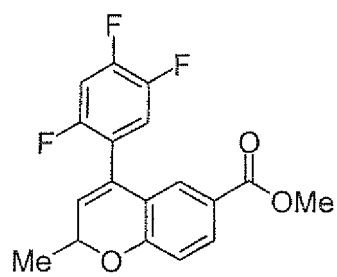 |
| 782 | 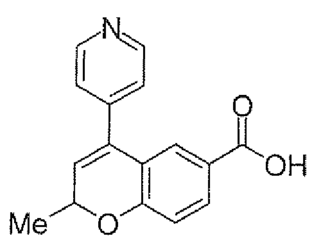 | | 788 | 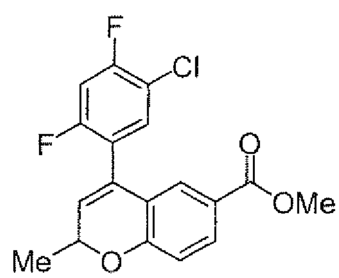 |

(continued)

| Prep | Str | | Prep | Str |
|---|---|---|---|---|
| 783 | 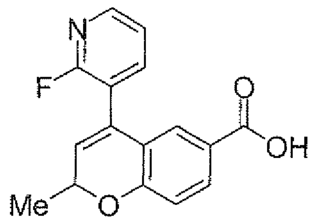 | | 789 | 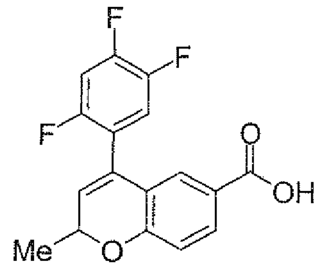 |
| 784 | 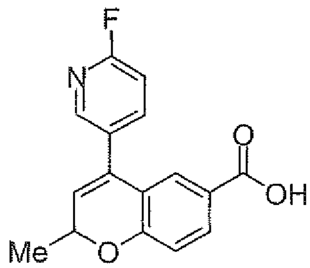 | | 790 | 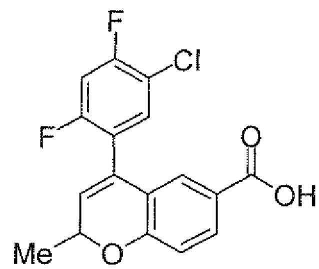 |
| 785 | 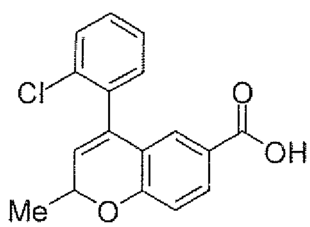 | | 791 | 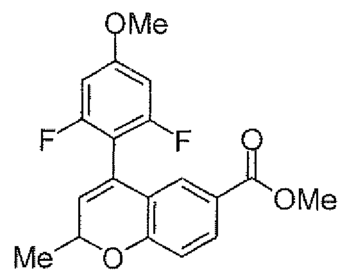 |
| 786 | 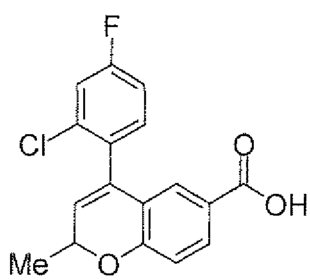 | | 792 | 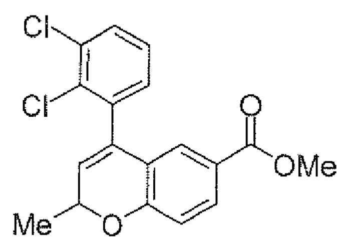 |

[Table 55]

| Prep | Str | | Prep | Str |
|---|---|---|---|---|
| 793 | 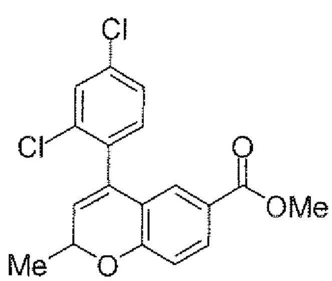 | | 799 | 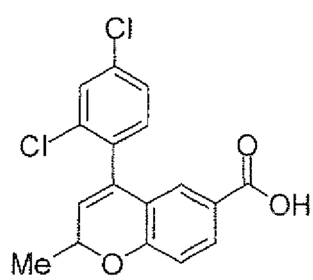 |

(continued)

| Prep | Str | Prep | Str |
|---|---|---|---|
| 794 | 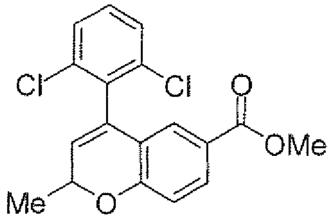 | 800 | 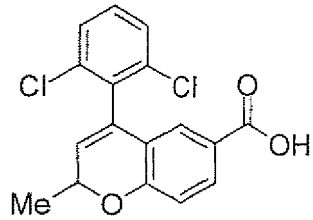 |
| 795 | 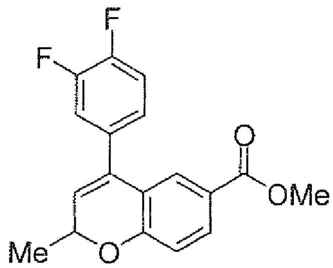 | 801 | 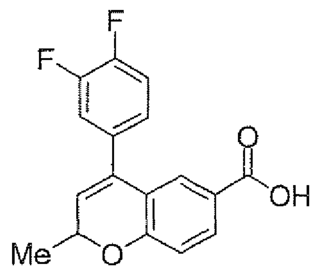 |
| 796 | 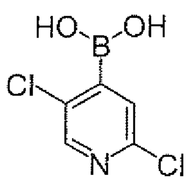 | 802 | 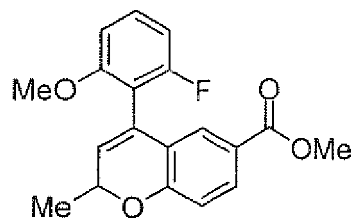 |
| 797 | 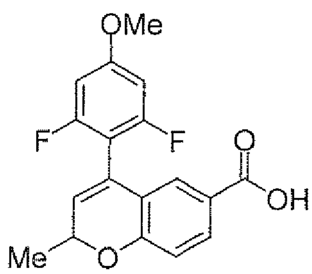 | 803 | 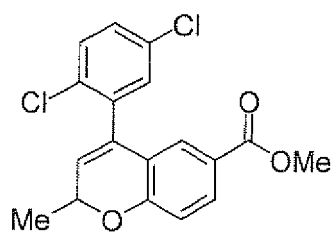 |
| 798 | 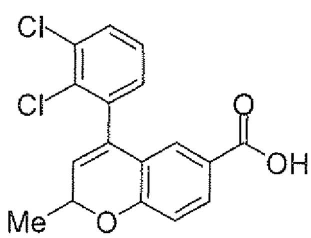 | 804 | 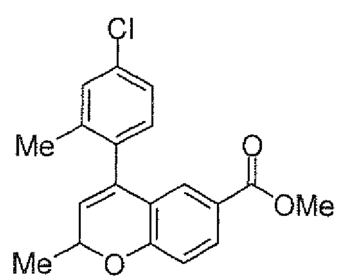 |

[Table 56]

| Prep | Str | Prep | Str |
|---|---|---|---|
| 805 | 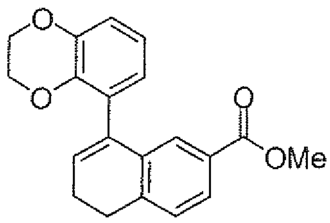 | 811 | 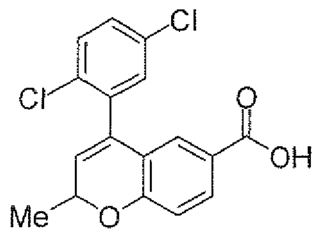 |
| 806 | 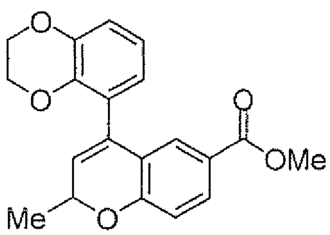 | 812 | 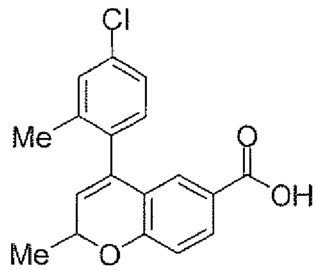 |
| 807 | 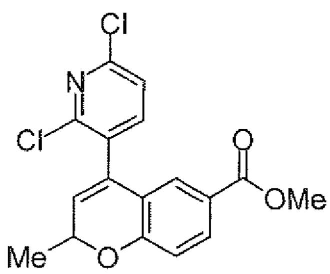 | 813 | 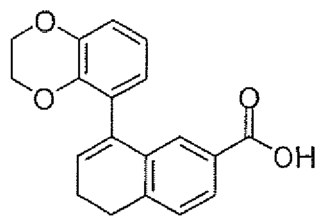 |
| 808 | 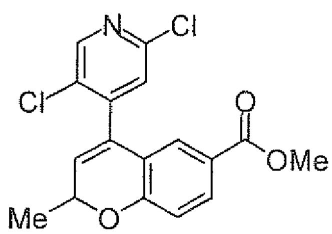 | 814 | 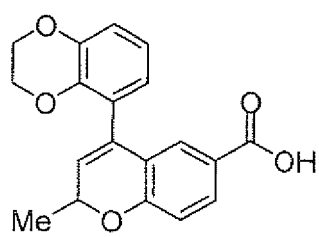 |
| 809 | 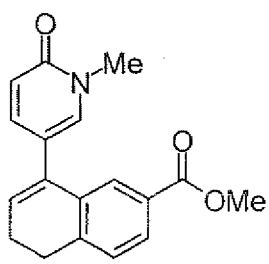 | 815 | 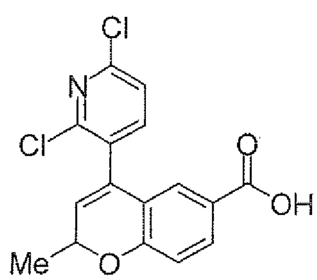 |
| 810 | 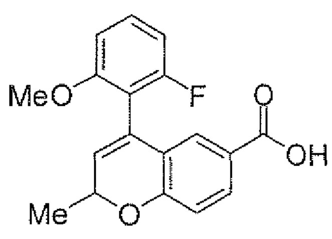 | 816 | 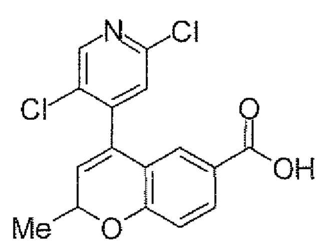 |

[Table 57]

| Prep | Str | | Prep | Str |
|---|---|---|---|---|
| 817 | 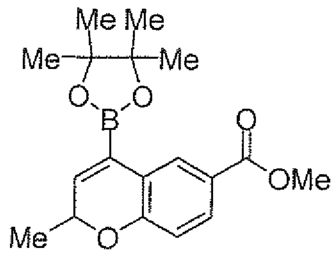 | | 824 | 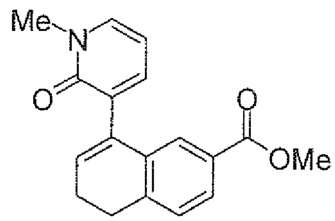 |
| 818 | 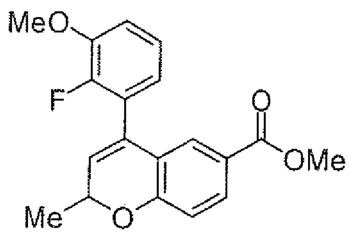 | | 825 | 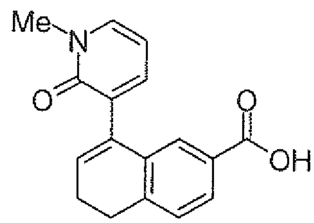 |
| 819 | 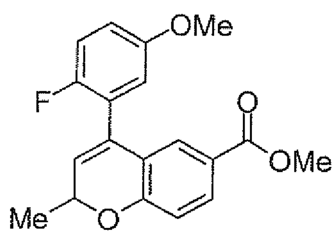 | | 826 | 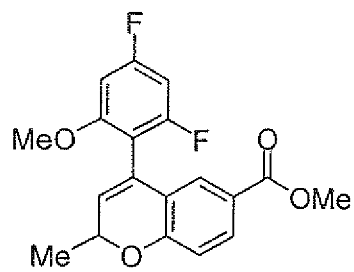 |
| 820 | 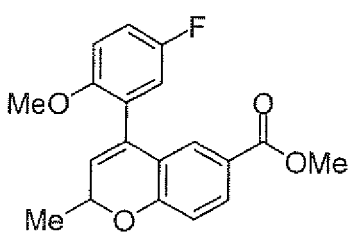 | | 827 | 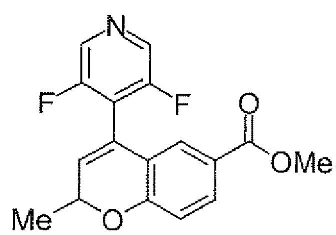 |
| 821 | 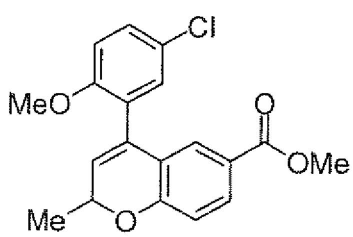 | | 828 | 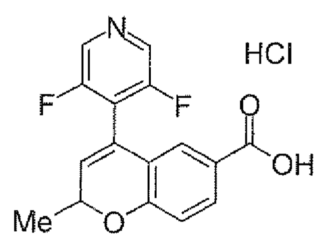 |

(continued)

| Prep | Str | | Prep | Str |
|---|---|---|---|---|
| 822 | 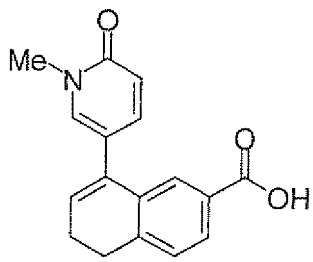 | | 829 | 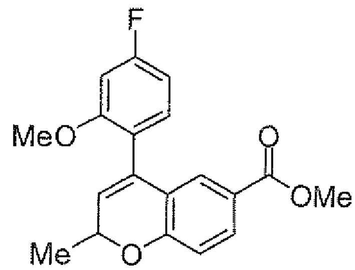 |
| 823 | 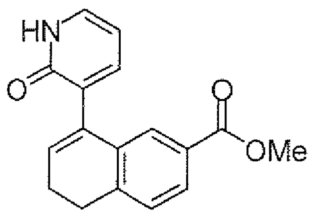 | | | |

[Table 58]

| Prep | Str | | Prep | Str |
|---|---|---|---|---|
| 830 | 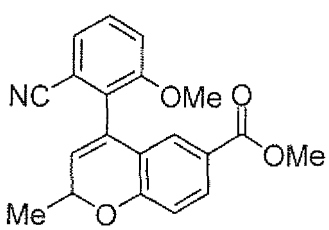 | | 837 | 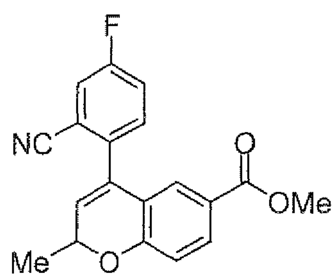 |
| 831 | 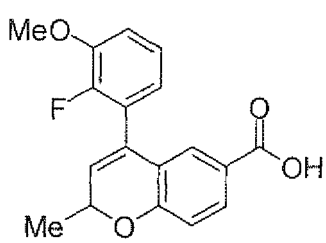 | | 838 | 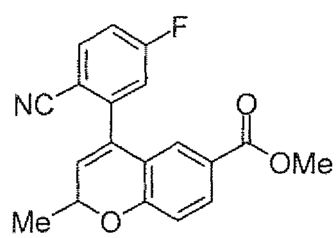 |
| 832 | 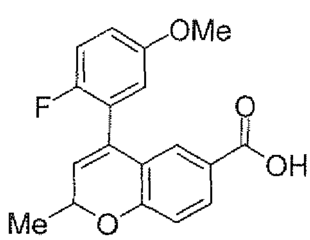 | | 839 | 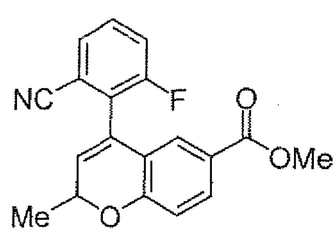 |

(continued)

| Prep | Str | | Prep | Str |
|---|---|---|---|---|
| 833 | 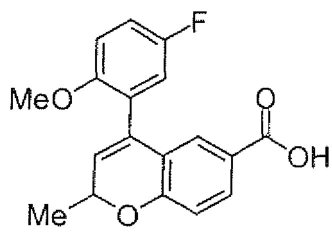 | | 840 | 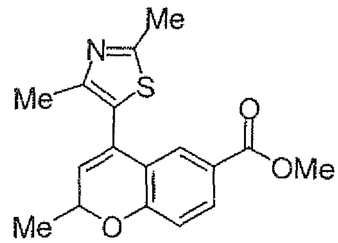 |
| 834 | 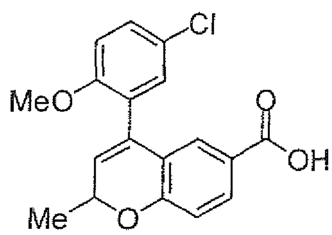 | | 841 | 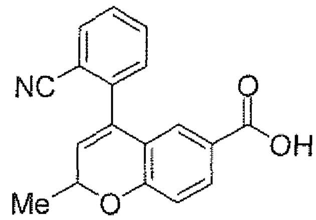 |
| 835 | 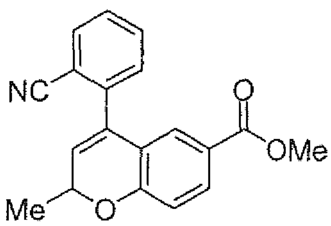 | | 842 | 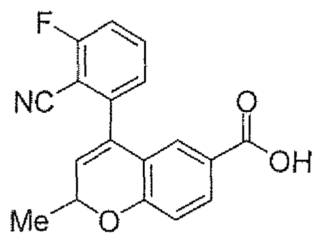 |
| 836 | 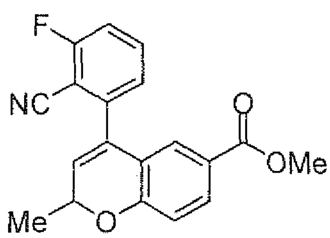 | | | |

[Table 59]

| Prep | Str | | Prep | Str |
|---|---|---|---|---|
| 843 | 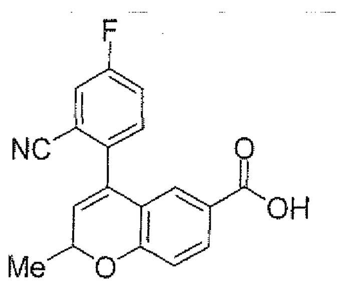 | | 849 | 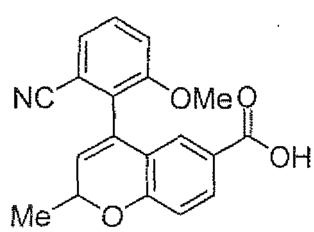 |

(continued)

| Prep | Str | Prep | Str |
|---|---|---|---|
| 844 | 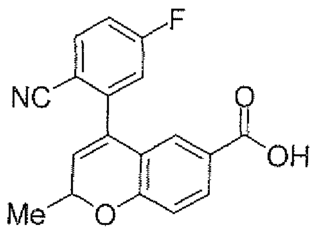 | 850 | 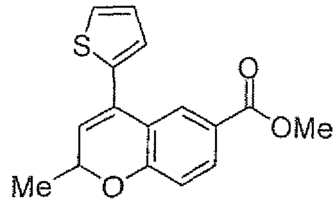 |
| 845 | 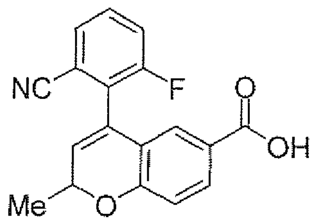 | 851 | 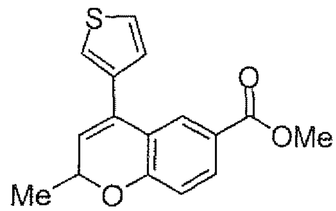 |
| 846 | 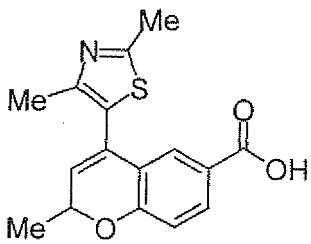 | 852 | 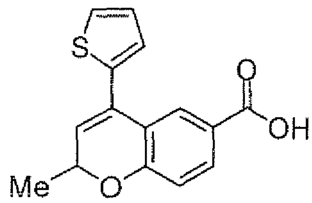 |
| 847 | 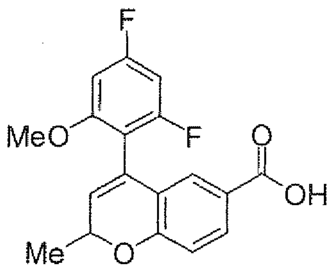 | 853 | 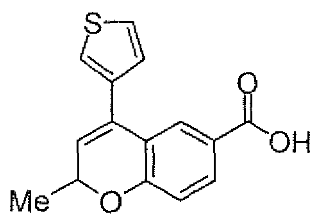 |
| 848 | 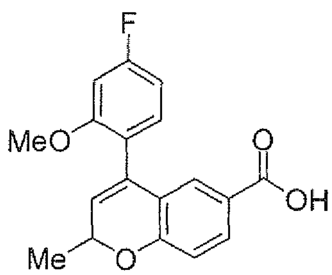 | | |

[Table 60]

| Prep | PSyn | Dat | Prep | PSyn | Dat | Prep | PSyn | Dat |
|---|---|---|---|---|---|---|---|---|
| 1 | 1 | FAB+:367 | 39 | 3 | FAB-: 279 | 76 | 3 | FAB+: 293 |
| 2 | 2 | FAB+:325 | 40 | 3 | FAB-: 279 | 77 | 1,2,3 | FAB+: 250 |
| 3 | 3 | FAB-:309 | 41 | 3 | FAB-: 267 | 78 | 2 | FAB+: 308 |

(continued)

| Prep | PSyn | Dat | Prep | PSyn | Dat | Prep | PSyn | Dat |
|---|---|---|---|---|---|---|---|---|
| 4 | 4 | EI+: 316 | 42 | 3 | FAB+: 285 | 79 | 3 | ESI+: 294 |
| 5 | 5 | EI+: 305 | 43 | 2 | FAB+: 283 | 80 | 2 | EI+: 292 |
| 6 | 6 | FAB+:273 | 44 | 2 | FAB+: 283 | 81 | 2 | EI+: 324 |
| 7 | 7 | EI+: 265 | 45 | 2 | FAB+: 299 | 82 | 2 | EI+: 324 |
| 8 | 8 | EI+: 243 | 46 | 2 | FAB+: 299 | 83 | 2 | EI+: 324 |
| 9 | 9 | CI+:219 | 47 | 3 | FAB-: 267 | 84 | 3 | FAB+: 311 |
| 10 | 10 | EI+:222 | 48 | 3 | FAB-: 267 | 85 | 3 | FAB+: 311 |
| 11 | 11 | EI+:220 | 49 | 3 | FAB+: 285 | 86 | 3 | FAB+: 311 |
| 12 | 12 | EI+:238 | 50 | 3 | FAB+: 285 | 87 | 3 | FAB+:252 |
| 13 | 13 | EI+:220 | 51 | 2 | FAB+: 279 | 88 | 3 | FAB-:255 |
| 14 | 14 | ESI-: 301 | 52 | 2 | FAB+: 279 | 89 | 3 | FAB-:239 |
| 15 | 15 | EI+: 284 | 53 | 2 | FAB+: 279 | 90 | 3 | FAB-:255 |
| 16 | 16 | EI+:226 | 54 | 2 | FAB+: 333 | 91 | 3 | FAB+:257 |
| 17 | 17 | ESI+: 375 | 55 | 2 | FAB+: 333 | 92 | 6 | EI+:270 |
| 18 | 18 | EI+:266 | 56 | 2 | FAB+: 333 | 93 | 3 | FAB-:257 |
| 20 | 20 | ESI+: 231 | 57 | 3 | FAB-: 263 | 94 | 3 | FAB-:279 |
| 21 | 21 | ESI+: 233 | 58 | 3 | FAB-: 263 | 95 | 2 | EI+:294 |
| 22 | 22 | FAB+:265 | 59 | 3 | FAB-: 263 | 96 | 3 | FAB-:341 |
| 23 | 23 | EI+:226 | 60 | 3 | FAB-: 317 | 97 | 2 | EI+:356 |
| 24 | 24 | EI+:220 | 61 | 3 | FAB-: 317 | 98 | 3 | FAB-:279 |
| 25 | 25 | ESI+:223 | 62 | 3 | FAB-: 317 | 99 | 2 | EI+:294 |
| 26 | 26 | EI+:218 | 63 | 2 | FAB+: 290 | 100 | 3 | FAB-:279 |
| 27 | 27 | EI+:220 | 64 | 2 | FAB+: 290 | 101 | 2 | EI+:294 |
| 28 | 28 | FAB+322 | 65 | 2 | ESI+: 290 | 102 | 2 | FAB+: 309 |
| 29 | 29 | ESI+: 343 | 66 | 3 | FAB-: 274 | 103 | 2 | FAB+: 322 |
| 30 | 2 | ESI+: 284 | 67 | 3 | FAB-: 274 | 104 | 3 | FAB+: 295 |
| 31 | 2 | FAB+: 265 | 68 | 3 | FAB-: 274 | 105 | 3 | FAB+: 309 |
| 32 | 1 | FAB+: 337 | 69 | 2 | FAB+: 307 | 106 | 2 | FAB+: 281 |
| 33 | 2 | FAB+: 295 | 70 | 2 | FAB+: 307 | 107 | 2 | FAB+: 281 |
| 34 | 2 | FAB+: 295 | 71 | 2 | EI+:270 | 108 | 2 | EI+: 280 |
| 35 | 2 | FAB+: 294 | 72 | 2 | EI+:265 | 109 | 2 | FAB+: 310 |
| 36 | 2 | FAB+: 283 | 73 | 2 | EI+:254 | 110 | 2 | EI+: 309 |
| 37 | 2 | EI+: 298 | 74 | 2 | EI+:270 | 111 | 2 | EI+: 309 |
| 38 | 3 | FAB-: 279 | 75 | 3 | FAB+: 293 | 112 | 3 | FAB-: 294 |

[Table 61]

| Prep | PSyn | Dat | | Prep | PSyn | Dat | | Prep | PSyn | Dat |
|---|---|---|---|---|---|---|---|---|---|---|
| 113 | 3 | FAB-: 294 | | 150 | 3 | FAB+: 268 | | 187 | 1 | FAB+:353 |
| 114 | 3 | FAB-: 294 | | 151 | 3 | FAB+:308 | | 188 | 3 | FAB-:269 |
| 115 | 3 | EI+: 266 | | 152 | 2 | FAB+:266 | | 189 | 2 | EI+:284 |
| 116 | 3 | EI+: 266 | | 153 | 3 | FAB-: 296 | | 190 | 3 | FAB-:319 |
| 117 | 3 | FAB-: 265 | | 154 | 3 | ESI+:308 | | 191 | 2 | ESI+:335 |
| 118 | 2 | FAB+: 313 | | 155 | 2 | FAB-: 333 | | 192 | 2 | EI+: 302 |
| 119 | 2 | EI+: 283 | | 156 | 2 | FAB+: 342 | | 193 | 2 | EI+: 302 |
| 120 | 3 | FAB+: 298 | | 157 | 2 | FAB+: 320 | | 194 | 3 | FAB+: 292 |
| 121 | 1 | FAB+:339 | | 158 | 1 | FAB+:355 | | 195 | 3 | EI+: 288 |
| 122 | 7 | EI+: 281 | | 159 | 3 | FAB+:312 | | 196 | 3 | EI+:289 |
| 123 | 3 | ESI+: 270 | | 160 | 2 | FAB+:282 | | 197 | 2 | EI+:300 |
| 124 | 4 | FAB+: 315 | | 161 | 2 | FAB+:295 | | 198 | 2 | EI+:310 |
| 125 | 8 | EI+: 259 | | 162 | 2 | FAB+:267 | | 199 | 2 | EI+:280 |
| 126 | 2 | FAB+: 320 | | 163 | 2 | FAB+:296 | | 200 | 2 | CI+:281 |
| 127 | 3 | ESI+: 306 | | 164 | 3 | FAB-:251 | | 201 | 2 | CI+:285 |
| 128 | 5 | EI+: 307 | | 165 | 3 | FAB-:281 | | 202 | 2 | EI+: 302 |
| 129 | 2 | FAB+: 322 | | 166 | 3 | EI+:280 | | 203 | 2 | EI+: 302 |
| 130 | 3 | EI+: 293 | | 167 | 3 | FAB-:267 | | 204 | 2 | EI+: 302 |
| 131 | 3 | ESI+: 308 | | 168 | 3 | FAB-:297 | | 205 | 2 | EI+: 320 |
| 132 | 2 | EI+: 284 | | 169 | 3 | ESI+:308 | | 206 | 3 | EI+: 289 |
| 133 | 2 | EI+: 281 | | 170 | 3 | FAB+:252 | | 207 | 3 | EI+: 288 |
| 134 | 2 | FAB+: 314 | | 171 | 3 | FAB-:276 | | 208 | 3 | EI+: 288 |
| 135 | 3 | ESI+: 271 | | 172 | 3 | FAB-:269 | | 209 | 3 | EI+: 307 |
| 136 | 3 | FAB+: 266 | | 173 | 2 | EI+:291 | | 210 | 2 | EI+:296 |
| 137 | 3 | EI+: 300 | | 174 | 3 | FAB-:276 | | 211 | 2 | EI+: 310 |
| 138 | 2 | EI+: 300 | | 175 | 2 | FAB+:283 | | 212 | 2 | EI+: 298 |
| 139 | 2 | EI+: 296 | | 176 | 2 | EI+:311 | | 213 | 3 | EI+: 285 |
| 140 | 2 | FAB+: 312 | | 177 | 3 | FAB-:293 | | 214 | 3 | FAB-: 295 |
| 141 | 2 | EI+: 282 | | 178 | 3 | FAB-:288 | | 215 | 3 | CI+:267 |
| 142 | 2 | EI+:291 | | 179 | 3 | FAB-: 319 | | 216 | 3 | FAB-:285 |
| 143 | 1 | FAB+:357 | | 180 | 3 | FAB+: 307 | | 217 | 3 | FAB-:265 |
| 144 | 2 | FAB+:284 | | 181 | 3 | FAB+: 329 | | 218 | 3 | EI+:297 |
| 145 | 1 | FAB+:351 | | 182 | 3 | FAB-:263 | | 219 | 2 | EI+: 303 |
| 146 | 2 | FAB+:279 | | 183 | 2 | FAB+:308 | | 220 | 2 | EI+: 298 |
| 147 | 2 | FAB+:304 | | 184 | 3 | FAB-:267 | | 221 | 3 | EI+: 288 |
| 148 | 3 | FAB+: 287 | | 185 | 3 | FAB-:296 | | 222 | 3 | EI+: 284 |
| 149 | 3 | FAB-: 281 | | 186 | 10 | EI+:224 | | 223 | 2 | FAB+: 295 |

[Table 62]

| Prep | PSyn | Dat | | Prep | PSyn | Dat | | Prep | PSyn | Dat |
|---|---|---|---|---|---|---|---|---|---|---|
| 224 | 3 | FAB-: 279 | | 261 | 1 | EI+:350 | | 298 | 3 | FAB+: 293 |
| 225 | 2 | EI+: 294 | | 262 | 2 | EI+:278 | | 299 | 3 | EI+: 302 |
| 226 | 3 | FAB-: 279 | | 263 | 3 | FAB-:263 | | 300 | 3 | FAB+: 305 |
| 227 | 2 | ESI+: 281 | | 264 | 2 | EI+303 | | 301 | 2 | ESI+: 333 |
| 228 | 2 | ESI+: 301 | | 265 | 3 | FAB-:288 | | 302 | 2 | ESI+: 319 |
| 229 | 2 | EI+: 318 | | 266 | 2 | EI+:305 | | 303 | 2 | EI+: 296 |
| 230 | 3 | FAB-: 303 | | 267 | 2 | EI+:334 | | 304 | 2 | EI+: 296 |
| 231 | 14 | FAB+: 271 | | 268 | 2 | EI+:310 | | 305 | 2 | EI+: 292 |
| 232 | 14 | FAB+: 273 | | 269 | 2 | EI+:280 | | 306 | 2 | EI+: 294 |
| 233 | 2 | FAB+:311 | | 270 | 3 | EI+:266 | | 307 | 3 | ESI-: 319 |
| 234 | 3 | FAB-:296 | | 271 | 3 | FAB-:295 | | 308 | 3 | ESI-: 303 |
| 235 | 3 | FAB-:281 | | 272 | 3 | FAB-:290 | | 309 | 1 | FAB+:353 |
| 236 | 3 | FAB-:327 | | 273 | 3 | EI+:320 | | 310 | 3 | FAB-:223 |
| 237 | 2 | EI+:292 | | 274 | 3 | FAB-:293 | | 311 | 3 | FAB-: 281 |
| 238 | 2 | EI+:292 | | 275 | 2 | EI+:332 | | 312 | 3 | FAB-: 281 |
| 239 | 28 | EI+:321 | | 276 | 3 | FAB-:317 | | 313 | 3 | FAB-: 277 |
| 240 | 2 | ESI+: 335 | | 277 | 8 | EI+: 259 | | 314 | 3 | FAB-: 279 |
| 241 | 3 | FAB-:269 | | 278 | 2 | ESI+: 335 | | 315 | 5 | EI+: 334 |
| 242 | 2 | EI+:342 | | 279 | 7 | EI+: 281 | | 316 | 3 | FAB-: 319 |
| 243 | 15 | EI+: 252 | | 280 | 2 | ESI+: 321 | | 318 | 2 | ESI+: 297 |
| 244 | 3 | EI+: 238 | | 281 | 2 | EI+: 317 | | 319 | 2 | ESI+: 331 |
| 245 | 5 | EI+: 309 | | 282 | 2 | EI+: 319 | | 320 | 5 | EI+: 336 |
| 246 | 3 | FAB-: 294 | | 283 | 2 | EI+: 319 | | 321 | 5 | EI+: 319 |
| 247 | 5 | EI+: 319 | | 284 | 3 | ESI-: 319 | | 324 | 3 | FAB-: 321 |
| 248 | 3 | FAB+: 306 | | 285 | 3 | ESI+: 321 | | 325 | 3 | FAB-: 304 |
| 249 | 2 | FAB+: 308 | | 286 | 3 | ES-: 305 | | 326 | 2 | ESI+:353 |
| 250 | 15 | FAB-: 253 | | 287 | 2 | EI+: 303 | | 327 | 3 | ESI-:315 |
| 251 | 2 | EI+: 309 | | 288 | 2 | EI+: 306 | | 328 | 3 | ESI-: 337 |
| 252 | 3 | ESI-: 292 | | 289 | 2 | EI+: 316 | | 329 | 2 | ESI+: 293 |
| 253 | 3 | FAB+: 296 | | 290 | 2 | EI+: 318 | | 330 | 2 | EI+:345 |
| 254 | 2 | ESI-: 311 | | 291 | 3 | FAB+: 306 | | 331 | 3 | FAB-:290 |
| 255 | 3 | ESI-: 297 | | 292 | 3 | FAB+: 303 | | 332 | 28 | EI+:321 |
| 256 | 2 | ESI+: 315 | | 293 | 3 | FAB+: 305 | | 333 | 2 | ESI+: 255 |
| 257 | 3 | ESI-: 299 | | 294 | 8 | EI+: 259 | | 334 | 2 | EI+:305 |
| 258 | 7 | EI+: 281 | | 295 | 2 | EI+: 319 | | 335 | 3 | FAB+: 361 |
| 259 | 3 | EI+:252 | | 296 | 3 | FAB-: 288 | | 336 | 1 | EI+:352 |
| 260 | 2 | ESI+:309 | | 297 | 3 | FAB-: 304 | | 337 | 26 | EI+:239 |

[Table 63]

| Prep | PSyn | Dat | | Prep | PSyn | Dat | | Prep | PSyn | Dat |
|---|---|---|---|---|---|---|---|---|---|---|
| 338 | 2 | EI+: 368 | | 375 | 2 | FAB+: 301 | | 413 | 3 | FAB+: 305 |
| 339 | 3 | ESI-: 353 | | 376 | 2 | FAB+: 301 | | 414 | 3 | FAB+: 305 |
| 340 | 2 | ESI+: 369 | | 377 | 2 | EI+: 300 | | 415 | 3 | FAB+: 319 |
| 341 | 2 | ESI+: 335 | | 378 | 2 | FAB+: 301 | | 416 | 20 | EI+: 244 |
| 342 | 2 | ESI+: 335 | | 379 | 5 | ESI+: 267 | | 417 | 2 | EI+: 292 |
| 343 | 3 | ESI+: 321 | | 380 | 5 | ESI+: 266 | | 418 | 2 | EI+: 300 |
| 344 | 3 | ESI+: 321 | | 381 | 5 | ESI+: 272 | | 419 | 2 | EI+: 303 |
| 345 | 3 | ESI+: 355 | | 382 | 3 | EI+: 286 | | 420 | 2 | ESI+: 349 |
| 346 | 2 | ESI+: 271 | | 383 | 3 | EI+: 286 | | 421 | 2 | CI+: 343 |
| 347 | 3 | ESI+: 257 | | 384 | 3 | EI+: 286 | | 422 | 1 | EI+:354 |
| 348 | 5 | EI+: 312 | | 385 | 3 | FAB-: 285 | | 423 | 3 | FAB-: 285 |
| 349 | 2 | EI+: 268 | | 386 | 3 | ESI+: 253 | | 424 | 3 | FAB-: 277 |
| 350 | 3 | ESI+: 255 | | 387 | 3 | ESI+: 253 | | 425 | 3 | FAB+: 290 |
| 351 | 3 | FAB-: 297 | | 388 | 2 | ESI+: 301 | | 426 | 18 | ESI+: 247 |
| 352 | 2 | ESI+: 311 | | 389 | 3 | ESI+: 252 | | 427 | 2 | FAB-: 401 |
| 353 | 2 | ESI+: 269 | | 390 | 3 | ESI+: 258 | | 428 | 2 | EI+: 400 |
| 354 | 3 | APCI+: 255 | | 391 | 2 | FAB+: 317 | | 429 | 2 | EI+: 308 |
| 355 | 27 | EI+: 208 | | 392 | 2 | FAB+: 317 | | 430 | 1 | ESI+: 365 |
| 356 | 2 | EI+: 308 | | 393 | 2 | FAB+: 331 | | 431 | 1 | ESI+: 379 |
| 357 | 2 | EI+: 322 | | 394 | 2 | FAB+: 319 | | 432 | 3 | EI+: 386 |
| 358 | 1 | Ki+: 340 | | 395 | 2 | FAB+: 319 | | 433 | 2 | ESI+: 293 |
| 359 | 5 | ESI+: 267 | | 396 | 2 | FAB+: 333 | | 434 | 2 | ESI+: 307 |
| 360 | 2 | ESI+: 267 | | 397 | 2 | ESI+: 333 | | 435 | 3 | CI: 388 |
| 361 | 2 | EI+: 330 | | 398 | 2 | ESI+: 319 | | 436 | 3 | ESI+: 295 |
| 362 | 2 | EI+: 318 | | 400 | 3 | ESI-: 285 | | 437 | 3 | ESI-: 277 |
| 363 | 2 | EI+: 332 | | 401 | 3 | ESI-: 317 | | 438 | 3 | ESI-: 291 |
| 364 | 2 | EI+: 320 | | 402 | 3 | EI+: 304 | | 439 | 3 | ESI-: 327 |
| 365 | 2 | EI+: 322 | | 403 | 3 | EI+: 316 | | 440 | 5 | EI+: 330 |
| 366 | 3 | FAB-: 293 | | 404 | 2 | ESI+: 313 | | 441 | 3 | ESI-: 315 |
| 367 | 3 | ESI+: 309 | | 405 | 3 | ESI+: 299 | | 442 | 3 | ESI-: 333 |
| 368 | 3 | FAB-: 315 | | 406 | 19 | ESI+: 263 | | 443 | 3 | ESI+: 270 |
| 369 | 3 | FAB+: 305 | | 407 | 2 | EI+: 333 | | 444 | 5 | ESI+: 284 |
| 370 | 3 | FAB+: 318 | | 408 | 3 | FAB-:319 | | 445 | 3 | ESI+: 270 |
| 371 | 3 | FAB+: 307 | | 409 | 14 | FAB-:225 | | 446 | 2 | ESI+: 296 |
| 372 | 3 | ESI-: 327 | | 410 | 3 | FAB+: 303 | | 447 | 3 | ESI+: 282 |
| 373 | 3 | FAB-: 307 | | 411 | 3 | FAB-: 301 | | 448 | 5 | ESI+: 280 |
| 374 | 3 | ESI+: 253 | | 412 | 3 | FAB+: 317 | | 449 | 3 | ESI+: 266 |

[Table 64]

| Prep | PSyn | Dat |
|---|---|---|
| 450 | 3 | FAB-:319 |
| 451 | 3 | FAB-:292 |
| 452 | 3 | FAB-:321 |
| 453 | 3 | FAB-:285 |
| 454 | 3 | FAB-:292 |
| 455 | 3 | FAB-:303 |
| 456 | 3 | FAB-:290 |
| 457 | 3 | FAB-:299 |
| 458 | 3 | FAB-:297 |
| 459 | 2 | EI+:312 |
| 460 | 3 | FAB-:297 |
| 461 | 2 | EI+:334 |
| 462 | 2 | EI+:318 |
| 463 | 2 | EI+:300 |
| 464 | 2 | EI+:307 |
| 465 | 2 | EI+:336 |
| 466 | 2 | EI+:314 |
| 467 | 2 | EI+:312 |
| 468 | 2 | EI+:307 |
| 469 | 5 | APCI+: 316 |
| 470 | 3 | ESI+: 302 |
| 471 | 5 | ESI+: 316 |
| 472 | 3 | ESI+: 302 |
| 473 | 2 | EI+: 309 |
| 474 | 1 | FAB+: 357 |
| 475 | 25 | EI+: 224 |
| 476 | 12 | EI+: 242 |
| 477 | 11 | EI+ 224 |
| 478 | 2 | EI+: 338 |
| 479 | 3 | FAB-: 323 |
| 480 | 3 | FAB-: 299 |
| 481 | 2 | EI+: 314 |
| 482 | 3 | FAB-: 294 |
| 483 | 3 | FAB-: 269 |
| 484 | 2 | EI+: 284 |

[Table 65]

| Prep | PSyn | Dat | | Prep | PSyn | Dat | | Prep | PSyn | Dat |
|---|---|---|---|---|---|---|---|---|---|---|
| 487 | 487 | EI+: 370 | | 524 | 3 | EI+: 298 | | 561 | 3 | EI+: 298 |
| 488 | 488 | EI+: 236 | | 525 | 3 | EI+: 320 | | 562 | 3 | FAB+: 301 |
| 489 | 489 | EI+: 314, 316 | | 526 | 3 | FAB-:292 | | 563 | 2 | ESI+: 323 |
| 490 | 490 | EI+: 298, 300 | | 527 | 3 | EI+: 316 | | 565 | 5 | ESI+: 280 |
| 491 | 491 | ESI+: 295, 297 | | 528 | 3 | EI+: 296 | | 566 | 5 | ESI+: 300 |
| 492 | 492 | EI+: 328 | | 529 | 3 | EI+: 282 | | 567 | 3 | ESI+: 286 |
| 493 | 493 | ESI+: 284 | | 530 | 2 | EI+: 314 | | 568 | 2 | EI+: 312 |
| 494 | 494 | ESI+: 282 | | 531 | 2 | EI+: 314 | | 569 | 2 | EI+: 328 |
| 495 | 495 | EI+: 240 | | 532 | 2 | EI+: 336 | | 570 | 2 | EI+: 316 |
| 496 | 496 | ESI-: 253 | | 533 | 2 | EI+: 330 | | 571 | 2 | EI+: 318 |
| 497 | 497 | ESI+: 249 | | 534 | 2 | EI+: 332 | | 572 | 5 | ESI+: 300 |
| 498 | 498 | CI+: 252 | | 535 | 3 | EI+: 300 | | 573 | 3 | ESI+: 286 |
| 499 | 499 | ESI+: 192 | | 536 | 3 | EI+: 300 | | 574 | 3 | ESI+: 266 |
| 500 | 500 | EI+: 241 | | 537 | 3 | EI+: 322 | | 575 | 5 | ESI+: 284 |
| 501 | 2 | ESI+: 266 | | 538 | 3 | EI+: 316 | | 576 | 3 | ESI+: 270 |
| 502 | 5 | EI+: 323 | | 539 | 3 | EI+: 318 | | 577 | 3 | EI+: 298 |
| 503 | 11 | FAB+: 357 | | 540 | 2 | EI+: 293 | | 578 | 3 | EI+: 314 |
| 504 | 496 | EI+: 266 | | 541 | 2 | FAB+: 334 | | 579 | 3 | EI+: 302 |
| 505 | 13 | ESI+: 249 | | 542 | 2 | EI+: 332 | | 580 | 3 | EI+: 304 |
| 506 | 1 | ESI+: 381 | | 543 | 2 | EI+: 348 | | 581 | 2 | FAB+: 313 |
| 507 | 29 | ESI+: 282 | | 544 | 2 | EI+: 292 | | 582 | 2 | FAB+: 313 |
| 508 | 2 | EI+: 308 | | 545 | 5 | ESI+: 280 | | 583 | 2 | FAB+: 317 |
| 509 | 3 | ESI+: 268 | | 546 | 3 | ESI+: 266 | | 584 | 2 | FAB+: 319 |
| 510 | 3 | FAB-: 293 | | 547 | 2 | ESI+: 296 | | 585 | 5 | EI+: 328 |
| 511 | 2 | EI+: 312 | | 548 | 3 | ESI+: 282 | | 586 | 3 | FAB-: 297 |
| 512 | 2 | EI+: 303 | | 549 | 2 | EI+: 334 | | 587 | 3 | FAB-: 297 |
| 513 | 2 | EI+: 292 | | 550 | 2 | EI+: 312 | | 588 | 3 | FAB-: 301 |
| 514 | 2 | EI+: 312 | | 551 | 2 | EI+: 336 | | 589 | 3 | FAB+: 305 |
| 515 | 2 | EI+: 312 | | 552 | 2 | EI+: 314 | | 590 | 3 | FAB-: 313 |
| 516 | 2 | EI+: 334 | | 553 | 3 | ESI+: 280 | | 591 | 2 | EI+: 310 |
| 517 | 2 | FAB+: 331 | | 554 | 3 | EI+: 319 | | 592 | 2 | EI+: 306 |
| 518 | 2 | FAB+: 310 | | 555 | 3 | EI+: 318 | | 593 | 3 | ESI-: 295 |
| 519 | 2 | FAB+: 296 | | 556 | 3 | FAB-: 331 | | 594 | 3 | EI+: 292 |
| 520 | 3 | FAB-: 297 | | 557 | 3 | EI+: 278 | | 595 | 2 | EI+: 362 |
| 521 | 3 | FAB+: 290 | | 558 | 6 | ESI+: 229 | | 596 | 2 | EI+: 364 |
| 522 | 3 | EI+: 278 | | 559 | 3 | ESI-: 213 | | 597 | 2 | EI+: 322 |
| 523 | 3 | EI+: 298 | | 560 | 3 | FAB-: 319 | | 598 | 2 | EI+: 314 |

[Table 66]

| Prep | PSyn | Dat | | Prep | PSyn | Dat | | Prep | PSyn | Dat |
|---|---|---|---|---|---|---|---|---|---|---|
| 599 | 2 | EI+: 344 | | 636 | 3 | ESI+: 312 | | 673 | 3 | FAB-: 271 |
| 600 | 3 | FAB+: 295 | | 637 | 3 | ESI+: 295 | | 674 | 5 | EI+: 307 |
| 601 | 3 | EI+: 348 | | 638 | 2 | EI+: 307 | | 675 | 2 | EI+: 319 |
| 602 | 3 | FAB+: 350 | | 639 | 5 | ESI+: 305 | | 676 | 5 | EI+: 330 |
| 603 | 3 | ESI+: 309 | | 640 | 3 | APCI-: 289 | | 677 | 3 | FAB-: 315 |
| 604 | 3 | EI+: 300 | | 641 | 3 | FAB-: 283 | | 678 | 3 | FAB-: 292 |
| 605 | 3 | FAB+: 330 | | 642 | 3 | EI+: 316 | | 679 | 3 | FAB-:304 |
| 606 | 2 | EI+: 337 | | 643 | 2 | EI+: 330 | | 680 | 492 | EI+: 316 |
| 607 | 3 | ESI-: 322 | | 644 | 2 | EI+: 348 | | 681 | 11 | EI+: 330 |
| 608 | 2 | EI+: 298 | | 645 | 3 | FAB-: 333 | | 682 | 5 | EI+: 307 |
| 609 | 2 | EI+: 298 | | 646 | 492 | EI+: 328 | | 683 | 5 | EI+: 307 |
| 610 | 2 | EI+: 316 | | 647 | 2 | EI+: 312 | | 684 | 3 | ESI+: 303 |
| 611 | 5 | ESI+: 272 | | 648 | 3 | EI+: 298 | | 685 | 3 | FAB-: 292 |
| 612 | 3 | ESI+: 258 | | 649 | 2 | EI+: 330 | | 686 | 3 | FAB-: 292 |
| 613 | 3 | ESI+: 270 | | 650 | 3 | EI+: 316 | | 687 | 27 | ESI+: 261 |
| 614 | 5 | EI+: 316 | | 651 | 3 | EI+: 296 | | 688 | 1 | EI+: 352 |
| 615 | 5 | EI+: 316 | | 652 | 2 | EI+: 310 | | 689 | 1 | EI+: 352 |
| 616 | 27 | FAB+: 221 | | 653 | 3 | EI+: 284 | | 690 | 13 | FAB+: 221 |
| 617 | 488 | EI+: 384 | | 654 | 3 | CI+: 303 | | 691 | 492 | EI+: 316 |
| 618 | 5 | EI+: 318 | | 655 | 2 | EI+: 319 | | 692 | 492 | EI+: 284 |
| 619 | 5 | EI+: 318 | | 656 | 3 | EI+: 305 | | 693 | 492 | EI+: 269 |
| 620 | 3 | FAB-: 301 | | 657 | 2 | EI+: 312 | | 694 | 1 | ESI+:393 |
| 621 | 3 | FAB-: 301 | | 658 | 3 | EI+: 298 | | 695 | 2 | ESI+: 375 |
| 622 | 7 | FAB+: 353 | | 659 | 492 | EI+: 316 | | 696 | 3 | ESI+: 361 |
| 623 | 3 | FAB-: 303 | | 660 | 492 | EI+: 316 | | 697 | 3 | FAB-: 301 |
| 624 | 3 | FAB-: 303 | | 661 | 2 | ESI+: 300 | | 698 | 492 | EI+: 304 |
| 625 | 2 | EI+: 334 | | 662 | 3 | ESI+: 286 | | 699 | 3 | ESI-: 269 |
| 626 | 3 | ESI-: 319 | | 663 | 3 | ESI+: 315 | | 701 | 11 | EI+: 220 |
| 627 | 5 | EI+: 307 | | 664 | 3 | ESI+: 315 | | 702 | 12 | EI+: 238 |
| 628 | 5 | EI+: 307 | | 665 | 3 | ESI-: 301 | | 703 | 13 | FAB+: 221 |
| 629 | 3 | FAB+: 294 | | 666 | 3 | EI+: 302 | | 704 | 11 | EI+: 220 |
| 630 | 3 | FAB+: 294 | | 667 | 3 | FAB+: 323 | | 705 | 12 | EI+: 238 |
| 631 | 5 | ESI+: 297 | | 668 | 492 | EI+: 298 | | 707 | 3 | EI+: 318 |
| 632 | 3 | ESI+: 283 | | 669 | 492 | EI+: 286 | | 708 | 3 | EI+: 282 |
| 633 | 5 | ESI+: 300 | | 670 | 492 | EI+: 286 | | 709 | 2 | EI+: 296 |
| 634 | 3 | ESI+: 286 | | 671 | 3 | FAB-: 283 | | 710 | 1 | EI+: 368 |
| 635 | 2 | APCI+: 326 | | 672 | 3 | FAB-: 271 | | 711 | 18 | ESI+:323 |

[Table 67]

| Prep | PSyn | Dat | Prep | PSyn | Dat | Prep | PSyn | Dat |
|---|---|---|---|---|---|---|---|---|
| 712 | 3 | ESI+: 309 | 749 | 5 | EI+: 299 | 786 | 3 | ESI-: 317 |
| 713 | 2 | EI+: 350 | 750 | 2 | ESI+: 309 | 787 | 2 | EI+: 334 |
| 714 | 3 | EI+: 336 | 751 | 3 | ESI-: 293 | 788 | 2 | EI+: 350 |
| 715 | 2 | ESI+: 335 | 752 | 5 | EI+: 333 | 789 | 3 | FAB+: 321 |
| 716 | 3 | ESI-: 319 | 753 | 3 | ESI+: 286 | 790 | 3 | FAB-: 335 |
| 717 | 3 | EI+: 334 | 754 | 3 | ESI+: 320 | 791 | 2 | EI+: 346 |
| 718 | 2 | EI+: 348 | 755 | 492 | EI+: 293 | 792 | 2 | EI+: 348 |
| 719 | 3 | EI+: 318 | 756 | 489 | EI+: 300, 302 | 793 | 2 | EI+: 348 |
| 720 | 2 | EI+: 332 | 757 | 3 | FAB-: 278 | 794 | 2 | EI+: 348 |
| 721 | 3 | EI+: 334 | 758 | 492 | EI+: 323 | 795 | 2 | EI+: 316 |
| 722 | 2 | EI+: 348 | 759 | 3 | ESI-: 308 | 796 | 499 | ESI+: 192 |
| 723 | 3 | EI+: 318 | 760 | 2 | ESI+: 317 | 797 | 3 | FAB+: 333 |
| 724 | 2 | EI+: 332 | 761 | 2 | ESI+: 316 | 798 | 3 | FAB-: 333 |
| 725 | 2 | EI+: 332 | 762 | 2 | ESI+: 282 | 799 | 3 | FAB-: 333 |
| 726 | 3 | FAB-: 319 | 763 | 3 | ESI-: 301 | 800 | 3 | FAB-: 333 |
| 727 | 2 | EI+: 334 | 764 | 3 | ESI-: 300 | 801 | 3 | FAB+: 303 |
| 728 | 492 | EI+: 312 | 765 | 3 | ESI-: 266 | 802 | 2 | EI+: 328 |
| 729 | 3 | FAB+: 256 | 766 | 2 | EI+: 316 | 803 | 2 | EI+: 348 |
| 730 | 3 | FAB-: 308 | 767 | 2 | EI+: 316 | 804 | 2 | EI+: 328 |
| 731 | 3 | FAB-: 297 | 768 | 2 | EI+: 332 | 805 | 2 | ESI+: 323 |
| 732 | 492 | EI+: 300 | 769 | 2 | EI+: 294 | 806 | 2 | ESI+: 337 |
| 733 | 2 | ESI+: 321 | 770 | 3 | EI+: 302 | 807 | 2 | ESI+: 350 |
| 734 | 2 | ESI+: 357 | 771 | 3 | EI+: 302 | 808 | 2 | ESI+: 350 |
| 735 | 2 | ESI+: 373 | 772 | 3 | FAB+: 319 | 809 | 17 | ESI+: 296 |
| 736 | 3 | ESI-: 305 | 773 | 3 | EI+: 280 | 810 | 3 | ESI+: 315 |
| 737 | 3 | ESI-: 341 | 774 | 5 | ESI+: 302 | 811 | 3 | FAB+: 335 |
| 738 | 3 | ESI-: 357 | 775 | 3 | ESI+: 288 | 812 | 3 | ESI+: 315 |
| 739 | 3 | FAB+: 287 | 776 | 4 | ESI+: 263 | 813 | 3 | ESI-: 307 |
| 740 | 492 | EI+: 342 | 777 | 2 | ESI+: 283 | 814 | 3 | ESI-: 323 |
| 741 | 3 | FAB-: 327 | 778 | 2 | ESI+: 300 | 815 | 3 | ESI-: 334 |
| 742 | 492 | FAB+: 270 | 779 | 2 | ESI+: 300 | 816 | 3 | ESI-: 334 |
| 743 | 3 | FAB+: 256 | 780 | 2 | ESI+: 315 | 817 | 4 | EI+: 330 |
| 744 | 2 | ESI+: 347 | 781 | 2 | ESI+: 333 | 818 | 2 | ESI+: 329 |
| 745 | 3 | ESI-: 331 | 782 | 3 | ESI+: 268 | 819 | 2 | ESI+: 329 |
| 746 | 18 | ESI+: 349 | 783 | 3 | ESI-: 284 | 820 | 2 | ESI+: 329 |
| 747 | 3 | ESI+: 335 | 784 | 3 | ESI-: 284 | 821 | 2 | ESI+: 345 |
| 748 | 1 | ESI+: 381 | 785 | 3 | ESI-: 299 | 822 | 3 | ESI+: 282 |

[Table 68]

| Prep | PSyn | Dat |
|---|---|---|
| 823 | 494 | ESI+: 282 |
| 824 | 17 | ESI+: 296 |
| 825 | 3 | ESI+: 282 |
| 826 | 5 | EI+: 346 |
| 827 | 5 | EI+: 317 |
| 828 | 3 | ESI+: 304 |
| 829 | 2 | EI+: 328 |
| 830 | 2 | EI+: 335 |
| 831 | 3 | ESI-: 313 |
| 832 | 3 | ESI-: 313 |
| 833 | 3 | ESI-: 313 |
| 834 | 3 | ESI-: 329 |
| 835 | 2 | A/E+: 306 |
| 836 | 5 | ESI+: 324 |
| 837 | 5 | A/E+: 324 |
| 838 | 5 | ESI+: 324 |
| 839 | 5 | ESI+: 324 |
| 840 | 5 | ESI+: 316 |
| 841 | 3 | ESI-: 290 |
| 842 | 3 | ESI-: 308 |
| 843 | 3 | ESI-: 308 |
| 844 | 3 | ESI-: 308 |
| 845 | 3 | ESI-: 308 |
| 846 | 3 | ESI-: 300 |
| 847 | 3 | ESI+: 333 |
| 848 | 3 | ESI+: 315 |
| 849 | 3 | ESI+: 322 |
| 850 | 5 | ESI+: 287 |
| 851 | 5 | ESI+: 287 |
| 852 | 3 | ESI-: 271 |
| 853 | 3 | ESI-: 271 |

[Table 69]

| Prep | PSyn | Dat (NMR) |
|---|---|---|
| 19 | 19 | $CDCl_3$: 1.30 (3H, d, J = 6.8 Hz), 1.83-1.92 (0.7H, m), 2.01-2.12 (0.7H, m), 2.19-2.30 (1H, m), 2.46-2.53 (0.7H, m), 2.62-2.68 (0.3H, m), 3.06-3.13 (2H, m), 3.93 (3H, s), 4.15-4.23 (0.7H, m), 4.40-4.46 (0.3H, m), 7.33-7.37 (1H, m), 8.12-8.17 (1H, m), 8.66-8.69 (1H, m) |
| 317 | 2 | $CDCl_3$: 1.68 (3H, s), 2.35-2.48 (2H, m), 2.87-2.96 (2H, m), 3.68 (3H, s), 3.77 (3H, s), 6.69-6.74 (2H, m), 6.96-7.01 (1H, m), 7.15-7.19 (2H, m), 7.71-7.74 (1H, m) |
| 322 | 3 | DMSO-$d^6$: 1.63 (3H, s), 2.35-2.39 (2H, m), 2.86-2.90 (2H, m), 3.68 (3H, s), 6.83-6.88 (1H, m), 6.99-7.06 (3H, m), 7.24-7.27 (1H, m), 7.62-7.65 (1H, m) |
| 323 | 3 | DMSO-$d^6$: 1.70 (3H, s), 2.40-2.44 (2H, m), 2.90-2.94 (2H, m), 7.05 (1H, s), 7.20-7.24 (1H, m), 7.29-7.36 (2H, m), 7.45-7.51 (1H, m), 7.67-7.69 (1H, m) |
| 399 | 2 | $CDCl_3$: 2.39-2.46 (2H, m), 2.88-2.94 (2H, m), 3.62 (3H, s), 3.81 (3H, s), 6.03-6.06 (1H, m), 6.73-6.79 (1H, m), 7.00-7.06 (1H, m), 7.19-7.22 (1H, m), 7.32-7.34 (1H, m), 7.79-7.82 (1H, m) |
| 485 | 485 | $CDCl_3$: 2.56-2.64 (2H, m), 2.83-2.91 (2H, m), 3.84 (3H, s), 3.93 (3H, s), 7.24 (1H, s), 7.97-8.03 (1H, m), 8.43-8.47 (1H, m), 12.38 (1H, brs) |
| 486 | 486 | $CDCl_3$: 2.68-2.78 (2H, m), 2.92-3.01 (2H, m), 5.24 (2H, s), 7.09-7.20 (2H, m), 7.24-7.48 (10H, m), 7.89-7.95 (1H, m) |
| 564 | 1 | $CDCl_3$: 2.80-2.88 (2H, m), 2.91-2.98 (2H, m), 3.89 (3H, s), 3.94 (3H, s), 7.31 (1H, d, J = 8.4 Hz), 8.01-8.05 (1H, m), 8.19 (1H, s) |

[Tables 70]

| Ex | Sal | Str | | Ex | Sal | Str |
|---|---|---|---|---|---|---|
| 1-01 | HCl | 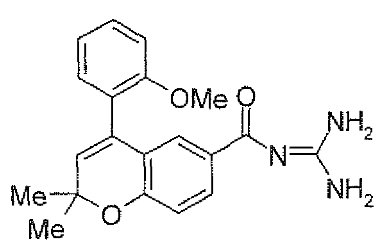 | | 1-08 | HCl | 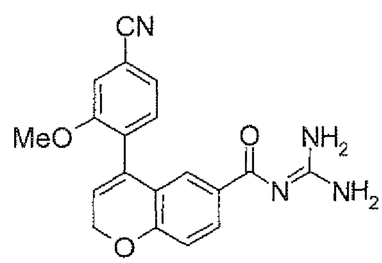 |
| 1-02 | | 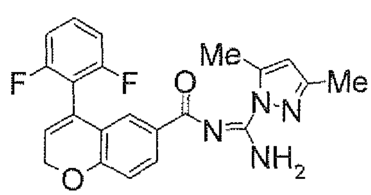 | | 1-09 | HCl | 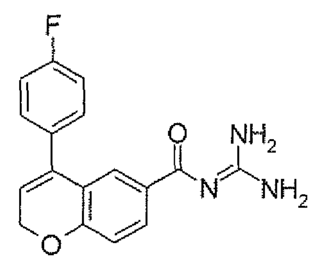 |
| 1-03 | HCl | 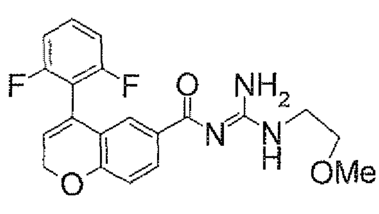 | | 1-10 | HCl | 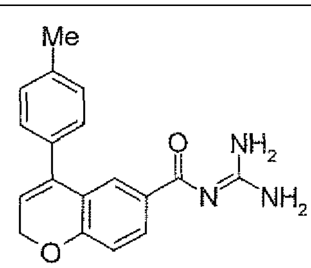 |

(continued)

| Ex | Sal | Str | | Ex | Sal | Str |
|---|---|---|---|---|---|---|
| 1-04 | HCl | 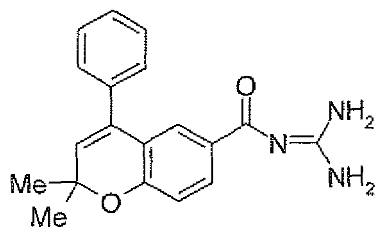 | | 1-11 | HCl | 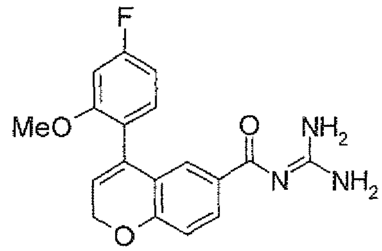 |
| 1-05 | HCl | 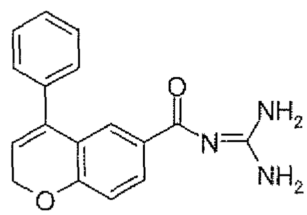 | | 1-12 | HCl | 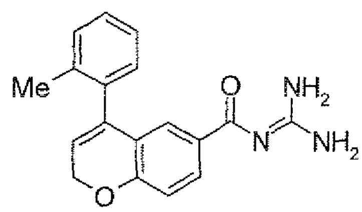 |
| 1-06 | HCl | 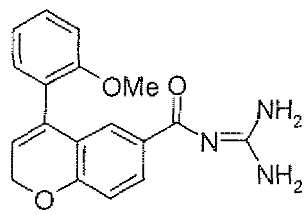 | | | | |
| 1-07 | HCl | 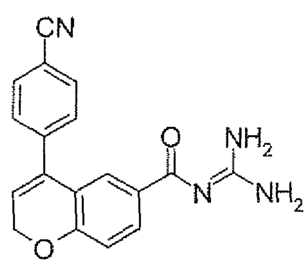 | | 1-13 | HCl | 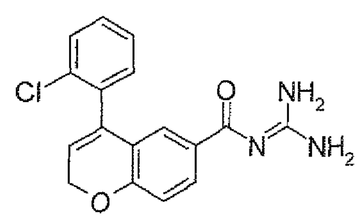 |

[Table 71]

| Ex | Sal | Str | | Ex | Sal | Str |
|---|---|---|---|---|---|---|
| 1-14 | HCl | 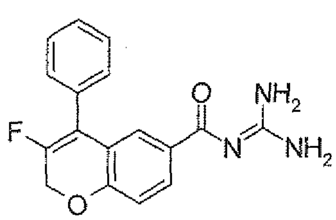 | | 1-20 | HCl | 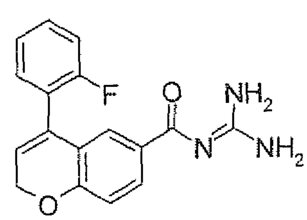 |

(continued)

| Ex | Sal | Str |
|---|---|---|
| 1-15 | HCl | 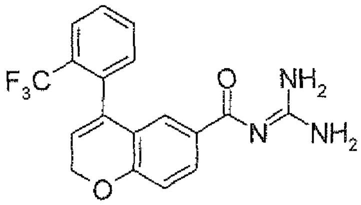 |
| 1-16 | HCl | 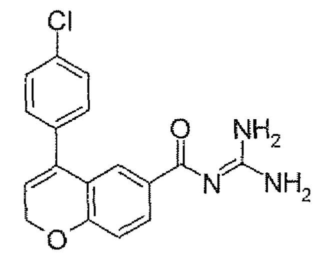 |
| 1-17 | HCl | 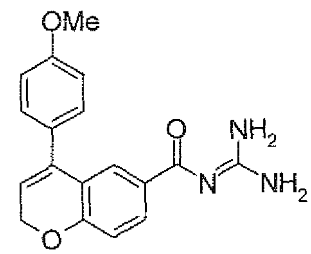 |
| 1-18 | HCl | 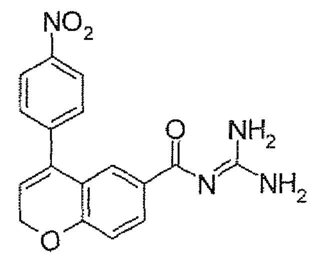 |
| 1-19 | HCl | 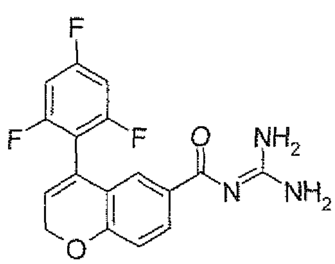 |
| 1-21 | HCl | 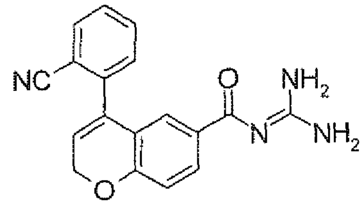 |
| 1-22 | HCl | 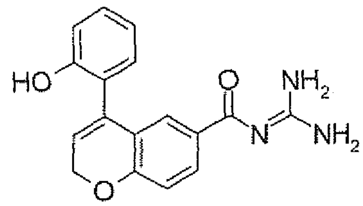 |
| 1-23 | HCl | 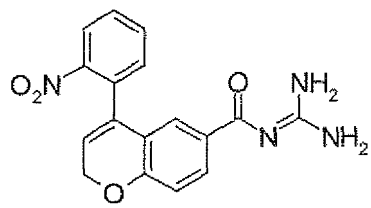 |
| 1-24 | HCl | 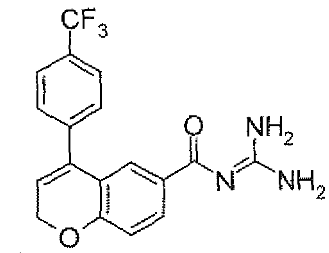 |
| 1-25 | HCl | 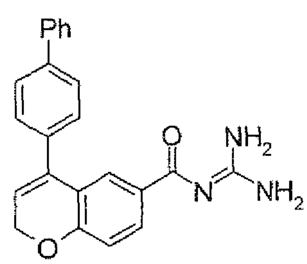 |
| 1-26 | HCl | 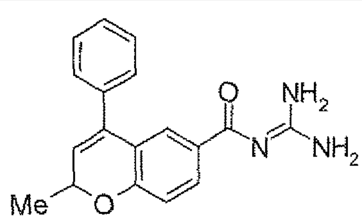 |

[Table 72]

| Ex | Sal | Str | Ex | Sal | Str |
|---|---|---|---|---|---|
| 1-27 | HCl | 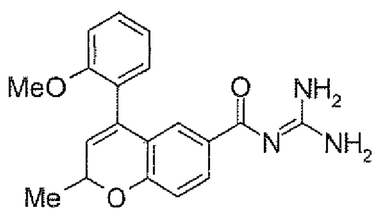 | 1-34 | HCl | 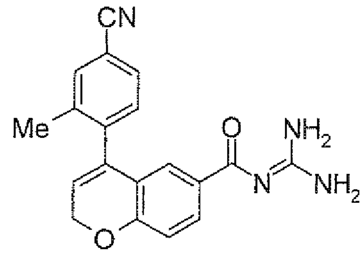 |
| 1-28 | HCl | 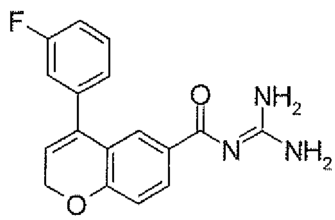 | 1-35 | HCl | 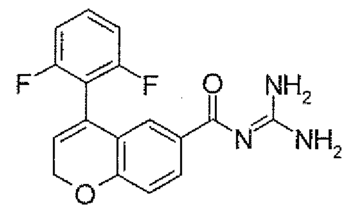 |
| 1-29 | HCl | 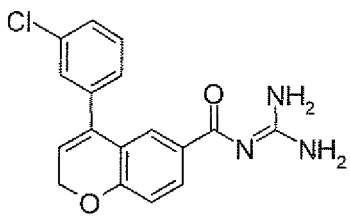 | 1-36 | HCl | 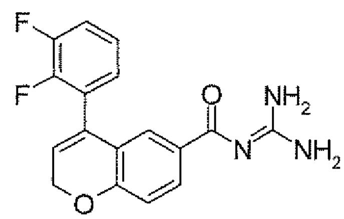 |
| 1-30 | HCl | 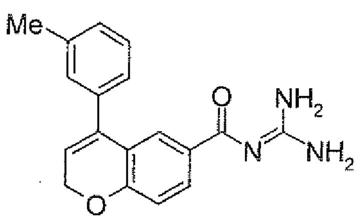 | 1-37 | HCl | 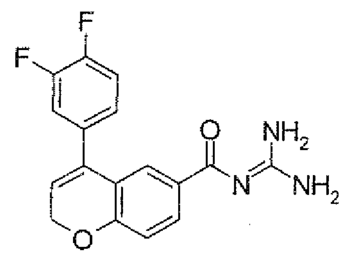 |
| 1-31 | HCl | 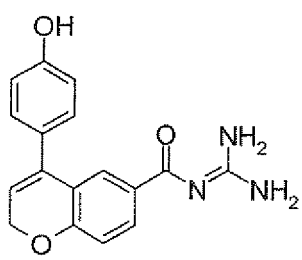 | 1-38 | HCl | 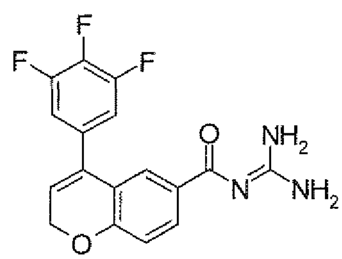 |
| 1-32 | HCl | 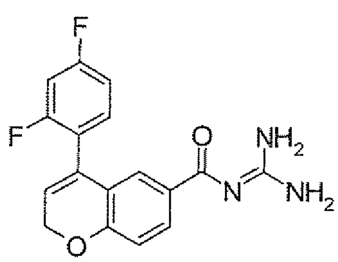 | 1-39 | HCl | 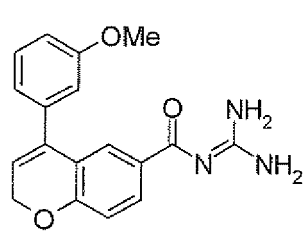 |

(continued)

| Ex | Sal | Str | | Ex | Sal | Str |
|---|---|---|---|---|---|---|
| 1-33 | HCl | 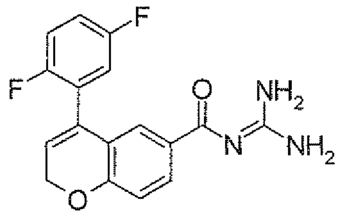 | | 1-40 | HCl | 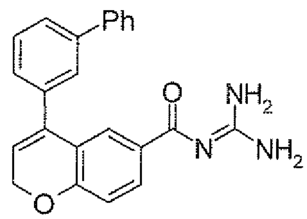 |

[Table 73]

| Ex | Sal | Str | | Ex | Sal | Str |
|---|---|---|---|---|---|---|
| 1-41 | HCl | 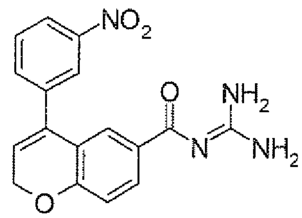 | | 1-48 | HCl | 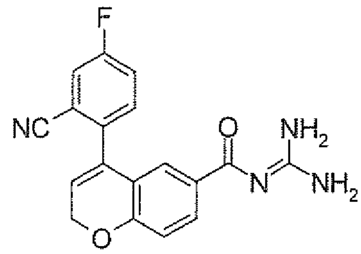 |
| 1-42 | HCl | 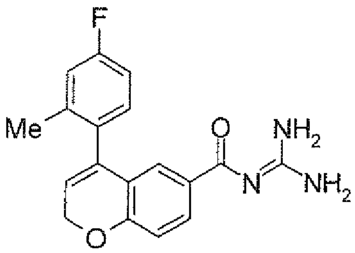 | | 1-49 | HCl | 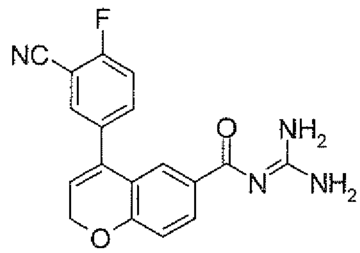 |
| 1-43 | HCl | 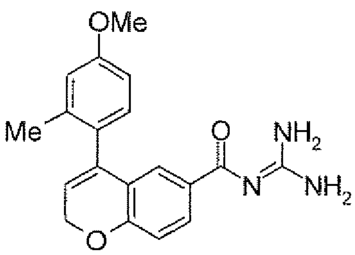 | | 1-50 | HCl | 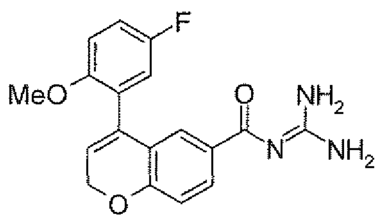 |
| 1-44 | HCl | 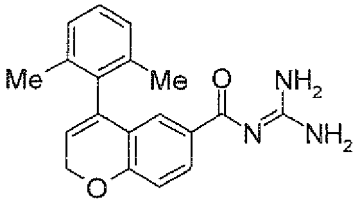 | | 1-51 | HCl | 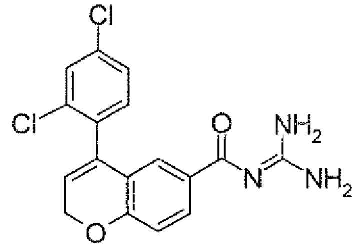 |

(continued)

| Ex | Sal | Str | | Ex | Sal | Str |
|---|---|---|---|---|---|---|
| 1-45 | HCl | 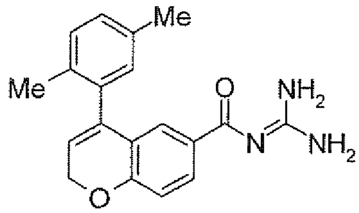 | | 1-52 | HCl | 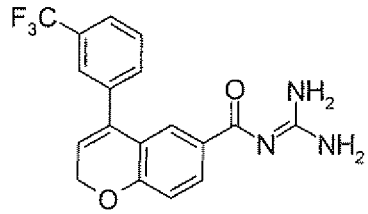 |
| 1-46 | HCl | 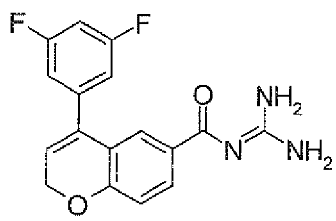 | | 1-53 | HCl | 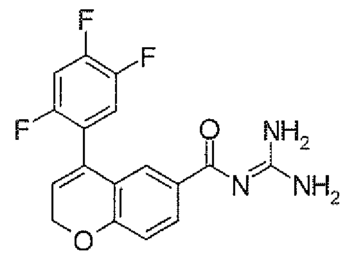 |
| 1-47 | HCl | 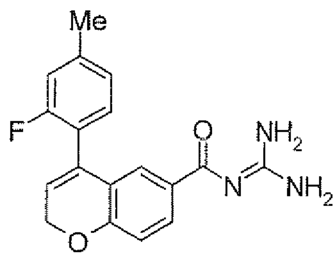 | | | | |

[Table 74]

| Ex | Sal | Str | | Ex | Sal | Str |
|---|---|---|---|---|---|---|
| 1-54 | HCl | 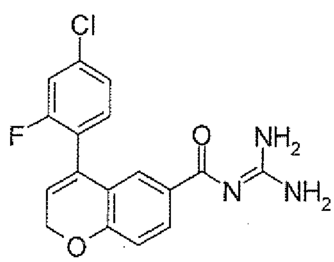 | | 1-60 | HCl | 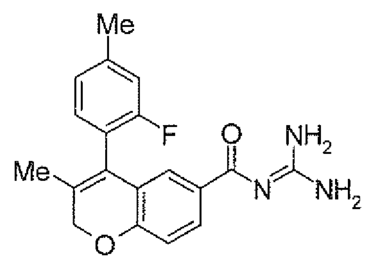 |
| 1-55 | HCl | 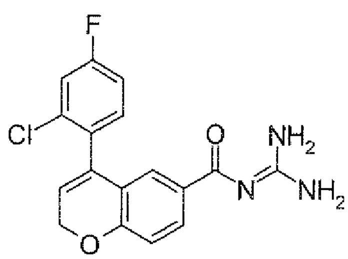 | | 1-61 | HCl | 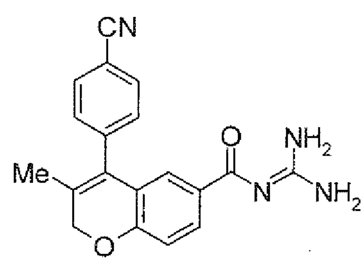 |

(continued)

| Ex | Sal | Str | Ex | Sal | Str |
|---|---|---|---|---|---|
| 1-56 | HCl | 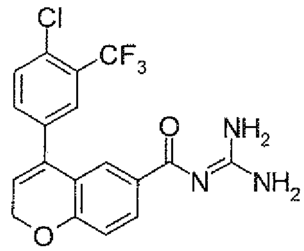 | 1-62 | HCl | 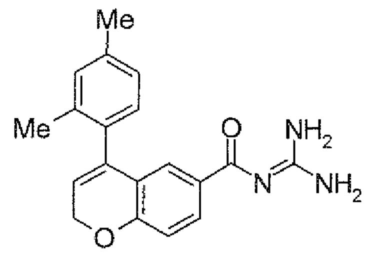 |
| 1-57 | HCl | 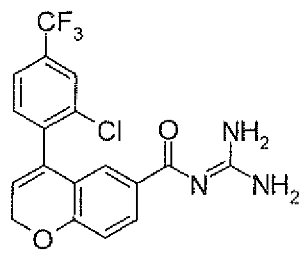 | 1-63 | HCl | 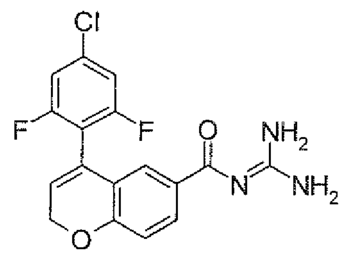 |
| 1-58 | HCl | 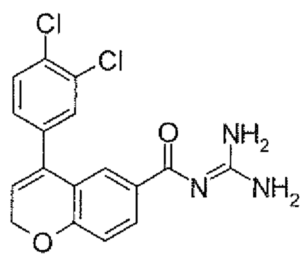 | 1-64 | HCl | 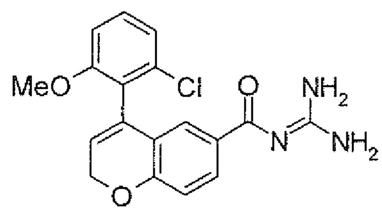 |
| 1-59 | HCl | 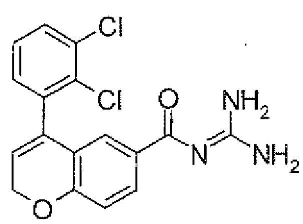 | 1-65 | HCl | 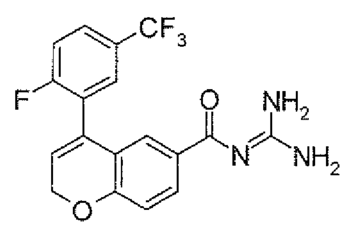 |

[Table 75]

| Ex | Sal | Str | | Ex | Sal | Str |
|---|---|---|---|---|---|---|
| 1-66 | HCl | 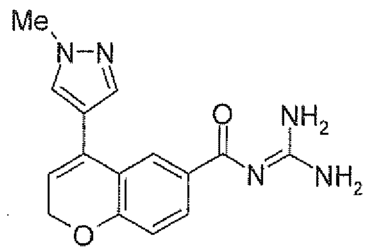 | | 1-72 | HCl | 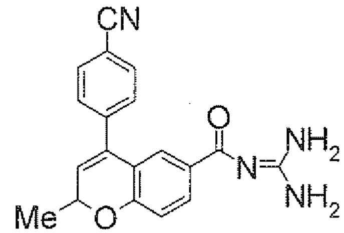 |
| 1-67 | HCl | 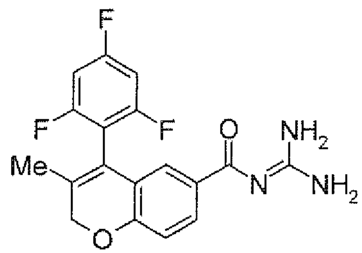 | | 1-73 | HCl | 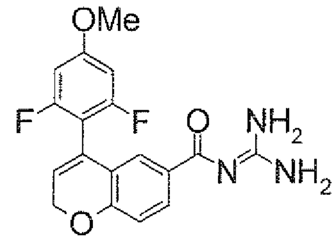 |
| 1-68 | HCl | 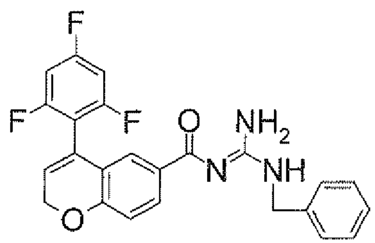 | | 1-74 | HCl | 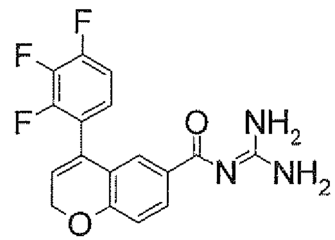 |
| 1-69 | HCl | 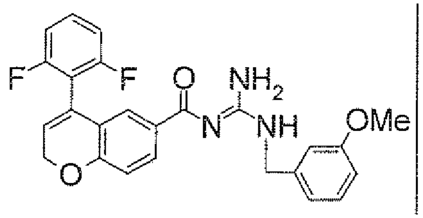 | | 1-75 | HCl | 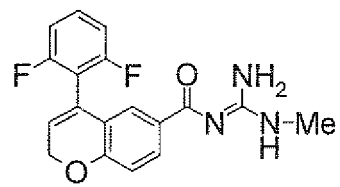 |

(continued)

| Ex | Sal | Str | | Ex | Sal | Str |
|---|---|---|---|---|---|---|
| 1-70 | HCl | 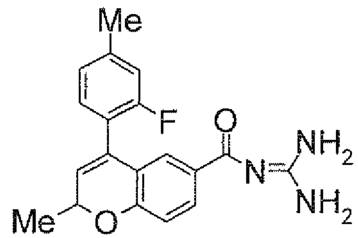 | | 1-76 | HCl | 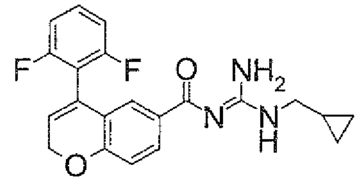 |
| 1-71 | HCl | 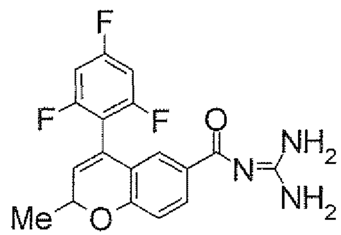 | | 1-77 | HCl | 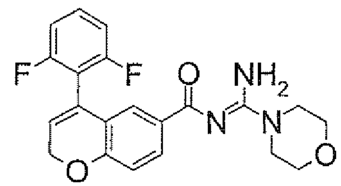 |
| | | | | 1-78 | 2HCl | 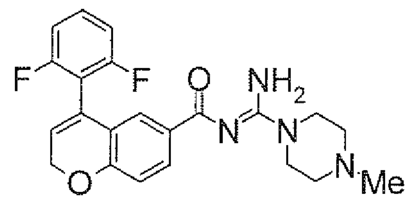 |

[Table 76]

| Ex | Sal | Str | | Ex | Sal | Str |
|---|---|---|---|---|---|---|
| 1-79 | HCl | 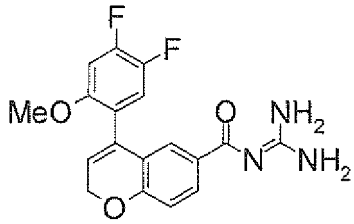 | | 1-85 | HCl | 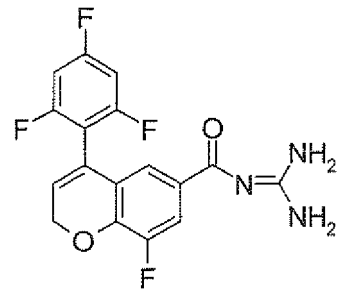 |
| 1-80 | HCl | 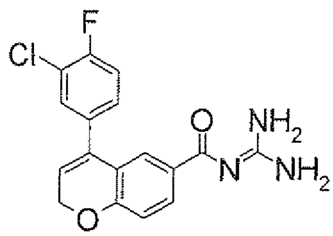 | | 1-86 | HCl | 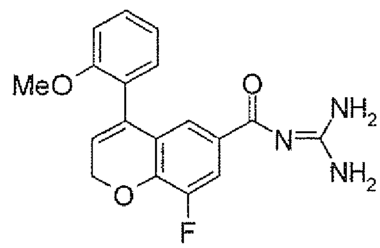 |
| 1-81 | HCl | 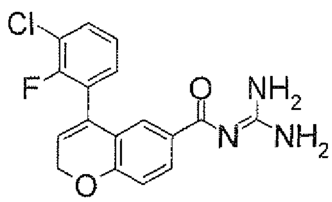 | | 1-87 | HCl | 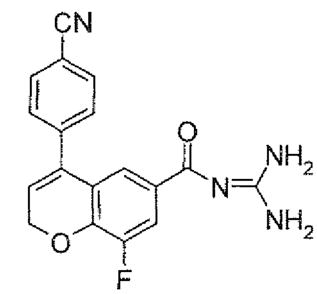 |
| 1-82 | HCl | 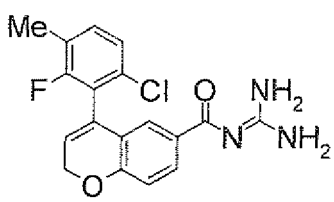 | | 1-88 | HCl | 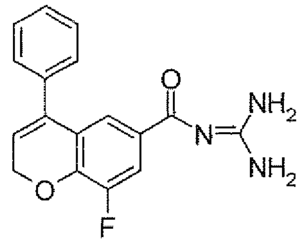 |
| 1-83 | HCl | 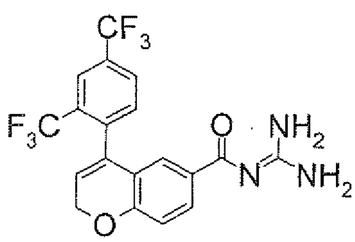 | | 1-89 | HCl | 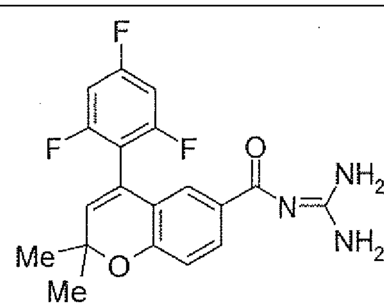 |

(continued)

| Ex | Sal | Str | | Ex | Sal | Str |
|---|---|---|---|---|---|---|
| 1-84 | | 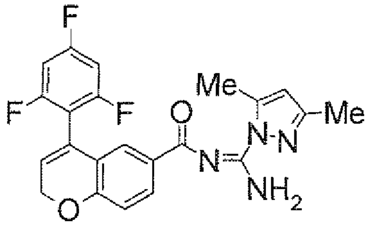 | | 1-90 | HCl | 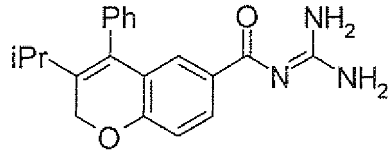 |

[Table 77]

| Ex | Sal | Str | | Ex | Sal | Str |
|---|---|---|---|---|---|---|
| 1-91 | HCl | 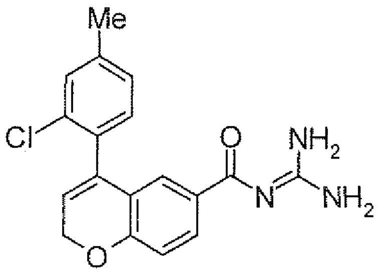 | | 1-97 | HCl | 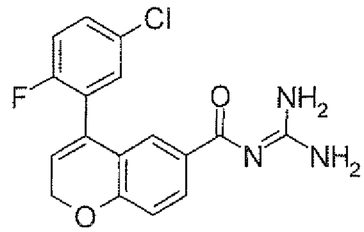 |
| 1-92 | HCl | 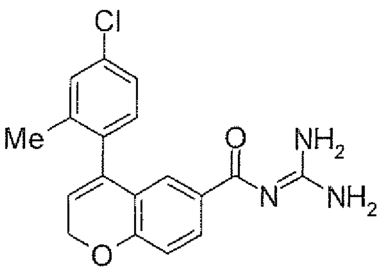 | | 1-98 | HCl | 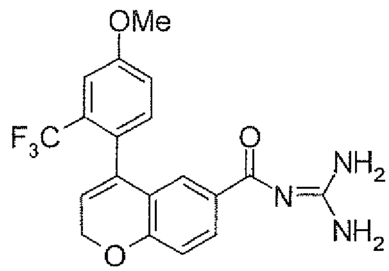 |
| 1-93 | HCl | 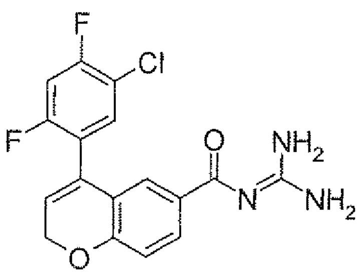 | | 1-99 | HCl | 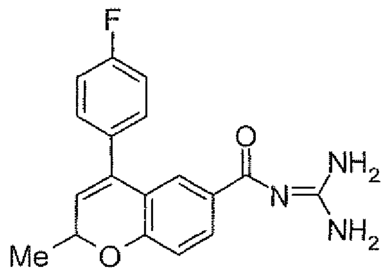 |
| 1-94 | HCl | 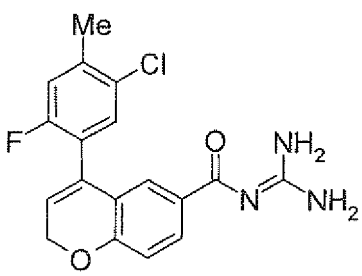 | | 1-100 | HCl | 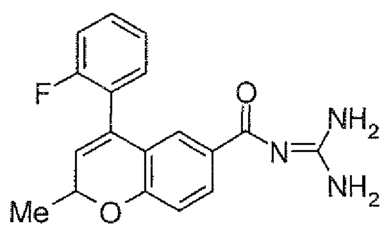 |

(continued)

| Ex | Sal | Str | | Ex | Sal | Str |
|---|---|---|---|---|---|---|
| 1-95 | HCl | 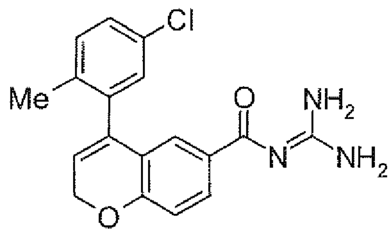 | | 1-101 | HCl | 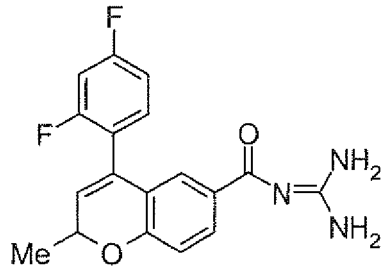 |
| 1-96 | HCl | 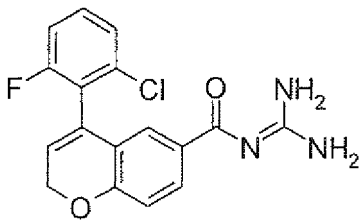 | | 1-102 | HCl | 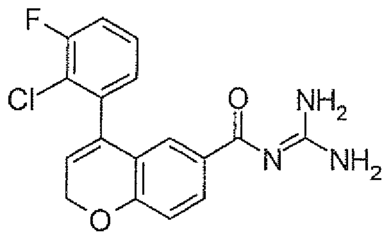 |

[Table 78]

| Ex | Sal | Str | | Ex | Sal | Str |
|---|---|---|---|---|---|---|
| 1-103 | HCl | 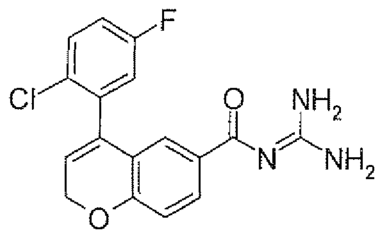 | | 1-109 | HCl | 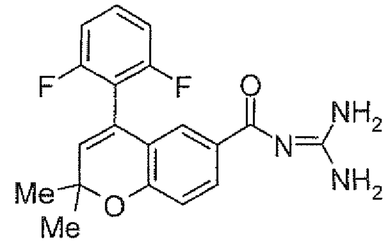 |
| 1-104 | HCl | 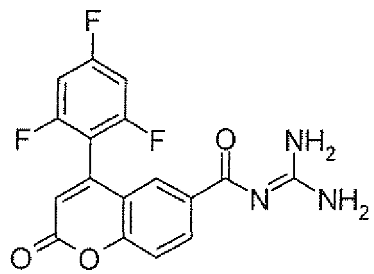 | | 1-110 | HCl | 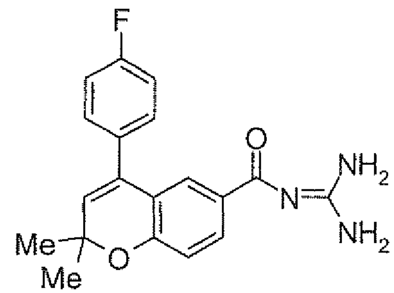 |
| 1-105 | HCl | 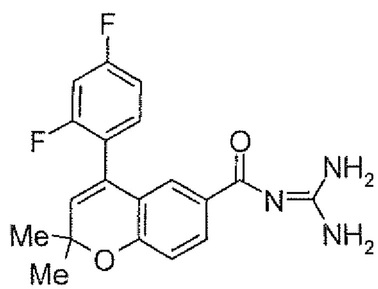 | | 1-111 | HCl | 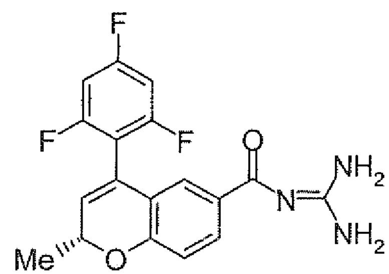 |

(continued)

| Ex | Sal | Str | | Ex | Sal | Str |
|---|---|---|---|---|---|---|
| 1-106 | HCl | 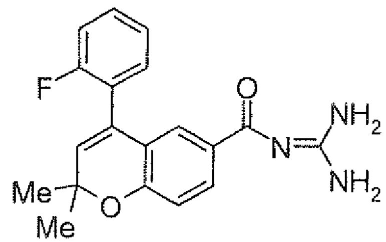 | | 1-112 | HCl | 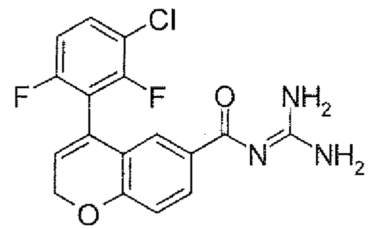 |
| 1-107 | HCl | 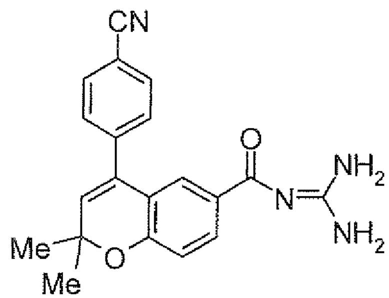 | | 1-113 | HCl | 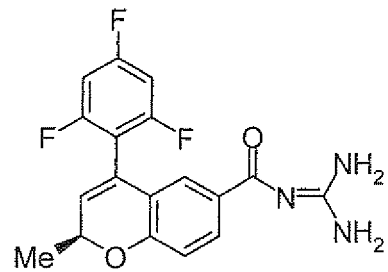 |
| 1-108 | HCl | 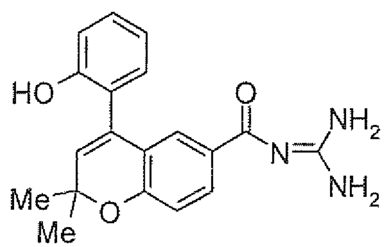 | | 1-114 | HCl | 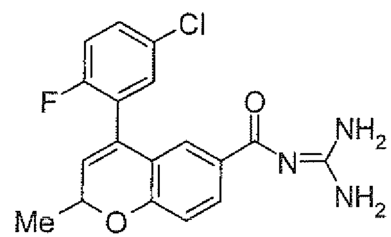 |

[Table 79]

| Ex | Sal | Str | | Ex | Sal | Str |
|---|---|---|---|---|---|---|
| 1-115 | HCl | 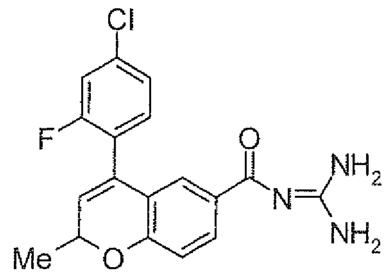 | | 1-121 | HCl | 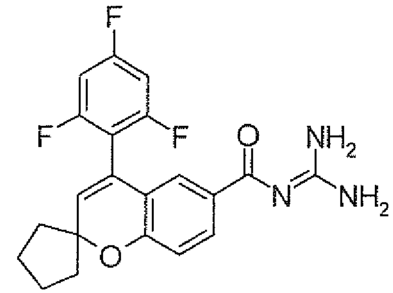 |
| 1-116 | HCl | 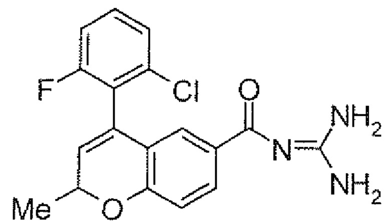 | | 1-122 | HCl | 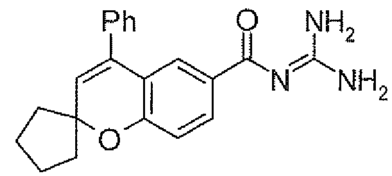 |
| 1-117 | HCl | 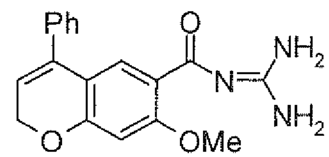 | | 1-123 | HCl | 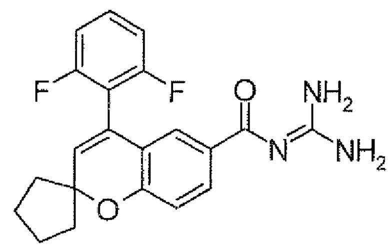 |
| 1-118 | HCl | 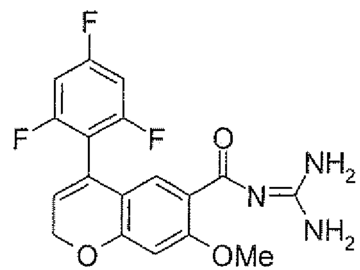 | | 1-124 | HCl | 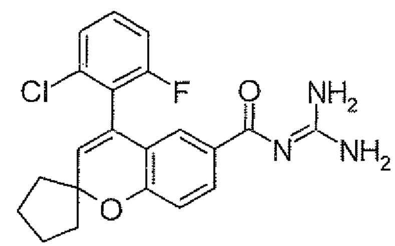 |

(continued)

| Ex | Sal | Str | Ex | Sal | Str |
|---|---|---|---|---|---|
| 1-119 | HCl | 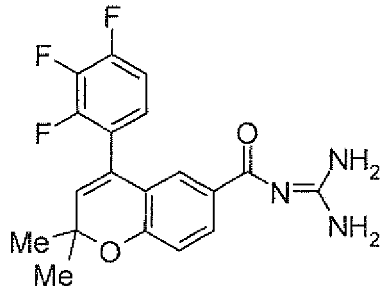 | 1-125 | HCl | 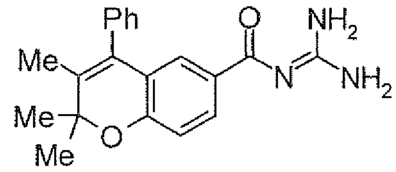 |
| 1-120 | HCl | 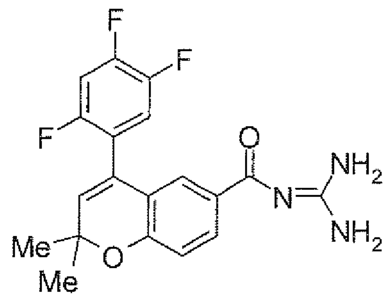 | 1-126 | HCl | 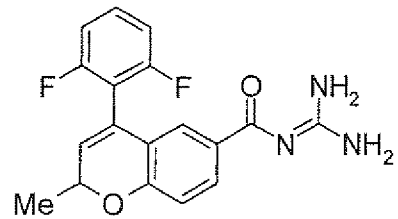 |
| | | | 1-127 | 2HCl | 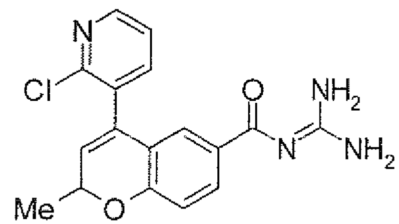 |

[Table 80]

| Ex | Sal | Str | | Ex | Sal | Str |
|---|---|---|---|---|---|---|
| 1-128 | 2HCl | 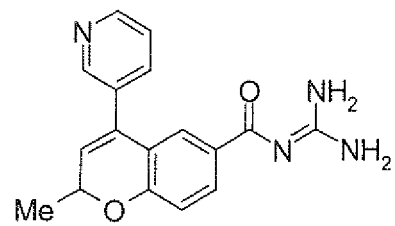 | | 1-135 | HCl | 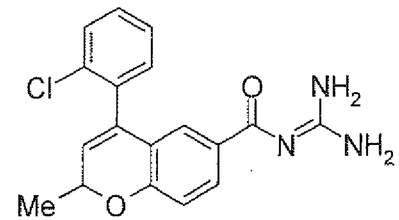 |
| 1-129 | HCl | 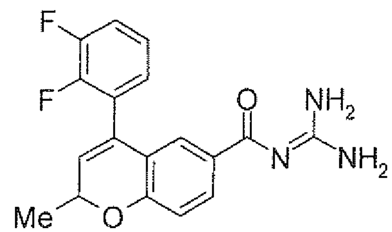 | | 1-136 | HCl | 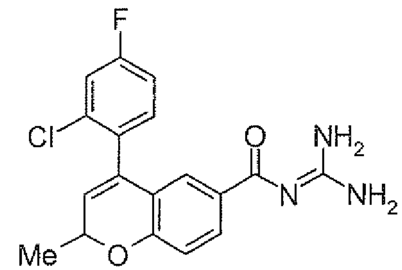 |
| 1-130 | HCl | 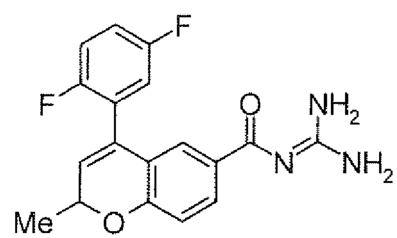 | | 1-137 | HCl | 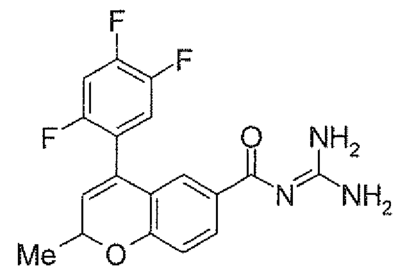 |
| 1-131 | HCl | 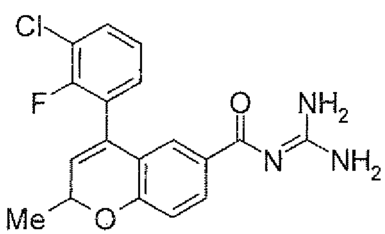 | | 1-138 | HCl | 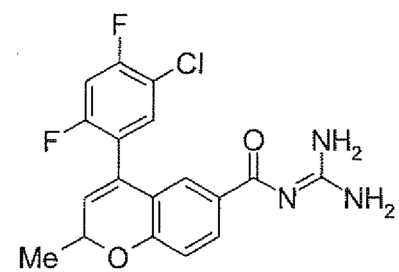 |

(continued)

| Ex | Sal | Str | | Ex | Sal | Str |
|---|---|---|---|---|---|---|
| 1-132 | HCl | 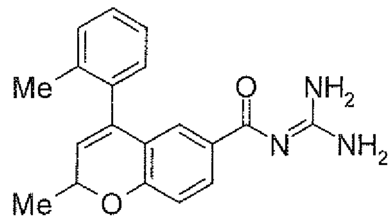 | | 1-139 | 2HCl | 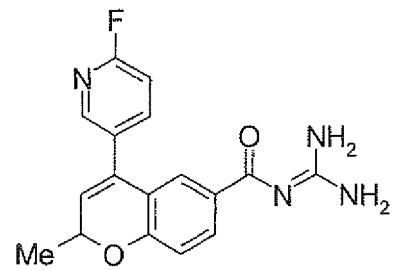 |
| 1-133 | HCl | 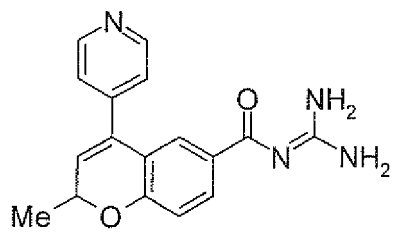 | | | | |
| 1-134 | 2HCl | 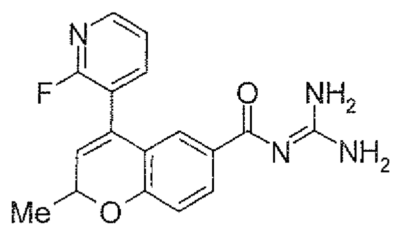 | | 1-140 | 2HCl | 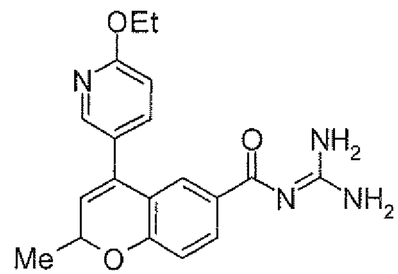 |

[Table 81]

| Ex | Sal | Str | | Ex | Sal | Str |
|---|---|---|---|---|---|---|
| 1-141 | HCl | 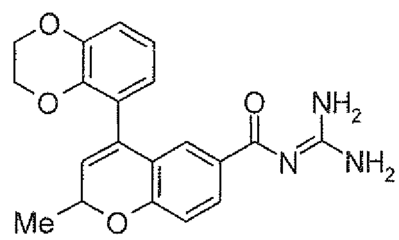 | | 1-147 | HCl | 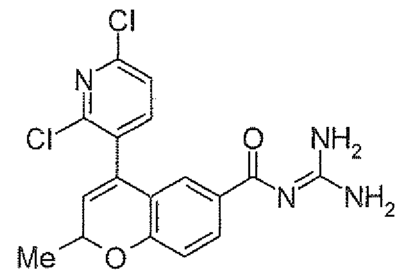 |
| 1-142 | HCl | 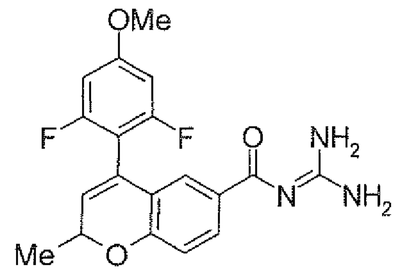 | | 1-148 | HCl | 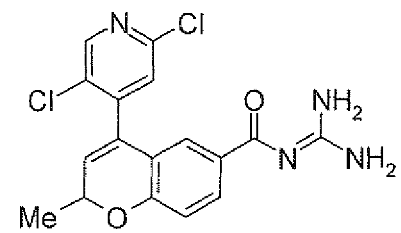 |
| 1-143 | HCl | 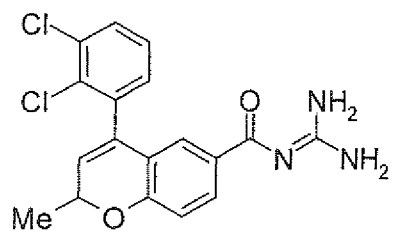 | | 1-149 | HCl | 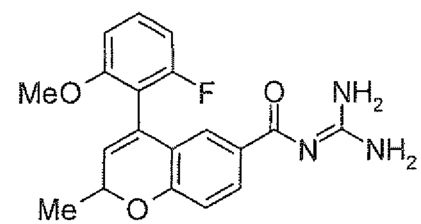 |
| 1-144 | HCl | 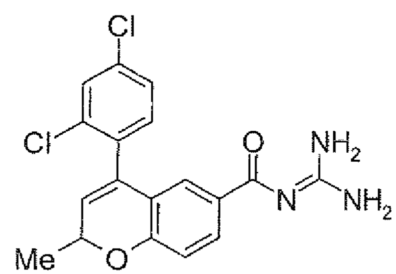 | | 1-150 | HCl | 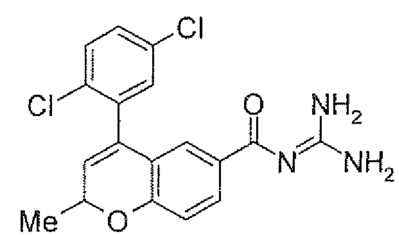 |

(continued)

| Ex | Sal | Str | | Ex | Sal | Str |
|---|---|---|---|---|---|---|
| 1-145 | HCl | 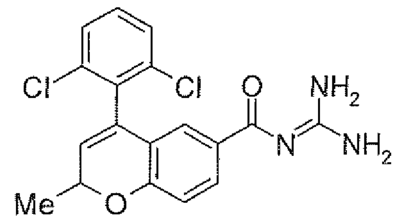 | | 1-151 | HCl | 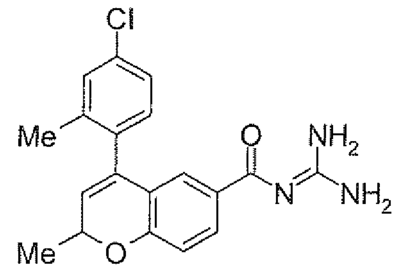 |
| 1-146 | HCl | 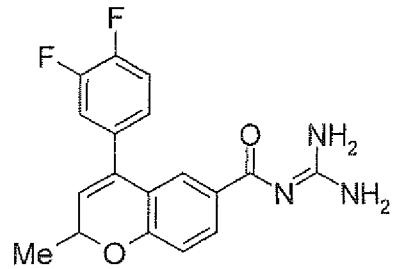 | | 1-152 | HCl | 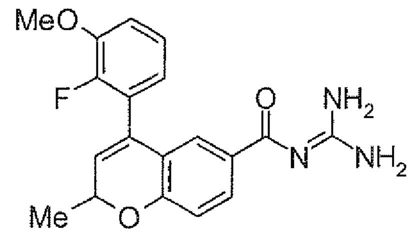 |

[Table 82]

| Ex | Sal | Str | | Ex | Sal | Str |
|---|---|---|---|---|---|---|
| 1-153 | HCl | 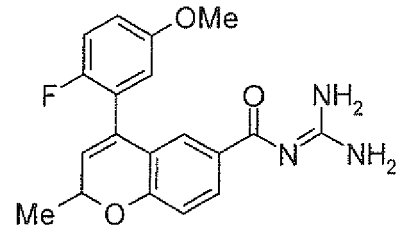 | | 1-160 | HCl | 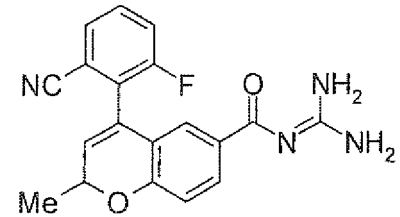 |
| 1-154 | HCl | 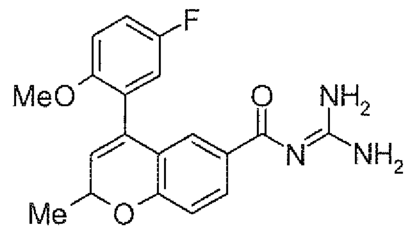 | | 1-161 | 2HCl | 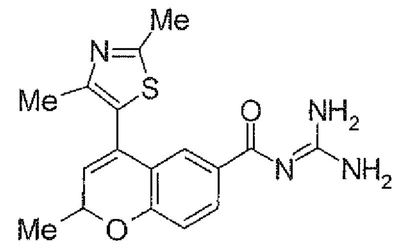 |
| 1-155 | HCl | 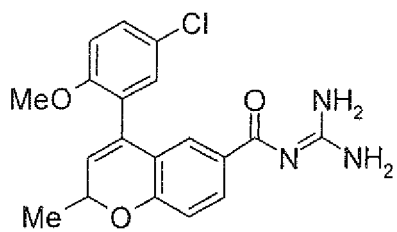 | | 1-162 | 2HCl | 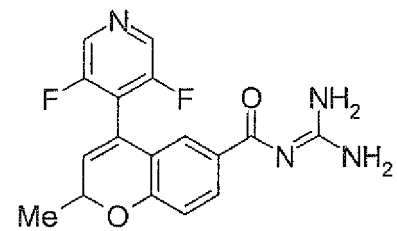 |
| 1-156 | HCl | 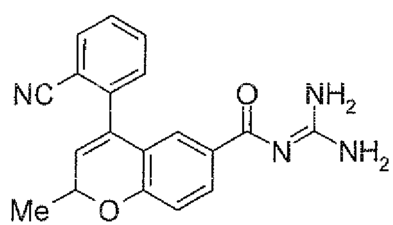 | | 1-163 | HCl | 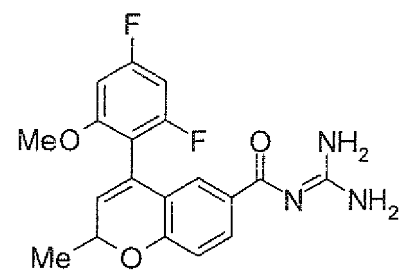 |

(continued)

| Ex | Sal | Str | | Ex | Sal | Str |
|---|---|---|---|---|---|---|
| 1-157 | HCl | 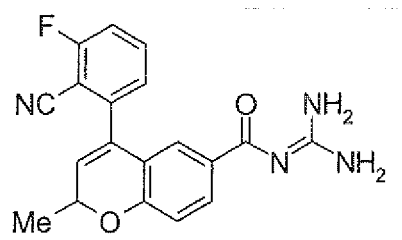 | | 1-164 | HCl | 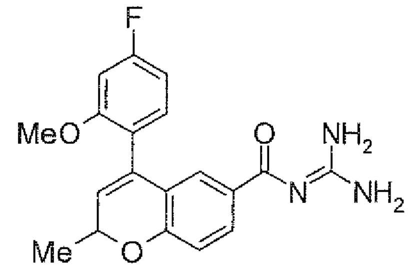 |
| 1-158 | HCl | 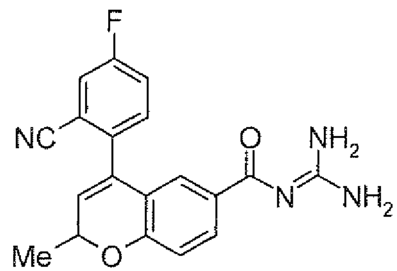 | | 1-165 | HCl | 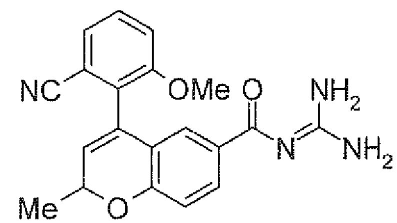 |
| 1-159 | HCl | 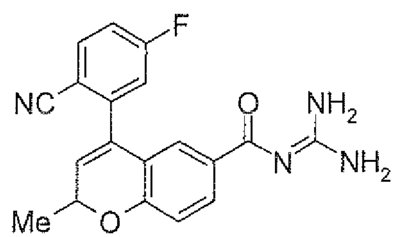 | | | | |

[Table 83]

| Ex | Sal | Str | | Ex | Sal | Str |
|---|---|---|---|---|---|---|
| 1-166 | HCl | 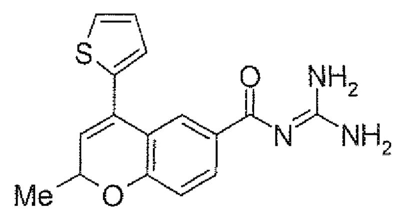 | | 2-06 | HCl | 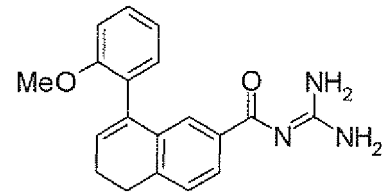 |
| 1-167 | HCl | 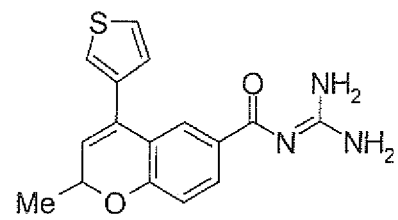 | | 2-07 | HCl | 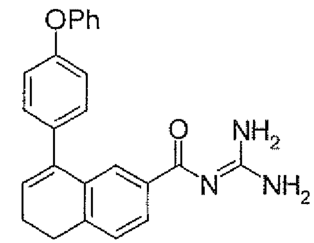 |
| 2-01 | Me-$SO_3$H | 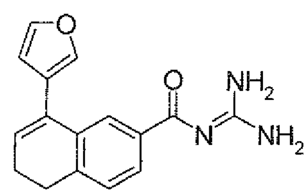 | | 2-08 | HCl | 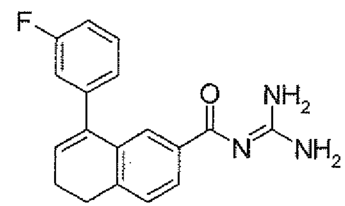 |
| 2-02 | HCl | 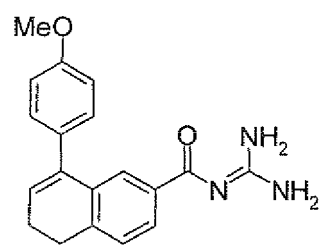 | | 2-09 | HCl | 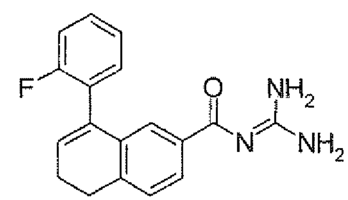 |

(continued)

| Ex | Sal | Str | | Ex | Sal | Str |
|---|---|---|---|---|---|---|
| 2-03 | HCl | 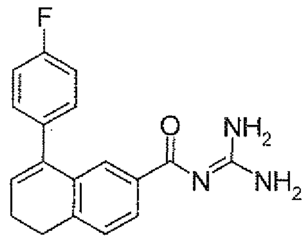 | | 2-10 | HCl | 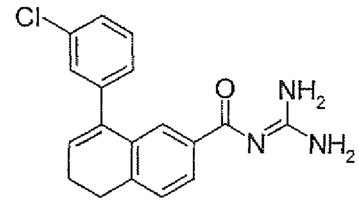 |
| 2-04 | HCl | 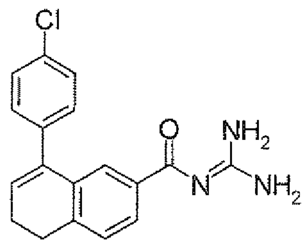 | | 2-11 | HCl | 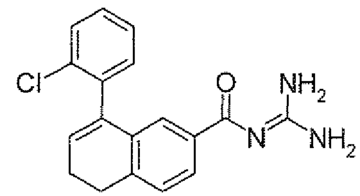 |
| 2-05 | HCl | 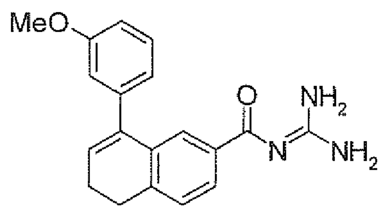 | | 2-12 | HCl | 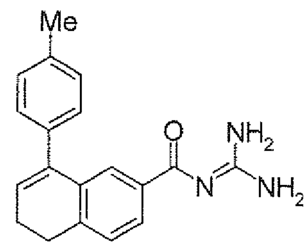 |

[Table 84]

| Ex | Sal | Str | | Ex | Sal | Str |
|---|---|---|---|---|---|---|
| 2-13 | HCl | 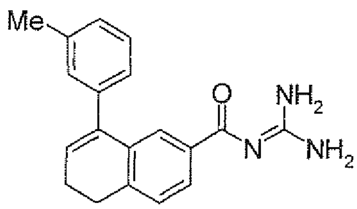 | | 2-20 | HCl | 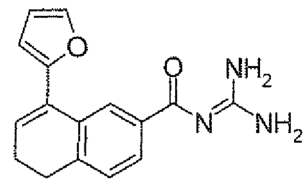 |
| 2-14 | HCl | 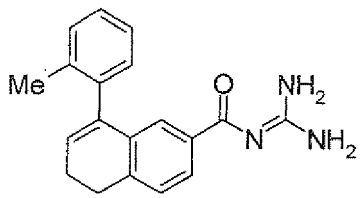 | | 2-21 | HCl | 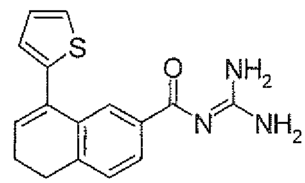 |
| 2-15 | HCl | 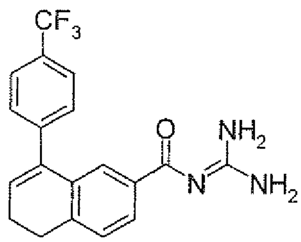 | | 2-22 | HCl | 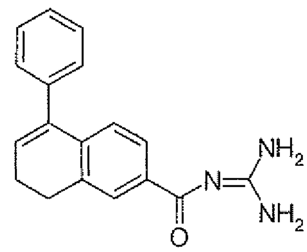 |
| 2-16 | HCl | 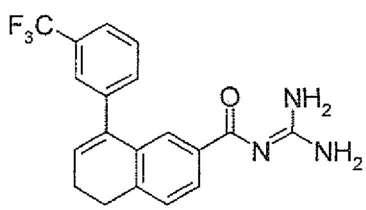 | | 2-23 | HCl | 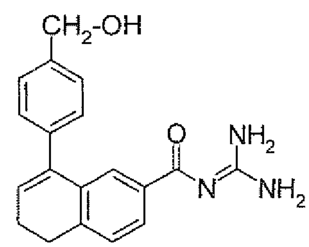 |
| 2-17 | HCl | 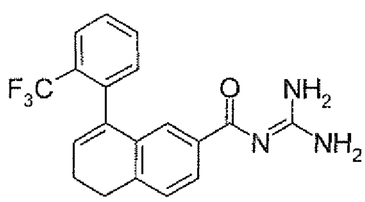 | | 2-24 | HCl | 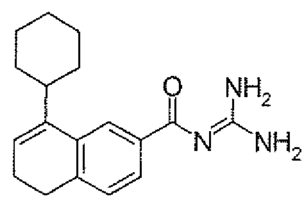 |
| 2-18 | 2HCl | 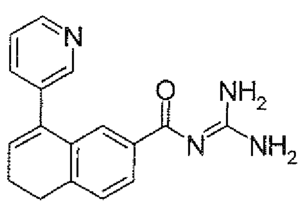 | | 2-25 | HCl | 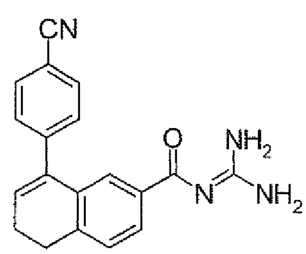 |

(continued)

| Ex | Sal | Str | | Ex | Sal | Str |
|---|---|---|---|---|---|---|
| 2-19 | HCl | 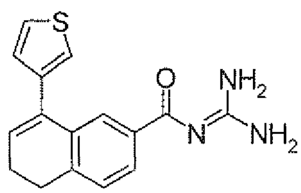 | | 2-26 | HCl | 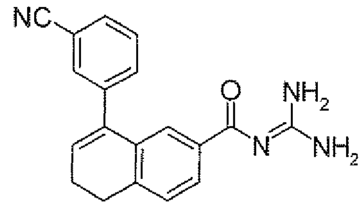 |

[Table 85]

| Ex | Sal | Str | | Ex | Sal | Str |
|---|---|---|---|---|---|---|
| 2-27 | HCl | 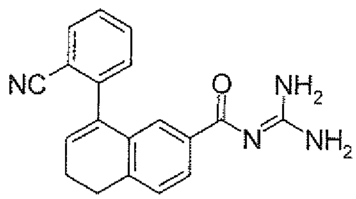 | | 2-34 | HCl | 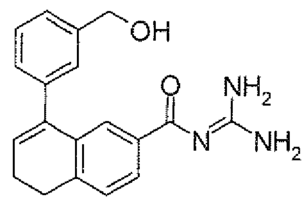 |
| 2-28 | HCl | 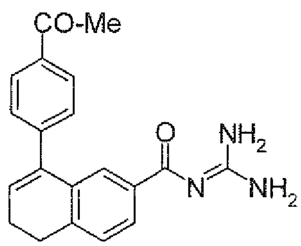 | | 2-35 | HCl | 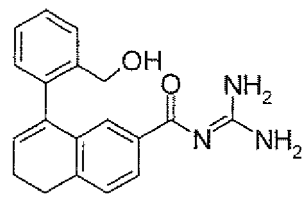 |
| 2-29 | HCl | 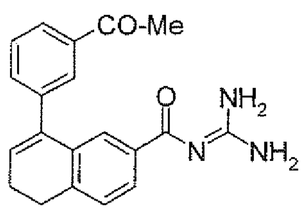 | | 2-36 | 2HCl | 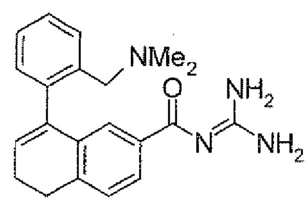 |
| 2-30 | HCl | 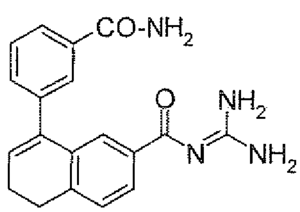 | | 2-37 | Me-$SO_3H$ | 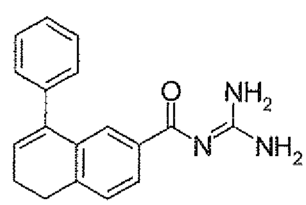 |
| 2-31 | HCl | 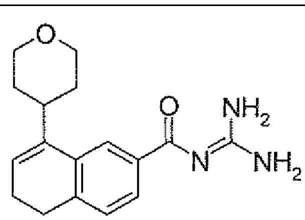 | | 2-38 | 2HCl | 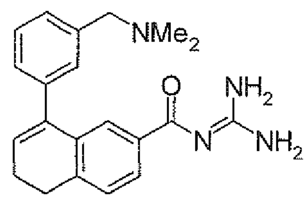 |

(continued)

| Ex | Sal | Str | | Ex | Sal | Str |
|---|---|---|---|---|---|---|
| 2-32 | 2HCl | 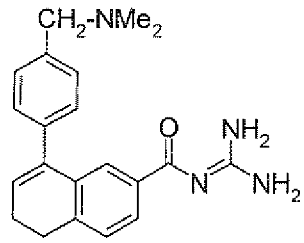 | | 2-39 | HCl | 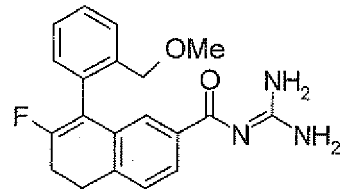 |
| 2-33 | HCl | 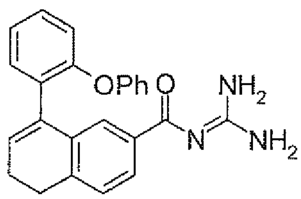 | | 2-40 | HCl | 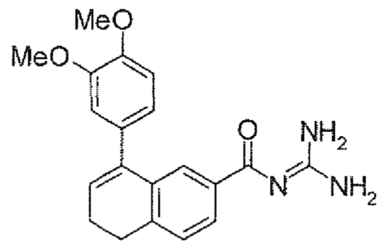 |

[Table 86]

| Ex | Sal | Str | | Ex | Sal | Str |
|---|---|---|---|---|---|---|
| 2-41 | HCl | 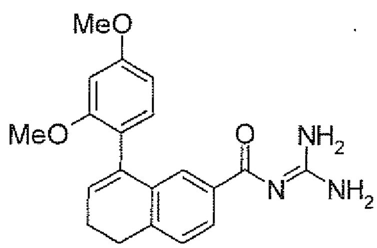 | | 2-48 | HCl | 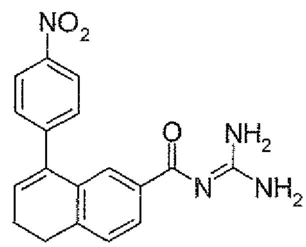 |
| 2-42 | HCl | 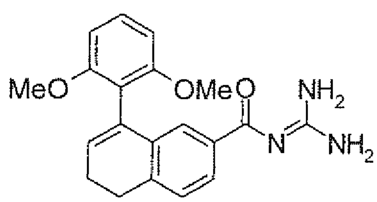 | | 2-49 | HCl | 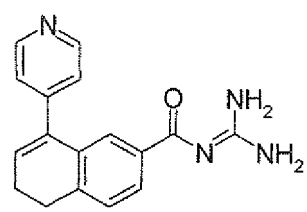 |
| 2-43 | HCl | 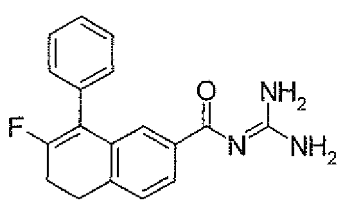 | | 2-50 | HCl | 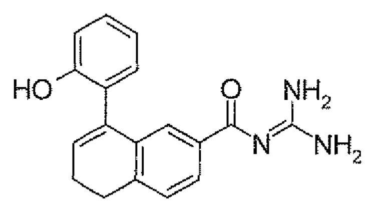 |
| 2-44 | HCl | 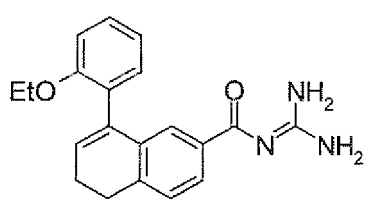 | | 2-51 | HCl | 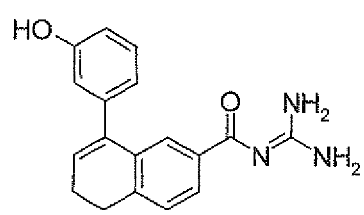 |

(continued)

| Ex | Sal | Str | | Ex | Sal | Str |
|---|---|---|---|---|---|---|
| 2-45 | HCl | 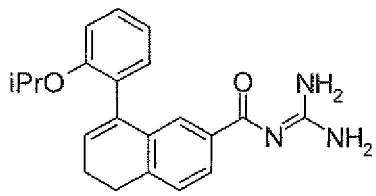 | | 2-52 | HCl | 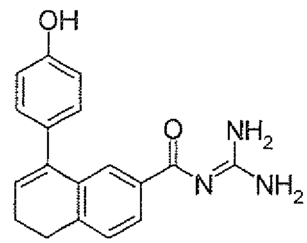 |
| 2-46 | HCl | 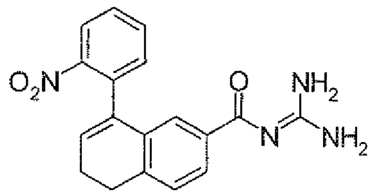 | | 2-53 | 2HCl | 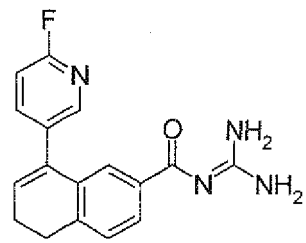 |
| 2-47 | HCl | 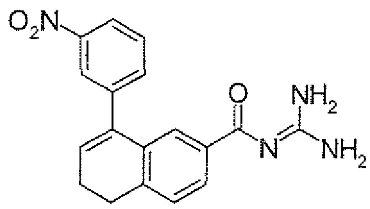 | | | | |

[Table 87]

| Ex | Sal | Str | | Ex | Sal | Str |
|---|---|---|---|---|---|---|
| 2-54 | HCl | 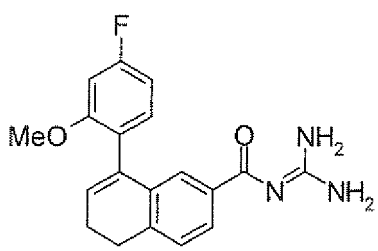 | | 2-60 | HCl | 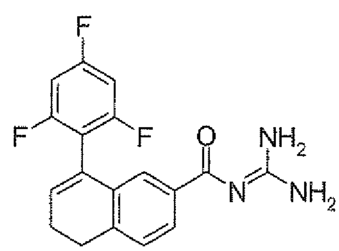 |
| 2-55 | HCl | 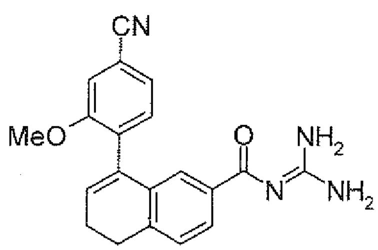 | | 2-61 | HCl | 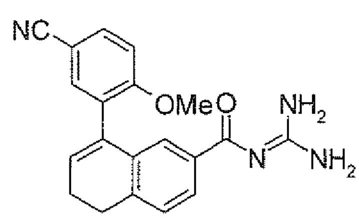 |

(continued)

| Ex | Sal | Str | | Ex | Sal | Str |
|---|---|---|---|---|---|---|
| 2-56 | HCl | 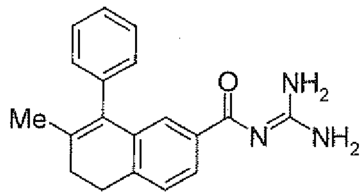 | | 2-62 | HCl | 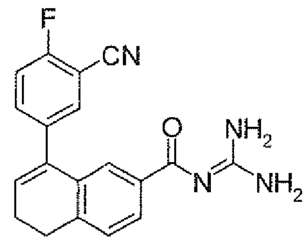 |
| 2-57 | HCl | 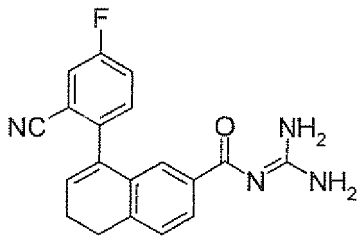 | | 2-63 | HCl | 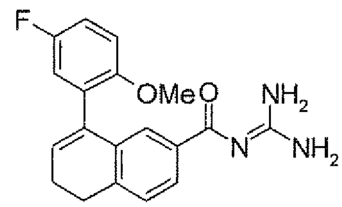 |
| 2-58 | HCl | 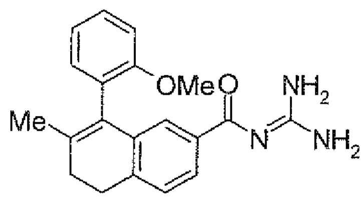 | | 2-64 | HCl | 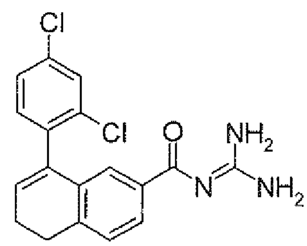 |
| 2-59 | HCl | 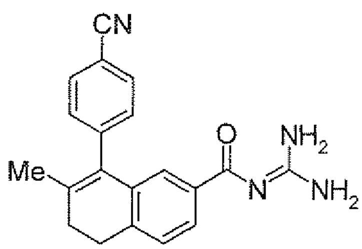 | | 2-65 | HCl | 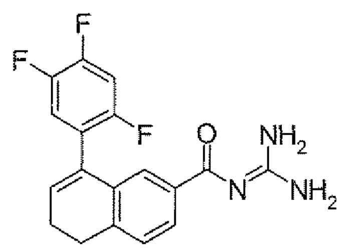 |

[Table 88]

| Ex | Sal | Str | | Ex | Sal | Str |
|---|---|---|---|---|---|---|
| 2-66 | HCl | 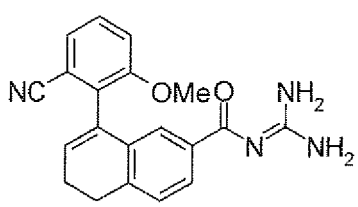 | | 2-72 | HCl | 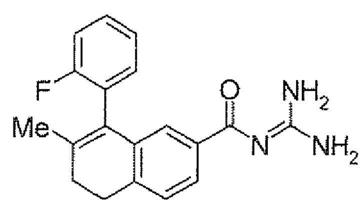 |

(continued)

| Ex | Sal | Str | Ex | Sal | Str |
|---|---|---|---|---|---|
| 2-67 | HCl | 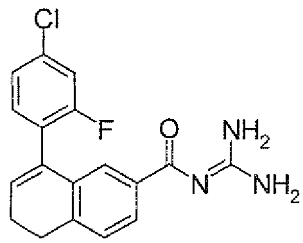 | 2-73 | HCl | 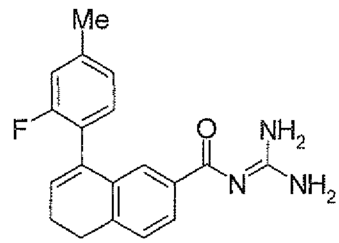 |
| 2-68 | HCl | 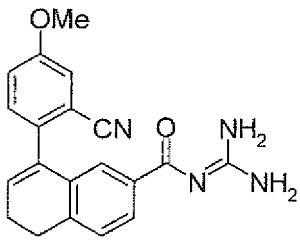 | 2-74 | HCl | 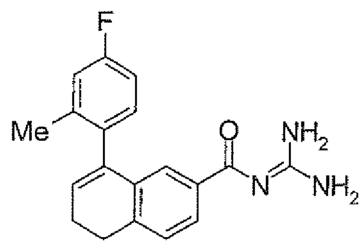 |
| 2-69 | HCl | 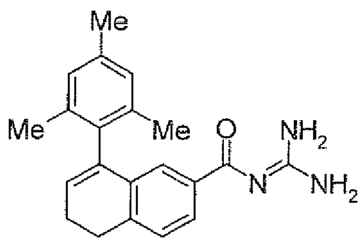 | 2-75 | HCl | 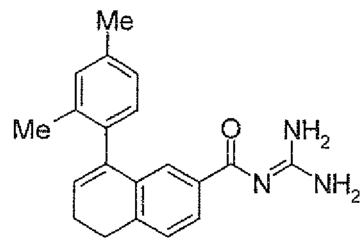 |
| 2-70 | HCl | 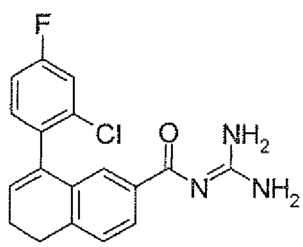 | 2-76 | HCl | 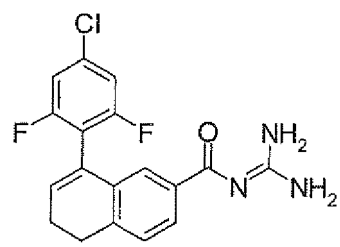 |
| 2-71 | HCl | 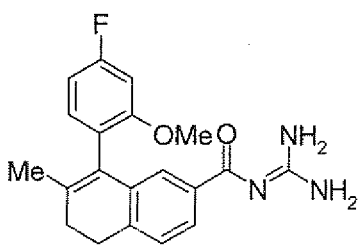 | 2-77 | HCl | 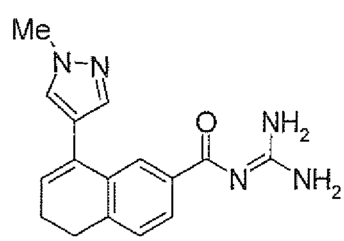 |

[Table 89]

| Ex | Sal | Str | Ex | Sal | Str |
|---|---|---|---|---|---|
| 2-78 | HCl | 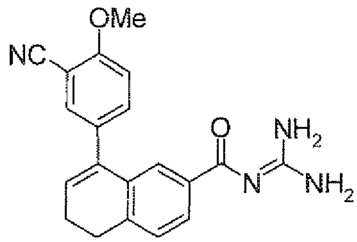 | 2-85 | HCl | 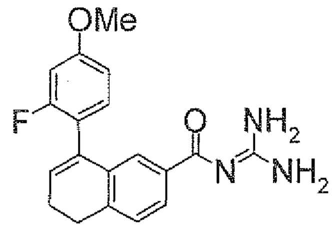 |
| 2-79 | HCl | 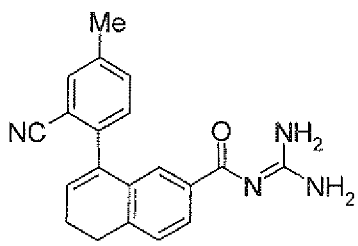 | 2-86 | 2HCl | 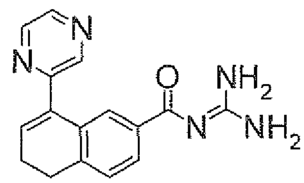 |
| 2-80 | HCl | 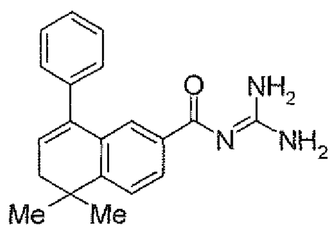 | 2-87 | HCl | 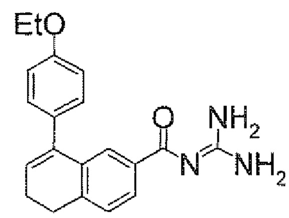 |
| 2-81 | HCl | 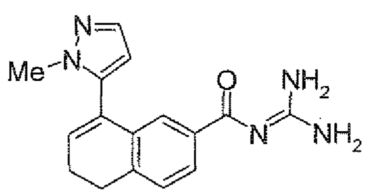 | 2-88 | HCl | 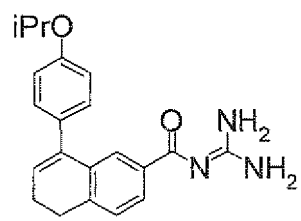 |
| 2-82 | HCl | 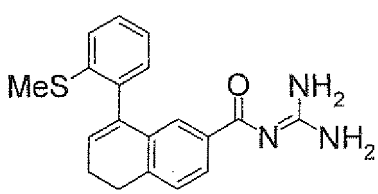 | 2-89 | 2HCl | 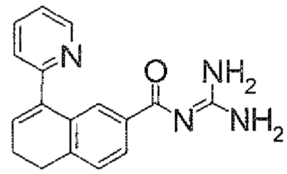 |
| 2-83 | HCl | 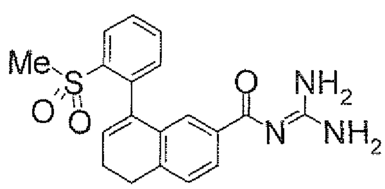 | 2-90 | HCl | 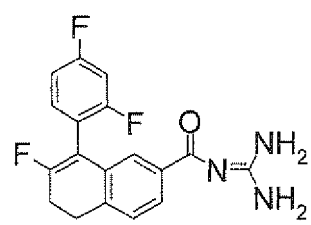 |

(continued)

| Ex | Sal | Str | | Ex | Sal | Str |
|---|---|---|---|---|---|---|
| 2-84 | 2HCl | 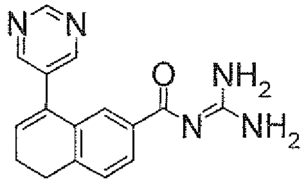 | | 2-91 | HCl | 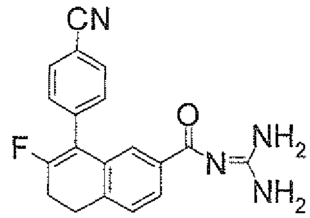 |

[Table 90]

| Ex | Sal | Str | | Ex | Sal | Str |
|---|---|---|---|---|---|---|
| 2-92 | 2HCl | 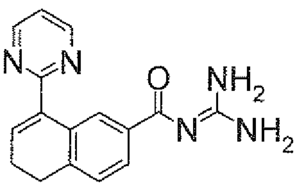 | | 2-99 | HCl | 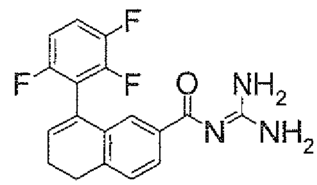 |
| 2-93 | HCl | 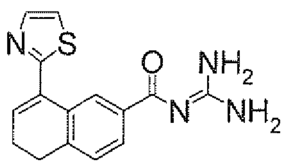 | | 2-100 | | 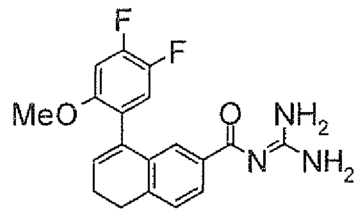 |
| 2-94 | HCl | 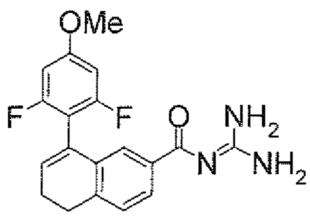 | | 2-101 | HCl | 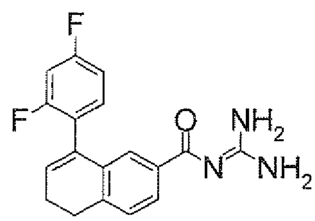 |
| 2-95 | HCl | 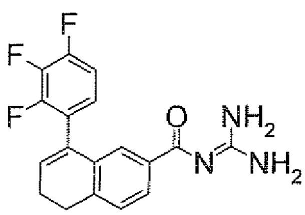 | | 2-102 | HCl | 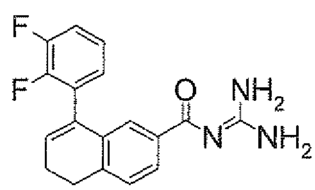 |
| 2-96 | HCl | 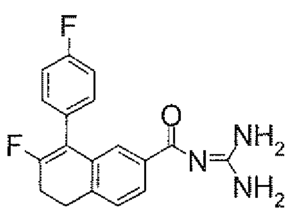 | | 2-103 | HCl | 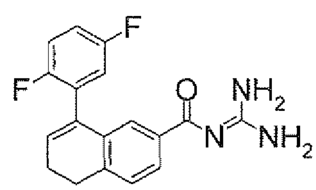 |

(continued)

| Ex | Sal | Str | | Ex | Sal | Str |
|---|---|---|---|---|---|---|
| 2-97 | HCl | 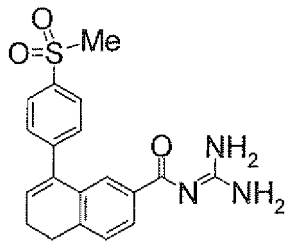 | | 2-104 | HCl | 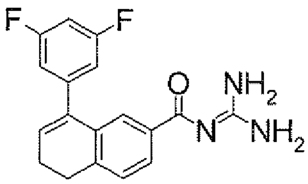 |
| 2-98 | HCl | 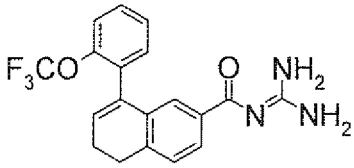 | | 2-105 | HCl | 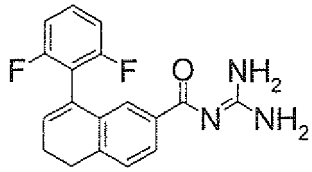 |

[Table 91]

| Ex | Sal | Str | | Ex | Sal | Str |
|---|---|---|---|---|---|---|
| 2-106 | HCl | 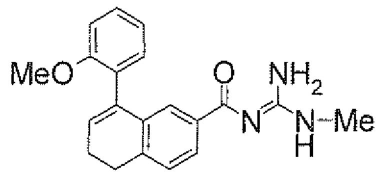 | | 2-113 | HCl | 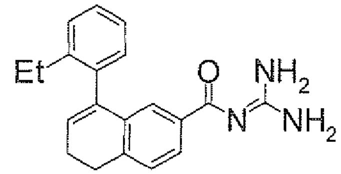 |
| 2-107 | HCl | 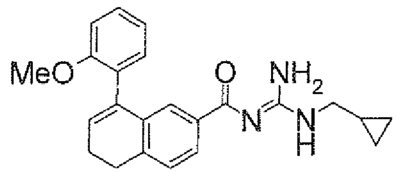 | | 2-114 | HCl | 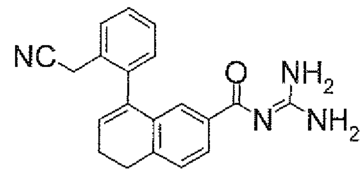 |
| 2-108 | HCl | 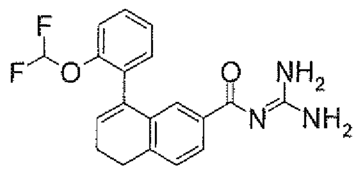 | | 2-115 | HCl | 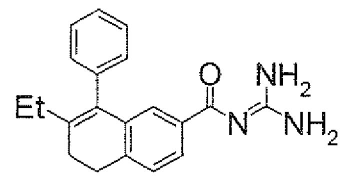 |
| 2-109 | HCl | 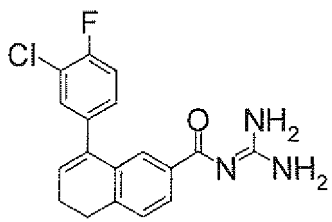 | | 2-116 | HCl | 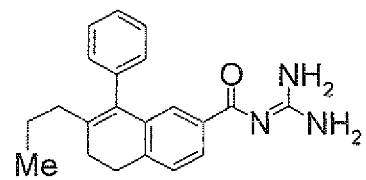 |

(continued)

| Ex | Sal | Str | | Ex | Sal | Str |
|---|---|---|---|---|---|---|
| 2-110 | HCl | 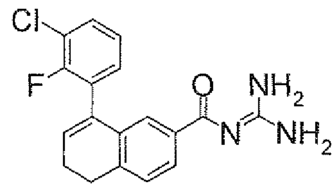 | | 2-117 | HCl | 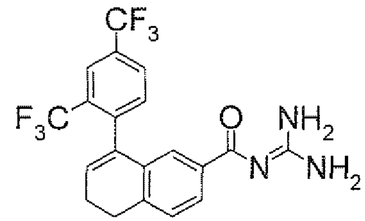 |
| 2-111 | HCl | 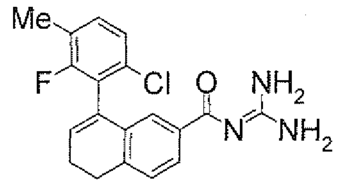 | | 2-118 | HCl | 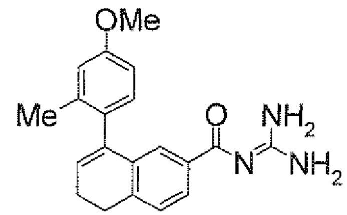 |
| 2-112 | HCl | 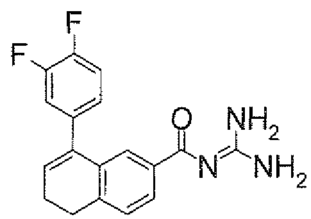 | | 2-119 | HCl | 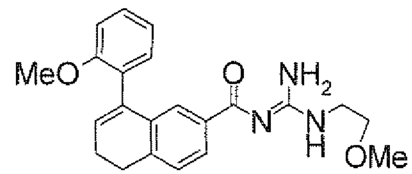 |

[Table 92]

| Ex | Sal | Str | | Ex | Sal | Str |
|---|---|---|---|---|---|---|
| 2-120 | HCl | 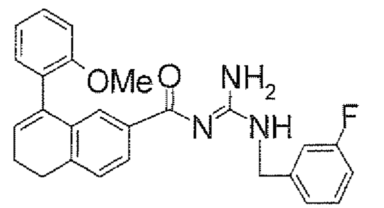 | | 2-127 | 2HCl | 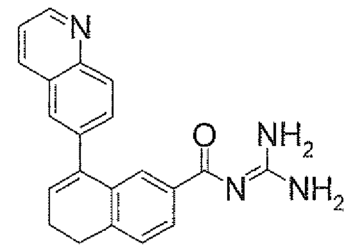 |
| 2-121 | HCl | 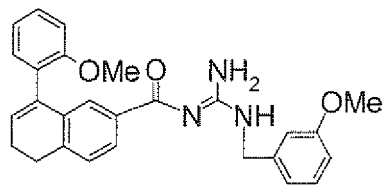 | | 2-128 | | 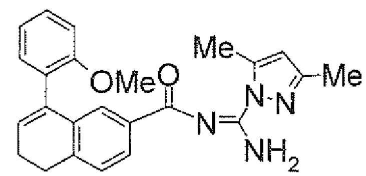 |
| 2-122 | 2HCl | 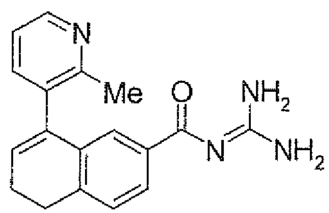 | | 2-129 | HCl | 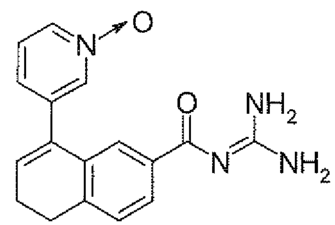 |
| 2-123 | 2HCl | 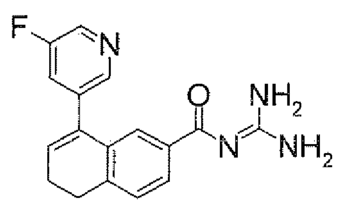 | | 2-130 | HCl | 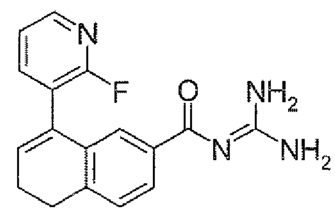 |

(continued)

| Ex | Sal | Str | | Ex | Sal | Str |
|---|---|---|---|---|---|---|
| 2-124 | 2HCl | 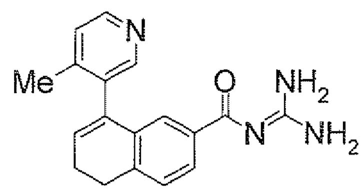 | | 2-131 | HCl | 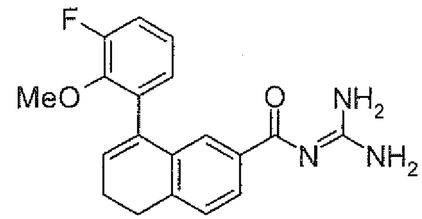 |
| 2-125 | 2HCl | 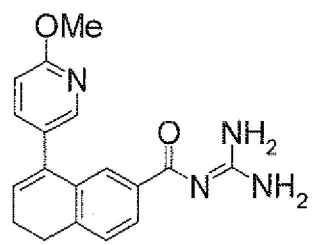 | | 2-132 | HCl | 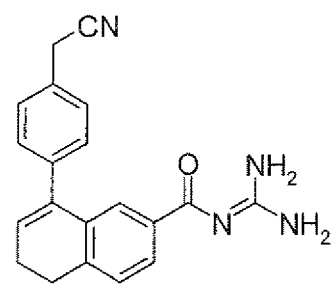 |
| 2-126 | 2HCl | 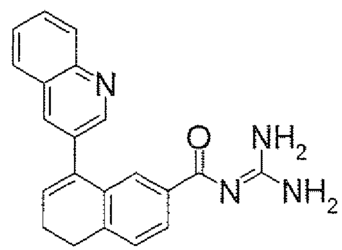 | | | | |

[Table 93]

| Ex | Sal | Str | | Ex | Sal | Str |
|---|---|---|---|---|---|---|
| 2-133 | HCl | 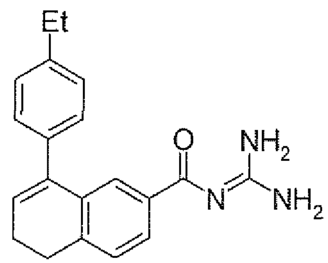 | | 2-139 | HCl | 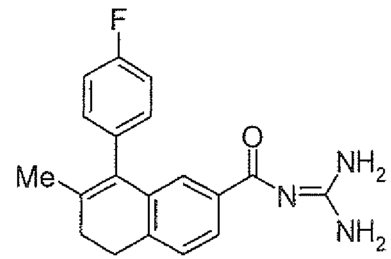 |
| 2-134 | HCl | 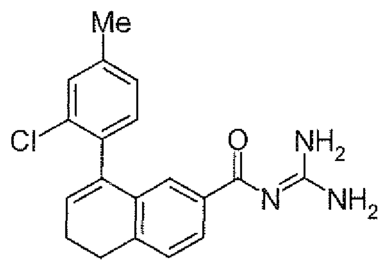 | | 2-140 | 2HCl | 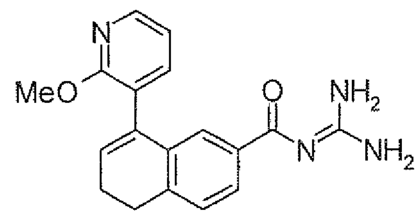 |
| 2-135 | HCl | 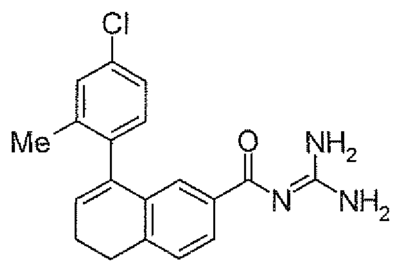 | | 2-141 | HCl | 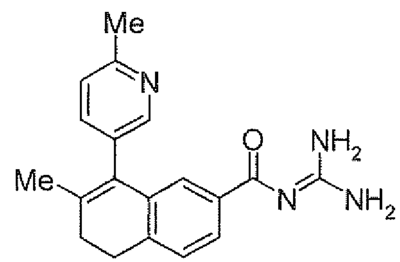 |

(continued)

| Ex | Sal | Str | | Ex | Sal | Str |
|---|---|---|---|---|---|---|
| 2-136 | HCl | 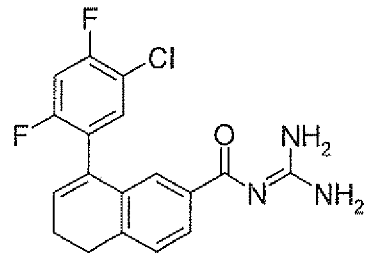 | | 2-142 | HCl | 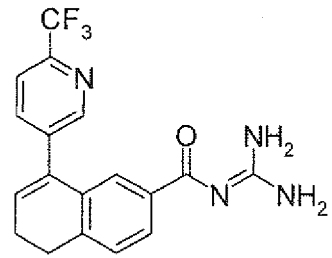 |
| 2-137 | HCl | 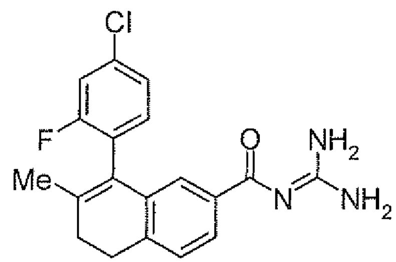 | | 2-143 | HCl | 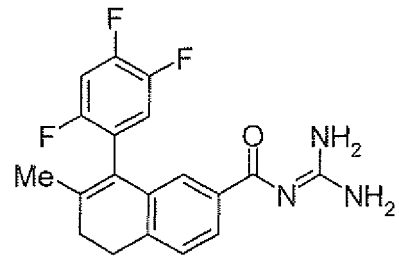 |
| 2-138 | HCl | 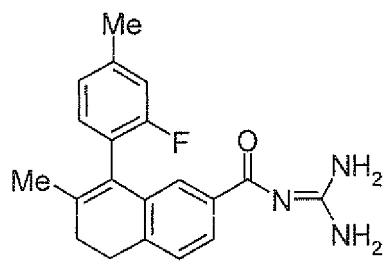 | | 2-144 | HCl | 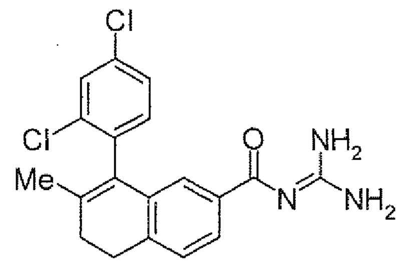 |

[Table 94]

| Ex | Sal | Str | | Ex | Sal | Str |
|---|---|---|---|---|---|---|
| 2-145 | HCl | 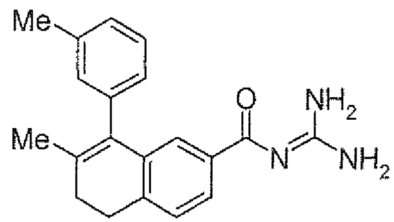 | | 2-152 | HCl | 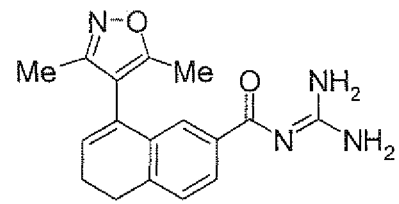 |
| 2-146 | HCl | 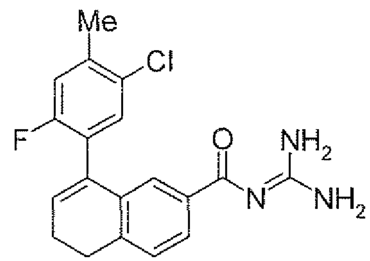 | | 2-153 | HCl | 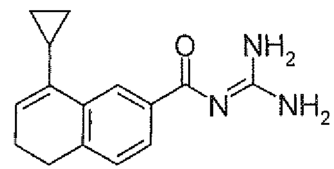 |
| 2-147 | HCl | 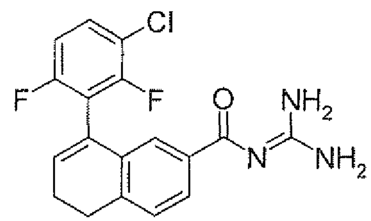 | | 2-154 | HCl | 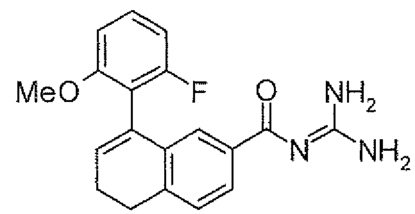 |
| 2-148 | HCl | 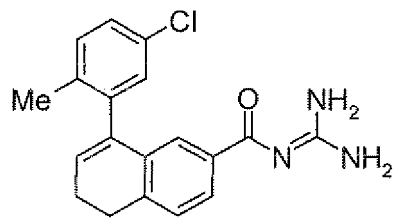 | | 2-155 | HCl | 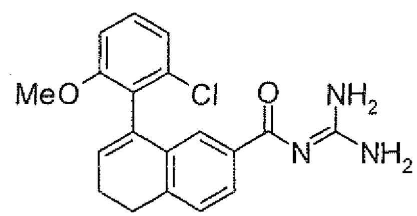 |

(continued)

| Ex | Sal | Str | Ex | Sal | Str |
|---|---|---|---|---|---|
| 2-149 | 2HCl | 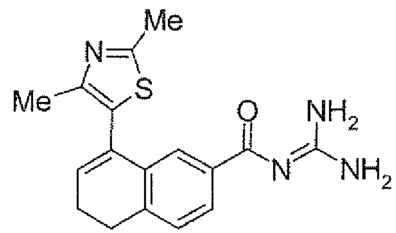 | 2-156 | HCl | 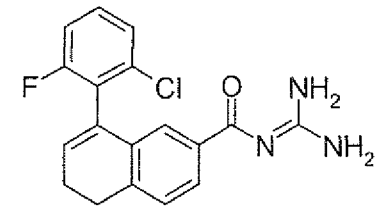 |
| 2-150 | 2HCl | 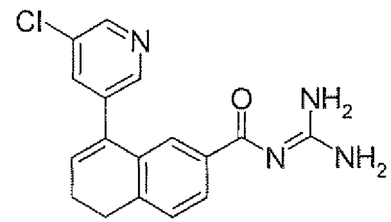 | 2-157 | 2HCl | 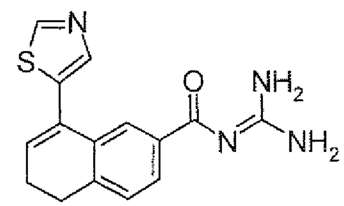 |
| 2-151 | 2HCl | 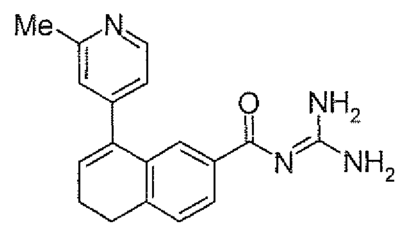 | 2-158 | HCl | 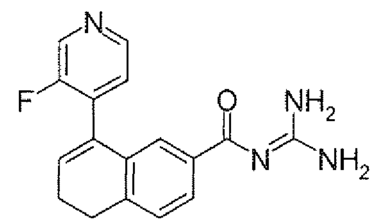 |

[Table 95]

| Ex | Sal | Str | Ex | Sal | Str |
|---|---|---|---|---|---|
| 2-159 | HCl | 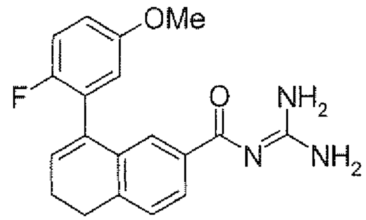 | 2-165 | HCl | 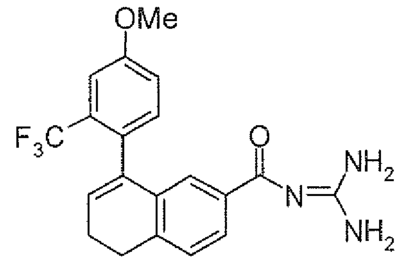 |
| 2-160 | HCl | 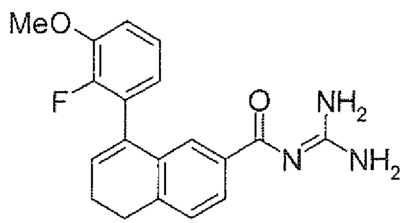 | 2-166 | HCl | 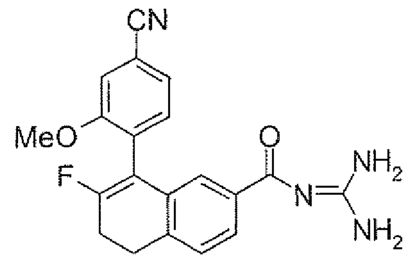 |
| 2-161 | HCl | 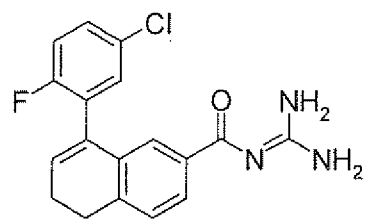 | 2-167 | HCl | 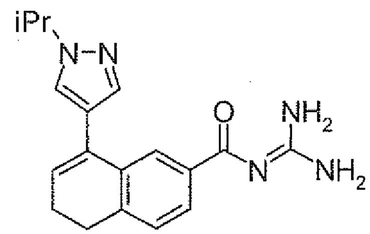 |
| 2-162 | HCl | 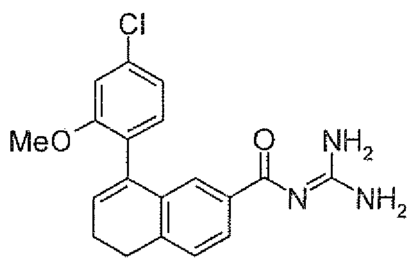 | 2-168 | HCl | 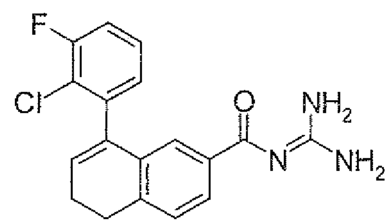 |

(continued)

| Ex | Sal | Str | | Ex | Sal | Str |
|---|---|---|---|---|---|---|
| 2-163 | HCl | 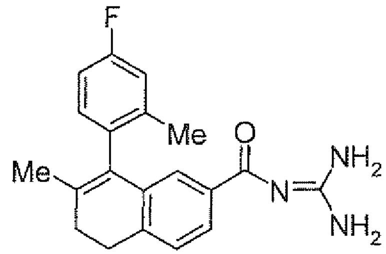 | | 2-169 | HCl | 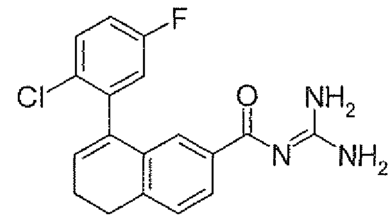 |
| 2-164 | HCl | 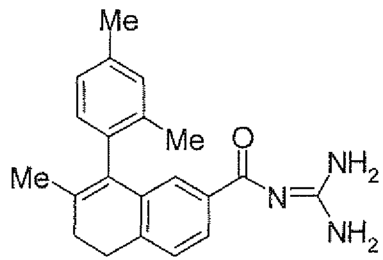 | | 2-170 | HCl | 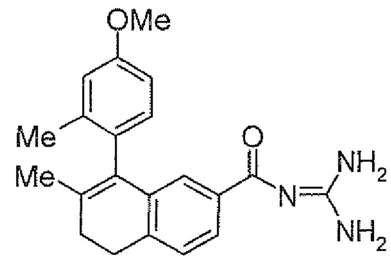 |

[Table 96]

| Ex | Sal | Str | | Ex | Sal | Str |
|---|---|---|---|---|---|---|
| 2-171 | 2HCl | 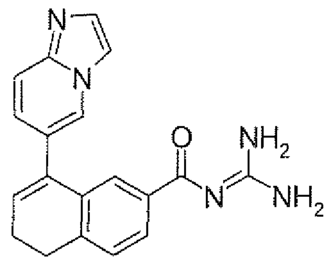 | | 2-178 | HCl | 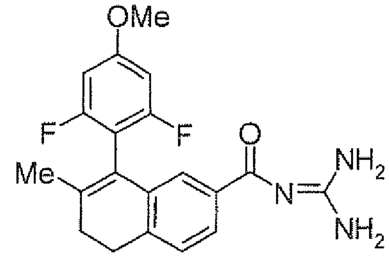 |
| 2-172 | HCl | 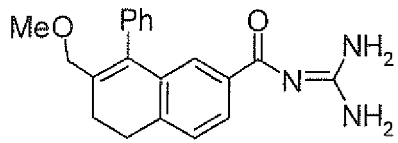 | | 2-179 | HCl | 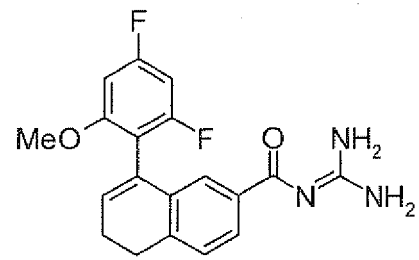 |
| 2-173 | 2HCl | 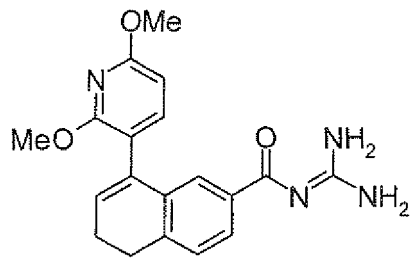 | | 2-180 | HCl | 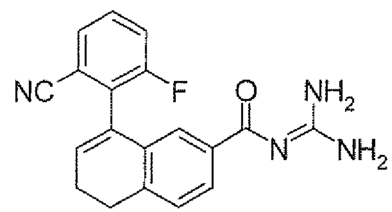 |
| 2-174 | 2HCl | 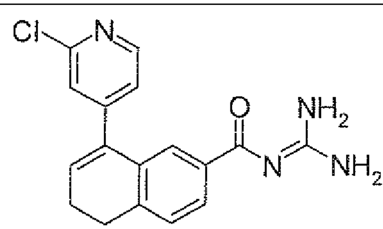 | | 2-181 | HCl | 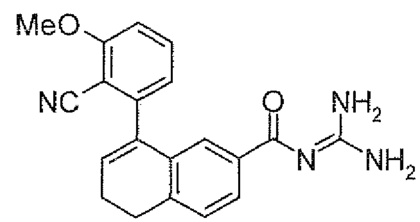 |

(continued)

| Ex | Sal | Str | | Ex | Sal | Str |
|---|---|---|---|---|---|---|
| 2-175 | HCl | 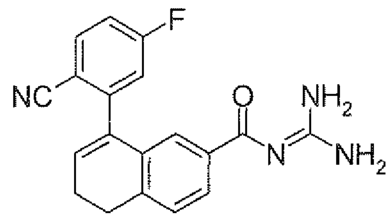 | | 2-182 | HCl | 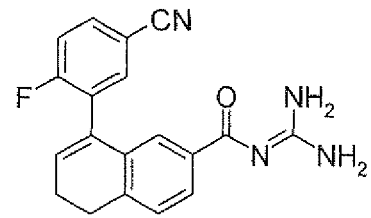 |
| 2-176 | 2HCl | 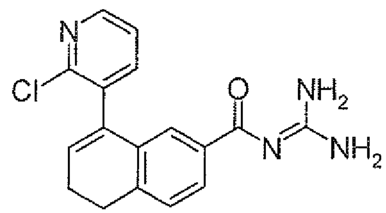 | | 2-183 | HCl | 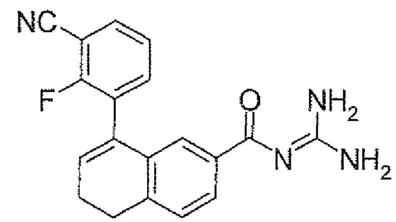 |
| 2-177 | HCl | 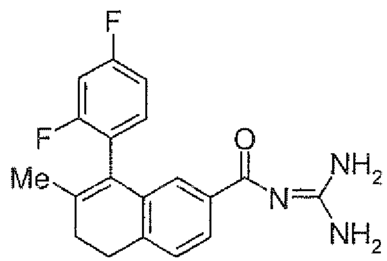 | | 2-184 | HCl | 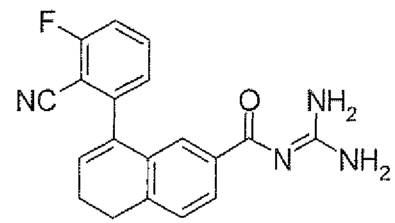 |

[Table 97]

| Ex | Sal | Str | | Ex | Sal | Str |
|---|---|---|---|---|---|---|
| 2-185 | HCl | 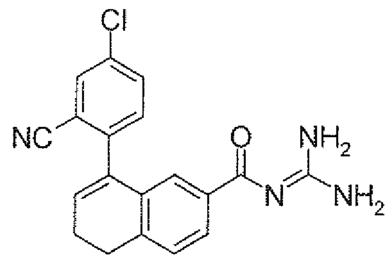 | | 2-191 | HCl | 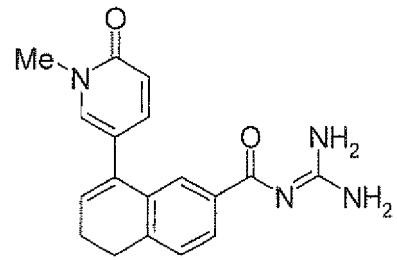 |
| 2-186 | HCl | 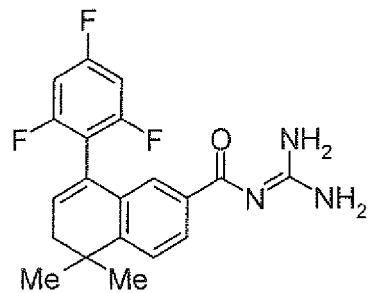 | | 2-192 | HCl | 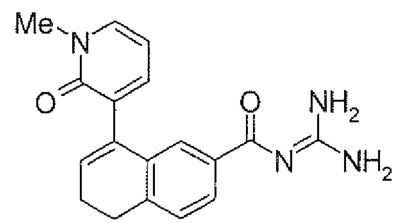 |
| 2-187 | 2HCl | 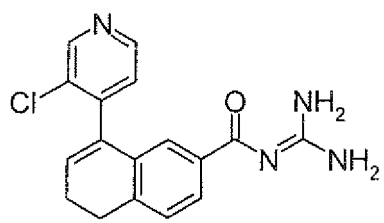 | | 3-01 | 3HCl | 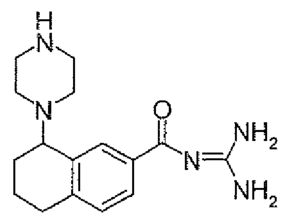 |
| 2-188 | 2HCl | 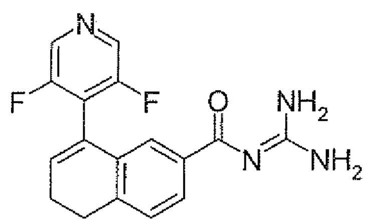 | | 3-02 | HCl | 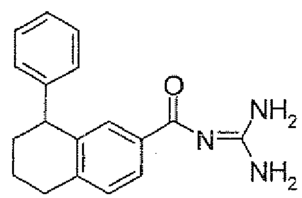 |

(continued)

| Ex | Sal | Str | Ex | Sal | Str |
|---|---|---|---|---|---|
| 2-189 | HCl | 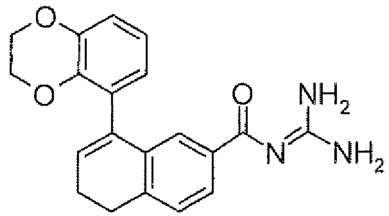 | 3-03 | HCl | 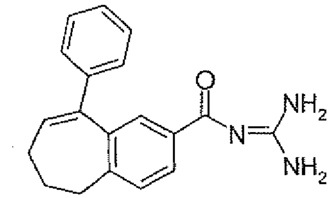 |
| 2-190 | 2HCl | 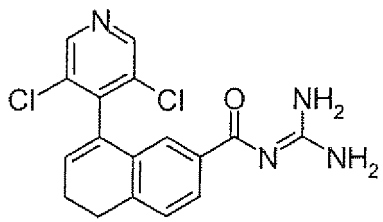 | 3-04 | HCl | 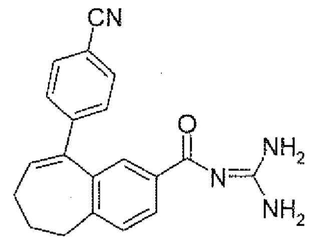 |
| | | | 3-05 | HCl | 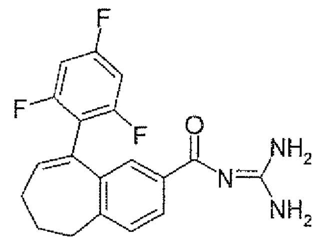 |

[Table 98]

| Ex | Sal | Str | Ex | Sal | Str |
|---|---|---|---|---|---|
| 3-06 | HCl | 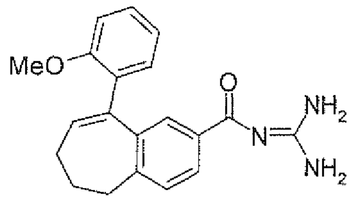 | 3-13 | HCl | 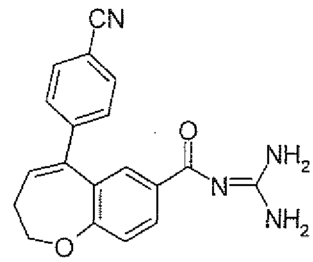 |
| 3-07 | HCl | 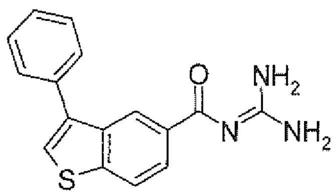 | 3-14 | HCl | 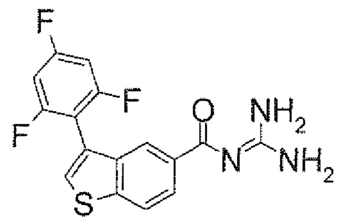 |
| 3-08 | HCl | 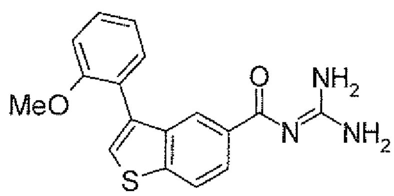 | 3-15 | HCl | 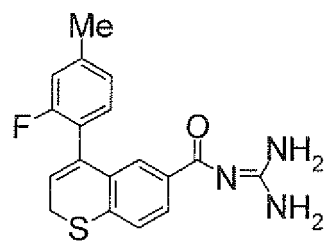 |
| 3-09 | HCl | 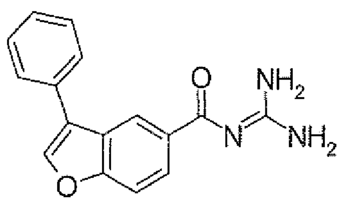 | 3-16 | HCl | 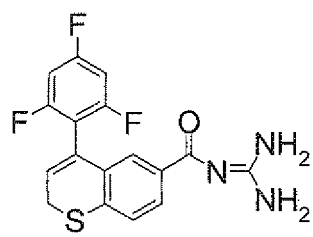 |
| 3-10 | HCl | 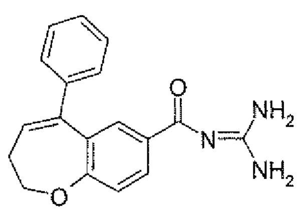 | 3-17 | HCl | 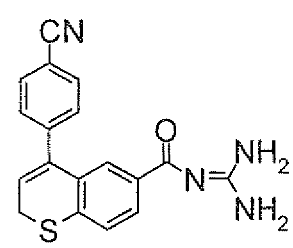 |
| 3-11 | HCl | 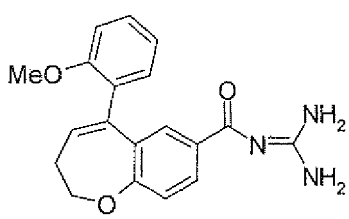 | | | |

(continued)

| Ex | Sal | Str | | Ex | Sal | Str |
|---|---|---|---|---|---|---|
| 3-12 | HCl | 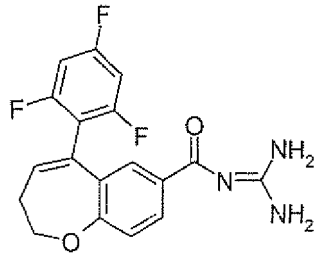 | | 3-18 | HCl | 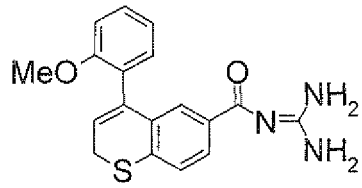 |

[Table 99]

| Ex | Sal | Str | Ex | Sal | Str |
|---|---|---|---|---|---|
| 3-19 | HCl | 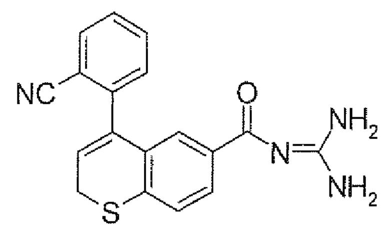 | 3-26 | HCl | 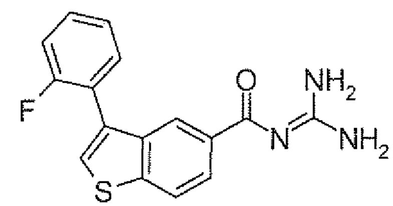 |
| 3-20 | HCl | 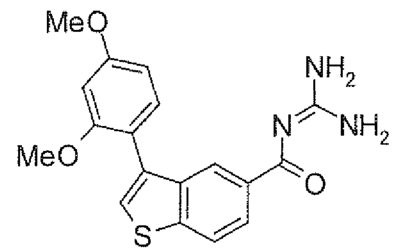 | 3-27 | HCl | 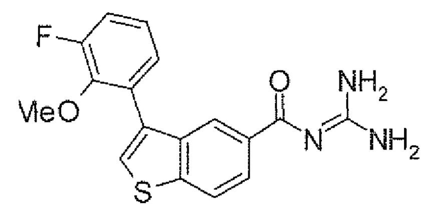 |
| 3-21 | HCl | 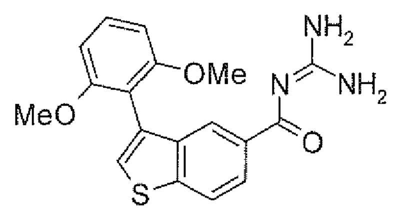 | 3-28 | HCl | 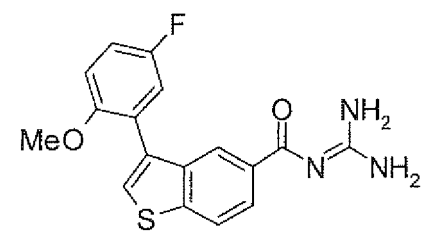 |
| 3-22 | HCl | 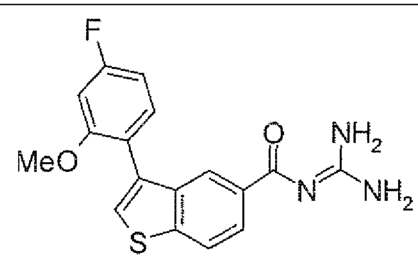 | 3-29 | HCl | 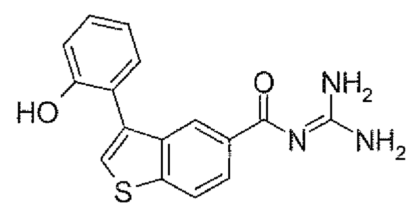 |

(continued)

| Ex | Sal | Str | | Ex | Sal | Str |
|---|---|---|---|---|---|---|
| 3-23 | HCl | 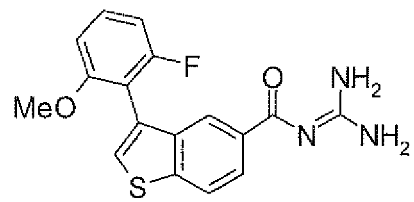 | | 3-30 | HCl | 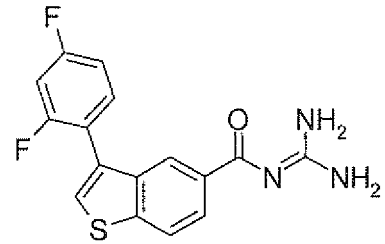 |
| 3-24 | HCl | 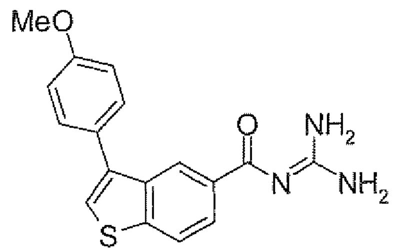 | | 3-31 | HCl | 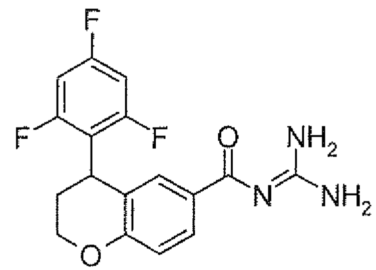 |
| 3-25 | HCl | 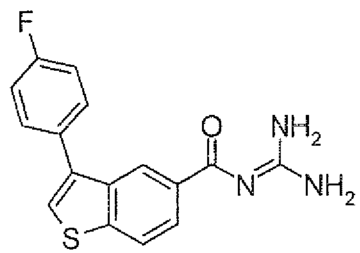 | | 3-32 | 2HCl | 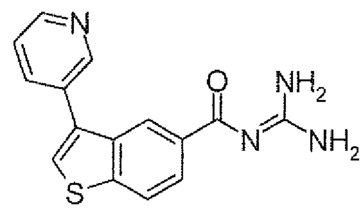 |

[Table 100]

| Ex | Sal | Str |
|---|---|---|
| 3-33 | HCl | 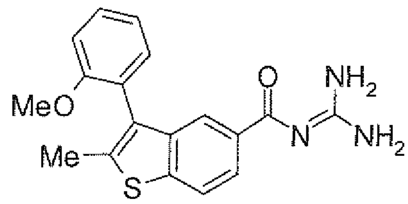 |
| 3-34 | HCl | 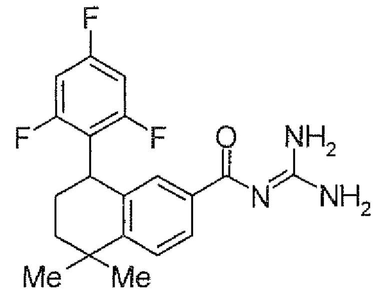 |
| 3-35 | HCl | 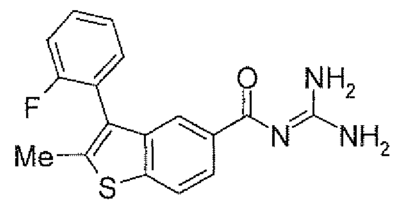 |
| 3-36 | HCl | 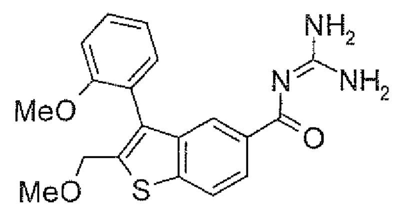 |
| 3-37 | 2HCl | 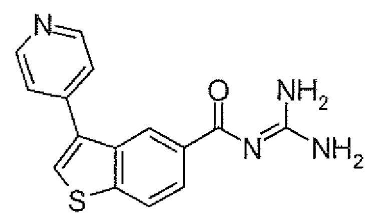 |
| 3-38 | HCl | 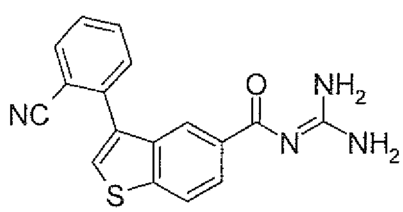 |
| 3-39 | HCl | 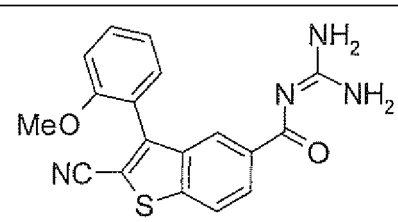 |

[Table 101]

| Ex | Syn | Dat | | Ex | Syn | Dat | | Ex | Syn | Dat |
|---|---|---|---|---|---|---|---|---|---|---|
| 1-01 | 1-01 | FAB+:352 | | 1-36 | 1-01 | EST+: 330 | | 1-73 | 1-01 | FAB+: 360 |
| 1-02 | 1-02 | ESI+: 409 | | 1-37 | 1-01 | ESI+: 330 | | 1-74 | 1-01 | FAB+: 348 |
| 1-03 | 1-03 | ESI+: 388 | | 1-38 | 1-01 | ESI+: 348 | | 1-75 | 1-03 | ESI+: 344 |
| 1-04 | 1-01 | FAB+:322 | | 1-39 | 1-01 | FAB+:324 | | 1-76 | 1-03 | ESI+: 384 |
| 1-05 | 1-01 | FAB+:294 | | 1-40 | 1-01 | EST+:370 | | 1-77 | 1-03 | ESI+: 400 |
| 1-06 | 1-01 | FAB+:324 | | 1-41 | 1-01 | FAB+:339 | | 1-78 | 1-03 | ESI+: 413 |
| 1-07 | 1-01 | FAB+:319 | | 1-42 | 1-01 | FAB+: 326 | | 1-79 | 1-01 | ESI+: 360 |
| 1-08 | 1-01 | ESI+: 349 | | 1-43 | 1-01 | FAB+: 338 | | 1-80 | 1-01 | FAB+: 346 |
| 1-09 | 1-01 | FAB+: 312 | | 1-44 | 1-01 | ESI+: 322 | | 1-81 | 1-01 | FAB+: 346 |
| 1-10 | 1-01 | FAB+: 308 | | 1-45 | 1-01 | EST+: 322 | | 1-82 | 1-01 | FAB+: 360 |
| 1-11 | 1-01 | FAB+: 342 | | 1-46 | 1-01 | FAB+: 330 | | 1-83 | 1-01 | ESI+: 430 |
| 1-12 | 1-01 (PSyn 3) | FAB+: 308 | | 1-47 | 1-01 | ESI+: 326 | | 1-84 | 1-02 | ESI+: 427 |
| | | | | 1-48 | 1-01 | ESI+: 337 | | 1-85 | 1-01 | ESI+: 366 |
| 1-13 | 1-01 (PSyn 3) | ESI+: 328 | | 1-49 | 1-01 | FAB+: 337 | | 1-86 | 1-01 | ESI+: 342 |
| | | | | 1-50 | 1-01 | ESI+: 342 | | 1-87 | 1-01 | ESI+: 337 |
| 1-14 | 1-01 | FAB+:312 | | 1-51 | 1-01 | ESI+: 362 | | 1-88 | 1-01 | ESI+: 312 |
| 1-15 | 1-01 | FAB+: 362 | | 1-52 | 1-01 | ESI+: 362 | | 1-89 | 1-01 | ESI+: 376 |
| 1-16 | 1-01 | ESI+: 328 | | 1-53 | 1-01 | ESI+: 348 | | 1-90 | 1-01 | FAB+: 336 |
| 1-17 | 1-01 | EST+: 324 | | 1-54 | 1-01 | ESI+: 346 | | 1-91 | 1-01 | ESI+: 342 |
| 1-18 | 1-01 | ESI+: 339 | | 1-55 | 1-01 | FAB+: 346 | | 1-92 | 1-01 | FAB+: 342 |
| 1-19 | 1-01 | ESI+: 348 | | 1-56 | 1-01 | ESI+: 396 | | 1-93 | 1-01 | ESI+: 364 |
| 1-20 | 1-01 | FAB+:312 | | 1-57 | 1-01 | FAB+: 396 | | 1-94 | 1-01 | ESI+: 360 |
| 1-21 | 1-01 | FAB+:319 | | 1-58 | 1-01 | ESI+: 362 | | 1-95 | 1-01 | ESI+: 342 |
| 1-22 | 1-01 | FAB+:310 | | 1-59 | 1-01 | ESI+: 362 | | 1-96 | 1-01 | ESI+: 346 |
| 1-23 | 1-01 | FAB+:339 | | 1-60 | 1-01 | FAB+:340 | | 1-97 | 1-01 | FAB+: 346 |
| 1-24 | 1-01 | ESI+:362 | | 1-61 | 1-01 | FAB+:333 | | 1-98 | 1-01 | FAB+: 392 |
| 1-25 | 1-01 | ESI+: 370 | | 1-62 | 1-01 | ESI+: 322 | | 1-99 | 1-01 | FAB+: 326 |
| 1-26 | 1-01 | FAB+:308 | | 1-63 | 1-01 | ESI+: 364 | | 1-100 | 1-01 | FAB+: 326 |
| 1-27 | 1-01 | FAB+:338 | | 1-64 | 1-01 | ESI+: 358 | | 1-101 | 1-01 | FAB+: 344 |
| 1-28 | 1-01 | FAB+:312 | | 1-65 | 1-01 | ESI+: 380 | | 1-102 | 1-01 | ESI+: 346 |
| 1-29 | 1-01 | FAB+:328 | | 1-66 | 1-01 | ESI+: 298 | | 1-103 | 1-01 | ESI+: 346 |
| 1-30 | 1-01 | FAB+:308 | | 1-67 | 1-01 | FAB+:362 | | 1-104 | 1-01 | FAB+: 362 |
| 1-31 | 1-01 | ESI+: 310 | | 1-68 | 1-03 | ESI+: 438 | | 1-105 | 1-01 | ESI+: 358 |
| 1-32 | 1-01 | ESI+: 330 | | 1-69 | 1-03 | ESI+: 450 | | 1-106 | 1-01 | ESI+: 340 |
| 1-33 | 1-01 | ESI+: 330 | | 1-70 | 1-01 | ESI+:340 | | 1-107 | 1-01 | ESI+: 347 |
| 1-34 | 1-01 | ESI+: 333 | | 1-71 | 1-01 | FAB+:362 | | 1-108 | 1-01 | ESI+: 338 |
| 1-35 | 1-01 | ESI+: 330 | | 1-72 | 1-01 | ESI+:333 | | 1-109 | 1-01 | ESI+: 358 |

[Table 102]

| Ex | Syn | Dat | Ex | Syn | Dat | Ex | Syn | Dat |
|---|---|---|---|---|---|---|---|---|
| 1-110 | 1-01 | ESI+:340 | 1-147 | 1-01 | ESI+: 377 | 2-16 | 1-01 | FAB+: 360 |
| 1-111 | 1-01 | ESI+: 362 | 1-148 | 1-01 | ESI+: 377 | 2-17 | 1-01 | FAB+: 360 |
| 1-112 | 1-01 | ESI+: 364 | 1-149 | 1-01 | ESI+: 356 | 2-18 | 1-01 | FAB+:293 |
| 1-113 | 1-01 | ESI+: 362 | 1-150 | 1-01 | ESI+: 376 | 2-19 | 1-01 | FAB+:298 |
| 1-114 | 1-01 | ESI+: 360 | 1-151 | 1-01 | ESI+: 356 | 2-20 | 1-01 | FAB+:282 |
| 1-115 | 1-01 | ESI+:360 | 1-152 | 1-01 | ESI+: 356 | 2-21 | 1-01 | FAB+:298 |
| 1-116 | 1-01 | ESI+: 360 | 1-153 | 1-01 | ESI+: 356 | 2-22 | 1-01 | ESI+: 292 |
| 1-117 | 1-01 | ESI+: 324 | 1-154 | 1-01 | ESI+: 356 | 2-23 | 1-01 | FAB+:322 |
| 1-118 | 1-01 | ESI+: 378 | 1-155 | 1-01 | ESI+: 372 | 2-24 | 1-01 | FAB+:298 |
| 1-119 | 1-01 | ESI+: 376 | 1-156 | 1-01 | ESI+: 333 | 2-25 | 1-01 | ESI+: 317 |
| 1-120 | 1-01 | ESI+: 376 | 1-157 | 1-01 | ESI+: 351 | 2-26 | 1-01 | ESI+: 317 |
| 1-121 | 1-01 | ESI+: 402 | 1-158 | 1-01 | ESI+: 351 | 2-27 | 1-01 | ESI+: 317 |
| 1-122 | 1-01 | ESI+: 348 | 1-159 | 1-01 | ESI+: 351 | 2-28 | 1-01 | FAB+: 334 |
| 1-123 | 1-01 | ESI+: 384 | 1-160 | 1-01 | ESI+: 351 | 2-29 | 1-01 | FAB+: 334 |
| 1-124 | 1-01 | ESI+: 400 | 1-161 | 1-01 | ESI+: 343 | 2-30 | 1-01 | ESI+: 335 |
| 1-125 | 1-01 | ESI+: 336 | 1-162 | 1-01 | ESI+: 345 | 2-31 | 1-01 | FAB+:300 |
| 1-126 | 1-01 | ESI+: 344 | 1-163 | 1-01 | ESI+: 374 | 2-32 | 1-01 | FAB+:349 |
| 1-127 | 1-01 | ESI+: 343 | 1-164 | 1-01 | ESI+: 356 | 2-33 | 1-01 | FAB+:384 |
| 1-128 | 1-01 | FAB+: 309 | 1-165 | 1-01 | ESI+: 363 | 2-34 | 1-01 | FAB+:322 |
| 1-129 | 1-01 | FAB+: 344 | 1-166 | 1-01 | ESI+: 314 | 2-35 | 1-01 | FAB+:322 |
| 1-130 | 1.01 | FAB+: 344 | 1-167 | 1-01 | ESI+: 314 | 2-36 | 1-01 | FAB+:349 |
| 1-131 | 1-01 | FAB+: 360 | 2-01 | 2-01 | ESI+: 282 | 2-37 | 1-02 (PSyn 3) | ESI+: 292 |
| 1-132 | 1-01 | ESI+: 322 | 2-02 | 1-01 | ESI+: 322 | | | |
| 1-133 | 1-01 | ESI+: 309 | 2-03 | 1-01 | ESI+: 310 | 2-38 | 1-01 | FAB+:349 |
| 1-134 | 1-01 | ESI+: 327 | 2-04 | 1-01 | ESI+: 326 | 2-39 | 1-01 | FAB+:340 |
| 1-135 | 1-01 | ESI+: 342 | 2-05 | 1-01 | ESI+: 322 | 2-40 | 1-01 | ESI+: 352 |
| 1-136 | 1-01 | ESI+: 360 | 2-06 | 1-01 | ESI+: 322 | 2-41 | 1-01 | ESI+: 352 |
| 1-137 | 1-01 | ESI+: 362 | 2-07 | 1-01 (PSyn2,3) | FAB+: 384 | 2-42 | 1-01 | ESI+: 352 |
| 1-138 | 1-01 | ESI+: 378 | | | | 2-43 | 1-01 | FAB+:310 |
| 1-139 | 1-01 | FAB+: 327 | 2-08 | 1-01 | ESI+: 310 | 2-44 | 1-01 | FAB+: 336 |
| 1-140 | 1-01 | FAB+: 353 | 2-09 | 1-01 | FAB+: 310 | 2-45 | 1-01 | FAB+: 350 |
| 1-141 | 1-01 | ESI+: 366 | 2-10 | 1-01 | ESI+: 326 | 2-46 | 1-01 | ESI+: 337 |
| 1-142 | 1-01 | FAB+: 374 | 2-11 | 1-01 | ESI+:326 | 2-47 | 1-01 | ESI+: 337 |
| 1-143 | 1-01 | FAB+: 376 | 2-12 | 1-01 | ESI+:306 | 2-48 | 1-01 | ESI+: 337 |
| 1-144 | 1-01 | FAB+: 376 | 2-13 | 1-01 | ESI+: 306 | 2-49 | 1-01 | FAB+:293 |
| 1-145 | 1-01 | FAB+: 376 | 2-14 | 1-01 | ESI+: 306 | 2-50 | 1-01 | EST+: 308 |
| 1-146 | 1-01 | FAB+: 344 | 2-15 | 1-01 | FAB+: 360 | 2-51 | 1-01 | ESI+: 308 |

[Table 103]

| Ex | Syn | Dat | | Ex | Syn | Dat | | Ex | Syn | Dat |
|---|---|---|---|---|---|---|---|---|---|---|
| 2-52 | 1-01 | ESI+: 308 | | 2-89 | 1-01 | ESI+: 293 | | 2-126 | 1-01 | ES1+: 343 |
| 2-53 | 1-01 | ESI+: 311 | | 2-90 | 1-01 | ESI+:346 | | 2-127 | 1-01 | ESI+: 343 |
| 2-54 | 1-01 | ESI+: 340 | | 2-91 | 1-01 | ESI+:335 | | 2-128 | 1-02 | ESI+: 401 |
| 2-55 | 1-01 | ESI+: 347 | | 2-92 | 1-01 | ESI+: 294 | | 2-129 | 1-01 | ESI+: 309 |
| 2-56 | 1-01 | FAB+:306 | | 2-93 | 1-01 | ESI+: 299 | | 2-130 | 1-01 | FAB+: 311 |
| 2-57 | 1-01 | FAB+: 335 | | 2-94 | 1-01 | FAB+: 358 | | 2-131 | 1-01 | ESI+: 340 |
| 2-58 | 1-01 | FAB+:336 | | 2-95 | 1-01 | FAB+: 346 | | 2-132 | 1-01 | ESI+: 331 |
| 2-59 | 1-01 | FAB+:331 | | 2-96 | 1-01 | ESI+:328 | | 2-133 | 1-01 | ESI+: 320 |
| 2-60 | 1-01 | ESI+: 346 | | 2-97 | 1-01 | FAB+: 370 | | 2-134 | 1-01 | ESI+: 340 |
| 2-61 | 1-01 | FAB+: 347 | | 2-98 | 1-01 | FAB+: 376 | | 2-135 | 1-01 | ESI+: 340 |
| 2-62 | 1-01 | ESI+: 335 | | 2-99 | 1-01 | ESI+: 346 | | 2-136 | 1-01 | ESI+: 362 |
| 2-63 | 1-01 | ESI+: 340 | | 2-100 | 1-01 | ESI+: 358 | | 2-137 | 1-01 | ESI+: 358 |
| 2-64 | 1-01 | ESI+: 362 | | 2-101 | 1-01 | FAB+: 328 | | 2-138 | 1-01 | ESI+: 338 |
| 2-65 | 1-01 | ESI+: 346 | | 2-102 | 1-01 | FAB+: 328 | | 2-139 | 1-01 | ESI+: 324 |
| 2-66 | 1-01 | ESI+: 347 | | 2-103 | 1-01 | FAB+: 328 | | 2-144 | 1-01 | ESI+: 323 |
| 2-67 | 1-01 | ESI+: 344 | | 2-104 | 1-01 | FAB+: 328 | | 2-141 | 1-01 | FAB+: 321 |
| 2-68 | 1-01 | FAB+: 347 | | 2-105 | 1-01 | ESI+: 328 | | 2-142 | 1-01 | FAB+: 361 |
| 2-69 | 1-01 | FAB+: 334 | | 2-106 | 1-03 | ESI+ 336 | | 2-143 | 1-01 | FAB+: 360 |
| 2-70 | 1-01 | FAB+: 344 | | 2-107 | 1-03 | ESI+: 376 | | 2-144 | 1-01 | FAB+: 374 |
| 2-71 | 1-01 | ESI+: 354 | | 2-108 | 1-01 | FAB+: 358 | | 2-145 | 1-01 | FAB+: 320 |
| 2-72 | 1-01 | ESI+: 324 | | 2-109 | 1-01 | FAB+: 344 | | 2-146 | 1-01 | ESI+: 358 |
| 2-73 | 1-01 | ESI+: 324 | | 2-110 | 1-01 | FAB+: 344 | | 2-147 | 1-01 | ESI+: 362 |
| 2-74 | 1-01 | ESI+: 324 | | 2-111 | 1-01 | FAB+: 358 | | 2-148 | 1-01 | ESI+: 340 |
| 2-75 | 1-01 | ESI+: 320 | | 2-112 | 1-01 l | FAB+: 328 | | 2-149 | 1-01 | FAB+: 327 |
| 2-76 | 1-01 | ESI+: 362 | | 2-113 | 1-01 | FAB+: 320 | | 2-150 | 1-01 | FAB+: 327 |
| 2-77 | 1-01 | ESI+: 296 | | 2-114 | 1-01 | FAB+: 331 | | 2-151 | 1-01 | ESI+: 307 |
| 2-78 | 1-01 | ESI+: 347 | | 2-115 | 1-01 | ESI+: 320 | | 2-152 | 1-01 | ESI+: 311 |
| 2-79 | 1-01 | ESI+: 331 | | 2-116 | 1-01 | ESI+: 334 | | 2-153 | 1-01 | FAB+: 256 |
| 2-80 | 2-01 | ESI+: 320 | | 2-117 | 1-01 | ESI+: 428 | | 2-154 | 1-01 | ESI+: 340 |
| 2-81 | 1-01 | ESI+: 296 | | 2-118 | 1-01 | ESI+: 336 | | 2-155 | 1-01 | ESI+: 356 |
| 2-82 | 2-01 | ESI+: 338 | | 2-119 | 1-03 | FAB+: 380 | | 2-156 | 1-01 | ESI+: 344 |
| 2-83 | 1-01 | ESI+: 370 | | 2-120 | 1-03 | FAB+: 430 | | 2-157 | 1-01 | FAB+: 299 |
| 2-84 | 1-01 | ESI+: 294 | | 2-121 | 1-03 | ESI+: 442 | | 2-158 | 1-01 | ESI+: 311 |
| 2-85 | 1-01 | ESI+: 340 | | 2-122 | 1-01 | ESI+: 307 | | 2-159 | 1-01 | FAB+: 340 |
| 2-86 | 1-01 | ESI+:294 | | 2-123 | 1-01 | ESI+: 311 | | 2-160 | 1-01 | FAB+: 340 |
| 2-87 | 1-01 | FAB+: 336 | | 2-124 | 1-01 | ESI+: 307 | | 2-161 | 1-01 | FAB+: 344 |
| 2-88 | 1-01 | FAB+: 350 | | 2-125 | 1-01 | ESI+: 323 | | 2-162 | 1-01 | FAB+: 356 |

[Table 104]

| Ex | Syn | Dat | Ex | Syn | Dat | Ex | Syn | Dat |
|---|---|---|---|---|---|---|---|---|
| 2-163 | 1-01 | ESI+: 338 | 2-186 | 1-01 | ESI+: 374 | 3-17 | 1-01 | ESI+:335 |
| 2-164 | 1-01 | ESI+: 334 | 2-187 | 1-01 | ESI+: 327 | 3-18 | 1-01 | ESI+:340 |
| 2-165 | 1-01 | FAB+: 390 | 2-188 | 1-01 | ESI+: 329 | 3-19 | 1-01 | ESI+: 335 |
| 2-166 | 1-01 | FAB+: 365 | 2-189 | 1-01 | ESI+: 350 | 3-20 | 1-01 | ESI+: 356 |
| 2-167 | 1-01 | FAB+: 324 | 2-190 | 1-01 | ESI+: 361 | 3-21 | 1-01 | ESI+: 356 |
| 2-168 | 1-01 | ESI+: 344 | 2-191 | 1-01 | ESI+: 323 | 3-22 | 1-01 | ESI+: 344 |
| 2-169 | 1-01 | ESI+: 344 | 2-192 | 1-01 | ESI+: 323 | 3-23 | 1-01 | ESI+: 344 |
| 2-170 | 1-01 | FAB+: 350 | 3-01 | 3-01 | FAB+: 302 | 3-24 | 1-01 | ESI+: 326 |
| 2-171 | 1-01 | ESI+: 332 | 3-02 | 1-01 | ESI+:294 | 3-25 | 1-01 | ESI+: 314 |
| 2-172 | 1-01 | ESI+: 336 | 3-03 | 1-01 | FAB+:306 | 3-26 | 1-01 | ESI+: 314 |
| 2-173 | 1-01 | ESI+: 353 | 3-04 | 1-01 | FAB+:331 | 3-27 | 1-01 | ESI+: 344 |
| 2-174 | 1-01 | ESI+: 327 | 3-05 | 1-01 | FAB+:360 | 3-28 | 1-01 | FAB+: 344 |
| 2-175 | 1-01 | ESI+: 335 | 3-06 | 1-01 | FAB+:336 | 3-29 | 1-01 | FAB+: 312 |
| 2-176 | 1-01 | FAB+: 327 | 3-07 | 1-01 | ESI+: 296 | 3-30 | 1-01 | ESI+: 332 |
| 2-177 | 1-01 | ESI+: 342 | 3-08 | 1-01 | ESI+: 326 | 3-31 | 1-01 | ESI+: 350 |
| 2-178 | 1-01 | ESI+: 372 | 3-09 | 1-01 | ESI+: 280 | 3-32 | 1-01 | ESI+: 297 |
| 2-179 | 1-01 | ESI+: 358 | 3-10 | 1-01 | FAB+:308 | 3-33 | 1-01 | ESI+: 340 |
| 2-180 | 1-01 | ESI+: 335 | 3-11 | 1-01 | FAB+:338 | 3-34 | 1-01 | ESI+: 376 |
| 2-181 | 1-01 | ESI+: 347 | 3-12 | 1-01 | FAB+:362 | 3-35 | 1-01 | FAB+: 328 |
| 2-182 | 1-01 | ESI+: 335 | 3-13 | 1-01 | FAB+:333 | 3-36 | 1-01 | ESI+: 370 |
| 2-183 | 1-01 | ESI+: 335 | 3-14 | 1-01 | ESI+: 350 | 3-37 | 1-01 | ESI+: 297 |
| 2-184 | 1-01 | ESI+: 335 | 3-15 | 1-01 | ESI+342 | 3-38 | 1-01 | ESI+: 321 |
| 2-185 | 1-01 | ESI+: 351 | 3-16 | 1-01 | FAB+:364 | 3-39 | 1-01 | ESI+: 351 |

[Table 105]

| Ex | Dat (NMR-DMSO-$d^6$) |
|---|---|
| 1-01 | 1.49 (6H, s), 3.66 (3H, s), 5.79 (1H, s), 6.95 (1H, d, J = 8.4 Hz), 7.03-7.06 (1H, m), 7.12 (1H, d, J = 8.4 Hz), 7.18-7.20 (1H, m), 7.24 (1H, d, J = 2.3 Hz), 7.40-7.45 (1H, m), 8.11-8.13 (1H, m), 8.45 (2H, brs), 8.66 (2H, brs), 9.10 (1H, brs) |
| 1-09 | 5.00 (2H, d, J = 3.8 Hz), 6.01 (1H, t, J = 3.8 Hz), 7.05 (1H, d, J = 8.6 Hz), 7.28-7.32 (2H, m), 7.40-7.44 (2H, m), 7.55 (1H, d, J = 2.2 Hz), 8.08-8.10 (1H, m), 8.42 (2H, brs), 8.58 (2H, brs), 11.72 (1H, brs) |
| 1-12 | 2.10 (3H, s), 5.00-5.16 (2H, m), 5.86 (1H, t, J = 3.6 Hz), 7.00 (1H, d, J = 8.6 Hz), 7.10 (1H, d, J = 2.3 Hz), 7.17 (1H, d, J = 7.1 Hz), 7.24-7.38 (3H, m), 8.12 (1H, dd, J = 8.1, 2.3 Hz), 8.42 (2H, brs), 8.61 (2H, brs), 11.77 (1H, brs) |
| 1-13 | 5.00-5.18 (2H, m), 5.98 (1H, t, J = 3.5 Hz), 7.01 (1H, d, J = 8.7 Hz), 7.13 (1H, d, J = 2.3 Hz), 7.37-7.62 (4H, m), 8.17 (1H, dd, J = 8.5, 2.3 Hz), 8.46 (2H, brs), 8.66 (2H, brs), 11.86 (1H, brs) |
| 1-16 | 5.01 (2H, d, J = 3.8 Hz), 6.04 (1H, t, J = 3.8 Hz), 7.05 (1H, d, J = 8.6 Hz), 7.40 (2H, d, J = 8.4 Hz), 7.52-7.55 (3H, m), 8.10-8.13 (1H, m), 8.45 (2H, brs), 8.63 (2H, brs), 11.81 (1H, brs) |

(continued)

| Ex | Dat (NMR-DMSO-d6) |
|---|---|
| 1-17 | 3.81 (3H, s), 4.98 (2H, d, J = 3.8 Hz), 5.95 (1H, t, J = 3.8 Hz), 7.01-7.05 (3H, m), 7.29 (2H, d, J = 8.6 Hz), 7.60 (1H, d, J = 2.0 Hz), 8.09-8.12 (1H, m), 8.43 (2H, brs), 8.60 (2H, brs), 11.74 (1H, brs) |
| 1-19 | 5.10 (2H, d, J = 3.6 Hz), 6.18 (1H, t, J = 3.6 Hz), 7.03 (1H, d, J = 2.6 Hz), 7.31-7.38 (3H, m), 8.08-8.11 (1H, m), 8.43 (2H, brs), 8.64 (2H, brs), 11.85 (1H, brs) |
| 1-27 | 1.47 (3H, d, J = 6.5 Hz), 3.66 (3H, s), 5.22-5.28 (1H, m), 5.81 (1H, d, J = 3.2 Hz), 6.94-6.96 (1H, m), 7.02-7.06 (1H, m), 7.12 (1H, d, J = 8.3 Hz), 7.19-7.23 (2H, m), 7.41-7.45 (1H, m), 8.17 (1H, dd, J = 8.4, 2.3 Hz), 8.51 (2H, brs), 8.73 (2H, brs), 9.11 (1H, brs) |
| 1-35 | 5.12 (2H, d, J = 3.6 Hz), 6.17 (1H, t, J = 3.6 Hz), 7.03 (1H, d, J = 8.6 Hz), 7.23-7.29 (3H, m), 7.53-7.61 (1H, m), 8.12-8.15 (1H, m), 8.44 (2H, brs), 8.62 (2H, brs), 11.81 (1H, brs) |
| 1-47 | 2.39 (3H, s), 5.05 (2H, d, J = 3.6 Hz), 6.02 (1H, t, J = 3.6 Hz), 7.01 (1H, d, J = 8.6 Hz), 7.12-7.17 (2H, m), 7.27 (1H, t, J = 8.2 Hz), 7.33 (1H, t, J = 2.2 Hz), 8.14-8.16 (1H, m), 8.47 (2H, brs), 8.64 (2H, brs), 11.83 (1H, brs) |
| 1-51 | 5.07-5.10 (2H, m), 6.00-6.03 (1H, m), 7.01-7.04 (1H, m), 7.11-7.14 (1H, m), 7.43-7.46 (1H, m), 7.55-7.79 (3H, m), 8.07 (2H, brs), 8.42 (2H, brs), 9.10 (1H, brs) |

[Table 106]

| Ex | Dat (NMR-DMSO-d6) |
|---|---|
| 1-66 | 3.89 (3H, s), 4.92 (2H, d, J = 3.6 Hz), 6.01-6.04 (1H, m), 7.02 (1H, d, J = 8.4 Hz), 7.62 (1H, s), 7.88 (1H, brs), 8.08-8.09 (2H, m), 8.53 (2H, brs), 8.75 (2H, brs), 11.92 (1H, s) |
| 1-69 | 3.75 (3H, s), 4.54 (2H, d, J = 9.0 Hz), 5.12 (2H, d, J = 4.0 Hz), 6.13-6.23 (1H, m), 6.84-7.07 (4H, m), 7.18-7.35 (4H, m), 7.48-7.62 (1H, m), 8.10-8.27 (1H, m), 8.94-9.28 (2H, m), 9.75-9.90 (1H, brs), 11.85-12.00 (1H, brs) |
| 1-71 | 1.47 (3H, d, J = 6.7 Hz), 5.29-5.35 (1H, m), 6.11 (1H, d, J = 3.4 Hz), 7.04 (1H, d, J = 8.5 Hz), 7.33-7.38 (3H, m), 8.11-8.14 (1H, m), 8.46 (2H, brs), 8.70 (2H, brs), 11.94 (1H, brs) |
| 1-73 | 3.85 (3H, s), 5.09 (2H, d, J = 3.6 Hz), 6.10 (1H, t, J = 3.6 Hz), 6.90 (2H, d, J = 9.6 Hz), 7.01 (1H, d, J = 8.5 Hz), 7.28 (1H, brs), 8.16 (1H, dd, J = 8.6, 2.3 Hz), 8.49 (2H, brs), 8.68 (2H, brs), 11.90 (1H, brs) |
| 1-75 | 2.88 (3H, d, J = 4.8 Hz), 5.11 (2H, d, J = 3.2 Hz), 6.14-6.20 (1H, m), 6.95-7.09 (1H, m), 7.16-7.36 (3H, m), 7.49-7.65 (1H, m), 8.10-8.27 (1H, m), 8.67-9.12 (2H, m), 9.34-9.51 (1H, m), 11.84-12.01 (1H, m) |
| 1-96 | 5.13 (2H, d, J = 3.5 Hz), 6.10 (1H ,t, J = 3.5 Hz), 7.03 (1H, d, J = 8.7 Hz), 7.09 (1H, d, J = 2.0 Hz), 7.36-7.43 (1H, m), 7.45-7.59 (2H, m), 8.05-8.13 (1H, m), 8.40 (2H, brs), 8.55 (2H, brs), 11.71 (1H, brs) |
| 1-144 | 1.41-1.55 (3H, m), 5.20-5.36 (1H, m), 5.94 (1H, brs), 7.05 (1H, d, J = 8.7 Hz), 7.12 (1H, d, J = 2.1 Hz), 7.43 (1H, d, J = 8.2 Hz), 7.56 (1H, dd, J = 8.2,2.0 Hz), 7.78 (1H, d, J = 2.0 Hz), 7.94-8.02 (1H, m), 8.36 (2H, brs), 8.42 (2H, brs), 11.50 (1H, brs) |
| 2-03 | 2.38-2.44 (2H, m), 2.89-2.93 (2H, m), 6.23 (1H, t, J = 4.6 Hz), 7.24-7.28 (2H, m), 7.36-7.40 (2H, m), 7.47-7.48 (2H, m), 8.08-8.10 (1H, m), 8.48 (2H, brs), 8.65 (2H, brs), 11.88 (1H, brs) |
| 2-04 | 2.39-2.44 (2H, m), 2.89-2.93 (2H, m), 6.26 (1H, t, J = 4.6 Hz), 7.36-7.47 (2H, m), 7.48-7.50 (4H, m), 8.08-8.11 (1H, m), 8.49 (2H, brs), 8.67 (2H, brs), 11.90 (1H, brs) |
| 2-06 | 2.41-2.42 (2H, m), 2.90-2.94 (2H, m), 3.62 (3H, s), 6.08 (1H, t, J = 4.5 Hz), 7.00-7.04 (1H, m), 7.09 (1H, d, J = 8.1 Hz), 7.17-7.19 (2H, m), 7.37-7.41 (2H, m), 8.03-8.06 (1H, m), 8.45 (2H, brs), 8.63 (2H, brs), 11.79 (1H, brs) |
| 2-13 | 2.34 (3H, s), 2.37-2.43 (2H, m), 2.88-2.92 (2H, m), 6.20 (1H, t, J = 4.6 Hz), 7.11 (1H, d, J = 7.7 Hz), 7.15 (1H, s), 7.19 (1H, d, J = 7.7 Hz), 7.31 (1H, t, J = 7.5 Hz), 7.46 (1H, d, J = 8.0 Hz), 7.51 (1H, d, J = 1.6 Hz), 8.13-8.15 (1H, m), 8.53 (2H, brs), 8.71 (2H, brs), 11.96 (1H, brs) |

[Table 107]

| Ex | Dat (NMR-DMSO-d6) |
|---|---|
| 2-27 | 2.47-2.51 (2H, m), 2.94-2.98 (2H, m), 6.32 (1H, t, J = 4.6 Hz), 7.17-7.18 (1H, m), 7.49-7.53 (2H, m), 7.59-7.63 (1H, m), 7.77-7.82 (1H, m), 7.93-7.96 (1H, m), 8.12-8.14 (1H, m), 8.50 (2H, brs), 8.66 (2H, brs), 11.93 (1H, brs) |
| 2-31 | 1.39-1.50 (2H, m), 1.69-1.72 (2H, m), 2.21-2.25 (2H, m), 2.72-2.76 (2H, m), 3.11-3.17 (1H, m), 3.57-3.62 (2H, m), 3.87-3.90 (2H, m), 5.98-6.00 (1H, m), 7.41 (1H, d, J = 7.8 Hz), 7.91 (1H, dd, J = 7.8, 1.5 Hz), 8.11 (1H, s), 8.45 (2H, brs), 8.86 (2H, brs), 12.08 (1H, brs) |
| 2-37 | 2.36 (3H, s), 2.38-2.46 (2H, m), 2.91 (2H, t, J = 8.0 Hz), 6.25 (1H, t, J = 4.6 Hz), 7.30-7.54 (7H, m), 7.76-7.82 (1H, m), 8.10-8.50 (4H, m), 11.14 (1H, brs) |
| 2-39 | 2.71-2.77 (2H, m), 3.15-3.19 (2H, m), 3.68 (3H, s), 7.03-7.10 (2H, m), 7.14-7.18 (2H, m), 7.41-7.45 (2H, m), 8.01 (1H, dd, J = 7.9, 1.8 Hz), 8.45 (2H, brs), 8.62 (2H, brs), 11.80 (1H, brs) |
| 2-43 | 2.73-2.79 (2H, m), 3.15-3.20 (2H, m), 7.26-7.41 (3H, m), 7.42-7.51 (4H, m), 7.93 (1H, d, J = 7.9 Hz), 8.39 (2H, brs), 8.45 (2H, brs), 11.59 (1H, brs) |
| 2-53 | 2.41-2.47 (2H, m), 2.90-2.95 (2H, m), 6.34 (1H, t, J = 4.6 Hz), 7.24-7.26 (1H, m), 7.46-7.50 (2H, m), 7.95-7.99 (1H, m), 8.08-8.11 (1H, m), 8.22-8.23 (1H, m), 8.49 (2H, brs), 8.69 (2H, brs), 11.95 (1H, brs) |
| 2-54 | 2.40-2.42 (2H, m), 2.89-2.93 (2H, m), 3.64 (3H, s), 6.08 (1H, t, J = 4.5 Hz), 6.82-6.86 (1H, m), 6.99-7.02 (1H, m), 7.17-7.22 (2H, m), 7.3 9-7.41 (1H, m), 8.06-8.09 (1H, m), 8.49 (2H, brs), 8.69 (2H, brs), 11.90 (1H, brs) |
| 2-59 | 1.72 (3H, s), 2.41-2.51 (2H, m), 2.94-2.98 (2H, m), 7.0 (1H, d, J = 1.5 Hz), 7.39-7.43 (3H, m), 7.93 (2H, d, J = 7.6 Hz), 8.01-8.04 (1H, m), 8.45 (2H, brs), 8.66 (2H, brs), 11.86 (1H, brs) |
| 2-60 | 2.46-2.51 (2H, m), 2.96 (2H, t, J = 8.1 Hz), 6.32 (1H, t, J = 4.6 Hz), 7.28-7.34 (3H, m), 7.47 (1H, d, J = 7.9 Hz), 8.14-8.16 (1H, m), 8.55 (2H, brs), 8.76 (2H, brs), 12.07 (1H, brs) |
| 2-61 | 2.43-2.50 (2H, m), 2.92 (2H, t, J = 8.0 Hz), 3.72 (3H, s), 6.17 (1H, t, J = 4.4 Hz), 7.12 (1H, d, J = 1.7 Hz), 7.28 (1H, d, J = 8.6 Hz), 7.43 (1H, d, J = 8.0 Hz), 7.65 (1H, d, J = 1.7 Hz), 7.89-7.91 (1H, m), 8.05-8.07 (1H, m), 8.47 (2H, brs), 8.66 (2H, brs), 11.86 (1H, brs) |
| 2-64 | 2.47-2.53 (2H, m), 2.91-2.97 (2H, m), 6.17-6.20 (1H, m), 7.09 (1H, s), 7.40-7.55 (3H, m), 7.72-7.74 (1H, m), 8.02-8.06 (1H, m), 8.43 (2H, brs), 8.58 (2H, brs), 11.78 (1H, brs) |
| 2-65 | 2.42-2.47 (2H, m), 2.91-2.95 (2H, m), 6.30-6.33 (1H, m), 7.33 (1H, s), 7.47-7.67 (3H, m), 8.00-8.06 (1H, m), 8.44 (2H, brs), 8.59 (2H, brs), 11.80 (1H, brs) |

[Table 108]

| Ex | Dat (NMR-DMSO-d6) |
|---|---|
| 2-67 | 2.43-2.48 (2H, m), 2.94 (2H, t, J = 8.0 Hz), 6.29 (1H, t, J = 4.6 Hz), 7.28 (1H, s), 7.37-7.54 (4H, m), 8.10-8.13 (1H, m), 8.50 (2H, brs), 8.68 (2H, brs), 11.93 (1H, brs) |
| 2-70 | 2.35-2.57 (2H, m), 2.85-3.03 (2H, m), 6.16 (1H, t, J = 4.5 Hz), 7.11 (1H, d, J = 1.6 Hz), 7.28-7.36 (1H, m), 7.38-7.50 (2H, m), 7.55 (1H, dd, J = 8.8, 2.6 Hz), 8.49 (2H, brs), 8.68 (2H, brs), 11.92 (1H, brs) |
| 2-76 | 2.40-2.57 (2H, m), 2.96 (2H, t, J = 8.1 Hz), 6.35 (1H, t, J = 4.5 Hz), 7.23 (1H, brs), 7.42-7.54 (3H, m), 8.00-8.08 (1H, m), 8.45 (2H, brs), 8.61 (2H, brs), 11.84 (1H, brs) |
| 2-77 | 2.31-2.36 (2H, m), 2.84 (2H, t, J = 7.9 Hz), 3.88 (3H, s), 6.25 (1H, t, J = 4.7 Hz), 7.44 (1H, d, J = 3.9 Hz), 7.56 (1H, s), 7.83 (1H, brs), 7.97 (1H, s), 8.04 (1H, d, J = 3.9 Hz), 8.56 (2H, brs), 8.77 (2H, brs), 11.99 (1H, s) |
| 2-90 | 2.73-2.79 (2H, m), 3.15-3.19 (2H, m), 7.11-7.20 (1H, m), 7.25-7.29 (1H, m), 7.41-7.57 (3H, m), 8.01-8.03 (1H, m), 8.44 (2H, brs), 8.61 (2H, brs), 11.84 (1H, brs) |
| 2-94 | 2.44-2.51 (2H, m), 2.94 (2H, t, J = 8.1 Hz), 3.84 (3H, s), 6.26 (1H, t, J = 4.5 Hz), 6.86 (2H, d, J = 9.6 Hz), 7.25 (1H, s), 7.45 (1H, d, J = 7.9 Hz), 8.14-8.17 (1H, m), 8.55 (2H, brs), 8.73 (2H, brs) |
| 2-95 | 2.43-2.51 (2H, m), 2.94 (2H, t, J = 8.0 Hz), 6.31 (1H, t, J = 4.6 Hz), 7.23-7.27 (1H, m), 7.38-7.49 (3H, m), 8.09-8.12 (1H, m), 8.51 (2H, brs), 8.76 (2H, brs), 12.04 (1H, brs) |

(continued)

| Ex | Dat (NMR-DMSO-d6) |
|---|---|
| 2-96 | 2.75-2.78 (2H, m), 3.15-3.20 (2H, m), 7.22-7.26 (1H, m), 7.30-7.35 (4H, m), 7.46 (1H, d, J = 7.9 Hz), 7.97-8.00 (1H, m), 8.41 (2H, brs), 8.53 (2H, brs), 11.71 (1H, brs) |
| 2-105 | 2.43-2.57 (2H, m), 2.97 (2H, t, J = 8.3 Hz), 6.32 (1H, t, J = 4.4 Hz), 7.07-7.32 (3H, m), 7.45-7.60 (2H, m), 8.12-8.20 (1H, m), 8.53 (2H, brs), 8.71 (2H, brs), 11.99 (1H, brs) |
| 2-119 | 2.34-2.46 (2H, m), 2.91 (2H, t, J = 8.0 Hz), 3.29 (3H, s), 3.35-3.55 (4H, m), 3.63 (3H, s), 6.00-6.14 (1H, m), 6.92-7.46 (6H, m), 8.15 (1H, d, J = 2.0 Hz), 8.74-9.34 (2H, m), 9.52 (1H, brs), 11.99 (1H, s) |
| 2-131 | 2.38-2.55 (2H, m), 2.94 (2H, t, J = 8.0 Hz), 3.61 (3H, d, J = 1.0 Hz), 6.15-6.24 (1H, m), 7.00-7.10 (1H, m), 7.12-7.25 (3H, m), 7.27-7.36 (1H, m), 7.45 (1H, d, J = 8.0 Hz), 8.08 (1H, dd, J = 7.9, 1.8 Hz), 8.46 (2H, brs), 8.65 (2H, brs), 11.85 (1H, brs) |
| 2-154 | 2.40-2.56 (2H, m), 2.94 (2H, t, J = 8.2 Hz), 3.69 (3H, s), 6.11 (1H, t, J = 4.5 Hz), 6.86-6.94 (1H, m), 6.97 (1H, d, J = 8.4 Hz), 7.08-7.14 (1H, m), 7.36-7.46 (2H, m), 8.04 (1H, dd, J = 7.8, 1.8 Hz), 8.44 (2H, brs), 8.60 (2H, brs), 11.77 (1H, brs) |

[Table 109]

| Ex | Dat (NMR-DMSO-d6) |
|---|---|
| 2-188 | 2.45-2.57 (2H, m), 2.97 (2H, t, J = 8.1 Hz), 6.49 (1H, t, J = 4.5 Hz), 7.27 (1H, brs), 7.51 (1H, d, J = 8.0 Hz), 8.02-8.07 (1H, m), 8.43 (2H, brs), 8.60 (2H, brs), 8.66 (2H, s), 11.85 (1H, brs) |
| 3-02 | 1.72-2.12 (4H, m), 2.85-3.01 (2H, m), 4.21-4.24 (1H, m), 7.07 (2H, d, J = 7.2 Hz), 7.19-7.23 (1H, m), 7.28-7.32 (2H, m), 7.36 (1H, d, J = 8.2 Hz), 7.48 (1H, s), 7.98-8.0 (1H, m), 8.50 (2H, brs), 8.67 (2H, brs), 9.10 (1H, brs) |
| 3-05 | 1.99-2.07 (2H, m), 2.17-2.24 (2H, m), 2.78-2.81 (2H, m), 6.47 (1H, t, J = 7.0 Hz), 7.24 (2H, t, J = 8.2 Hz), 7.46 (1H, d, J = 1.8 Hz), 7.55 (1H, d, J = 7.9 Hz), 8.04-8.06 (1H, m), 8.46 (2H, brs), 8.62 (2H, brs), 11.87 (1H, brs) |
| 3-08 | 3.74 (3H, s), 7.11 (1H, d, J = 7.4 Hz), 7.22 (1H, d, J = 8.2 Hz), 7.41-7.43 (1H, m), 7.46-7.50 (1H, m), 7.89 (1H, s), 8.15-8.20 (2H, m), 8.27 (1H, d, J = 8.6 Hz), 8.55 (2H, brs), 8.69 (2H, brs), 11.96 (1H, brs) |
| 3-09 | 7.44 (1H, t, J= 7.4 Hz), 7.54 (2H, t, J = 7.4 Hz), 7.89-7.92 (3H, m), 8.12-8.15 (1H, m), 8.54 (2H, brs), 8.57 (1H, s), 8.72 (1H, d, J = 1.6 Hz), 8.80 (2H, brs), 12.24 (1H, brs) |
| 3-12 | 2.50-2.82 (2H, m), 4.37-4.40 (2H, m), 6.33 (1H, t, J = 5.0 Hz), 7.23 (1H, d, J = 8.4 Hz), 7.30-7.34 (2H, m), 7.40 (1H, m), 8.11-8.14 (1H, m), 8.41 (2H, brs), 8.58 (2H, brs), 9.09 (1H, brs) |
| 3-33 | 2.38 (3H, s), 3.72 (3H, s), 7.08-7.16 (1H, m), 7.20-7.31 (2H, m), 7.46-7.53 (1H, m), 7.78-7.83 (1H, m), 7.97-8.05 (1H, m), 8.17 (1H, d, J = 8.4 Hz), 8.44 (2H, brs), 8.49 (2H, brs), 11.65 (1H, brs) |

(Test Examples)

The pharmacological activity of the compound of the present invention was confirmed by the following test.

Test Example 1

Acquisition of HEK293 cells for forced expressions of a human 5-$HT_{5A}$ receptor
The ORF (open reading frame; protein coding region) of a human 5-$HT_{5A}$ receptor (Genbank AF498985) was cloned from a human hippocampus cDNA library, and then inserted into a pCR2.1 vector (Invitrogen), and *Escherichia coli* containing the plasmid was cultured in a large amount. Next, the full-length cDNA sequence of the human 5-$HT_{5A}$ receptor was analyzed, and recombined into a pCDNA3.1 vector (Invitrogen) as an expression vector and cultured in a large amount. HEK293 established cells (ATCC) derived from the human fetal kidney were seeded, the expression plasmid (1 μg) obtained above were added thereto with LIPOFECTAMINE 2000 (Invitrogen; 2 μl), the gene was transfected intoHEK293 cells, and the expression cells were screened with a drug-resistant marker, Geneticin (G418 sulfate 500 μg/ml; Kanto Chemical Co., Inc.). Thus prepared recombinant cells which express the gene were cultured in a

medium containing D-MEM (Dulbecco's modified eagle medium, Sigma), 10% FCS (Fetal calf serum: fetal bovine serum), 1% Pc./Sm (Penicillin/Streptomycin, Invitrogen), and 500 μg/ml G418 for 3 days. These experimental operations follow an manual for gene operation experiment and an instruction appended in a reagent, and the like, such as a known method (Sambrook, J. et al, Molecular Cloning-A Laboratory Manual", Cold Spring Harabor laboratory, NY, 1989).

Test Example 2

Test on a human 5-$HT_{5A}$ receptor binding inhibition

(1) Preparation of a membrane from HEK293 cells for forced expressions of a human 5-$HT_{5A}$ receptor

HEK293 cells for forced expressions of a human 5-$HT_{5A}$ receptor were cultured in a F500 plate, and scraped with a scraper. After centrifugation, a precipitate was collected, and an incubation buffer (50 mM Tris (HCl) (pH 7.4), 10 mM $MgSO_4$, and 0.5 mM EDTA (ethylenediamine tetraacetic acid)) were added thereto. After homogenization, it was further centrifuged, and the precipitate was added with the incubation buffer, followed by thoroughly suspending. This operation was repeated, and a protein concentration was measured, thereby completing the preparation of a membrane.

(2) Test on a human 5-$HT_{5A}$ receptor binding inhibition

A solution of a compound to be tested and 100 μM 5-CT (5-carboxamidetriptamine) in DMSO was added to a 96-well plate at 2 μl/well, and suspended in an incubation buffer, and a membrane from HEK293 cells for forced expressions of a human 5-$HT_{5A}$ receptor prepared at 200 μg/ml was added at 100 μl/well. After incubation at room temperature for 15 minutes, a [$^3$H]5-CT solution (2 nM [$^3$H]5-CT, incubation buffer) was added thereto at 100 μl/well.

Separately, 100 μl of the solution was distributed into a liquid scintillation vial, and 2 ml of Aquasol II (registered trademark) was added thereto, followed by stirring. Then, a radioactivity was measured by a liquid scintillation counter. It was incubated at 37°C for 60 minutes. The reaction mixture was sucked into 96-well GF/C filter plate that had been pre-treated with 0.2% polyethyleneimine, and washed six times with an ice-cooled, 50 mM Tris (pH 7.5) buffer. The GF/C filter plate was dried.

Microscint TMPS (registered trademark) was added thereto at 40 μl/well. A radioactivity remaining on the GF/C filter plate was measured by a top counter.

The [$^3$H]5-CT binding inhibiting activity by the compound to be tested in each experiment was determined as an $IC_{50}$ value with a radioactivity upon addition of DMSO alone being 0% inhibition, and a radioactivity upon addition of 1 μM 5-CT being 100% inhibition. Separately, a Ki value was calculated from the Kd value of the [$^3$H]5-CT determined from Scatchard analysis, by the following equation.

$$Ki = IC_{50}\ (1+\text{Concentraion of ligands added}/Kd\ (4.95\ nM))$$

As a result of this test, it was demonstrated that the compound (I) as an active ingredient of the medicine of the present invention has a potent human 5-$HT_{5A}$ receptor binding inhibiting activity.

For example, the compound of Example 1-14 exhibited a Ki value of 0.97 nM, while the compound of Example 2-56 exhibited a Ki value of 2.3 nM. The compounds of Examples 1-05 to 1-13, 1-16 to 1-20, 1-22, 1-30 to 1-36, 1-42 to 1-45, 1-47, 1-51, 1-54, 1-55, 1-59 to 1-64, 1-67, 1-71, 1-73 to 1-75, 1-81, 1-82, 1-91, 1-92, 1-96, 1-102, 1-103, 1-111 to 1-113, 1-118, 2-02 to 2-04, 2-06, 2-09, 2-11 to 2-13, 2-19, 2-21, 2-25, 2-37, 2-39, 2-43, 2-48, 2-51, 2-52, 2-54, 2-55, 2-59, 2-60, 2-67, 2-70, 2-72 to 2-76, 2-85, 2-91, 2-95, 2-96, 2-99, 2-101, 2-105, 2-110, 2-134, 2-135, 2-137 to 2-139, 2-143, 2-144, 2-154 to 2-156, 2-158, 2-163, 2-164, 2-166, 2-168, 2-170, 2-177, 2-178, 3-08, 3-12, 3-14, 3-16, 3-21 to 3-23, 3-25, 3-26, 3-29, 3-30, 3-33, and 3-35 each exhibited a Ki value in a range from 0.3 nM to 3 nM.

Furthermore, the compounds of Examples 1-03, 1-15, 1-21, 1-23 to 1-29, 1-37 to 1-41, 1-46, 1-48, 1-50, 1-52, 1-53, 1-56 to 1-58, 1-65, 1-68 to 1-70, 1-72, 1-76, 1-79, 1-80, 1-85, 1-86, 1-88 to 1-90, 1-93 to 1-95, 1-97 to 1-101, 1-105, 1-109, 1-114 to 1-117, 1-121, 1-126, 1-129 to 1-132, 1-135 to 1-138, 1-142 to 1-145, 1-148 to 1-151, 1-162 to 1-164, 1-166, 1-167, 2-01, 2-05, 2-08, 2-10, 2-14 to 2-18, 2-20, 2-22 to 2-24, 2-26 to 2-29, 2-31, 2-32, 2-34, 2-38, 2-40 to 2-42, 2-46, 2-47, 2-49, 2-50, 2-53, 2-57, 2-58, 2-61, 2-63 to 2-65, 2-69, 2-71, 2-78, 2-79, 2-81, 2-82, 2-87, 2-90, 2-94, 2-100, 2-102 to 2-104, 2-106 to 2-109, 2-111 to 2-113, 2-115, 2-118 to 2-122, 2-124 to 2-127, 2-130 to 2-133, 2-136, 2-140, 2-141, 2-145 to 2-149, 2-151 to 2-153, 2-157, 2-159 to 2-162, 2-165, 2-169, 2-171, 2-173, 2-174, 2-176, 2-179, 2-180, 2-182 to 2-184, 2-186 to 2-188, 2-190, 3-02, 3-05, 3-07, 3-10, 3-11, 3-13, 3-15, 3-17, 3-19, 3-20, 3-24, 3-27, 3-28, 3-31, 3-38, and 3-39 each exhibited a Ki value in a range from 3 nM to 30 nM,

Furthermore, the compounds of Examples 1-01, 1-04, 1-49, 1-66, 1-83, 1-87, 1-104, 1-110, 1-119, 1-120, 1-122

to 1-125, 1-127, 1-128, 1-133, 1-134, 1-139 to 1-141, 1-146, 1-147, 1-152 to 1-161, 1-165, 2-07, 2-30, 2-33, 2-35, 2-36, 2-44, 2-45, 2-62, 2-66, 2-68, 2-77, 2-80, 2-86, 2-88, 2-89, 2-93, 2-97, 2-98, 2-114, 2-116, 2-117, 2-123, 2-142, 2-150, 2-167, 2-175, 2-181, 2-185, 2-189, 2-191, 3-01, 3-03, 3-04, 3-06, 3-09, 3-18, 3-32, 3-34, 3-36, and 3-37 each exhibited a Ki value in a range from 30 nM to 300 nM.

As described above, it was confirmed that the compound (I) has a 5-$HT_{5A}$ receptor affinity.

Test Example 3

Various drug evaluations on a drug for increasing the motion of mice (methamphetamine, MK-801) (radiated infrared motion measurement)

An effect of the compound (I) on improvement on schizophrenia by was evaluated by measuring the inhibited motion through the administration of the compound in a model having a symptom caused by methamphetamine (which is hereinafter simply referred to MAP) and MK-801.

(1) Animal

Species: Male ICR mouse

(2) Operation procedure

An animal was taken out of a breeding cage, orally administered with a compound to be tested, and then placed into a cage for breeding. After 30 minutes, the animal was put into a cage for measurement, and the motion with the compound to be tested alone was measured. Further, after 30 to 90 minutes, the animal was taken out, and intraperitoneally administered with a drug for increasing the motion (MAP; 1 mg/kg or MK-801; 0.3 mg/kg, dissolved in a physiological saline, respectively). Then, the motion for a certain period of time (60 minutes) was measured by using a motion measurement device (CompACT AMS from Muromachi Kikai Co., Ltd.) by means of an infrared sensor.

(3) Analysis

For a normal mouse (a mouse administered with physiological saline) and a mouse administered with a drug for increasing the motion, a Student's T test was performed for evaluation for each interval. For a group administered with the compound to be tested, an assay was performed using a solvent (vehicle) group and a Dunnett's T test. For the evaluation, if there was a significant difference ($P<0.05$), it was considered that there is an effect.

As a result of this test, the compound of the present invention inhibited the increase in the motion of the mouse. For example, the compound of Example 2-37 significantly inhibited the hyperactivity caused by methamphetamine at a dose of 0.03 mg/kg. The compound of Example 1-47 significantly inhibited the hyperactivity caused by MK-801 at a dose of 0.1 mg/kg, and the compound of Example 2-06 significantly inhibited the hyperactivity at a dose of 0.03 mg/kg.

As described above, it was confirmed that the compound (I) has an effect of improving schizophrenia.

Test Example 4

An improvement effect for spontaneous alternation behavior caused by Scoporamine or MK-801 in mice

An effect of the compound (I) on improvement on cognitive impairment was evaluated by using a known performance test method as a model with short-term learning disorder.

(1) Animal

Species: Male ddY mouse

(2) Measurement method

The compound to be tested was administered orally 10 to 30 minutes before the test, and 0.5 mg/kg Scoporamine or 0.15 mg/kg MK-801 (for a normal group, physiological saline) was administered intraperitoreally, and the test was performed after 20 minutes. Also, the normal group (group administered with physiological saline) and a control group (group administered with 0.5 mg/kg Scoporamine or 0.15 mg/kg MK-801) were administered orally with a solvent (vehicle) upon administration of the compound to be test.

A mouse was placed at the end of one arm of a Y-maze having arms with the same length in three directions, and then explored freely and the number of arm entries was counted for 8 minutes. Furthermore, a spontaneous alternation behavior was defined as entries into all three different arms on consecutive occasions. The ratio of the number of the behaviors to the total number of entries was calculated as an alternation rate by the following formula:

Alternation rate (%) = (Number of spontaneous alternation behaviors/Total number of entries $-$ 2)$\times$100.

(3) Data analysis

If a significant difference between the normal group and the control group (Student's t test) was approved in the alternation rate (%), it was considered to have learning disorder by the administration of Scoporamine or MK-801. By carrying out a Dunnett's test on the group administered with the compound to be tested relative to the control group, the presence or absence of an action of the compound to be tested on learning disorder was evaluated. For each assay, it was considered that there was a significant difference when $p<0.05$.

As a result of the test, it was found that the compound of the present invention inhibited the spontaneous alternation behavior of the mouse. For example, the compound of Example 2-37 significantly improved the alternation rate caused by Scoporamine at a dose of 0.03 mg/kg.

As a result of the test, it was confirmed that the compound (I) has an effect on cognitive impairment.

Test Example 5

An improvement effect for a disorder of PCP-induced prepulse inhibition (PPI) in rats

If a sound stimulus is given to a human, a startle reaction occurs, but for a normal human, this startle reaction is inhibited when the sound stimulus is preceded by a weak sound stimulus. In a similar manner, this inhibiting action is lowered in a patient with schizophrenia. It is known that if a rat is administered with PCP (phencyclidine), a similar symptom to schizophrenia of a human occurs. Using this model, an effect of the compound (I) on improvement of information processing disorder included in cognitive impairment of schizophrenia was evaluated.

An effect of the compound (I) on improvement of schizophrenia was evaluated by using a known model with prepulse inhibition disorder caused by PCP as a model having a disease condition. Specifically, it follows the method as described in "Neuropsychopharmacology, 1989; 2: 61-66, Mansbach, R.S. and Geyer, M.A. and Brain Research, 1998; 781: 227-235". As a result of the test, it was found that the compound (I) improves prepulse inhibition (PPI) disorder caused by PCP.

As a result of this test, it was confirmed that the compound (I) also has an effect on information processing disorder included in cognitive impairment of schizophrenia.

Test Example 6

Evaluation of a drug for water maze learning disorder in an old rats

An effect of the compound (I) on improvement of schizophrenia was evaluated by using a known model with water maze learning disorder as a model having a disease condition. Specifically, it follows the method described in J Pharmacol Exp Ther, 1996; 279: 1157-73, Yamazaki M. et al.

As a result of this test, it was confirmed that the compound (I) also has an effect for dementia.

Test Example 7

Evaluation of a drug in a forced swimming test in DBA/2 mice

An effect of the compound (I) on improvement of depression was evaluated by using a known forced swimming test with a model to be evaluated. Specifically, it follows the method described in Behav Brain Res. 2005; 156(1): 153-162, Ducottet C. et al.

As a result of this test, it was confirmed that the compound (I) has an effect for depression.

From the above-described results of the tests, it can be seen that the compound of the present invention is effective for treatment or prevention of 5-$HT_{5A}$ receptor-related diseases, and in particular, treatment or prevention of dementia, schizophrenia (including symptoms such as positive symptom, negative symptom, cognitive impairment, and

mood disorder), bipolar disorder, attention deficit hyperactivity disorder, neuroses (panic disorder, obsessive-compulsive disorder, and the like), autism, mood disorder (anxiety disorder, depressive disorder), sleep disorder, neurodegenerative diseases, or cerebral infarction.

A pharmaceutical preparation containing one or two or more kinds of the compound (I) or a salt thereof as an active ingredient can be prepared by using pharmaceutical carriers, excipients, and the like that are each usually used in the art, by a method that is usually used.

Administration may be made in any form for either oral administration by tablets, pills, capsules, granules, powders, and solutions, or parenteral administration by injections for intraarticular injection, intravenous injection, and intramuscular injection, suppositories, ophthalmic solutions, ophthalmic oinments, percutaneous liquids, oinments, percutaneous patches, transmucosal liquids, transmucosal patches, and inhalations.

Regarding the solid composition for oral administration according to the present invention, tablets, powders, granules, or the like are used. In such a solid composition, one, or two or more active ingredients are mixed with at least one inactive excipient such as lactose, mannitol, glucose, hydroxypropyl cellulose, microcrystalline cellulose, starch, polyvinyl pyrrolidone, and/or magnesium meta-silicate alminate. According to a conventional method, the composition may contain inactive additives; for example, a lubricant such as magnesium stearate, a disintegrator such as carboxymethylstarch sodium, a stabilizing agent, and a dissolution promotor. As occasion demands, tablets or pills may be coated with a sugar, or a film of a gastric or enteric material.

The liquid composition for oral administration includes pharmaceutically acceptable emulsions, solutions, suspensions, syrups, elixirs, and the like, and contains an inert diluent that is commonly used, such as purified water or ethanol. In addition to the inert diluent, this liquid composition may contain an auxiliary agent such as a solubilizing agent, a moistening agent, and a suspending agent, a sweetener, a flavor, an aroma, and an antiseptic.

Injections for parenteral administration include aqueous or non-aqueous sterile solutions, suspensions, and emulsions. Examples of the aqueous solvent include distilled water for injection, and physiological saline. Examples of the non-aqueous solvent include propylene glycol, polyethylene glycol, vegetable oils such as olive oil, alcohols such as ethanol, and Polysorbate 80 (Pharmacopeia). Such a composition may further contain a tonicity agent, an antiseptic, a moistening agent, an emulsifying agent, a dispersing agent, a stabilizing agent, and a dissolution promotor. These are sterilized, for example, by filtration through a bacterium-retaining filter, blending of bactericides, or irradiation. In addition, these can also be used by producing a sterile solid composition, and dissolving or suspending it in sterile water or a sterile solvent for injection prior to its use.

Examples of the drug for external use include ointments, plasters, creams, jellies, cataplasms, sprays, lotions, ophthalmic solutions, and ophthalmic ointments. The drug contains commonly used ointment bases, lotion bases, aqueous or non-aqueous solutions, suspensions, emulsions, and the like. Examples of the ointment bases or lotion bases include polyethylene glycol, propylene glycol, white vaseline, bleached bee wax, polyoxyethylene hydrogenated castor oil, glyceryl monostearate, stearyl alcohol, cetyl alcohol, lauromacrogol, and sorbitan sesquioleate.

A transmucosal agent such as an inhalations and a transmucosal agent can be used in a solid, liquid or semi-solid state, and may be produced in accordance with a conventionally known method. For example, a known excipient, and also a pH adjusting agent, an antiseptic, a surfactant, a lubricant, a stabilizer, a viscosity-increasing agent, and the like may be appropriately added thereto. For their administration, an appropriate device for inhalation or blowing may be used. For example, a compound may be administered alone or as a powder of a formulated mixture, or as a solution or suspension by combining it with a pharmaceutically acceptable carrier, using a conventionally known device or sprayer, such as a measured administration inhalation device. The dry powder inhaler or the like may be for single or multiple administration use, and a dry powder or a powder-containing capsule may be used. Alternatively, this may be in a form such as a high pressure aerosol spray which uses an appropriate propellant, for example, a suitable gas such as chlorofluoroalkane, hydrofluoroalkane, or carbon dioxide.

It is suitable that the daily dose is usually from about 0.0001 to 100 mg/kg per body weight in the case of oral administration, preferably 0.0001 to 10 mg/kg, and even more preferably 0.0001 to 1 mg/kg, and the preparation is administered in one portion or dividing it into 2 to 4 portions. Also, in the case of intravenous administration, the daily dose is administered suitably in a range from about 0.00001 to 1 mg/kg per body weight, and the preparation is administered once a day or two or more times a day. In the case of drugs for external use or transmucosal administration, the drug is administered usually in a range from about 0.0001 to 10 mg/kg per body weight, once a day or two or more times a day. The dose is appropriately decided, depending on individual cases by taking into consideration the symptom, age, sex and the like. The content of the active ingredients in the preparation is from 0.0001 to 50%, and more preferably 0.001 to 50%.

## INDUSTRIAL AVAILABILITY

The compound of the present invention has an advantage that it has a potent 5-$HT_{5A}$ receptor modulating action, and an excellent pharmacological action based on the 5-$HT_{5A}$ receptor modulating action. The pharmaceutical

composition of the present invention can be used for treatment or prevention of 5-$HT_{5A}$ receptor-mediated diseases, and in particular, for treatment or prevention of dementia, schizophrenia, bipolar disorder, or attention deficit hyperactivity disorder.

## Claims

**1.** A bicyclic acylguanidine derivative represented by the following general formula (I), or a salt thereof.

[Chem. 11]

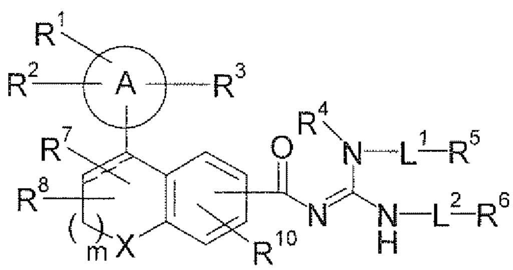

(I)

(the symbols in the formula have the following meanings:

[Chem. 12]

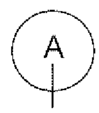

:

phenyl, cycloalkyl, monocyclic or bicyclic heteroaryl, a monocyclic oxygen-containing saturated heterocyclic group, or a monocyclic nitrogen-containing saturated heterocyclic group,
$R^1$, $R^2$, and $R^3$: the same with or different from each other, each representing H, lower alkyl, halogen, halogeno-lower alkyl, -CN, -$NO_2$, -$NR^bR^c$, -$OR^a$, -O-halogeno-lower alkyl, -$SR^a$, -C(O)$R^a$, -$CO_2R^a$, -C(O)$NR^bR^c$, -$SO_2$-lower alkyl,
-$NR^b$C(O)$R^a$, lower alkylene-$OR^a$, lower alkylene-$NR^bR^c$, lower alkylene-CN, phenyl, -O-phenyl, or $R^1$ and $R^2$ in combination represent oxo or -O-$(CH_2)_n$-O-,
n: 1, 2, or 3,
$R^a$, $R^b$ and $R^c$: the same with or different from each other, each representing H or lower alkyl,
$R^7$ and $R^8$: the same with or different from each other, each representing H, lower alkyl, halogen, lower alkylene-$OR^a$, or $R^7$ and $R^8$ in combination represent oxo, or $R^7$ and $R^8$ may be combined together to form a $C_{2-5}$ alkylene chain which forms a $C_{3-6}$ cycloalkyl ring with a carbon atom to which they bond,
dotted line: a bond or inexistence, and it represents, together with the solid line, that a ring bond at this moiety is a single bond or a double bond,
X: O, S or $CR^{9a}R^{9b}$,
$R^{9a}$ and $R^{9b}$: the same with or different from each other, each representing H or lower alkyl,
m: 0, 1, or 2,
$R^4$: H or lower alkyl,
$L^1$ and $L^2$: the same with or different from each other, each representing a bond or lower alkylene,
$R^5$ and $R^6$: the same with or different from each other, each representing H, -$OR^a$, -$NR^bR^c$, phenyl, or cycloalkyl, in which $R^5$ may form a monocyclic nitrogen-containing heterocyclic group together with $R^4$ and $L^1$, and a nitrogen atom to which they are bonded, in which phenyl, cycloalkyl, and a monocyclic nitrogen-containing heterocyclic group may be substituted with lower alkyl, halogen, or -$OR^a$, and
$R^{10}$: H, halogen, or -$OR^a$.)

2. The compound according to claim 1 or a salt thereof, wherein $R^4$ and $R^5$ are each H, $R^6$ is H, methyl, or methoxy, and $L^1$ and $L^2$ are each a bond.

3. The compound according to claim 2 or a salt thereof, wherein $R^6$ is H.

4. The compound according to claim 3 or a salt thereof, wherein A is phenyl or pyridyl.

5. The compound according to claim 4 or a salt thereof, wherein X is $CR^{9a}R^{9b}$.

6. The compound according to claim 4 or a salt thereof, wherein X is O.

7. The compound according to claim 1 or a salt thereof, which is selected from the group consisting of N-(diaminomethylene)-4-(4-fluorophenyl)-2H-chromene-6-carboxamide, N-(diaminomethylene)-4-(2-methylphenyl)-2H-chromene-6-carboxamide, 4-(2-chlorophenyl)-N-(diaminomethylene)-2H-chromene-6-carboxamide, N-(diaminomethylene)-4-(2,4,6-trifluorophenyl)-2H-chromene-6-carboxamide, N-(diaminomethylene)-4-(2,6-difluorophenyl)-2H-chromene-6-carboxamide, N-(diaminomethylene)-4-(2-fluoro-4-methylphenyl)-2H-chromene-6-carboxamide, N-(diaminomethylene)-4-(2,4-dichlorophenyl)-2H-chromene-6-carboxamide, N-(diaminomethylene)-4-(2,6-difluoro-4-methoxyphenyl)-2H-chromene-6-carboxamide, 4-(2-chloro-6-fluorophenyl)-N-(diaminomethylene)-2H-chromene-6-carboxamide, N-(diaminomethylene)-4-(2,4-dichlorophenyl)-2-methyl-2H-chromene-6-carboxamide, N-(diaminomethylene)-8-(4-fluorophenyl)-5,6-dihydronaphthalene-2-carboxamide, N-(diaminomethylene)-8-(2-methoxyphenyl)-5,6-dihydronaphthalene-2-carboxamide, N-(diaminomethylene)-8-(3-methylphenyl)-5,6-dihydronaphthalene-2-carboxamide, 8-(2-cyanophenyl)-N-(diaminomethylene)-5,6-dihydronaphthalene-2-carboxamide, N-(diaminomethylene)-8-phenyl-5,6-dihydronaphthalene-2-carboxamide, N-(diaminomethylene)-7-fluoro-8-(2-methoxyphenyl)-5,6-dihydronaphthalene-2-carboxamide, 8-(4-cyanophenyl)-N-(diaminomethylene)-7-methyl-5,6-dihydronaphthalene-2-carboxamide, N-(diaminomethylene)-8-(2,4,6-trifluorophenyl)-5,6-dihydronaphthalene-2-carboxamide, 8- (5-cyano-2-methoxyphenyl)-N-(diaminomethylene)-5,6-dihydronaphthalene-2-carboxamide, 8-(2-chloro-4-fluorophenyl)-N-(diaminomethylene)-5,6-dihydronaphthalene-2-carboxamide, 8-(4-chloro-2,6-difluorophenyl)-N-(diaminomethylene)-5,6-dihydronaphthalene-2-carboxamide, N-(diaminomethylene)-8-(2,6-difluoro-4-methoxyphenyl)-5,6-dlhydronaphthalene-2-carboxamide, N-(diaminomethylene)-8-(2,6-difluorophenyl)-5,6-dihydronaphthalene-2-carboxamide, N-{(1E)-amino[(2-(methoxyethyl)amino]methylene}-8-(2-methoxyphenyl)-5,6-dihydronaphthalene-2-carboxamide, N-(diaminomethylene)-8-(3-fluoro-2-methoxyphenyl)-5,6-dihydronaphthalene-2-carboxamide, N-(diaminomethylene)-8-(2-fluoro-6-methoxyphenyl)-5,6-dihydronaphthalene-2-carboxamide, N-(diaminomethylene)-8-(3,5-difluoropyridin-4-yl)-5,6-dihydronaphthalene-2-carboxamide, N-(diaminomethylene)-3-(2-methoxyphenyl)-1-benzothiophene5-carboxamide, and N-(diaminomethylene)-3-(2-methoxyphenyl)-2-methyl-1-benzothiophene5-carboxamide.

8. A pharmaceutical composition comprising the compound according to claim 1 or a salt thereof, and a pharmaceutically acceptable carrier.

9. The pharmaceutical composition according to claim 8, which is a 5-$HT_{5A}$ receptor modulator.

10. The pharmaceutical composition according to claim 9, which is an agent for preventing or treating dementia, schizophrenia, bipolar disorder, or attention deficit hyperactivity disorder.

11. A use of the derivative according to claim 1 or a salt thereof, for the preparation of an agent for preventing or treating dementia, schizophrenia, bipolar disorder, or attention deficit hyperactivity disorder.

12. A method for preventing or treating dementia, schizophrenia, bipolar disorder, or attention deficit hyperactivity disorder, comprising administering a therapeutically effective amount of the derivative according to claim 1 or a salt thereof to a patient.

## INTERNATIONAL SEARCH REPORT

International application No.
PCT/JP2008/064257

A. CLASSIFICATION OF SUBJECT MATTER
*C07C279/22*(2006.01)i, *A61K31/341*(2006.01)i, *A61K31/352*(2006.01)i, *A61K31/495*(2006.01)i, *A61P25/28*(2006.01)i, *A61P43/00*(2006.01)i, *C07D295/12*(2006.01)i, *C07D307/52*(2006.01)i, *C07D311/04*(2006.01)i
According to International Patent Classification (IPC) or to both national classification and IPC

B. FIELDS SEARCHED
Minimum documentation searched (classification system followed by classification symbols)
C07C279/22, A61K31/341, A61K31/352, A61K31/495, A61P25/28, A61P43/00, C07D295/12, C07D307/52, C07D311/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| E,A | WO 2008/096791 A1 (Astellas Pharma Inc.), 14 August, 2008 (14.08.08), Claims | 1-11 |
| A | WO 2007/018168 A1 (Astellas Pharma Inc.), 15 February, 2007 (15.02.07), Claims | 1-11 |
| A | WO 2005/080322 A1 (Yamanouchi Pharmaceutical Co., Ltd.), 01 September, 2005 (01.09.05), Claims | 1-11 |
| A | WO 2005/082871 A2 (ABBOTT GMBH & CO. KG.), 09 September, 2005 (09.09.05), Claims | 1-11 |

☐ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 October, 2008 (06.10.08) | 14 October, 2008 (14.10.08) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**
Information on patent family members

International application No.
PCT/JP2008/064257

| | | |
|---|---|---|
| WO 2008/096791 A1 | 2008.08.14 | (Family: none) |
| WO 2007/018168 A1 | 2007.02.15 | EP 1923387 A1 |
| WO 2005/080322 A1 | 2005.09.01 | NO 200601149 A<br>BR 200506139 A<br>AU 2005214249 A1<br>MX 2006002391 A1<br>EP 1728784 A1<br>CN 1842517 A<br>KR 2007000389 A<br>IN 200601180 P1 |
| WO 2005/082871 A2 | 2005.09.09 | DE 102004008141 A1<br>EP 1716127 A2<br>JP 2007-523113 A<br>MX 2006009434 A1<br>US 2007/0299074 A1 |

Form PCT/ISA/210 (patent family annex) (April 2007)

**INTERNATIONAL SEARCH REPORT**

International application No.
PCT/JP2008/064257

**Box No. II Observations where certain claims were found unsearchable (Continuation of item 2 of first sheet)**

This international search report has not been established in respect of certain claims under Article 17(2)(a) for the following reasons:

1. [x] Claims Nos.: 12
because they relate to subject matter not required to be searched by this Authority, namely:
Claim 12 pertains to a method for treatment of a human body by therapy and thus relates to a subject matter which this International Searching Authority is not required, under the provisions of PCT Rule 39.1(iv), to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee.

[ ] The additional search fees were accompanied by the applicant's protest but the applicable protest fee was not paid within the time limit specified in the invitation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5082871 A
- JP 6135978 A
- JP 9077753 A


### Non-patent literature cited in the description


- *Neuron,* 1999, vol. 22, 581-591
- *Molecular Brain Research,* 1998, vol. 56, 1-8
- *Neuroreport,* 2000, vol. 11, 2017-2020
- *Mol. Psychiatr.,* 2001, vol. 6, 217-219
- *J. Psychiatr. Res.,* 2004, vol. 38, 371-376
- **Rolf Bergmann et al.** *Journal of Medicinal Chemistry,* 1990, vol. 33, 492-504
- *Prog. Med.,* 1985, vol. 5, 2157-2161
- Bunshi Sekkei. Iyakuhin no Kaihatsu. Hirokawa Shoten, 1990, vol. 7, 163-198
- Green's Protective Groups in Organic Synthesis. 2006
- Yuki Gosei. Jikken Kagaku Koza. Maruzen, 1992, vol. 20, 300
- Metal-Catalyzed Cross-Coupling Reactions. VCH Publishers Inc, 1997
- Reductions in Organic Chemistry. ACS, 1996
- *J. Chem. Soc., Perkin Trans.,* 1999, vol. 1, 2421-2423
- *Tetrahedron Asymmetry,* 2003, vol. 14, 1529-1534
- *Tetrahedron,* 2003, vol. 59, 9641-9648
- *Tetrahedron,* 2000, vol. 56, 8133-8140
- **Sambrook, J. et al.** Molecular Cloning-A Laboratory Manual. Cold Spring Harabor laboratory, 1989
- *Neuropsychopharmacology,* 1989, vol. 2, 61-66
- **Mansbach, R.S. ; Geyer, M.A.** *Brain Research,* 1998, vol. 781, 227-235
- *J Pharmacol Exp Ther,* 1996, vol. 279, 1157-73
- **Ducottet C.** *Behav Brain Res.,* 2005, vol. 156 (1), 153-162